(19) Europäisches Patentamt
European Patent Office
Office européen des brevets

(11) EP 1 525 315 B1

(12) **EUROPÄISCHE PATENTSCHRIFT**

(45) Veröffentlichungstag und Bekanntmachung des Hinweises auf die Patenterteilung:
**03.03.2010 Patentblatt 2010/09**

(21) Anmeldenummer: **03764981.1**

(22) Anmeldetag: **14.07.2003**

(51) Int Cl.:
***C12N 15/82*** *(2006.01)* ***C12N 15/24*** *(2006.01)*
***A01H 5/10*** *(2006.01)*

(86) Internationale Anmeldenummer:
**PCT/EP2003/007589**

(87) Internationale Veröffentlichungsnummer:
**WO 2004/009820 (29.01.2004 Gazette 2004/05)**

(54) **VERFAHREN ZUM ERREICHEN EINER PATHOGENRESISTENZ IN PFLANZEN**

METHOD FOR OBTAINING THE PATHOGENIC RESISTANCE IN PLANTS

PROCEDE DE GENERATION D'UNE RESISTANCE A DES AGENTS PATHOGENES DANS DES PLANTES

(84) Benannte Vertragsstaaten:
**AT BE BG CH CY CZ DE DK EE ES FI FR GB GR HU IE IT LI LU MC NL PT RO SE SI SK TR**
Benannte Erstreckungsstaaten:
**AL LT LV MK**

(30) Priorität: **22.07.2002 DE 10233327**

(43) Veröffentlichungstag der Anmeldung:
**27.04.2005 Patentblatt 2005/17**

(73) Patentinhaber: **BASF Plant Science GmbH**
**67056 Ludwigshafen (DE)**

(72) Erfinder:
- **KOGEL, Karl-Heinz**
  **35457 Lollar (DE)**
- **HÜCKELHOVEN, Ralph**
  **35392 Giessen (DE)**
- **TRUJILLO, Marco**
  **35394 Giessen (DE)**

(74) Vertreter: **Bieberbach, Andreas**
**BASF SE**
**Global Intellectual Property**
**GVX / K - C 6**
**67056 Ludwigshafen (DE)**

(56) Entgegenhaltungen:
- **TORRES MIGUEL ANGEL ET AL: "Arabidopsis gp91phox homologues AtrbohD and AtrbohF are required for accumulation of reactive oxygen intermediates in the plant defense response" PROCEEDINGS OF THE NATIONAL ACADEMY OF SCIENCES OF THE UNITED STATES, Bd. 99, Nr. 1, 8. Januar 2002 (2002-01-08), Seiten 517-522, XP002261415 January 8, 2002 ISSN: 0027-8424 in der Anmeldung erwähnt**
- **HUECKELHOVEN RALPH ET AL: "Tissue-specific superoxide generation at interaction sites in resistant and susceptibile near-isogenic barley lines attacked by the powdery mildew fungus (Erysiphe graminis f. sp. hordei)" MOLECULAR PLANT-MICROBE INTERACTIONS, Bd. 11, Nr. 4, April 1998 (1998-04), Seiten 292-300, XP009020951 ISSN: 0894-0282 in der Anmeldung erwähnt**
- **SAGI MOSHE ET AL: "Superoxide production by plant homologues of the gp91phox NADPH oxidase. Modulation of activity by calcium and by tobacco mosaic virus infection" PLANT PHYSIOLOGY (ROCKVILLE), Bd. 126, Nr. 3, Juli 2001 (2001-07), Seiten 1281-1290, XP002261416 ISSN: 0032-0889**
- **BOLWELL G PAUL ET AL: "The apoplastic oxidative burst in response to biotic stress in plants: A three-component system" JOURNAL OF EXPERIMENTAL BOTANY, Bd. 53, Nr. 372, Mai 2002 (2002-05), Seiten 1367-1376, XP002261417 ISSN: 0022-0957**

EP 1 525 315 B1

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

- **HUECKELHOVEN R ET AL: "Functional studies on the role of reactive oxygen intermediates in the resistance of barley against powdery mildew." PLANT PROTECTION SCIENCE, Bd. 38, Nr. Special Issue 2, 2002, Seiten 458-460, XP001155865 ISSN: 1212-2580**
- **BORDEN STEPHANIE ET AL: "Hydrogen peroxide plays a critical role in the defence response of tomato to Cladosporium fulvum." PHYSIOLOGICAL AND MOLECULAR PLANT PATHOLOGY, Bd. 61, Nr. 4, Oktober 2002 (2002-10), Seiten 227-236, XP002261418 ISSN: 0885-5765**
- **MAHALINGAM RAMAMURTHY ET AL: "Stress response, cell death and signalling: The many faces of reactive oxygen species." PHYSIOLOGIA PLANTARUM, Bd. 119, Nr. 1, September 2003 (2003-09), Seiten 56-58, XP002261419 ISSN: 0031-9317 (ISSN print)**

SEQUENZPROTOKOLL

<110> BASF Plant Science GmbH

<120> Verfahren zum Erreichen einer Pathogenresistenz in Pflanzen

<130> AE20020416

<140>
<141>

<160> 24

<170> PatentIn Ver. 2.1

<210> 1
<211> 337
<212> DNA
<213> Hordeum vulgare

<220>
<221> CDS
<222> (2)..(337)
<223> coding for NADPH-oxidase (fragment)

<400> 1

```
g ttt aaa gga atc atg aat gag att gct gaa cta gat caa agg aat atc 49
  Phe Lys Gly Ile Met Asn Glu Ile Ala Glu Leu Asp Gln Arg Asn Ile
  1               5                   10                  15

att gag atg cac aac tat ctc aca agt gtt tat gag gaa ggg gat gct   97
Ile Glu Met His Asn Tyr Leu Thr Ser Val Tyr Glu Glu Gly Asp Ala
            20                  25                  30

cgg tca gca ctc atc aca atg ctg caa gct ctc aac cat gcc aag aat   145
Arg Ser Ala Leu Ile Thr Met Leu Gln Ala Leu Asn His Ala Lys Asn
        35                  40                  45

ggt gtc gat gta gtg tct ggm act cga gtc cgg aca cat ttt gca aga   193
Gly Val Asp Val Val Ser Xaa Thr Arg Val Arg Thr His Phe Ala Arg
    50                  55                  60

cca aat ttt aag agg gtg ctg tct aag gta gcc gcc aaa cat cct tat   241
Pro Asn Phe Lys Arg Val Leu Ser Lys Val Ala Ala Lys His Pro Tyr
65                  70                  75                  80

gcc aag ata gga gtg ttc tat tgc gga gct cca gtt ctg gcg cag gaa   289
Ala Lys Ile Gly Val Phe Tyr Cys Gly Ala Pro Val Leu Ala Gln Glu
                85                  90                  95

cta agc aac ctt tgc cat gag ttc aat ggc aaa tgc acg aca aaa ttc   337
Leu Ser Asn Leu Cys His Glu Phe Asn Gly Lys Cys Thr Thr Lys Phe
            100                 105                 110
```

<210> 2
<211> 112
<212> PRT
<213> Hordeum vulgare

<400> 2

```
Phe Lys Gly Ile Met Asn Glu Ile Ala Glu Leu Asp Gln Arg Asn Ile
1               5                   10                  15

Ile Glu Met His Asn Tyr Leu Thr Ser Val Tyr Glu Glu Gly Asp Ala
            20                  25                  30

Arg Ser Ala Leu Ile Thr Met Leu Gln Ala Leu Asn His Ala Lys Asn
        35                  40                  45

Gly Val Asp Val Val Ser Xaa Thr Arg Val Arg Thr His Phe Ala Arg
    50                  55                  60

Pro Asn Phe Lys Arg Val Leu Ser Lys Val Ala Ala Lys His Pro Tyr
65                  70                  75                  80

Ala Lys Ile Gly Val Phe Tyr Cys Gly Ala Pro Val Leu Ala Gln Glu
                85                  90                  95

Leu Ser Asn Leu Cys His Glu Phe Asn Gly Lys Cys Thr Thr Lys Phe
            100                 105                 110
```

<210> 3
<211> 2832
<212> DNA

<213> Oryza sativa

<220>
<221> CDS
<222> (1)..(2829)
<223> coding for NADPH-oxidase

<400> 3

```
atg agg ggc ggc gcc tcc tcg gga ccc cag cga tgg ggc tcg gcg ggg      48
Met Arg Gly Gly Ala Ser Ser Gly Pro Gln Arg Trp Gly Ser Ala Gly
 1               5                  10                  15

acg aca ccg cgg tcg ctg agc acg ggc tcg tcg ccg cgc ggg tcc gac      96
Thr Thr Pro Arg Ser Leu Ser Thr Gly Ser Ser Pro Arg Gly Ser Asp
            20                  25                  30

gac cgg agc tcc gac gac ggg gag gag ctg gtc gag gtc acg ctc gac     144
Asp Arg Ser Ser Asp Asp Gly Glu Glu Leu Val Glu Val Thr Leu Asp
        35                  40                  45

ctg cag gac gac gac acc att gtg ctt cgg agc gtc gag ccc gcg gcg     192
Leu Gln Asp Asp Asp Thr Ile Val Leu Arg Ser Val Glu Pro Ala Ala
    50                  55                  60

gcg gcg gcg gcg ggg gtg ggg gcg ggg gcg ggg gcg gcg tcg gcg cgg     240
Ala Ala Ala Ala Gly Val Gly Ala Gly Ala Gly Ala Ala Ser Ala Arg
65                  70                  75                  80

ggg gag ctc acg ggt ggc ccg tcg tcg tcg tcg tcg cgg tcg agg tcg     288
Gly Glu Leu Thr Gly Gly Pro Ser Ser Ser Ser Ser Arg Ser Arg Ser
                85                  90                  95

ccg tcg atc cgg agg agc tcg tcg cac cgg ctg ctg cag ttc tcg cag     336
Pro Ser Ile Arg Arg Ser Ser Ser His Arg Leu Leu Gln Phe Ser Gln
            100                 105                 110

gag ctc aag gcg gag gcc atg gcc cgg gcg cgg cag ttc tcg cag gac     384
Glu Leu Lys Ala Glu Ala Met Ala Arg Ala Arg Gln Phe Ser Gln Asp
        115                 120                 125

ctg acc aag cgg ttc ggc cgc agc cac agc cgc agc gaa gcg cag gcg     432
Leu Thr Lys Arg Phe Gly Arg Ser His Ser Arg Ser Glu Ala Gln Ala
    130                 135                 140

ccg tcg ggc ctc gag tcc gcg ctc gcc gcc cgc gcc gcg cgg cgg cag     480
Pro Ser Gly Leu Glu Ser Ala Leu Ala Ala Arg Ala Ala Arg Arg Gln
145                 150                 155                 160

cgc gcg cag ctc gac cgc aca cgc tcc ggc gcc cac aag gcg ctc cgc     528
Arg Ala Gln Leu Asp Arg Thr Arg Ser Gly Ala His Lys Ala Leu Arg
                165                 170                 175
```

```
ggc ctc cgc ttc atc agc agc aac aag gcc aac aac gcc tgg atg gag      576
Gly Leu Arg Phe Ile Ser Ser Asn Lys Ala Asn Asn Ala Trp Met Glu
            180                 185                 190

gtg cag gcc aac ttc gac cgc ctc gcc cgc gac ggc tac ctc tcc cgc      624
Val Gln Ala Asn Phe Asp Arg Leu Ala Arg Asp Gly Tyr Leu Ser Arg
        195                 200                 205

tcc gac ttc gcc gaa tgc atc ggg atg acg gaa tcg aag gag ttc gcg      672
Ser Asp Phe Ala Glu Cys Ile Gly Met Thr Glu Ser Lys Glu Phe Ala
    210                 215                 220

ctc gag ctg ttc gac acg ctg agc cgg cga cga cag atg aag gtg gac      720
Leu Glu Leu Phe Asp Thr Leu Ser Arg Arg Arg Gln Met Lys Val Asp
225                 230                 235                 240

acg att aac aag gat gaa ctc cgc gag atc tgg cag cag atc acc gat      768
Thr Ile Asn Lys Asp Glu Leu Arg Glu Ile Trp Gln Gln Ile Thr Asp
                245                 250                 255

aac agc ttc gac tcc cgt ctc caa atc ttc ttc gaa atg gtg gat aag      816
Asn Ser Phe Asp Ser Arg Leu Gln Ile Phe Phe Glu Met Val Asp Lys
            260                 265                 270

aac gcg gac ggc cgg att acg gag gcg gag gtg aaa gag att att atg      864
Asn Ala Asp Gly Arg Ile Thr Glu Ala Glu Val Lys Glu Ile Ile Met
        275                 280                 285

ttg agc gcg tct gcc aat aaa ctg tcg agg ctt aag gag caa gca gaa      912
Leu Ser Ala Ser Ala Asn Lys Leu Ser Arg Leu Lys Glu Gln Ala Glu
    290                 295                 300

gag tac gcc gct ttg atc atg gag gag ctt gat cct gaa ggg ctc ggc      960
Glu Tyr Ala Ala Leu Ile Met Glu Glu Leu Asp Pro Glu Gly Leu Gly
305                 310                 315                 320

tac att gag cta tgg caa ttg gag aca ctt ctg ttg cag aaa gat acc      1008
Tyr Ile Glu Leu Trp Gln Leu Glu Thr Leu Leu Leu Gln Lys Asp Thr
                325                 330                 335

tat atg aac tat agt cag gcc ctt agt tac aca agc caa gca ctg agc      1056
Tyr Met Asn Tyr Ser Gln Ala Leu Ser Tyr Thr Ser Gln Ala Leu Ser
            340                 345                 350

cag aat ctt gca ggg cta agg aag aag agt tca atc cgc aaa ata agc      1104
Gln Asn Leu Ala Gly Leu Arg Lys Lys Ser Ser Ile Arg Lys Ile Ser
        355                 360                 365

acc tct tta agc tac tat ttc gag gac aac tgg aaa cgt tta tgg gtg      1152
Thr Ser Leu Ser Tyr Tyr Phe Glu Asp Asn Trp Lys Arg Leu Trp Val
    370                 375                 380

ctt gca ttg tgg att ggg ata atg gct gga ctg ttc acc tgg aaa ttc      1200
Leu Ala Leu Trp Ile Gly Ile Met Ala Gly Leu Phe Thr Trp Lys Phe
385                 390                 395                 400

atg cag tat cgt aac cga tat gtc ttt gat gtg atg ggc tac tgt gtc      1248
Met Gln Tyr Arg Asn Arg Tyr Val Phe Asp Val Met Gly Tyr Cys Val
                405                 410                 415

aca aca gca aaa gga gct gct gaa acc cta aag ctg aat atg gca att      1296
Thr Thr Ala Lys Gly Ala Ala Glu Thr Leu Lys Leu Asn Met Ala Ile
            420                 425                 430

atc ctc ctg cca gta tgc cgt aac acc att act tgg ttg cga agt aca      1344
Ile Leu Leu Pro Val Cys Arg Asn Thr Ile Thr Trp Leu Arg Ser Thr
        435                 440                 445
```

```
agg gct gca cgg gca cta cct ttt gat gac aac atc aac ttc cac aag    1392
Arg Ala Ala Arg Ala Leu Pro Phe Asp Asp Asn Ile Asn Phe His Lys
    450                 455                 460

act att gca gca gca att gtg gtt ggt ata atc ctc cat gca ggg aac    1440
Thr Ile Ala Ala Ala Ile Val Val Gly Ile Ile Leu His Ala Gly Asn
465                 470                 475                 480

cac ctt gta tgc gat ttt cca cgg tta ata aaa tca tca gat gag aag    1488
His Leu Val Cys Asp Phe Pro Arg Leu Ile Lys Ser Ser Asp Glu Lys
                485                 490                 495

tat gct cct ttg ggc cag tat ttt ggg gaa ata aag cca aca tat ttt    1536
Tyr Ala Pro Leu Gly Gln Tyr Phe Gly Glu Ile Lys Pro Thr Tyr Phe
            500                 505                 510

aca ttg gtc aaa gga gtg gag ggc atc act ggg gta atc atg gtt gta    1584
Thr Leu Val Lys Gly Val Glu Gly Ile Thr Gly Val Ile Met Val Val
        515                 520                 525

tgc atg ata att gct ttt act cta gca acc cgg tgg ttc cgc cgt agc    1632
Cys Met Ile Ile Ala Phe Thr Leu Ala Thr Arg Trp Phe Arg Arg Ser
    530                 535                 540

ttg gtt aag ctt cca agg cca ttt gac aaa ctg act ggc ttc aat gcc    1680
Leu Val Lys Leu Pro Arg Pro Phe Asp Lys Leu Thr Gly Phe Asn Ala
545                 550                 555                 560

ttt tgg tat tct cat cat ctg ttc atc att gtg tat atc gcg ctc att    1728
Phe Trp Tyr Ser His His Leu Phe Ile Ile Val Tyr Ile Ala Leu Ile
                565                 570                 575

gtt cat gga gag tgt cta tac ctt att cat gtc tgg tac aga aga acg    1776
Val His Gly Glu Cys Leu Tyr Leu Ile His Val Trp Tyr Arg Arg Thr
            580                 585                 590

aca tgg atg tat ctt tca gtg cct gtt tgc ttg tat gta ggg gag agg    1824
Thr Trp Met Tyr Leu Ser Val Pro Val Cys Leu Tyr Val Gly Glu Arg
        595                 600                 605

att cta agg ttc ttc agg tct ggc agt tat tct gtc cgg cta ttg aag    1872
Ile Leu Arg Phe Phe Arg Ser Gly Ser Tyr Ser Val Arg Leu Leu Lys
    610                 615                 620

gtg gcc ata tat cca ggt aat gtt ttg aca ctg caa atg tcc aag cct    1920
Val Ala Ile Tyr Pro Gly Asn Val Leu Thr Leu Gln Met Ser Lys Pro
625                 630                 635                 640

ccc acg ttc cgt tac aag agt gga caa tat atg ttt gtt caa tgt cca    1968
Pro Thr Phe Arg Tyr Lys Ser Gly Gln Tyr Met Phe Val Gln Cys Pro
                645                 650                 655

gca gtg tct ccc ttt gaa tgg cat ccc ttc tca att act tca gca cct    2016
Ala Val Ser Pro Phe Glu Trp His Pro Phe Ser Ile Thr Ser Ala Pro
            660                 665                 670

ggg gat gac tac ctc agc att cat gtt cga caa ctt ggt gat tgg aca    2064
Gly Asp Asp Tyr Leu Ser Ile His Val Arg Gln Leu Gly Asp Trp Thr
        675                 680                 685

cga gaa ctc aag aga gta ttt gct gca gct tgt gag ccc cca gcg ggt    2112
Arg Glu Leu Lys Arg Val Phe Ala Ala Ala Cys Glu Pro Pro Ala Gly
    690                 695                 700

ggt aaa agc ggc ctt ctt agg gca gat gag aca act aag aaa atc tta    2160
Gly Lys Ser Gly Leu Leu Arg Ala Asp Glu Thr Thr Lys Lys Ile Leu
705                 710                 715                 720
```

```
ccc aag ctt ctg att gat gga ccg tat ggt tct cct gct cag gat tac     2208
Pro Lys Leu Leu Ile Asp Gly Pro Tyr Gly Ser Pro Ala Gln Asp Tyr
                725                 730                 735

agc aag tat gat gtt tta tta ctt gtt gga tta gga att ggt gcg aca     2256
Ser Lys Tyr Asp Val Leu Leu Leu Val Gly Leu Gly Ile Gly Ala Thr
            740                 745                 750

ccc ttt att agc ata tta aaa gat ctt ctg aat aac atc atc aaa atg     2304
Pro Phe Ile Ser Ile Leu Lys Asp Leu Leu Asn Asn Ile Ile Lys Met
        755                 760                 765

gag gaa gag gag gat gct tct act gat ctt tat cca cca atg ggt cgg     2352
Glu Glu Glu Glu Asp Ala Ser Thr Asp Leu Tyr Pro Pro Met Gly Arg
    770                 775                 780

aat aag cca cat gtt gat ctg ggc aca ctt atg acg att acc tca aga     2400
Asn Lys Pro His Val Asp Leu Gly Thr Leu Met Thr Ile Thr Ser Arg
785                 790                 795                 800

cca aag aag atc ttg aag acc aca aat gct tac ttt tac tgg gtg aca     2448
Pro Lys Lys Ile Leu Lys Thr Thr Asn Ala Tyr Phe Tyr Trp Val Thr
                805                 810                 815

cgt gag caa ggc tct ttt gat tgg ttc aaa gga gtc atg aat gaa att     2496
Arg Glu Gln Gly Ser Phe Asp Trp Phe Lys Gly Val Met Asn Glu Ile
            820                 825                 830

gct gac ttg gat caa agg aat atc att gag atg cac aac tac cta aca     2544
Ala Asp Leu Asp Gln Arg Asn Ile Ile Glu Met His Asn Tyr Leu Thr
        835                 840                 845

agc gtc tat gag gag ggg gat gcc agg tca gca ctc atc acc atg ctc     2592
Ser Val Tyr Glu Glu Gly Asp Ala Arg Ser Ala Leu Ile Thr Met Leu
    850                 855                 860

caa gct ctg aac cat gcc aag aat gga gtt gat att gtc tct ggg aca     2640
Gln Ala Leu Asn His Ala Lys Asn Gly Val Asp Ile Val Ser Gly Thr
865                 870                 875                 880

aaa gtc cgg aca cat ttt gca cga cca aat tgg aga aag gtc ctt tct     2688
Lys Val Arg Thr His Phe Ala Arg Pro Asn Trp Arg Lys Val Leu Ser
                885                 890                 895

aaa att tcc tcc aag cat cca tat gcc aaa ata ggt gta ttc tac tgt     2736
Lys Ile Ser Ser Lys His Pro Tyr Ala Lys Ile Gly Val Phe Tyr Cys
            900                 905                 910

gga gct cca gtc ctg gca caa gaa cta agc aaa ctt tgc cat gaa ttc     2784
Gly Ala Pro Val Leu Ala Gln Glu Leu Ser Lys Leu Cys His Glu Phe
        915                 920                 925

aac ggg aaa tgc aca acg aag ttc gaa ttc cat aag gag cat ttc tga     2832
Asn Gly Lys Cys Thr Thr Lys Phe Glu Phe His Lys Glu His Phe
    930                 935                 940
```

<210> 4
<211> 943
<212> PRT
<213> Oryza sativa

<400> 4

```
Met Arg Gly Gly Ala Ser Ser Gly Pro Gln Arg Trp Gly Ser Ala Gly
 1               5                  10                  15
Thr Thr Pro Arg Ser Leu Ser Thr Gly Ser Ser Pro Arg Gly Ser Asp
            20                  25                  30
```

```
Asp Arg Ser Ser Asp Asp Gly Glu Glu Leu Val Glu Val Thr Leu Asp
        35                  40                  45

Leu Gln Asp Asp Asp Thr Ile Val Leu Arg Ser Val Glu Pro Ala Ala
    50                  55                  60

Ala Ala Ala Ala Gly Val Gly Ala Gly Ala Gly Ala Ala Ser Ala Arg
65                  70                  75                  80

Gly Glu Leu Thr Gly Gly Pro Ser Ser Ser Ser Ser Arg Ser Arg Ser
                85                  90                  95

Pro Ser Ile Arg Arg Ser Ser Ser His Arg Leu Leu Gln Phe Ser Gln
            100                 105                 110

Glu Leu Lys Ala Glu Ala Met Ala Arg Ala Arg Gln Phe Ser Gln Asp
        115                 120                 125

Leu Thr Lys Arg Phe Gly Arg Ser His Ser Arg Ser Glu Ala Gln Ala
    130                 135                 140

Pro Ser Gly Leu Glu Ser Ala Leu Ala Ala Arg Ala Ala Arg Arg Gln
145                 150                 155                 160

Arg Ala Gln Leu Asp Arg Thr Arg Ser Gly Ala His Lys Ala Leu Arg
                165                 170                 175

Gly Leu Arg Phe Ile Ser Ser Asn Lys Ala Asn Asn Ala Trp Met Glu
            180                 185                 190

Val Gln Ala Asn Phe Asp Arg Leu Ala Arg Asp Gly Tyr Leu Ser Arg
        195                 200                 205

Ser Asp Phe Ala Glu Cys Ile Gly Met Thr Glu Ser Lys Glu Phe Ala
    210                 215                 220

Leu Glu Leu Phe Asp Thr Leu Ser Arg Arg Arg Gln Met Lys Val Asp
225                 230                 235                 240

Thr Ile Asn Lys Asp Glu Leu Arg Glu Ile Trp Gln Gln Ile Thr Asp
                245                 250                 255

Asn Ser Phe Asp Ser Arg Leu Gln Ile Phe Phe Glu Met Val Asp Lys
            260                 265                 270

Asn Ala Asp Gly Arg Ile Thr Glu Ala Glu Val Lys Glu Ile Ile Met
        275                 280                 285

Leu Ser Ala Ser Ala Asn Lys Leu Ser Arg Leu Lys Glu Gln Ala Glu
    290                 295                 300

Glu Tyr Ala Ala Leu Ile Met Glu Glu Leu Asp Pro Glu Gly Leu Gly
305                 310                 315                 320

Tyr Ile Glu Leu Trp Gln Leu Glu Thr Leu Leu Leu Gln Lys Asp Thr
                325                 330                 335

Tyr Met Asn Tyr Ser Gln Ala Leu Ser Tyr Thr Ser Gln Ala Leu Ser
            340                 345                 350

Gln Asn Leu Ala Gly Leu Arg Lys Lys Ser Ser Ile Arg Lys Ile Ser
        355                 360                 365

Thr Ser Leu Ser Tyr Tyr Phe Glu Asp Asn Trp Lys Arg Leu Trp Val
    370                 375                 380

Leu Ala Leu Trp Ile Gly Ile Met Ala Gly Leu Phe Thr Trp Lys Phe
385                 390                 395                 400

Met Gln Tyr Arg Asn Arg Tyr Val Phe Asp Val Met Gly Tyr Cys Val
                405                 410                 415
```

Thr Thr Ala Lys Gly Ala Ala Glu Thr Leu Lys Leu Asn Met Ala Ile
420 425 430

Ile Leu Leu Pro Val Cys Arg Asn Thr Ile Thr Trp Leu Arg Ser Thr
435 440 445

Arg Ala Ala Arg Ala Leu Pro Phe Asp Asp Asn Ile Asn Phe His Lys
450 455 460

Thr Ile Ala Ala Ala Ile Val Val Gly Ile Ile Leu His Ala Gly Asn
465 470 475 480

His Leu Val Cys Asp Phe Pro Arg Leu Ile Lys Ser Ser Asp Glu Lys
485 490 495

Tyr Ala Pro Leu Gly Gln Tyr Phe Gly Glu Ile Lys Pro Thr Tyr Phe
500 505 510

Thr Leu Val Lys Gly Val Glu Gly Ile Thr Gly Val Ile Met Val Val
515 520 525

Cys Met Ile Ile Ala Phe Thr Leu Ala Thr Arg Trp Phe Arg Arg Ser
530 535 540

Leu Val Lys Leu Pro Arg Pro Phe Asp Lys Leu Thr Gly Phe Asn Ala
545 550 555 560

Phe Trp Tyr Ser His His Leu Phe Ile Ile Val Tyr Ile Ala Leu Ile
565 570 575

Val His Gly Glu Cys Leu Tyr Leu Ile His Val Trp Tyr Arg Arg Thr
580 585 590

Thr Trp Met Tyr Leu Ser Val Pro Val Cys Leu Tyr Val Gly Glu Arg
595 600 605

Ile Leu Arg Phe Phe Arg Ser Gly Ser Tyr Ser Val Arg Leu Leu Lys
610 615 620

Val Ala Ile Tyr Pro Gly Asn Val Leu Thr Leu Gln Met Ser Lys Pro
625 630 635 640

Pro Thr Phe Arg Tyr Lys Ser Gly Gln Tyr Met Phe Val Gln Cys Pro
645 650 655

Ala Val Ser Pro Phe Glu Trp His Pro Phe Ser Ile Thr Ser Ala Pro
660 665 670

Gly Asp Asp Tyr Leu Ser Ile His Val Arg Gln Leu Gly Asp Trp Thr
675 680 685

Arg Glu Leu Lys Arg Val Phe Ala Ala Ala Cys Glu Pro Pro Ala Gly
690 695 700

Gly Lys Ser Gly Leu Leu Arg Ala Asp Glu Thr Thr Lys Lys Ile Leu
705 710 715 720

Pro Lys Leu Leu Ile Asp Gly Pro Tyr Gly Ser Pro Ala Gln Asp Tyr
725 730 735

Ser Lys Tyr Asp Val Leu Leu Leu Val Gly Leu Gly Ile Gly Ala Thr
740 745 750

Pro Phe Ile Ser Ile Leu Lys Asp Leu Leu Asn Asn Ile Ile Lys Met
755 760 765

Glu Glu Glu Glu Asp Ala Ser Thr Asp Leu Tyr Pro Pro Met Gly Arg
770 775 780

Asn Lys Pro His Val Asp Leu Gly Thr Leu Met Thr Ile Thr Ser Arg
785 790 795 800

```
Pro Lys Lys Ile Leu Lys Thr Thr Asn Ala Tyr Phe Tyr Trp Val Thr
                805                 810                 815
Arg Glu Gln Gly Ser Phe Asp Trp Phe Lys Gly Val Met Asn Glu Ile
            820                 825                 830
Ala Asp Leu Asp Gln Arg Asn Ile Ile Glu Met His Asn Tyr Leu Thr
        835                 840                 845
Ser Val Tyr Glu Glu Gly Asp Ala Arg Ser Ala Leu Ile Thr Met Leu
    850                 855                 860
Gln Ala Leu Asn His Ala Lys Asn Gly Val Asp Ile Val Ser Gly Thr
865                 870                 875                 880
Lys Val Arg Thr His Phe Ala Arg Pro Asn Trp Arg Lys Val Leu Ser
                885                 890                 895
Lys Ile Ser Ser Lys His Pro Tyr Ala Lys Ile Gly Val Phe Tyr Cys
            900                 905                 910
Gly Ala Pro Val Leu Ala Gln Glu Leu Ser Lys Leu Cys His Glu Phe
        915                 920                 925
Asn Gly Lys Cys Thr Thr Lys Phe Glu Phe His Lys Glu His Phe
    930                 935                 940
```

<210> 5
<211> 2889
<212> DNA
<213> Nicotiana tabacum

<220>
<221> CDS
<222> (1)..(2886)
<223> coding for NADPH-oxidase

<400> 5

```
atg agg ggt tta cct ggg cat gaa cgc cgg tgg aca tcc gat acg gta    48
Met Arg Gly Leu Pro Gly His Glu Arg Arg Trp Thr Ser Asp Thr Val
 1               5                  10                  15

tct tcc gac aag gat ttt agt ggt gaa tta tcg ccg gga gct gat tcc    96
Ser Ser Asp Lys Asp Phe Ser Gly Glu Leu Ser Pro Gly Ala Asp Ser
            20                  25                  30

ggc tat aat tcc ggt ttt gct tcc gag gag ttt gtt gaa gtc acg ctt    144
Gly Tyr Asn Ser Gly Phe Ala Ser Glu Glu Phe Val Glu Val Thr Leu
        35                  40                  45

gat ctt cag gat gat gat acc att att cta cgg agc gtt gaa ccg gct    192
Asp Leu Gln Asp Asp Asp Thr Ile Ile Leu Arg Ser Val Glu Pro Ala
    50                  55                  60

act gtg att aac att gac gct cct gat ctt ccc gcc gga gtc ggt att    240
Thr Val Ile Asn Ile Asp Ala Pro Asp Leu Pro Ala Gly Val Gly Ile
65                  70                  75                  80

tcc gga gtt tca att gaa act ccg acg tca gca tcg gtg tcg gaa tct    288
Ser Gly Val Ser Ile Glu Thr Pro Thr Ser Ala Ser Val Ser Glu Ser
                85                  90                  95

cga tcg ccg acg atc cgc cgg agt tca tct agt aaa ctt cgt cag ttt    336
Arg Ser Pro Thr Ile Arg Arg Ser Ser Ser Ser Lys Leu Arg Gln Phe
            100                 105                 110
```

```
tca cag gag ttg aaa gct gag gcg gtt gcg aaa gcg agg cag ttt tca     384
Ser Gln Glu Leu Lys Ala Glu Ala Val Ala Lys Ala Arg Gln Phe Ser
        115                 120                 125

caa gag ctg aag gcg gag tta agg aga ttc tca tgg agc cat ggg cat     432
Gln Glu Leu Lys Ala Glu Leu Arg Arg Phe Ser Trp Ser His Gly His
    130                 135                 140

gcg tct cgc gcg ttt tcg ccc tcg tcg ttt ttt caa aac gcc gtc gtt     480
Ala Ser Arg Ala Phe Ser Pro Ser Ser Phe Phe Gln Asn Ala Val Val
145                 150                 155                 160

gga aca ggt aac ggc gtg gac tcg gct tta gcg gca cgt gca tta cgt     528
Gly Thr Gly Asn Gly Val Asp Ser Ala Leu Ala Ala Arg Ala Leu Arg
                165                 170                 175

cgg caa cgc gcg cag ctt gat cgg act cgt tcc agc gcc cat aga gct     576
Arg Gln Arg Ala Gln Leu Asp Arg Thr Arg Ser Ser Ala His Arg Ala
            180                 185                 190

ctt cgt aga ctc aaa ttc att agc aat aac aaa acc aat gga tgg aat     624
Leu Arg Arg Leu Lys Phe Ile Ser Asn Asn Lys Thr Asn Gly Trp Asn
        195                 200                 205

gaa gtt gaa aac aat ttc tca aag ctc gct aaa gac ggt tat ctt tac     672
Glu Val Glu Asn Asn Phe Ser Lys Leu Ala Lys Asp Gly Tyr Leu Tyr
    210                 215                 220

cgt tcc gat ttc gca caa tgc ata ggt atg aag gat tcg aag gaa ttt     720
Arg Ser Asp Phe Ala Gln Cys Ile Gly Met Lys Asp Ser Lys Glu Phe
225                 230                 235                 240

gca ttg gaa tta ttt gat gct ttg agt aga aga aga aga tta aag gtt     768
Ala Leu Glu Leu Phe Asp Ala Leu Ser Arg Arg Arg Arg Leu Lys Val
                245                 250                 255

gat aaa att agc aag gag gaa ttg tat gag tac tgg tct caa atc acc     816
Asp Lys Ile Ser Lys Glu Glu Leu Tyr Glu Tyr Trp Ser Gln Ile Thr
            260                 265                 270

gat cag agt ttc gat tct cgg ctt cag atc tcc ttc gac atg gtg gac     864
Asp Gln Ser Phe Asp Ser Arg Leu Gln Ile Ser Phe Asp Met Val Asp
        275                 280                 285

aag aat gaa gat ggt cga att gct gaa gag gaa gta aaa gag atc atc     912
Lys Asn Glu Asp Gly Arg Ile Ala Glu Glu Glu Val Lys Glu Ile Ile
    290                 295                 300

atg cta agt gca tct gca aac aag tta tca aga tta aaa gaa caa gca     960
Met Leu Ser Ala Ser Ala Asn Lys Leu Ser Arg Leu Lys Glu Gln Ala
305                 310                 315                 320

gag gag tat gca gct tta atc atg gaa gaa tta gat cct gaa aga ctc    1008
Glu Glu Tyr Ala Ala Leu Ile Met Glu Glu Leu Asp Pro Glu Arg Leu
                325                 330                 335

ggc tac att gag cta tgg cag ctg gaa aca ctt ctc ctc caa aag gac    1056
Gly Tyr Ile Glu Leu Trp Gln Leu Glu Thr Leu Leu Leu Gln Lys Asp
            340                 345                 350

act tac ctc aac tac agt caa gca cta agt tac acg agc caa gcc ttg    1104
Thr Tyr Leu Asn Tyr Ser Gln Ala Leu Ser Tyr Thr Ser Gln Ala Leu
        355                 360                 365

agc caa aac ctt cac gga tta agg aag aaa agc cca ata aaa aga atg    1152
Ser Gln Asn Leu His Gly Leu Arg Lys Lys Ser Pro Ile Lys Arg Met
    370                 375                 380
```

```
agc aca aaa ctt gtc tat tca ttg caa gaa aac tgg aag aga att tgg      1200
Ser Thr Lys Leu Val Tyr Ser Leu Gln Glu Asn Trp Lys Arg Ile Trp
385                 390                 395                 400

gtt ctc act tta tgg att ttg ata atg att ggg ctt ttt ctt tgg aag      1248
Val Leu Thr Leu Trp Ile Leu Ile Met Ile Gly Leu Phe Leu Trp Lys
                405                 410                 415

ttc tat cag tac aaa aac aag agt gca ttc cgt gtc atg ggt tat tgc      1296
Phe Tyr Gln Tyr Lys Asn Lys Ser Ala Phe Arg Val Met Gly Tyr Cys
            420                 425                 430

ctt gtc acg gct aag ggc gct gct gag act ctc aag ttc aac atg gct      1344
Leu Val Thr Ala Lys Gly Ala Ala Glu Thr Leu Lys Phe Asn Met Ala
        435                 440                 445

ctt ata tta ttg cca gta tgc aga aac act att aca tgg ctc agg tcc      1392
Leu Ile Leu Leu Pro Val Cys Arg Asn Thr Ile Thr Trp Leu Arg Ser
    450                 455                 460

acc aag ttg agc cat ttt gta ccc ttt gac gac aac atc aac ttt cac      1440
Thr Lys Leu Ser His Phe Val Pro Phe Asp Asp Asn Ile Asn Phe His
465                 470                 475                 480

aag act gtc gct gca gcc att gtc act ggt atc ata ctc cat gct ggt      1488
Lys Thr Val Ala Ala Ala Ile Val Thr Gly Ile Ile Leu His Ala Gly
                485                 490                 495

aac cat ctt gta tgt gat ttc cca agg ctt ata cat gca gat gat caa      1536
Asn His Leu Val Cys Asp Phe Pro Arg Leu Ile His Ala Asp Asp Gln
            500                 505                 510

gat tat caa agt ttt ttg tcg aat gat ttt ggc caa agt aag cct gga      1584
Asp Tyr Gln Ser Phe Leu Ser Asn Asp Phe Gly Gln Ser Lys Pro Gly
        515                 520                 525

tac ata gac ctt gtt aaa gga gtt gag ggt gtg acg gga ata ata atg      1632
Tyr Ile Asp Leu Val Lys Gly Val Glu Gly Val Thr Gly Ile Ile Met
    530                 535                 540

gta atc ctt atg gcc att gct ttc act ctt gct aca cga tgg ttt aga      1680
Val Ile Leu Met Ala Ile Ala Phe Thr Leu Ala Thr Arg Trp Phe Arg
545                 550                 555                 560

cgg agc ctc att aag ttg ccc aaa cct ttt gat aga ctc act ggc ttc      1728
Arg Ser Leu Ile Lys Leu Pro Lys Pro Phe Asp Arg Leu Thr Gly Phe
                565                 570                 575

aat gca ttc tgg tat tca cac cac ctt ctt gtc att gtc tac atc cta      1776
Asn Ala Phe Trp Tyr Ser His His Leu Leu Val Ile Val Tyr Ile Leu
            580                 585                 590

ctg atc atc cat ggc acg ttc ctc ttc ctt gtg cat aaa tgg tac tcc      1824
Leu Ile Ile His Gly Thr Phe Leu Phe Leu Val His Lys Trp Tyr Ser
        595                 600                 605

aag acg acg tgg atg tat cta gca gtt cct gtg ctt ctc tac gca ggg      1872
Lys Thr Thr Trp Met Tyr Leu Ala Val Pro Val Leu Leu Tyr Ala Gly
    610                 615                 620

gaa aga act ctt aga ttc ttc cgg tca ggc ttg tac act gtc cgg ctt      1920
Glu Arg Thr Leu Arg Phe Phe Arg Ser Gly Leu Tyr Thr Val Arg Leu
625                 630                 635                 640

ctg aaa gta gca ata tat cct gga aat gtc ctc act cta caa atg tct      1968
Leu Lys Val Ala Ile Tyr Pro Gly Asn Val Leu Thr Leu Gln Met Ser
                645                 650                 655
```

```
aag cct cct caa ttt cga tac aaa agt gga caa tat atg ttt gtc cag      2016
Lys Pro Pro Gln Phe Arg Tyr Lys Ser Gly Gln Tyr Met Phe Val Gln
            660                 665                 670

tgt cca gct gtt tct cca ttc gag tgg cat cca ttt tcc att act tca      2064
Cys Pro Ala Val Ser Pro Phe Glu Trp His Pro Phe Ser Ile Thr Ser
        675                 680                 685

gct cct ggg gat gac tac ttg agc att cac atc cgg caa ctt ggt gac      2112
Ala Pro Gly Asp Asp Tyr Leu Ser Ile His Ile Arg Gln Leu Gly Asp
    690                 695                 700

tgg act caa gaa ctc aag cgg gtc ttt tct gag gct tgc gag cgg cca      2160
Trp Thr Gln Glu Leu Lys Arg Val Phe Ser Glu Ala Cys Glu Arg Pro
705                 710                 715                 720

gag gct gga aag agt ggc ctg ctc aga gct gac gaa aac act aag aaa      2208
Glu Ala Gly Lys Ser Gly Leu Leu Arg Ala Asp Glu Asn Thr Lys Lys
                725                 730                 735

agt ttg cca aag cta tta ata gat gga cct tac gga gct cca gca caa      2256
Ser Leu Pro Lys Leu Leu Ile Asp Gly Pro Tyr Gly Ala Pro Ala Gln
            740                 745                 750

gat tac cga aaa tat gat gtc ttg ctg ctt gtt ggt ctt ggc att gga      2304
Asp Tyr Arg Lys Tyr Asp Val Leu Leu Leu Val Gly Leu Gly Ile Gly
        755                 760                 765

gca acg ccg ttc ata agt atc ctg aaa gac ttg ctc gtt aac atc gtg      2352
Ala Thr Pro Phe Ile Ser Ile Leu Lys Asp Leu Leu Val Asn Ile Val
    770                 775                 780

aaa atg gag gag caa gca gat tta gcc tca gat ttc agt ggg aac tca      2400
Lys Met Glu Glu Gln Ala Asp Leu Ala Ser Asp Phe Ser Gly Asn Ser
785                 790                 795                 800

gac atg agc gtt gcg aca agt gaa caa cca gct ctc aac aag att tct      2448
Asp Met Ser Val Ala Thr Ser Glu Gln Pro Ala Leu Asn Lys Ile Ser
                805                 810                 815

ctg aaa agg aga aag agc act cta aga acc aca aat gca tat ttt tat      2496
Leu Lys Arg Arg Lys Ser Thr Leu Arg Thr Thr Asn Ala Tyr Phe Tyr
            820                 825                 830

tgg gtg acc cgg gag caa gga tca ttt gat tgg ttc aaa ggc gtt atg      2544
Trp Val Thr Arg Glu Gln Gly Ser Phe Asp Trp Phe Lys Gly Val Met
        835                 840                 845

aac gaa gtg gct gaa ctt gat caa agg ggg gtc atc gag atg cat aac      2592
Asn Glu Val Ala Glu Leu Asp Gln Arg Gly Val Ile Glu Met His Asn
    850                 855                 860

tac ttg acg agt gtt tat gag gaa ggg gat gct cgt tca gct ctc att      2640
Tyr Leu Thr Ser Val Tyr Glu Glu Gly Asp Ala Arg Ser Ala Leu Ile
865                 870                 875                 880

acc atg gtc cag gca ctt aac cat gct aag aat ggg gtt gat att gta      2688
Thr Met Val Gln Ala Leu Asn His Ala Lys Asn Gly Val Asp Ile Val
                885                 890                 895

tca ggc acc agg gtg agg aca cat ttt gct agg cca aat tgg aag aaa      2736
Ser Gly Thr Arg Val Arg Thr His Phe Ala Arg Pro Asn Trp Lys Lys
            900                 905                 910

gta ttt tcc aag acc tta acc aag cat gca aat gca aga ata ggg gtt      2784
Val Phe Ser Lys Thr Leu Thr Lys His Ala Asn Ala Arg Ile Gly Val
        915                 920                 925
```

```
ttc tac tgt ggt gca ccc gta tta gca aaa gaa ctc agc aaa ctc tgc    2832
Phe Tyr Cys Gly Ala Pro Val Leu Ala Lys Glu Leu Ser Lys Leu Cys
    930                 935                 940

aaa gag tat aat caa aag ggt gca aca aag ttc gag ttt cac aaa gaa    2880
Lys Glu Tyr Asn Gln Lys Gly Ala Thr Lys Phe Glu Phe His Lys Glu
945                 950                 955                 960

cat ttt tag                                                        2889
His Phe
```

<210> 6
<211> 962
<212> PRT
<213> Nicotiana tabacum

<400> 6

```
Met Arg Gly Leu Pro Gly His Glu Arg Arg Trp Thr Ser Asp Thr Val
 1               5                  10                  15
Ser Ser Asp Lys Asp Phe Ser Gly Glu Leu Ser Pro Gly Ala Asp Ser
            20                  25                  30
Gly Tyr Asn Ser Gly Phe Ala Ser Glu Glu Phe Val Glu Val Thr Leu
        35                  40                  45
Asp Leu Gln Asp Asp Asp Thr Ile Ile Leu Arg Ser Val Glu Pro Ala
    50                  55                  60
Thr Val Ile Asn Ile Asp Ala Pro Asp Leu Pro Ala Gly Val Gly Ile
65                  70                  75                  80
Ser Gly Val Ser Ile Glu Thr Pro Thr Ser Ala Ser Val Ser Glu Ser
                85                  90                  95
Arg Ser Pro Thr Ile Arg Arg Ser Ser Ser Ser Lys Leu Arg Gln Phe
            100                 105                 110
Ser Gln Glu Leu Lys Ala Glu Ala Val Ala Lys Ala Arg Gln Phe Ser
        115                 120                 125
Gln Glu Leu Lys Ala Glu Leu Arg Arg Phe Ser Trp Ser His Gly His
    130                 135                 140
Ala Ser Arg Ala Phe Ser Pro Ser Ser Phe Phe Gln Asn Ala Val Val
145                 150                 155                 160
Gly Thr Gly Asn Gly Val Asp Ser Ala Leu Ala Ala Arg Ala Leu Arg
                165                 170                 175
Arg Gln Arg Ala Gln Leu Asp Arg Thr Arg Ser Ser Ala His Arg Ala
            180                 185                 190
Leu Arg Arg Leu Lys Phe Ile Ser Asn Asn Lys Thr Asn Gly Trp Asn
        195                 200                 205
Glu Val Glu Asn Asn Phe Ser Lys Leu Ala Lys Asp Gly Tyr Leu Tyr
    210                 215                 220
Arg Ser Asp Phe Ala Gln Cys Ile Gly Met Lys Asp Ser Lys Glu Phe
225                 230                 235                 240
Ala Leu Glu Leu Phe Asp Ala Leu Ser Arg Arg Arg Arg Leu Lys Val
                245                 250                 255
Asp Lys Ile Ser Lys Glu Glu Leu Tyr Glu Tyr Trp Ser Gln Ile Thr
            260                 265                 270
Asp Gln Ser Phe Asp Ser Arg Leu Gln Ile Ser Phe Asp Met Val Asp
        275                 280                 285
```

Lys Asn Glu Asp Gly Arg Ile Ala Glu Glu Glu Val Lys Glu Ile Ile
290 295 300

Met Leu Ser Ala Ser Ala Asn Lys Leu Ser Arg Leu Lys Glu Gln Ala
305 310 315 320

Glu Glu Tyr Ala Ala Leu Ile Met Glu Glu Leu Asp Pro Glu Arg Leu
325 330 335

Gly Tyr Ile Glu Leu Trp Gln Leu Glu Thr Leu Leu Leu Gln Lys Asp
340 345 350

Thr Tyr Leu Asn Tyr Ser Gln Ala Leu Ser Tyr Thr Ser Gln Ala Leu
355 360 365

Ser Gln Asn Leu His Gly Leu Arg Lys Lys Ser Pro Ile Lys Arg Met
370 375 380

Ser Thr Lys Leu Val Tyr Ser Leu Gln Glu Asn Trp Lys Arg Ile Trp
385 390 395 400

Val Leu Thr Leu Trp Ile Leu Ile Met Ile Gly Leu Phe Leu Trp Lys
405 410 415

Phe Tyr Gln Tyr Lys Asn Lys Ser Ala Phe Arg Val Met Gly Tyr Cys
420 425 430

Leu Val Thr Ala Lys Gly Ala Ala Glu Thr Leu Lys Phe Asn Met Ala
435 440 445

Leu Ile Leu Leu Pro Val Cys Arg Asn Thr Ile Thr Trp Leu Arg Ser
450 455 460

Thr Lys Leu Ser His Phe Val Pro Phe Asp Asp Asn Ile Asn Phe His
465 470 475 480

Lys Thr Val Ala Ala Ala Ile Val Thr Gly Ile Ile Leu His Ala Gly
485 490 495

Asn His Leu Val Cys Asp Phe Pro Arg Leu Ile His Ala Asp Asp Gln
500 505 510

Asp Tyr Gln Ser Phe Leu Ser Asn Asp Phe Gly Gln Ser Lys Pro Gly
515 520 525

Tyr Ile Asp Leu Val Lys Gly Val Glu Gly Val Thr Gly Ile Ile Met
530 535 540

Val Ile Leu Met Ala Ile Ala Phe Thr Leu Ala Thr Arg Trp Phe Arg
545 550 555 560

Arg Ser Leu Ile Lys Leu Pro Lys Pro Phe Asp Arg Leu Thr Gly Phe
565 570 575

Asn Ala Phe Trp Tyr Ser His His Leu Leu Val Ile Val Tyr Ile Leu
580 585 590

Leu Ile Ile His Gly Thr Phe Leu Phe Leu Val His Lys Trp Tyr Ser
595 600 605

Lys Thr Thr Trp Met Tyr Leu Ala Val Pro Val Leu Leu Tyr Ala Gly
610 615 620

Glu Arg Thr Leu Arg Phe Phe Arg Ser Gly Leu Tyr Thr Val Arg Leu
625 630 635 640

Leu Lys Val Ala Ile Tyr Pro Gly Asn Val Leu Thr Leu Gln Met Ser
645 650 655

Lys Pro Pro Gln Phe Arg Tyr Lys Ser Gly Gln Tyr Met Phe Val Gln
660 665 670

Cys Pro Ala Val Ser Pro Phe Glu Trp His Pro Phe Ser Ile Thr Ser
675 680 685

Ala Pro Gly Asp Asp Tyr Leu Ser Ile His Ile Arg Gln Leu Gly Asp
690 695 700

Trp Thr Gln Glu Leu Lys Arg Val Phe Ser Glu Ala Cys Glu Arg Pro
705 710 715 720

Glu Ala Gly Lys Ser Gly Leu Leu Arg Ala Asp Glu Asn Thr Lys Lys
725 730 735

Ser Leu Pro Lys Leu Leu Ile Asp Gly Pro Tyr Gly Ala Pro Ala Gln
740 745 750

Asp Tyr Arg Lys Tyr Asp Val Leu Leu Leu Val Gly Leu Gly Ile Gly
755 760 765

Ala Thr Pro Phe Ile Ser Ile Leu Lys Asp Leu Leu Val Asn Ile Val
770 775 780

Lys Met Glu Glu Gln Ala Asp Leu Ala Ser Asp Phe Ser Gly Asn Ser
785 790 795 800

Asp Met Ser Val Ala Thr Ser Glu Gln Pro Ala Leu Asn Lys Ile Ser
805 810 815

Leu Lys Arg Arg Lys Ser Thr Leu Arg Thr Thr Asn Ala Tyr Phe Tyr
820 825 830

Trp Val Thr Arg Glu Gln Gly Ser Phe Asp Trp Phe Lys Gly Val Met
835 840 845

Asn Glu Val Ala Glu Leu Asp Gln Arg Gly Val Ile Glu Met His Asn
850 855 860

Tyr Leu Thr Ser Val Tyr Glu Glu Gly Asp Ala Arg Ser Ala Leu Ile
865 870 875 880

Thr Met Val Gln Ala Leu Asn His Ala Lys Asn Gly Val Asp Ile Val
885 890 895

Ser Gly Thr Arg Val Arg Thr His Phe Ala Arg Pro Asn Trp Lys Lys
900 905 910

Val Phe Ser Lys Thr Leu Thr Lys His Ala Asn Ala Arg Ile Gly Val
915 920 925

Phe Tyr Cys Gly Ala Pro Val Leu Ala Lys Glu Leu Ser Lys Leu Cys
930 935 940

Lys Glu Tyr Asn Gln Lys Gly Ala Thr Lys Phe Glu Phe His Lys Glu
945 950 955 960

His Phe

<210> 7
<211> 3733
<212> DNA
<213> Solanum tuberosum

<220>
<221> CDS
<222> (92)..(2980)
<223> coding for NADPH-oxidase

<400> 7

ggcacgagaa taaccaaaac ttttggtcag gcttctgcag aaaactctgt tttcaacata 60

```
tatttattta ttgtgctttg atttgggaca a atg agg ggt tta cct ggg cat     112
                                   Met Arg Gly Leu Pro Gly His
                                   1               5

gaa cgc cgg tgg acg tcg gat acg gta tct tcc ggc aag gat tta agt    160
Glu Arg Arg Trp Thr Ser Asp Thr Val Ser Ser Gly Lys Asp Leu Ser
        10                  15                  20

ggt gag tca tcg ccg gga act gat tcc ggg aat att tcc ggt ttt gct    208
Gly Glu Ser Ser Pro Gly Thr Asp Ser Gly Asn Ile Ser Gly Phe Ala
    25                  30                  35

tcc gag gag ttt gtt gaa gtt ata ctt gat ctt cag gat gat gat acg    256
Ser Glu Glu Phe Val Glu Val Ile Leu Asp Leu Gln Asp Asp Asp Thr
40                  45                  50                  55

att att cta cgg agc gtt gaa ccg gct act gta atc aac att gat gct    304
Ile Ile Leu Arg Ser Val Glu Pro Ala Thr Val Ile Asn Ile Asp Ala
                60                  65                  70

tct gat cct gct acc gga gtc ggt att ggt gga gta tcg att gaa act    352
Ser Asp Pro Ala Thr Gly Val Gly Ile Gly Gly Val Ser Ile Glu Thr
            75                  80                  85

ccg gcg tcg ctg act tcg acg tcg gga act cga tcg ccg acg atg cgt    400
Pro Ala Ser Leu Thr Ser Thr Ser Gly Thr Arg Ser Pro Thr Met Arg
        90                  95                  100

cgg agt aca tcg aat aaa tta cgt cag ttt tca cag gag ttg aaa gct    448
Arg Ser Thr Ser Asn Lys Leu Arg Gln Phe Ser Gln Glu Leu Lys Ala
    105                 110                 115

gag gct gtc gcg aaa gcg aag cat ttc tcg caa gag ctt aaa gcg gag    496
Glu Ala Val Ala Lys Ala Lys His Phe Ser Gln Glu Leu Lys Ala Glu
120                 125                 130                 135

cta agg aga ttc tca tgg agc cat gga cat gcg tct cgc act ttt tcg    544
Leu Arg Arg Phe Ser Trp Ser His Gly His Ala Ser Arg Thr Phe Ser
                140                 145                 150

ccg gcg tcg ttt ttc caa aac gcc gtc gtc ggt aca ggc aac ggt gta    592
Pro Ala Ser Phe Phe Gln Asn Ala Val Val Gly Thr Gly Asn Gly Val
            155                 160                 165

gat tcg gct tta gca gct cga gca tta cga cgg cag cgc gct cag ctc    640
Asp Ser Ala Leu Ala Ala Arg Ala Leu Arg Arg Gln Arg Ala Gln Leu
        170                 175                 180

gat cgg act cgt tcc agc gct cac aag gct ctt cgt gga ctc aaa ttc    688
Asp Arg Thr Arg Ser Ser Ala His Lys Ala Leu Arg Gly Leu Lys Phe
    185                 190                 195

atc agc aat aac aaa act aac gga tgg aat gaa gtt gaa aac aat ttt    736
Ile Ser Asn Asn Lys Thr Asn Gly Trp Asn Glu Val Glu Asn Asn Phe
200                 205                 210                 215

gct aag ctc gct aaa gac ggt tac ctt tat cgc tcc gat ttc gca caa    784
Ala Lys Leu Ala Lys Asp Gly Tyr Leu Tyr Arg Ser Asp Phe Ala Gln
                220                 225                 230

tgc atc ggt atg aag gat tca aag gaa ttt gca ttg gaa ttg ttt gat    832
Cys Ile Gly Met Lys Asp Ser Lys Glu Phe Ala Leu Glu Leu Phe Asp
            235                 240                 245

gct ttg agt aga aga aga aga ttg aag gtt gat aag att agc aaa gag    880
Ala Leu Ser Arg Arg Arg Arg Leu Lys Val Asp Lys Ile Ser Lys Glu
        250                 255                 260
```

```
gaa ttg tat gag tat tgg tct caa atc acc gat cag agt ttc gat tct     928
Glu Leu Tyr Glu Tyr Trp Ser Gln Ile Thr Asp Gln Ser Phe Asp Ser
    265                 270                 275

cgg ctt cag atc ttc ttc gac atg gtg gac aag aat gaa gat ggt cga     976
Arg Leu Gln Ile Phe Phe Asp Met Val Asp Lys Asn Glu Asp Gly Arg
280                 285                 290                 295

att ggt gaa gaa gaa gta aaa gag atc atc atg cta agt gcc tct gca     1024
Ile Gly Glu Glu Glu Val Lys Glu Ile Ile Met Leu Ser Ala Ser Ala
                300                 305                 310

aac aaa tta tca aga tta aaa gaa caa gca gag gag tat gca gct ctg     1072
Asn Lys Leu Ser Arg Leu Lys Glu Gln Ala Glu Glu Tyr Ala Ala Leu
            315                 320                 325

atc atg gaa gaa tta gat cct gaa aga ctt ggc tac att gag cta tgg     1120
Ile Met Glu Glu Leu Asp Pro Glu Arg Leu Gly Tyr Ile Glu Leu Trp
        330                 335                 340

cag ctg gaa acg ctt ctc ctc caa aag gac act tac ctc aac tac agt     1168
Gln Leu Glu Thr Leu Leu Leu Gln Lys Asp Thr Tyr Leu Asn Tyr Ser
    345                 350                 355

caa gca cta agc tac aca agc caa gct ttg agc caa aac ctg caa ggg     1216
Gln Ala Leu Ser Tyr Thr Ser Gln Ala Leu Ser Gln Asn Leu Gln Gly
360                 365                 370                 375

ttg agg aag aga agc cca ata aga aga atg agc aca aaa ctt gtc tat     1264
Leu Arg Lys Arg Ser Pro Ile Arg Arg Met Ser Thr Lys Leu Val Tyr
                380                 385                 390

tca ctg caa gag aat tgg aag aga att tgg gtt ctg gtc ttg tgg att     1312
Ser Leu Gln Glu Asn Trp Lys Arg Ile Trp Val Leu Val Leu Trp Ile
            395                 400                 405

ttg ata atg att gga ctt ttt ctt tgg aag ttc tat ctg tac aaa cag     1360
Leu Ile Met Ile Gly Leu Phe Leu Trp Lys Phe Tyr Leu Tyr Lys Gln
        410                 415                 420

aaa agt gca ttt caa gtt atg ggt tat tgc ctt cta aca gct aag ggt     1408
Lys Ser Ala Phe Gln Val Met Gly Tyr Cys Leu Leu Thr Ala Lys Gly
    425                 430                 435

gct gct gag act cta aag ttc aac atg gct ttg ata ttg ttg cca gtt     1456
Ala Ala Glu Thr Leu Lys Phe Asn Met Ala Leu Ile Leu Leu Pro Val
440                 445                 450                 455

tgc agg aac acc att aca ttc ctc agg tct act aaa ttg agt tgt ttt     1504
Cys Arg Asn Thr Ile Thr Phe Leu Arg Ser Thr Lys Leu Ser Cys Phe
                460                 465                 470

gta ccc ttt gat gac aac atc aac ttc cac aag act gtt gct gca gcc     1552
Val Pro Phe Asp Asp Asn Ile Asn Phe His Lys Thr Val Ala Ala Ala
            475                 480                 485

att gtt act ggt atc ata ctc cat gcc ggt aat cat ctt gta tgt gat     1600
Ile Val Thr Gly Ile Ile Leu His Ala Gly Asn His Leu Val Cys Asp
        490                 495                 500

ttc cca aag ctt ata cat gca aat aat acg aat tat cag aaa tat ttg     1648
Phe Pro Lys Leu Ile His Ala Asn Asn Thr Asn Tyr Gln Lys Tyr Leu
    505                 510                 515

gtg aat gat ttt ggc cca agc cag cct cag tac ata gat ctt gtt aaa     1696
Val Asn Asp Phe Gly Pro Ser Gln Pro Gln Tyr Ile Asp Leu Val Lys
520                 525                 530                 535
```

```
gga gtg gag ggt gtg aca gga ata ata atg gta atc ctc atg gcc att      1744
Gly Val Glu Gly Val Thr Gly Ile Ile Met Val Ile Leu Met Ala Ile
                540                 545                 550

gct ttc act ctt gca acg cga tgg ttt agg cgg agc ctc att aag ttt      1792
Ala Phe Thr Leu Ala Thr Arg Trp Phe Arg Arg Ser Leu Ile Lys Phe
            555                 560                 565

ccc aaa cct ttt gat aga ctc act ggt ttc aat gcg ttc tgg tac tcg      1840
Pro Lys Pro Phe Asp Arg Leu Thr Gly Phe Asn Ala Phe Trp Tyr Ser
        570                 575                 580

cac cac ctt ctc atc att gtc tac atc gta ctg atc atc cat ggc aca      1888
His His Leu Leu Ile Ile Val Tyr Ile Val Leu Ile Ile His Gly Thr
    585                 590                 595

ttc ctc tac ctt gtg cat aac tgg tac tcc aaa acg aca tgg atg tat      1936
Phe Leu Tyr Leu Val His Asn Trp Tyr Ser Lys Thr Thr Trp Met Tyr
600                 605                 610                 615

cta gca gtt cct gta ctt ctc tac gca ggg gaa aga act ctt aga ttc      1984
Leu Ala Val Pro Val Leu Leu Tyr Ala Gly Glu Arg Thr Leu Arg Phe
                620                 625                 630

ttc cga tca ggc tta tat aca gtc cgg ctt cta aaa gta gca ata tat      2032
Phe Arg Ser Gly Leu Tyr Thr Val Arg Leu Leu Lys Val Ala Ile Tyr
            635                 640                 645

cct gga aat gtc ctt act ctg caa atg tct aag cct ccg caa ttt cga      2080
Pro Gly Asn Val Leu Thr Leu Gln Met Ser Lys Pro Pro Gln Phe Arg
        650                 655                 660

tac aag agt gga caa tat atg ttt gtc cag tgt cca gct gtt tct cca      2128
Tyr Lys Ser Gly Gln Tyr Met Phe Val Gln Cys Pro Ala Val Ser Pro
    665                 670                 675

ttc gag tgg cat cca ttt tcc att act tca gct cct ggg gat gac tac      2176
Phe Glu Trp His Pro Phe Ser Ile Thr Ser Ala Pro Gly Asp Asp Tyr
680                 685                 690                 695

ttg agc att cat atc cga caa ctt ggt gac tgg act caa gaa ctc aag      2224
Leu Ser Ile His Ile Arg Gln Leu Gly Asp Trp Thr Gln Glu Leu Lys
                700                 705                 710

cgg gtg ttt tcc gag gct tgc gag cag cca gag gct gga aag agt ggc      2272
Arg Val Phe Ser Glu Ala Cys Glu Gln Pro Glu Ala Gly Lys Ser Gly
            715                 720                 725

ctg ctc aga gct gac gaa aac acc aaa aca agt ttg cca aag cta ttg      2320
Leu Leu Arg Ala Asp Glu Asn Thr Lys Thr Ser Leu Pro Lys Leu Leu
        730                 735                 740

ata gat gga cct tat gga gct cca gca caa gat tac cga aag tat gat      2368
Ile Asp Gly Pro Tyr Gly Ala Pro Ala Gln Asp Tyr Arg Lys Tyr Asp
    745                 750                 755

gtc tta ctg ctt gtt ggt ctt ggc att gga gca act ccc ttt ata agt      2416
Val Leu Leu Leu Val Gly Leu Gly Ile Gly Ala Thr Pro Phe Ile Ser
760                 765                 770                 775

atc ctg aaa gac ttg ctc aaa aac atc gtc aca atg gag gag caa gca      2464
Ile Leu Lys Asp Leu Leu Lys Asn Ile Val Thr Met Glu Glu Gln Ala
                780                 785                 790

gat tta gtc tcg gat ttt tca ggg aac tca gac atg agc gct gca aca      2512
Asp Leu Val Ser Asp Phe Ser Gly Asn Ser Asp Met Ser Ala Ala Thr
            795                 800                 805
```

```
agt gaa caa cca gct ctc aac aag att tct cca aaa aag aga aag agt    2560
Ser Glu Gln Pro Ala Leu Asn Lys Ile Ser Pro Lys Lys Arg Lys Ser
        810                 815                 820

act cta aaa acc aca aat gca tat ttt tat tgg gtg acc cgg gag caa    2608
Thr Leu Lys Thr Thr Asn Ala Tyr Phe Tyr Trp Val Thr Arg Glu Gln
    825                 830                 835

gga tca ttt gat tgg ttc aaa ggt gtt atg aac gaa gtg gct gaa ctt    2656
Gly Ser Phe Asp Trp Phe Lys Gly Val Met Asn Glu Val Ala Glu Leu
840                 845                 850                 855

gat caa agg ggg gtc atc gag atg cat aac tac tta acg agt gtt tat    2704
Asp Gln Arg Gly Val Ile Glu Met His Asn Tyr Leu Thr Ser Val Tyr
                860                 865                 870

gag gaa ggg gat gca cgt tca gct ctc att acc atg gtc cag gcg ctt    2752
Glu Glu Gly Asp Ala Arg Ser Ala Leu Ile Thr Met Val Gln Ala Leu
            875                 880                 885

aac cat gct aag aat ggg gtt gat att gta tca ggc acc agt gtg agg    2800
Asn His Ala Lys Asn Gly Val Asp Ile Val Ser Gly Thr Ser Val Arg
        890                 895                 900

aca cat ttt gcc aga ccg aat tgg agg aaa gta ttt tcc aag acc tta    2848
Thr His Phe Ala Arg Pro Asn Trp Arg Lys Val Phe Ser Lys Thr Leu
    905                 910                 915

acc aag cat gca aat gca aga ata gga gtt ttc tac tgc ggt gca ccc    2896
Thr Lys His Ala Asn Ala Arg Ile Gly Val Phe Tyr Cys Gly Ala Pro
920                 925                 930                 935

ata tta gct aaa gaa ctc agc aaa ctc tgc aaa gag ttt aac caa aag    2944
Ile Leu Ala Lys Glu Leu Ser Lys Leu Cys Lys Glu Phe Asn Gln Lys
                940                 945                 950

ggc aca acg aag ttc gag ttt cac aaa gaa cat ttt tagaaggccc         2990
Gly Thr Thr Lys Phe Glu Phe His Lys Glu His Phe
            955                 960

tggagtacaa ttaatcttgc atcaacggta cacacatcgg taaaccagta tttaccacat 3050
ctatctttgg tacctgattt gatgattcta ctgaagacat aacattagta aggaataagt 3110
cagagacaaa ttgtacataa taggaggaag cacatttaca gagaaaatac ataccaatat 3170
gatatgtgta taggttttgt atattcagtc atctgttatc acataccaaa cttcagaact 3230
ccaaaaggga gactctgctt tggtctgatg gcttagaata tgggagggaa aaaaagacga 3290
caattgaatg gtcacgatac acatgaagaa tgagaatatt gggaaacagc taataagaag 3350
ttgaccttct tgataaagaa acactatgaa aatggcaagc atgaaaggac agacaatcat 3410
ggcttggatg gggaaaacaa aatacaattt tgaaagaaga agataatatt agtaggagta 3470
gtgggggact gatagctttg ttggtggaac ttataatggg gctaagggaa tccttccaaa 3530
aaatgtctat gtagtaacta ctttttcttt tgctttgtga gtattttttg ggtatttta  3590
atatactact tattagataa gaggatagaa aatacgtgta tatgcaattc ttattagtaa 3650
agtttatctg tagtagttct ttaatctgga gaaaggtact atcaaaggaa atatctcatc 3710
gaaaaaaaaa aaaaaaaaaa aaa                                         3733
```

<210> 8

<211> 963
<212> PRT
<213> Solanum tuberosum

<400> 8

```
Met Arg Gly Leu Pro Gly His Glu Arg Arg Trp Thr Ser Asp Thr Val
 1               5                  10                  15
Ser Ser Gly Lys Asp Leu Ser Gly Glu Ser Ser Pro Gly Thr Asp Ser
            20                  25                  30
Gly Asn Ile Ser Gly Phe Ala Ser Glu Glu Phe Val Glu Val Ile Leu
        35                  40                  45
Asp Leu Gln Asp Asp Asp Thr Ile Ile Leu Arg Ser Val Glu Pro Ala
    50                  55                  60
Thr Val Ile Asn Ile Asp Ala Ser Asp Pro Ala Thr Gly Val Gly Ile
65                  70                  75                  80
Gly Gly Val Ser Ile Glu Thr Pro Ala Ser Leu Thr Ser Thr Ser Gly
                85                  90                  95
Thr Arg Ser Pro Thr Met Arg Arg Ser Thr Ser Asn Lys Leu Arg Gln
            100                 105                 110
Phe Ser Gln Glu Leu Lys Ala Glu Ala Val Ala Lys Ala Lys His Phe
        115                 120                 125
Ser Gln Glu Leu Lys Ala Glu Leu Arg Arg Phe Ser Trp Ser His Gly
    130                 135                 140
His Ala Ser Arg Thr Phe Ser Pro Ala Ser Phe Phe Gln Asn Ala Val
145                 150                 155                 160
Val Gly Thr Gly Asn Gly Val Asp Ser Ala Leu Ala Ala Arg Ala Leu
                165                 170                 175
Arg Arg Gln Arg Ala Gln Leu Asp Arg Thr Arg Ser Ser Ala His Lys
            180                 185                 190
Ala Leu Arg Gly Leu Lys Phe Ile Ser Asn Asn Lys Thr Asn Gly Trp
        195                 200                 205
Asn Glu Val Glu Asn Asn Phe Ala Lys Leu Ala Lys Asp Gly Tyr Leu
    210                 215                 220
Tyr Arg Ser Asp Phe Ala Gln Cys Ile Gly Met Lys Asp Ser Lys Glu
225                 230                 235                 240
Phe Ala Leu Glu Leu Phe Asp Ala Leu Ser Arg Arg Arg Arg Leu Lys
                245                 250                 255
Val Asp Lys Ile Ser Lys Glu Glu Leu Tyr Glu Tyr Trp Ser Gln Ile
            260                 265                 270
Thr Asp Gln Ser Phe Asp Ser Arg Leu Gln Ile Phe Phe Asp Met Val
        275                 280                 285
Asp Lys Asn Glu Asp Gly Arg Ile Gly Glu Glu Glu Val Lys Glu Ile
    290                 295                 300
Ile Met Leu Ser Ala Ser Ala Asn Lys Leu Ser Arg Leu Lys Glu Gln
305                 310                 315                 320
Ala Glu Glu Tyr Ala Ala Leu Ile Met Glu Glu Leu Asp Pro Glu Arg
                325                 330                 335
Leu Gly Tyr Ile Glu Leu Trp Gln Leu Glu Thr Leu Leu Leu Gln Lys
            340                 345                 350
Asp Thr Tyr Leu Asn Tyr Ser Gln Ala Leu Ser Tyr Thr Ser Gln Ala
        355                 360                 365
```

```
Leu Ser Gln Asn Leu Gln Gly Leu Arg Lys Arg Ser Pro Ile Arg Arg
    370                 375                 380

Met Ser Thr Lys Leu Val Tyr Ser Leu Gln Glu Asn Trp Lys Arg Ile
385                 390                 395                 400

Trp Val Leu Val Leu Trp Ile Leu Ile Met Ile Gly Leu Phe Leu Trp
                405                 410                 415

Lys Phe Tyr Leu Tyr Lys Gln Lys Ser Ala Phe Gln Val Met Gly Tyr
            420                 425                 430

Cys Leu Leu Thr Ala Lys Gly Ala Ala Glu Thr Leu Lys Phe Asn Met
        435                 440                 445

Ala Leu Ile Leu Leu Pro Val Cys Arg Asn Thr Ile Thr Phe Leu Arg
    450                 455                 460

Ser Thr Lys Leu Ser Cys Phe Val Pro Phe Asp Asp Asn Ile Asn Phe
465                 470                 475                 480

His Lys Thr Val Ala Ala Ala Ile Val Thr Gly Ile Ile Leu His Ala
                485                 490                 495

Gly Asn His Leu Val Cys Asp Phe Pro Lys Leu Ile His Ala Asn Asn
            500                 505                 510

Thr Asn Tyr Gln Lys Tyr Leu Val Asn Asp Phe Gly Pro Ser Gln Pro
        515                 520                 525

Gln Tyr Ile Asp Leu Val Lys Gly Val Glu Gly Val Thr Gly Ile Ile
    530                 535                 540

Met Val Ile Leu Met Ala Ile Ala Phe Thr Leu Ala Thr Arg Trp Phe
545                 550                 555                 560

Arg Arg Ser Leu Ile Lys Phe Pro Lys Pro Phe Asp Arg Leu Thr Gly
                565                 570                 575

Phe Asn Ala Phe Trp Tyr Ser His His Leu Leu Ile Ile Val Tyr Ile
            580                 585                 590

Val Leu Ile Ile His Gly Thr Phe Leu Tyr Leu Val His Asn Trp Tyr
        595                 600                 605

Ser Lys Thr Thr Trp Met Tyr Leu Ala Val Pro Val Leu Leu Tyr Ala
    610                 615                 620

Gly Glu Arg Thr Leu Arg Phe Phe Arg Ser Gly Leu Tyr Thr Val Arg
625                 630                 635                 640

Leu Leu Lys Val Ala Ile Tyr Pro Gly Asn Val Leu Thr Leu Gln Met
                645                 650                 655

Ser Lys Pro Pro Gln Phe Arg Tyr Lys Ser Gly Gln Tyr Met Phe Val
            660                 665                 670

Gln Cys Pro Ala Val Ser Pro Phe Glu Trp His Pro Phe Ser Ile Thr
        675                 680                 685

Ser Ala Pro Gly Asp Asp Tyr Leu Ser Ile His Ile Arg Gln Leu Gly
    690                 695                 700

Asp Trp Thr Gln Glu Leu Lys Arg Val Phe Ser Glu Ala Cys Glu Gln
705                 710                 715                 720

Pro Glu Ala Gly Lys Ser Gly Leu Leu Arg Ala Asp Glu Asn Thr Lys
                725                 730                 735

Thr Ser Leu Pro Lys Leu Leu Ile Asp Gly Pro Tyr Gly Ala Pro Ala
            740                 745                 750
```

Gln Asp Tyr Arg Lys Tyr Asp Val Leu Leu Leu Val Gly Leu Gly Ile
755 760 765

Gly Ala Thr Pro Phe Ile Ser Ile Leu Lys Asp Leu Leu Lys Asn Ile
770 775 780

Val Thr Met Glu Glu Gln Ala Asp Leu Val Ser Asp Phe Ser Gly Asn
785 790 795 800

Ser Asp Met Ser Ala Ala Thr Ser Glu Gln Pro Ala Leu Asn Lys Ile
805 810 815

Ser Pro Lys Lys Arg Lys Ser Thr Leu Lys Thr Thr Asn Ala Tyr Phe
820 825 830

Tyr Trp Val Thr Arg Glu Gln Gly Ser Phe Asp Trp Phe Lys Gly Val
835 840 845

Met Asn Glu Val Ala Glu Leu Asp Gln Arg Gly Val Ile Glu Met His
850 855 860

Asn Tyr Leu Thr Ser Val Tyr Glu Glu Gly Asp Ala Arg Ser Ala Leu
865 870 875 880

Ile Thr Met Val Gln Ala Leu Asn His Ala Lys Asn Gly Val Asp Ile
885 890 895

Val Ser Gly Thr Ser Val Arg Thr His Phe Ala Arg Pro Asn Trp Arg
900 905 910

Lys Val Phe Ser Lys Thr Leu Thr Lys His Ala Asn Ala Arg Ile Gly
915 920 925

Val Phe Tyr Cys Gly Ala Pro Ile Leu Ala Lys Glu Leu Ser Lys Leu
930 935 940

Cys Lys Glu Phe Asn Gln Lys Gly Thr Thr Lys Phe Glu Phe His Lys
945 950 955 960

Glu His Phe

<210> 9
<211> 3316
<212> DNA
<213> Lycopersicon esculentum

<220>
<221> CDS
<222> (146)..(3112)
<223> coding for NADPH-oxidase

<400> 9

```
cgccactcgt gccgaattcg gcacgaggct ctgaaaaact tttcatacaa agccaatcta 60
tttctctctc tttcttttgg tcaggcttct acagaaaact ctgttttcaa cgtatattta 120
tttattgtca tttgatttgg gacag atg agg ggt tta cct ggg cat gaa cgc   172
                            Met Arg Gly Leu Pro Gly His Glu Arg
                            1               5
cgg tgg acg tcg gat acg gtg tct tcc ggg aag gat tta agt ggt gag   220
Arg Trp Thr Ser Asp Thr Val Ser Ser Gly Lys Asp Leu Ser Gly Glu
10                  15                  20                  25
tca tcg ccg gga act gat tcc ggg aat att tcc ggt ttt gct tcg gag   268
Ser Ser Pro Gly Thr Asp Ser Gly Asn Ile Ser Gly Phe Ala Ser Glu
                30                  35                  40
```

```
gag ttt gtt gaa gtt ata ctt gat ctt cag gat gat gat acg att att      316
Glu Phe Val Glu Val Ile Leu Asp Leu Gln Asp Asp Asp Thr Ile Ile
            45                  50                  55

tta cgg agc gtt gaa ccg gct act gta atc aac att gat ggt tct gat      364
Leu Arg Ser Val Glu Pro Ala Thr Val Ile Asn Ile Asp Gly Ser Asp
        60                  65                  70

cct gct tcc gga gtc ggt att ggt gga gca tcg att gaa act ccg gcg      412
Pro Ala Ser Gly Val Gly Ile Gly Gly Ala Ser Ile Glu Thr Pro Ala
    75                  80                  85

tcg gtg acg tcg acg tcg gaa act cga tcg ccg atg atg cgt cgg agt      460
Ser Val Thr Ser Thr Ser Glu Thr Arg Ser Pro Met Met Arg Arg Ser
90                  95                  100                 105

aca tct aat aag ttt cgt cag ttt tca cag gag ttg aaa gct gag gct      508
Thr Ser Asn Lys Phe Arg Gln Phe Ser Gln Glu Leu Lys Ala Glu Ala
                110                 115                 120

gtt gcg aaa gcg aag cat ttc tcg caa gag ctt aaa gcg gag cta agg      556
Val Ala Lys Ala Lys His Phe Ser Gln Glu Leu Lys Ala Glu Leu Arg
            125                 130                 135

aga ttc tca tgg agc cat gga cat gcg tct cgt gct ttt tcg ccg gcg      604
Arg Phe Ser Trp Ser His Gly His Ala Ser Arg Ala Phe Ser Pro Ala
        140                 145                 150

tcg ttt ttc caa aac gct gtc gtc gga aca ggc aac ggt gta gac tcg      652
Ser Phe Phe Gln Asn Ala Val Val Gly Thr Gly Asn Gly Val Asp Ser
    155                 160                 165

gct tta gcg gct cga gca tta cgt cgg cag cgt gct cag ctc gac cgg      700
Ala Leu Ala Ala Arg Ala Leu Arg Arg Gln Arg Ala Gln Leu Asp Arg
170                 175                 180                 185

act cgt tcc agc gca cac aag gct ctt cgt gga ctc aaa ttc atc agc      748
Thr Arg Ser Ser Ala His Lys Ala Leu Arg Gly Leu Lys Phe Ile Ser
                190                 195                 200

aat aac aaa act aac gga tgg aat gaa gtt gaa aac aat ttc gct aag      796
Asn Asn Lys Thr Asn Gly Trp Asn Glu Val Glu Asn Asn Phe Ala Lys
            205                 210                 215

ctc gct aaa gac ggt tac ctt tat cgt tcc gat ttc gca caa tgc atc      844
Leu Ala Lys Asp Gly Tyr Leu Tyr Arg Ser Asp Phe Ala Gln Cys Ile
        220                 225                 230

ggt cag tac tca cgc cgg cga tca cta cag ttt aat tat aga tta att      892
Gly Gln Tyr Ser Arg Arg Arg Ser Leu Gln Phe Asn Tyr Arg Leu Ile
    235                 240                 245

aca tta att ttg att aat tat ttg gtt aaa ggt atg aag gat tca aag      940
Thr Leu Ile Leu Ile Asn Tyr Leu Val Lys Gly Met Lys Asp Ser Lys
250                 255                 260                 265

gaa ttt gcg ttg gaa ttg ttt gat gct tta agt aga aga aga aga ttg      988
Glu Phe Ala Leu Glu Leu Phe Asp Ala Leu Ser Arg Arg Arg Arg Leu
                270                 275                 280

aag gtt gat aag att agc caa gag gaa ttg tat gag tat tgg tct caa     1036
Lys Val Asp Lys Ile Ser Gln Glu Glu Leu Tyr Glu Tyr Trp Ser Gln
            285                 290                 295

atc acc gat cag agt ttc gat tct cgg ctt cag atc ttc ttc gac atg     1084
Ile Thr Asp Gln Ser Phe Asp Ser Arg Leu Gln Ile Phe Phe Asp Met
        300                 305                 310
```

```
gtg gac aag aat gaa gat ggt cga att ggt gaa gaa gaa gta aaa gag    1132
Val Asp Lys Asn Glu Asp Gly Arg Ile Gly Glu Glu Glu Val Lys Glu
    315                 320                 325

atc atc atg cta agt gcc tct gca aac aaa tta tca aga tta aaa gaa    1180
Ile Ile Met Leu Ser Ala Ser Ala Asn Lys Leu Ser Arg Leu Lys Glu
330                 335                 340                 345

caa gca gag gag tat gca gct ctg atc atg gaa gaa tta gat cct gaa    1228
Gln Ala Glu Glu Tyr Ala Ala Leu Ile Met Glu Glu Leu Asp Pro Glu
                350                 355                 360

aga ctt ggc tac att gag cta tgg cag ctg gaa aca ctt ctc ctc caa    1276
Arg Leu Gly Tyr Ile Glu Leu Trp Gln Leu Glu Thr Leu Leu Leu Gln
            365                 370                 375

aag gac act tac ctc aac tac agt caa gca cta agc tac aca agc caa    1324
Lys Asp Thr Tyr Leu Asn Tyr Ser Gln Ala Leu Ser Tyr Thr Ser Gln
        380                 385                 390

gct ttg agc caa aat ctg caa ggg ttg agg aag aga agc cca ata aga    1372
Ala Leu Ser Gln Asn Leu Gln Gly Leu Arg Lys Arg Ser Pro Ile Arg
    395                 400                 405

aga atg agc aca aaa ctt gtc tat tca ctg caa gag aat tgg aag aga    1420
Arg Met Ser Thr Lys Leu Val Tyr Ser Leu Gln Glu Asn Trp Lys Arg
410                 415                 420                 425

att tgg gtt ctg gtc ttg tgg att ttg ata atg att gga ctt ttt ctt    1468
Ile Trp Val Leu Val Leu Trp Ile Leu Ile Met Ile Gly Leu Phe Leu
                430                 435                 440

tgg aag ttc tat cag tac aaa cag aaa agt gca ttt caa gtc atg ggt    1516
Trp Lys Phe Tyr Gln Tyr Lys Gln Lys Ser Ala Phe Gln Val Met Gly
            445                 450                 455

tat tgc ctt cta aca gct aag ggt gct gct gag act ctc aag ttc aac    1564
Tyr Cys Leu Leu Thr Ala Lys Gly Ala Ala Glu Thr Leu Lys Phe Asn
        460                 465                 470

atg gct tta ata ttg ttg cca gta tgc agg aac acc att aca ttc ctc    1612
Met Ala Leu Ile Leu Leu Pro Val Cys Arg Asn Thr Ile Thr Phe Leu
    475                 480                 485

agg tct act aaa ttg agc tgt ttt gta ccc ttt gat gac aac ata aac    1660
Arg Ser Thr Lys Leu Ser Cys Phe Val Pro Phe Asp Asp Asn Ile Asn
490                 495                 500                 505

ttt cac aag act gtt gct gca gcc att gtc act ggt atc ata ctc cat    1708
Phe His Lys Thr Val Ala Ala Ala Ile Val Thr Gly Ile Ile Leu His
                510                 515                 520

gcc ggt aat cac ctt gta tgt gat ttc cca aag ctt ata cat gca aat    1756
Ala Gly Asn His Leu Val Cys Asp Phe Pro Lys Leu Ile His Ala Asn
            525                 530                 535

agt acg aat tat cag aaa tat ttg gtg aat gat ttt ggc cca agc cag    1804
Ser Thr Asn Tyr Gln Lys Tyr Leu Val Asn Asp Phe Gly Pro Ser Gln
        540                 545                 550

cct cag tac ata gat ctt gtt aaa gga gtg gag ggt gtg act gga ata    1852
Pro Gln Tyr Ile Asp Leu Val Lys Gly Val Glu Gly Val Thr Gly Ile
    555                 560                 565

gtt atg gta atc ctc atg gcc att gct ttc act ctt gca acg cga tgg    1900
Val Met Val Ile Leu Met Ala Ile Ala Phe Thr Leu Ala Thr Arg Trp
570                 575                 580                 585
```

```
ttt agg cgg agc ctc att aag tta ccc aaa cct ttt gat aga ctc act      1948
Phe Arg Arg Ser Leu Ile Lys Leu Pro Lys Pro Phe Asp Arg Leu Thr
                590                 595                 600

ggt ttc aat gcg ttc tgg tac tcg cac cac ctt ctc atc att gtc tac      1996
Gly Phe Asn Ala Phe Trp Tyr Ser His His Leu Leu Ile Ile Val Tyr
            605                 610                 615

atc gta ctg atc atc cat ggc aca ttc ctc tac ctt gtg cat aac tgg      2044
Ile Val Leu Ile Ile His Gly Thr Phe Leu Tyr Leu Val His Asn Trp
        620                 625                 630

tac tcc aaa acg aca tgg atg tat ata gca gtt cct gta ctt ctt tac      2092
Tyr Ser Lys Thr Thr Trp Met Tyr Ile Ala Val Pro Val Leu Leu Tyr
    635                 640                 645

gca ggg gaa aga act ctt aga ttc ttc cga tca ggc tta tac agt gtc      2140
Ala Gly Glu Arg Thr Leu Arg Phe Phe Arg Ser Gly Leu Tyr Ser Val
650                 655                 660                 665

cgg ctt cta aaa gta gca ata tat cct gga aat gtc ctt act ctg caa      2188
Arg Leu Leu Lys Val Ala Ile Tyr Pro Gly Asn Val Leu Thr Leu Gln
                670                 675                 680

atg tct aag cct ccg caa ttt cga tac aag agt gga cag tat atg ttt      2236
Met Ser Lys Pro Pro Gln Phe Arg Tyr Lys Ser Gly Gln Tyr Met Phe
            685                 690                 695

gtc cag tgt cca gct gtt tct cca ttc gag tgg cat cca ttt tcc att      2284
Val Gln Cys Pro Ala Val Ser Pro Phe Glu Trp His Pro Phe Ser Ile
        700                 705                 710

act tca gct cct ggg gat gac tac ttg agc att cat atc cga caa ctt      2332
Thr Ser Ala Pro Gly Asp Asp Tyr Leu Ser Ile His Ile Arg Gln Leu
    715                 720                 725

ggt gac tgg act caa gaa ctc aag cga gtg ttt tcc gag gct tgc gag      2380
Gly Asp Trp Thr Gln Glu Leu Lys Arg Val Phe Ser Glu Ala Cys Glu
730                 735                 740                 745

cag cca gag gct gga aag agt ggc ctg ctc aga gct gac gaa aac acc      2428
Gln Pro Glu Ala Gly Lys Ser Gly Leu Leu Arg Ala Asp Glu Asn Thr
                750                 755                 760

aaa aca agt ttg cca aag cta tta ata gat gga cct tat gga gct cca      2476
Lys Thr Ser Leu Pro Lys Leu Leu Ile Asp Gly Pro Tyr Gly Ala Pro
            765                 770                 775

gca caa gat tac cgg aag tat gat gtc tta ctg ctt gtt ggt ctt ggc      2524
Ala Gln Asp Tyr Arg Lys Tyr Asp Val Leu Leu Leu Val Gly Leu Gly
        780                 785                 790

att gga gca act ccc ttt ata agt atc ctg aaa gac ttg ctc aaa aac      2572
Ile Gly Ala Thr Pro Phe Ile Ser Ile Leu Lys Asp Leu Leu Lys Asn
    795                 800                 805

atc gtc gca atg gag gag caa gca gat tta gtc tcg gat ttc agt gga      2620
Ile Val Ala Met Glu Glu Gln Ala Asp Leu Val Ser Asp Phe Ser Gly
810                 815                 820                 825

aac tcg gac atg agt gct gca aca agt gaa caa cca gct ctc aac aag      2668
Asn Ser Asp Met Ser Ala Ala Thr Ser Glu Gln Pro Ala Leu Asn Lys
                830                 835                 840

att tct cca aaa aag aga aag agt act cta aaa acc aca aat gca tat      2716
Ile Ser Pro Lys Lys Arg Lys Ser Thr Leu Lys Thr Thr Asn Ala Tyr
            845                 850                 855
```

```
ttt tat tgg gtg acc cgg gag caa gga tca ttt gat tgg ttc aaa ggt   2764
Phe Tyr Trp Val Thr Arg Glu Gln Gly Ser Phe Asp Trp Phe Lys Gly
        860                 865                 870

gtt atg aat gaa gtg gct gaa ctt gat caa agg ggt gtc atc gag atg   2812
Val Met Asn Glu Val Ala Glu Leu Asp Gln Arg Gly Val Ile Glu Met
    875                 880                 885

cat aac tac ttg acg agt gtt tat gag gaa ggg gat gca cgt tca gct   2860
His Asn Tyr Leu Thr Ser Val Tyr Glu Glu Gly Asp Ala Arg Ser Ala
890                 895                 900                 905

ctc att acc atg gtc cag gca ctt aac cat gct aag aat ggg gtt gat   2908
Leu Ile Thr Met Val Gln Ala Leu Asn His Ala Lys Asn Gly Val Asp
                910                 915                 920

att gta tca ggc acc agt gtg agg aca cat ttc gcc agg ccg aat tgg   2956
Ile Val Ser Gly Thr Ser Val Arg Thr His Phe Ala Arg Pro Asn Trp
            925                 930                 935

agg aaa gta ttt tcc aag acc tta acc aag cat gca aat gca aga ata   3004
Arg Lys Val Phe Ser Lys Thr Leu Thr Lys His Ala Asn Ala Arg Ile
        940                 945                 950

gga gtt ttc tac tgt ggt gca ccc ata tta gct aaa gaa ctc agc caa   3052
Gly Val Phe Tyr Cys Gly Ala Pro Ile Leu Ala Lys Glu Leu Ser Gln
    955                 960                 965

ctc tgc aaa gag ttt aac caa aag ggc aca aca aag ttc gag ttt cac   3100
Leu Cys Lys Glu Phe Asn Gln Lys Gly Thr Thr Lys Phe Glu Phe His
970                 975                 980                 985

aaa gaa cat ttt tagaagggcc tggagtatga ttaatcttgc atcaacggta       3152
Lys Glu His Phe

cacacatcta tcttcggtac cttatttgat tattctactg aagagataac attagtaagg 3212

aataagtcag agataaattg tacataatag ggaagaagac tatttcaaga gaaaatacat 3272

accaataaga tgtgaaaaaa aaaaaaaaaa aaaaactcgt gccg                  3316
```

<210> 10
<211> 989
<212> PRT
<213> Lycopersicon esculentum

<400> 10

```
Met Arg Gly Leu Pro Gly His Glu Arg Arg Trp Thr Ser Asp Thr Val
 1               5                  10                  15

Ser Ser Gly Lys Asp Leu Ser Gly Glu Ser Ser Pro Gly Thr Asp Ser
            20                  25                  30

Gly Asn Ile Ser Gly Phe Ala Ser Glu Glu Phe Val Glu Val Ile Leu
        35                  40                  45

Asp Leu Gln Asp Asp Asp Thr Ile Ile Leu Arg Ser Val Glu Pro Ala
    50                  55                  60

Thr Val Ile Asn Ile Asp Gly Ser Asp Pro Ala Ser Gly Val Gly Ile
65                  70                  75                  80

Gly Gly Ala Ser Ile Glu Thr Pro Ala Ser Val Thr Ser Thr Ser Glu
                85                  90                  95

Thr Arg Ser Pro Met Met Arg Arg Ser Thr Ser Asn Lys Phe Arg Gln
            100                 105                 110

Phe Ser Gln Glu Leu Lys Ala Glu Ala Val Ala Lys Ala Lys His Phe
        115                 120                 125
```

```
Ser Gln Glu Leu Lys Ala Glu Leu Arg Arg Phe Ser Trp Ser His Gly
    130                 135                 140

His Ala Ser Arg Ala Phe Ser Pro Ala Ser Phe Phe Gln Asn Ala Val
145                 150                 155                 160

Val Gly Thr Gly Asn Gly Val Asp Ser Ala Leu Ala Ala Arg Ala Leu
                165                 170                 175

Arg Arg Gln Arg Ala Gln Leu Asp Arg Thr Arg Ser Ser Ala His Lys
            180                 185                 190

Ala Leu Arg Gly Leu Lys Phe Ile Ser Asn Asn Lys Thr Asn Gly Trp
        195                 200                 205

Asn Glu Val Glu Asn Asn Phe Ala Lys Leu Ala Lys Asp Gly Tyr Leu
    210                 215                 220

Tyr Arg Ser Asp Phe Ala Gln Cys Ile Gly Gln Tyr Ser Arg Arg Arg
225                 230                 235                 240

Ser Leu Gln Phe Asn Tyr Arg Leu Ile Thr Leu Ile Leu Ile Asn Tyr
                245                 250                 255

Leu Val Lys Gly Met Lys Asp Ser Lys Glu Phe Ala Leu Glu Leu Phe
            260                 265                 270

Asp Ala Leu Ser Arg Arg Arg Arg Leu Lys Val Asp Lys Ile Ser Gln
        275                 280                 285

Glu Glu Leu Tyr Glu Tyr Trp Ser Gln Ile Thr Asp Gln Ser Phe Asp
    290                 295                 300

Ser Arg Leu Gln Ile Phe Phe Asp Met Val Asp Lys Asn Glu Asp Gly
305                 310                 315                 320

Arg Ile Gly Glu Glu Glu Val Lys Glu Ile Ile Met Leu Ser Ala Ser
                325                 330                 335

Ala Asn Lys Leu Ser Arg Leu Lys Glu Gln Ala Glu Glu Tyr Ala Ala
            340                 345                 350

Leu Ile Met Glu Glu Leu Asp Pro Glu Arg Leu Gly Tyr Ile Glu Leu
        355                 360                 365

Trp Gln Leu Glu Thr Leu Leu Leu Gln Lys Asp Thr Tyr Leu Asn Tyr
    370                 375                 380

Ser Gln Ala Leu Ser Tyr Thr Ser Gln Ala Leu Ser Gln Asn Leu Gln
385                 390                 395                 400

Gly Leu Arg Lys Arg Ser Pro Ile Arg Arg Met Ser Thr Lys Leu Val
                405                 410                 415

Tyr Ser Leu Gln Glu Asn Trp Lys Arg Ile Trp Val Leu Val Leu Trp
            420                 425                 430

Ile Leu Ile Met Ile Gly Leu Phe Leu Trp Lys Phe Tyr Gln Tyr Lys
        435                 440                 445

Gln Lys Ser Ala Phe Gln Val Met Gly Tyr Cys Leu Leu Thr Ala Lys
    450                 455                 460

Gly Ala Ala Glu Thr Leu Lys Phe Asn Met Ala Leu Ile Leu Leu Pro
465                 470                 475                 480

Val Cys Arg Asn Thr Ile Thr Phe Leu Arg Ser Thr Lys Leu Ser Cys
                485                 490                 495

Phe Val Pro Phe Asp Asp Asn Ile Asn Phe His Lys Thr Val Ala Ala
            500                 505                 510
```

```
Ala Ile Val Thr Gly Ile Ile Leu His Ala Gly Asn His Leu Val Cys
        515                 520                 525

Asp Phe Pro Lys Leu Ile His Ala Asn Ser Thr Asn Tyr Gln Lys Tyr
    530                 535                 540

Leu Val Asn Asp Phe Gly Pro Ser Gln Pro Gln Tyr Ile Asp Leu Val
545                 550                 555                 560

Lys Gly Val Glu Gly Val Thr Gly Ile Val Met Val Ile Leu Met Ala
                565                 570                 575

Ile Ala Phe Thr Leu Ala Thr Arg Trp Phe Arg Arg Ser Leu Ile Lys
            580                 585                 590

Leu Pro Lys Pro Phe Asp Arg Leu Thr Gly Phe Asn Ala Phe Trp Tyr
        595                 600                 605

Ser His His Leu Leu Ile Ile Val Tyr Ile Val Leu Ile Ile His Gly
    610                 615                 620

Thr Phe Leu Tyr Leu Val His Asn Trp Tyr Ser Lys Thr Thr Trp Met
625                 630                 635                 640

Tyr Ile Ala Val Pro Val Leu Leu Tyr Ala Gly Glu Arg Thr Leu Arg
                645                 650                 655

Phe Phe Arg Ser Gly Leu Tyr Ser Val Arg Leu Leu Lys Val Ala Ile
            660                 665                 670

Tyr Pro Gly Asn Val Leu Thr Leu Gln Met Ser Lys Pro Pro Gln Phe
        675                 680                 685

Arg Tyr Lys Ser Gly Gln Tyr Met Phe Val Gln Cys Pro Ala Val Ser
    690                 695                 700

Pro Phe Glu Trp His Pro Phe Ser Ile Thr Ser Ala Pro Gly Asp Asp
705                 710                 715                 720

Tyr Leu Ser Ile His Ile Arg Gln Leu Gly Asp Trp Thr Gln Glu Leu
                725                 730                 735

Lys Arg Val Phe Ser Glu Ala Cys Glu Gln Pro Glu Ala Gly Lys Ser
            740                 745                 750

Gly Leu Leu Arg Ala Asp Glu Asn Thr Lys Thr Ser Leu Pro Lys Leu
        755                 760                 765

Leu Ile Asp Gly Pro Tyr Gly Ala Pro Ala Gln Asp Tyr Arg Lys Tyr
    770                 775                 780

Asp Val Leu Leu Leu Val Gly Leu Gly Ile Gly Ala Thr Pro Phe Ile
785                 790                 795                 800

Ser Ile Leu Lys Asp Leu Leu Lys Asn Ile Val Ala Met Glu Glu Gln
                805                 810                 815

Ala Asp Leu Val Ser Asp Phe Ser Gly Asn Ser Asp Met Ser Ala Ala
            820                 825                 830

Thr Ser Glu Gln Pro Ala Leu Asn Lys Ile Ser Pro Lys Lys Arg Lys
        835                 840                 845

Ser Thr Leu Lys Thr Thr Asn Ala Tyr Phe Tyr Trp Val Thr Arg Glu
    850                 855                 860

Gln Gly Ser Phe Asp Trp Phe Lys Gly Val Met Asn Glu Val Ala Glu
865                 870                 875                 880

Leu Asp Gln Arg Gly Val Ile Glu Met His Asn Tyr Leu Thr Ser Val
                885                 890                 895
```

```
Tyr Glu Glu Gly Asp Ala Arg Ser Ala Leu Ile Thr Met Val Gln Ala
            900                 905                 910
Leu Asn His Ala Lys Asn Gly Val Asp Ile Val Ser Gly Thr Ser Val
        915                 920                 925
Arg Thr His Phe Ala Arg Pro Asn Trp Arg Lys Val Phe Ser Lys Thr
    930                 935                 940
Leu Thr Lys His Ala Asn Ala Arg Ile Gly Val Phe Tyr Cys Gly Ala
945                 950                 955                 960
Pro Ile Leu Ala Lys Glu Leu Ser Gln Leu Cys Lys Glu Phe Asn Gln
                965                 970                 975
Lys Gly Thr Thr Lys Phe Glu Phe His Lys Glu His Phe
            980                 985
```

<210> 11
<211> 3080
<212> DNA
<213> Arabidopsis thaliana

<220>
<221> CDS
<222> (15)..(2846)
<223> coding for NADPH-oxidase

<400> 11

```
ccgactttgg atct atg aaa ccg ttc tca aag aac gat cgg cga cgg tgg    50
                Met Lys Pro Phe Ser Lys Asn Asp Arg Arg Arg Trp
                1               5                   10

tca ttt gat tca gtt tcc gcc gga aaa acc gcc gtc gga agt gca tca    98
Ser Phe Asp Ser Val Ser Ala Gly Lys Thr Ala Val Gly Ser Ala Ser
        15                  20                  25

act tca ccg gga act gaa tac tcc att aac ggt gat caa gag ttc gtt    146
Thr Ser Pro Gly Thr Glu Tyr Ser Ile Asn Gly Asp Gln Glu Phe Val
    30                  35                  40

gaa gtc aca atc gat ctt caa gac gat gac aca atc gtt ctt cgt agc    194
Glu Val Thr Ile Asp Leu Gln Asp Asp Asp Thr Ile Val Leu Arg Ser
45                  50                  55                  60

gtc gag cca gca acc gcc att aat gtc atc gga gat atc tcc gac gac    242
Val Glu Pro Ala Thr Ala Ile Asn Val Ile Gly Asp Ile Ser Asp Asp
                65                  70                  75

aac acc gga ata atg act ccg gtt tcg att tcg aga tct ccg acg atg    290
Asn Thr Gly Ile Met Thr Pro Val Ser Ile Ser Arg Ser Pro Thr Met
            80                  85                  90

aaa cga act tca tct aat cgg ttc cga caa ttc tca caa gag ctt aaa    338
Lys Arg Thr Ser Ser Asn Arg Phe Arg Gln Phe Ser Gln Glu Leu Lys
        95                  100                 105

gcc gaa gct gtg gcg aaa gcg aaa cag tta tct cag gag ttg aaa cga    386
Ala Glu Ala Val Ala Lys Ala Lys Gln Leu Ser Gln Glu Leu Lys Arg
    110                 115                 120

ttc tca tgg tct cgt tct ttc tca ggt aac tta acc act act agt acc    434
Phe Ser Trp Ser Arg Ser Phe Ser Gly Asn Leu Thr Thr Thr Ser Thr
125                 130                 135                 140

gcc gct aat caa agc ggc ggt gct ggt ggt ggt ttg gtg aac tcg gct    482
Ala Ala Asn Gln Ser Gly Gly Ala Gly Gly Gly Leu Val Asn Ser Ala
                145                 150                 155
```

```
tta gaa gcg cga gcg ttg cga aag caa cgt gct cag tta gat cgg act    530
Leu Glu Ala Arg Ala Leu Arg Lys Gln Arg Ala Gln Leu Asp Arg Thr
            160                 165                 170

cgg tct agt gct caa aga gct ctt cgt ggt ttg aga ttc att agc aat    578
Arg Ser Ser Ala Gln Arg Ala Leu Arg Gly Leu Arg Phe Ile Ser Asn
        175                 180                 185

aag caa aag aac gtt gat ggt tgg aac gat gtt caa tca aat ttc gaa    626
Lys Gln Lys Asn Val Asp Gly Trp Asn Asp Val Gln Ser Asn Phe Glu
    190                 195                 200

aaa ttc gaa aaa aat ggt tac atc tat cgc tcc gat ttc gct caa tgc    674
Lys Phe Glu Lys Asn Gly Tyr Ile Tyr Arg Ser Asp Phe Ala Gln Cys
205                 210                 215                 220

ata gga atg aaa gat tcg aaa gaa ttt gca ttg gaa ctg ttc gat gca    722
Ile Gly Met Lys Asp Ser Lys Glu Phe Ala Leu Glu Leu Phe Asp Ala
                225                 230                 235

ttg agt aga aga aga aga tta aaa gta gag aaa atc aat cac gat gag    770
Leu Ser Arg Arg Arg Arg Leu Lys Val Glu Lys Ile Asn His Asp Glu
            240                 245                 250

ctt tat gag tat tgg tca caa atc aac gac gag agt ttt gat tct cgt    818
Leu Tyr Glu Tyr Trp Ser Gln Ile Asn Asp Glu Ser Phe Asp Ser Arg
        255                 260                 265

ctc cag atc ttc ttc gac ata gtg gac aag aat gaa gat ggg aga att    866
Leu Gln Ile Phe Phe Asp Ile Val Asp Lys Asn Glu Asp Gly Arg Ile
    270                 275                 280

aca gaa gag gaa gta aaa gag ata ata atg ttg agt gca tct gca aat    914
Thr Glu Glu Glu Val Lys Glu Ile Ile Met Leu Ser Ala Ser Ala Asn
285                 290                 295                 300

aag cta tca aga tta aag gaa caa gca gag gaa tat gca gct ttg att    962
Lys Leu Ser Arg Leu Lys Glu Gln Ala Glu Glu Tyr Ala Ala Leu Ile
                305                 310                 315

atg gaa gag tta gat cct gaa aga ctt ggc tac ata gag cta tgg caa    1010
Met Glu Glu Leu Asp Pro Glu Arg Leu Gly Tyr Ile Glu Leu Trp Gln
            320                 325                 330

cta gag act ttg ctt cta caa aaa gac aca tac ctc aat tac agt caa    1058
Leu Glu Thr Leu Leu Leu Gln Lys Asp Thr Tyr Leu Asn Tyr Ser Gln
        335                 340                 345

gca ttg agc tat acg agc caa gca ttg agc caa aac ctt caa ggg tta    1106
Ala Leu Ser Tyr Thr Ser Gln Ala Leu Ser Gln Asn Leu Gln Gly Leu
    350                 355                 360

agg gga aag agt cga ata cat aga atg agt tcg gat ttc gtc tac att    1154
Arg Gly Lys Ser Arg Ile His Arg Met Ser Ser Asp Phe Val Tyr Ile
365                 370                 375                 380

atg caa gag aat tgg aaa agg ata tgg gtt tta tcc tta tgg atc atg    1202
Met Gln Glu Asn Trp Lys Arg Ile Trp Val Leu Ser Leu Trp Ile Met
                385                 390                 395

atc atg atc gga tta ttc ttg tgg aaa ttc ttc caa tac aag caa aaa    1250
Ile Met Ile Gly Leu Phe Leu Trp Lys Phe Phe Gln Tyr Lys Gln Lys
            400                 405                 410

gat gca ttt cat gtg atg gga tat tgt tta ctc aca gcc aaa gga gca    1298
Asp Ala Phe His Val Met Gly Tyr Cys Leu Leu Thr Ala Lys Gly Ala
        415                 420                 425
```

```
gct gaa aca ctt aaa ttc aac atg gct cta ata ctt ttc cca gtt tgc    1346
Ala Glu Thr Leu Lys Phe Asn Met Ala Leu Ile Leu Phe Pro Val Cys
    430                 435                 440

aga aac acc att act tgg ctt aga tcc aca aga ctc tct tac ttc gtt    1394
Arg Asn Thr Ile Thr Trp Leu Arg Ser Thr Arg Leu Ser Tyr Phe Val
445                 450                 455                 460

cct ttt gat gat aat atc aac ttc cac aag aca att gct gga gcc att    1442
Pro Phe Asp Asp Asn Ile Asn Phe His Lys Thr Ile Ala Gly Ala Ile
                465                 470                 475

gta gta gct gtg atc ctt cat att gga gac cat ctt gct tgt gat ttc    1490
Val Val Ala Val Ile Leu His Ile Gly Asp His Leu Ala Cys Asp Phe
            480                 485                 490

cct aga att gtt aga gcc acc gaa tac gat tac aat cgg tat ctg ttt    1538
Pro Arg Ile Val Arg Ala Thr Glu Tyr Asp Tyr Asn Arg Tyr Leu Phe
        495                 500                 505

cat tac ttt caa aca aaa cag cca aca tac ttc gac ctc gtt aag gga    1586
His Tyr Phe Gln Thr Lys Gln Pro Thr Tyr Phe Asp Leu Val Lys Gly
    510                 515                 520

cct gaa gga atc act ggg att tta atg gtc att ttg atg att att tca    1634
Pro Glu Gly Ile Thr Gly Ile Leu Met Val Ile Leu Met Ile Ile Ser
525                 530                 535                 540

ttc aca tta gca aca aga tgg ttt agg cgt aac cta gtc aag ctt cct    1682
Phe Thr Leu Ala Thr Arg Trp Phe Arg Arg Asn Leu Val Lys Leu Pro
                545                 550                 555

aag cca ttt gat cga cta acc ggt ttt aac gcc ttt tgg tat tcg cat    1730
Lys Pro Phe Asp Arg Leu Thr Gly Phe Asn Ala Phe Trp Tyr Ser His
            560                 565                 570

cat ttg ttc gtc att gtt tat atc ttg ctt att ctt cat ggt atc ttc    1778
His Leu Phe Val Ile Val Tyr Ile Leu Leu Ile Leu His Gly Ile Phe
        575                 580                 585

ctc tat ttc gcc aag cct tgg tat gtt cgt acg aca tgg atg tat ctt    1826
Leu Tyr Phe Ala Lys Pro Trp Tyr Val Arg Thr Thr Trp Met Tyr Leu
    590                 595                 600

gca gta cca gtt tta ctc tat ggt gga gaa aga aca ctt agg tac ttc    1874
Ala Val Pro Val Leu Leu Tyr Gly Gly Glu Arg Thr Leu Arg Tyr Phe
605                 610                 615                 620

cgt tct ggt tct tat tcg gtt cga ctg ctt aag gtt gct ata tat cct    1922
Arg Ser Gly Ser Tyr Ser Val Arg Leu Leu Lys Val Ala Ile Tyr Pro
                625                 630                 635

ggt aat gtt cta acg cta caa atg tcg aaa cca act caa ttt cgt tac    1970
Gly Asn Val Leu Thr Leu Gln Met Ser Lys Pro Thr Gln Phe Arg Tyr
            640                 645                 650

aaa agc gga caa tac atg ttt gtc caa tgt cct gcg gtt tcg cca ttc    2018
Lys Ser Gly Gln Tyr Met Phe Val Gln Cys Pro Ala Val Ser Pro Phe
        655                 660                 665

gag tgg cat cca ttc tca att act tcc gca cct gaa gat gat tat atc    2066
Glu Trp His Pro Phe Ser Ile Thr Ser Ala Pro Glu Asp Asp Tyr Ile
    670                 675                 680

agc att cac att aga caa ctt ggt gat tgg act caa gaa ctc aaa aga    2114
Ser Ile His Ile Arg Gln Leu Gly Asp Trp Thr Gln Glu Leu Lys Arg
685                 690                 695                 700
```

```
gta ttc tct gaa gtt tgt gag cca ccg gtt ggc ggt aaa agc gga ctt     2162
Val Phe Ser Glu Val Cys Glu Pro Pro Val Gly Gly Lys Ser Gly Leu
                705                 710                 715

ctc aga gcc gac gaa aca aca aag aaa agt ttg cca aag cta ttg ata     2210
Leu Arg Ala Asp Glu Thr Thr Lys Lys Ser Leu Pro Lys Leu Leu Ile
            720                 725                 730

gat gga ccg tac ggt gca cca gca caa gat tat agg aaa tat gat gtt     2258
Asp Gly Pro Tyr Gly Ala Pro Ala Gln Asp Tyr Arg Lys Tyr Asp Val
        735                 740                 745

ctc tta tta gtt ggt ctt ggc att ggt gca act cca ttt atc agt atc     2306
Leu Leu Leu Val Gly Leu Gly Ile Gly Ala Thr Pro Phe Ile Ser Ile
    750                 755                 760

ttg aaa gat ttg ctt aac aac att gtt aaa atg gaa gag cat gcg gat     2354
Leu Lys Asp Leu Leu Asn Asn Ile Val Lys Met Glu Glu His Ala Asp
765                 770                 775                 780

tcg atc tcg gat ttc agt aga tca tca gaa tac agc aca gga agc aac     2402
Ser Ile Ser Asp Phe Ser Arg Ser Ser Glu Tyr Ser Thr Gly Ser Asn
                785                 790                 795

ggt gac acg cca aga cga aag aga ata cta aaa acc aca aat gct tat     2450
Gly Asp Thr Pro Arg Arg Lys Arg Ile Leu Lys Thr Thr Asn Ala Tyr
            800                 805                 810

ttc tac tgg gtc aca aga gaa caa ggc tct ttt gat tgg ttc aaa ggt     2498
Phe Tyr Trp Val Thr Arg Glu Gln Gly Ser Phe Asp Trp Phe Lys Gly
        815                 820                 825

gtc atg aac gaa gtt gca gaa ctt gac caa cgg ggt gtg ata gag atg     2546
Val Met Asn Glu Val Ala Glu Leu Asp Gln Arg Gly Val Ile Glu Met
    830                 835                 840

cat aac tat tta aca agt gtg tat gaa gaa ggt gat gct cgt tct gct     2594
His Asn Tyr Leu Thr Ser Val Tyr Glu Glu Gly Asp Ala Arg Ser Ala
845                 850                 855                 860

ctc att aca atg gtt caa gct ctt aat cat gcc aaa aat ggt gtc gac     2642
Leu Ile Thr Met Val Gln Ala Leu Asn His Ala Lys Asn Gly Val Asp
                865                 870                 875

att gtc tct ggc act agg gtc aga aca cac ttt gca aga cct aat tgg     2690
Ile Val Ser Gly Thr Arg Val Arg Thr His Phe Ala Arg Pro Asn Trp
            880                 885                 890

aag aag gtt ctc aca aag cta agt tcc aag cat tgc aat gca aga aca     2738
Lys Lys Val Leu Thr Lys Leu Ser Ser Lys His Cys Asn Ala Arg Thr
        895                 900                 905

gga gtg ttt tat tgc gga gta ccg gtt tta ggg aag gag ctt agc aaa     2786
Gly Val Phe Tyr Cys Gly Val Pro Val Leu Gly Lys Glu Leu Ser Lys
    910                 915                 920

cta tgc aac aca ttc aat caa aaa ggt tca acc aag ttt gaa ttt cac     2834
Leu Cys Asn Thr Phe Asn Gln Lys Gly Ser Thr Lys Phe Glu Phe His
925                 930                 935                 940

aag gag cat ttc taaaagacaa gaaggaagaa gccaaaagcc ctctagattc         2886
Lys Glu His Phe

tttaatatct caaatttagc cacttatagt ataaaggcaa tctcttcact atttaattca 2946

aagtgattaa acgttaacac actgtcaaaa gtgagtgtgt taacgtttag ctccacacgt 3006

tctaggtttt tatacaccga ggcatacgtg taaatatacg agacagaaga aattcaaggg 3066
```

ggtttgatag aagc 3080

<210> 12
<211> 944
<212> PRT
<213> Arabidopsis thaliana

<400> 12

```
Met Lys Pro Phe Ser Lys Asn Asp Arg Arg Arg Trp Ser Phe Asp Ser
1               5                   10                  15
Val Ser Ala Gly Lys Thr Ala Val Gly Ser Ala Ser Thr Ser Pro Gly
            20                  25                  30
Thr Glu Tyr Ser Ile Asn Gly Asp Gln Glu Phe Val Glu Val Thr Ile
        35                  40                  45
Asp Leu Gln Asp Asp Asp Thr Ile Val Leu Arg Ser Val Glu Pro Ala
    50                  55                  60
Thr Ala Ile Asn Val Ile Gly Asp Ile Ser Asp Asp Asn Thr Gly Ile
65                  70                  75                  80
Met Thr Pro Val Ser Ile Ser Arg Ser Pro Thr Met Lys Arg Thr Ser
                85                  90                  95
Ser Asn Arg Phe Arg Gln Phe Ser Gln Glu Leu Lys Ala Glu Ala Val
            100                 105                 110
Ala Lys Ala Lys Gln Leu Ser Gln Glu Leu Lys Arg Phe Ser Trp Ser
        115                 120                 125
Arg Ser Phe Ser Gly Asn Leu Thr Thr Thr Ser Thr Ala Ala Asn Gln
    130                 135                 140
Ser Gly Gly Ala Gly Gly Gly Leu Val Asn Ser Ala Leu Glu Ala Arg
145                 150                 155                 160
Ala Leu Arg Lys Gln Arg Ala Gln Leu Asp Arg Thr Arg Ser Ser Ala
                165                 170                 175
Gln Arg Ala Leu Arg Gly Leu Arg Phe Ile Ser Asn Lys Gln Lys Asn
            180                 185                 190
Val Asp Gly Trp Asn Asp Val Gln Ser Asn Phe Glu Lys Phe Glu Lys
        195                 200                 205
Asn Gly Tyr Ile Tyr Arg Ser Asp Phe Ala Gln Cys Ile Gly Met Lys
    210                 215                 220
Asp Ser Lys Glu Phe Ala Leu Glu Leu Phe Asp Ala Leu Ser Arg Arg
225                 230                 235                 240
Arg Arg Leu Lys Val Glu Lys Ile Asn His Asp Glu Leu Tyr Glu Tyr
                245                 250                 255
Trp Ser Gln Ile Asn Asp Glu Ser Phe Asp Ser Arg Leu Gln Ile Phe
            260                 265                 270
Phe Asp Ile Val Asp Lys Asn Glu Asp Gly Arg Ile Thr Glu Glu Glu
        275                 280                 285
Val Lys Glu Ile Ile Met Leu Ser Ala Ser Ala Asn Lys Leu Ser Arg
    290                 295                 300
Leu Lys Glu Gln Ala Glu Glu Tyr Ala Ala Leu Ile Met Glu Glu Leu
305                 310                 315                 320
Asp Pro Glu Arg Leu Gly Tyr Ile Glu Leu Trp Gln Leu Glu Thr Leu
                325                 330                 335
```

```
Leu Leu Gln Lys Asp Thr Tyr Leu Asn Tyr Ser Gln Ala Leu Ser Tyr
            340                 345                 350
Thr Ser Gln Ala Leu Ser Gln Asn Leu Gln Gly Leu Arg Gly Lys Ser
        355                 360                 365
Arg Ile His Arg Met Ser Ser Asp Phe Val Tyr Ile Met Gln Glu Asn
    370                 375                 380
Trp Lys Arg Ile Trp Val Leu Ser Leu Trp Ile Met Ile Met Ile Gly
385                 390                 395                 400
Leu Phe Leu Trp Lys Phe Phe Gln Tyr Lys Gln Lys Asp Ala Phe His
                405                 410                 415
Val Met Gly Tyr Cys Leu Leu Thr Ala Lys Gly Ala Ala Glu Thr Leu
            420                 425                 430
Lys Phe Asn Met Ala Leu Ile Leu Phe Pro Val Cys Arg Asn Thr Ile
        435                 440                 445
Thr Trp Leu Arg Ser Thr Arg Leu Ser Tyr Phe Val Pro Phe Asp Asp
    450                 455                 460
Asn Ile Asn Phe His Lys Thr Ile Ala Gly Ala Ile Val Val Ala Val
465                 470                 475                 480
Ile Leu His Ile Gly Asp His Leu Ala Cys Asp Phe Pro Arg Ile Val
                485                 490                 495
Arg Ala Thr Glu Tyr Asp Tyr Asn Arg Tyr Leu Phe His Tyr Phe Gln
            500                 505                 510
Thr Lys Gln Pro Thr Tyr Phe Asp Leu Val Lys Gly Pro Glu Gly Ile
        515                 520                 525
Thr Gly Ile Leu Met Val Ile Leu Met Ile Ile Ser Phe Thr Leu Ala
    530                 535                 540
Thr Arg Trp Phe Arg Arg Asn Leu Val Lys Leu Pro Lys Pro Phe Asp
545                 550                 555                 560
Arg Leu Thr Gly Phe Asn Ala Phe Trp Tyr Ser His His Leu Phe Val
                565                 570                 575
Ile Val Tyr Ile Leu Leu Ile Leu His Gly Ile Phe Leu Tyr Phe Ala
            580                 585                 590
Lys Pro Trp Tyr Val Arg Thr Thr Trp Met Tyr Leu Ala Val Pro Val
        595                 600                 605
Leu Leu Tyr Gly Gly Glu Arg Thr Leu Arg Tyr Phe Arg Ser Gly Ser
    610                 615                 620
Tyr Ser Val Arg Leu Leu Lys Val Ala Ile Tyr Pro Gly Asn Val Leu
625                 630                 635                 640
Thr Leu Gln Met Ser Lys Pro Thr Gln Phe Arg Tyr Lys Ser Gly Gln
                645                 650                 655
Tyr Met Phe Val Gln Cys Pro Ala Val Ser Pro Phe Glu Trp His Pro
            660                 665                 670
Phe Ser Ile Thr Ser Ala Pro Glu Asp Asp Tyr Ile Ser Ile His Ile
        675                 680                 685
Arg Gln Leu Gly Asp Trp Thr Gln Glu Leu Lys Arg Val Phe Ser Glu
    690                 695                 700
Val Cys Glu Pro Pro Val Gly Gly Lys Ser Gly Leu Leu Arg Ala Asp
705                 710                 715                 720
```

```
Glu Thr Thr Lys Lys Ser Leu Pro Lys Leu Leu Ile Asp Gly Pro Tyr
                725                 730                 735

Gly Ala Pro Ala Gln Asp Tyr Arg Lys Tyr Asp Val Leu Leu Leu Val
            740                 745                 750

Gly Leu Gly Ile Gly Ala Thr Pro Phe Ile Ser Ile Leu Lys Asp Leu
        755                 760                 765

Leu Asn Asn Ile Val Lys Met Glu Glu His Ala Asp Ser Ile Ser Asp
    770                 775                 780

Phe Ser Arg Ser Ser Glu Tyr Ser Thr Gly Ser Asn Gly Asp Thr Pro
785                 790                 795                 800

Arg Arg Lys Arg Ile Leu Lys Thr Thr Asn Ala Tyr Phe Tyr Trp Val
                805                 810                 815

Thr Arg Glu Gln Gly Ser Phe Asp Trp Phe Lys Gly Val Met Asn Glu
            820                 825                 830

Val Ala Glu Leu Asp Gln Arg Gly Val Ile Glu Met His Asn Tyr Leu
        835                 840                 845

Thr Ser Val Tyr Glu Glu Gly Asp Ala Arg Ser Ala Leu Ile Thr Met
    850                 855                 860

Val Gln Ala Leu Asn His Ala Lys Asn Gly Val Asp Ile Val Ser Gly
865                 870                 875                 880

Thr Arg Val Arg Thr His Phe Ala Arg Pro Asn Trp Lys Lys Val Leu
                885                 890                 895

Thr Lys Leu Ser Ser Lys His Cys Asn Ala Arg Thr Gly Val Phe Tyr
            900                 905                 910

Cys Gly Val Pro Val Leu Gly Lys Glu Leu Ser Lys Leu Cys Asn Thr
        915                 920                 925

Phe Asn Gln Lys Gly Ser Thr Lys Phe Glu Phe His Lys Glu His Phe
    930                 935                 940
```

<210> 13
<211> 3035
<212> DNA
<213> Arabidopsis thaliana

<220>
<221> CDS
<222> (132)..(2894)
<223> coding for NADPH-oxidase

<400> 13

```
tcaaacacct tttgagagcg gttatttttt ctctatcaac taatacagta accttacggg 60
tgtttatttg tatagatctc tgtggttttc ttggccaact ctagtgagat ctttttcgtt 120
tctcgaattc g atg aaa atg aga cga ggc aat tca agt aac gac cat gaa  170
             Met Lys Met Arg Arg Gly Asn Ser Ser Asn Asp His Glu
              1               5                   10

ctt ggg att cta cga gga gct aac tcg gac acc aac tcg gac acg gag   218
Leu Gly Ile Leu Arg Gly Ala Asn Ser Asp Thr Asn Ser Asp Thr Glu
     15                  20                  25

agc atc gct agc gac cgt ggt gcc ttt agc ggt ccg ctt ggc cgg cct   266
Ser Ile Ala Ser Asp Arg Gly Ala Phe Ser Gly Pro Leu Gly Arg Pro
 30                  35                  40                  45
```

```
aaa cgt gcg tcc aag aaa aac gca aga ttc gcc gac gat ctt ccc aag    314
Lys Arg Ala Ser Lys Lys Asn Ala Arg Phe Ala Asp Asp Leu Pro Lys
                 50                  55                  60

aga agc aat agt gtt gct ggc ggc cgt ggt gat gac gat gag tac gtg    362
Arg Ser Asn Ser Val Ala Gly Gly Arg Gly Asp Asp Asp Glu Tyr Val
             65                  70                  75

gag atc acg cta gac atc agg gac gac tcg gtg gcc gtc cat agt gtc    410
Glu Ile Thr Leu Asp Ile Arg Asp Asp Ser Val Ala Val His Ser Val
         80                  85                  90

caa caa gca gct gga ggt gga ggc cac ctg gag gac ccg gag cta gcc    458
Gln Gln Ala Ala Gly Gly Gly Gly His Leu Glu Asp Pro Glu Leu Ala
     95                 100                 105

ctt ctt acg aag aag act ctc gag agc agc ctc aac aac acc acc tcc    506
Leu Leu Thr Lys Lys Thr Leu Glu Ser Ser Leu Asn Asn Thr Thr Ser
110                 115                 120                 125

tta tct ttc ttc cga agc acc tcc tca cgc atc aag aac gcc tcc cgc    554
Leu Ser Phe Phe Arg Ser Thr Ser Ser Arg Ile Lys Asn Ala Ser Arg
                130                 135                 140

gag ctc cgc cgc gtg ttc tct aga cgt ccc tcc ccg gcc gtg cgg cgg    602
Glu Leu Arg Arg Val Phe Ser Arg Arg Pro Ser Pro Ala Val Arg Arg
            145                 150                 155

ttt gac cgc acg agc tcc gcg gcc atc cac gca ctc aaa ggt ctc aag    650
Phe Asp Arg Thr Ser Ser Ala Ala Ile His Ala Leu Lys Gly Leu Lys
        160                 165                 170

ttc att gcc acc aag acg gcc gca tgg ccg gcc gtc gac caa cgt ttc    698
Phe Ile Ala Thr Lys Thr Ala Ala Trp Pro Ala Val Asp Gln Arg Phe
    175                 180                 185

gat aaa ctc tcc gct gat tcc aac ggc ctc tta ctc tct gcc aag ttt    746
Asp Lys Leu Ser Ala Asp Ser Asn Gly Leu Leu Leu Ser Ala Lys Phe
190                 195                 200                 205

tgg gaa tgc tta gga atg aat aag gaa tct aaa gac ttc gct gac cag    794
Trp Glu Cys Leu Gly Met Asn Lys Glu Ser Lys Asp Phe Ala Asp Gln
                210                 215                 220

ctc ttt aga gca tta gct cgc cgg aat aac gtc tcc ggc gat gca atc    842
Leu Phe Arg Ala Leu Ala Arg Arg Asn Asn Val Ser Gly Asp Ala Ile
            225                 230                 235

aca aag gaa cag ctt agg ata ttc tgg gaa cag atc tca gac gaa agc    890
Thr Lys Glu Gln Leu Arg Ile Phe Trp Glu Gln Ile Ser Asp Glu Ser
        240                 245                 250

ttt gat gcc aaa ctc caa gtc ttt ttt gac atg gtg gac aaa gat gaa    938
Phe Asp Ala Lys Leu Gln Val Phe Phe Asp Met Val Asp Lys Asp Glu
    255                 260                 265

gat ggg cga gta aca gaa gaa gag gtg gct gag att att agt ctt agt    986
Asp Gly Arg Val Thr Glu Glu Glu Val Ala Glu Ile Ile Ser Leu Ser
270                 275                 280                 285

gct tct gca aac aag ctc tca aat att caa aag caa gcc aaa gaa tat    1034
Ala Ser Ala Asn Lys Leu Ser Asn Ile Gln Lys Gln Ala Lys Glu Tyr
                290                 295                 300

gcg gca ctg ata atg gaa gag ttg gac cca gac aat gct ggg ttt att    1082
Ala Ala Leu Ile Met Glu Glu Leu Asp Pro Asp Asn Ala Gly Phe Ile
            305                 310                 315
```

```
atg atc gaa aac ttg gaa atg ttg cta tta caa gca ccg aac cag tcg      1130
Met Ile Glu Asn Leu Glu Met Leu Leu Leu Gln Ala Pro Asn Gln Ser
        320                 325                 330

gtg cgg atg gga gac agc agg ata ctt agt cag atg tta agt cag aag      1178
Val Arg Met Gly Asp Ser Arg Ile Leu Ser Gln Met Leu Ser Gln Lys
    335                 340                 345

ctt aga ccg gca aaa gag agc aac cct tta ttg aga tgg tcg gag aaa      1226
Leu Arg Pro Ala Lys Glu Ser Asn Pro Leu Leu Arg Trp Ser Glu Lys
350                 355                 360                 365

atc aaa tat ttc ata ctt gat aat tgg cag aga tta tgg atc atg atg      1274
Ile Lys Tyr Phe Ile Leu Asp Asn Trp Gln Arg Leu Trp Ile Met Met
                370                 375                 380

tta tgg ctt ggc atc tgt ggt ggc ctc ttt act tat aaa ttc att cag      1322
Leu Trp Leu Gly Ile Cys Gly Gly Leu Phe Thr Tyr Lys Phe Ile Gln
            385                 390                 395

tac aag aac aaa gct gcc tat ggt gtg atg ggt tat tgt gtt tgt gtc      1370
Tyr Lys Asn Lys Ala Ala Tyr Gly Val Met Gly Tyr Cys Val Cys Val
        400                 405                 410

gcc aaa gga ggc gcc gag act ctc aaa ttc aac atg gct ctc ata ttg      1418
Ala Lys Gly Gly Ala Glu Thr Leu Lys Phe Asn Met Ala Leu Ile Leu
    415                 420                 425

ttg cct gtt tgt cga aac acc atc act tgg ctt agg aac aag acc aag      1466
Leu Pro Val Cys Arg Asn Thr Ile Thr Trp Leu Arg Asn Lys Thr Lys
430                 435                 440                 445

ctt ggt act gtc gtt cct ttt gat gat agt ctt aac ttc cac aag gtt      1514
Leu Gly Thr Val Val Pro Phe Asp Asp Ser Leu Asn Phe His Lys Val
                450                 455                 460

att gca agc ggg ata gtc gtc ggt gtt ttg ctc cat gcg ggt gcc cat      1562
Ile Ala Ser Gly Ile Val Val Gly Val Leu Leu His Ala Gly Ala His
            465                 470                 475

tta acg tgt gat ttt cca cgt tta att gcc gcg gat gag gac acc tat      1610
Leu Thr Cys Asp Phe Pro Arg Leu Ile Ala Ala Asp Glu Asp Thr Tyr
        480                 485                 490

gag ccg atg gaa aaa tac ttt ggg gat caa ccg act agc tac tgg tgg      1658
Glu Pro Met Glu Lys Tyr Phe Gly Asp Gln Pro Thr Ser Tyr Trp Trp
    495                 500                 505

ttt gtg aaa gga gtg gaa gga tgg act ggc att gtg atg gtt gtg cta      1706
Phe Val Lys Gly Val Glu Gly Trp Thr Gly Ile Val Met Val Val Leu
510                 515                 520                 525

atg gct ata gcc ttt aca ctc gct acg cct tgg ttc cga cgt aac aag      1754
Met Ala Ile Ala Phe Thr Leu Ala Thr Pro Trp Phe Arg Arg Asn Lys
                530                 535                 540

ctt aac tta cct aac ttc ctc aag aag ctt acc ggt ttc aac gcc ttt      1802
Leu Asn Leu Pro Asn Phe Leu Lys Lys Leu Thr Gly Phe Asn Ala Phe
            545                 550                 555

tgg tac acc cac cat ttg ttc atc att gtt tat gct ctt ctc att gtc      1850
Trp Tyr Thr His His Leu Phe Ile Ile Val Tyr Ala Leu Leu Ile Val
        560                 565                 570

cat ggt atc aag ctc tac ctc aca aag att tgg tat cag aag acg aca      1898
His Gly Ile Lys Leu Tyr Leu Thr Lys Ile Trp Tyr Gln Lys Thr Thr
    575                 580                 585
```

```
tgg atg tat ctt gct gta ccc atc ctt cta tat gca tct gag agg ctg    1946
Trp Met Tyr Leu Ala Val Pro Ile Leu Leu Tyr Ala Ser Glu Arg Leu
590                 595                 600                 605

ctc cgt gct ttc aga tca agc atc aaa ccg gtt aag atg atc aag gtg    1994
Leu Arg Ala Phe Arg Ser Ser Ile Lys Pro Val Lys Met Ile Lys Val
                610                 615                 620

gct gtt tac ccc ggg aac gtg ttg tct cta cac atg acg aag cca caa    2042
Ala Val Tyr Pro Gly Asn Val Leu Ser Leu His Met Thr Lys Pro Gln
            625                 630                 635

gga ttc aaa tac aaa agt gga cag ttc atg ttg gtg aac tgc cga gcc    2090
Gly Phe Lys Tyr Lys Ser Gly Gln Phe Met Leu Val Asn Cys Arg Ala
        640                 645                 650

gta tct cca ttc gaa tgg cat cct ttc tca atc aca tca gct ccc gga    2138
Val Ser Pro Phe Glu Trp His Pro Phe Ser Ile Thr Ser Ala Pro Gly
    655                 660                 665

gac gat tac ctg agc gta cat atc cgc act ctc ggt gac tgg aca cgt    2186
Asp Asp Tyr Leu Ser Val His Ile Arg Thr Leu Gly Asp Trp Thr Arg
670                 675                 680                 685

aag ctc agg acc gtt ttc tcc gag gtt tgc aaa cct cct acc gcc ggt    2234
Lys Leu Arg Thr Val Phe Ser Glu Val Cys Lys Pro Pro Thr Ala Gly
                690                 695                 700

aaa agc ggt ctt ctc cga gca gac gga gga gat gga aac ctc ccg ttc    2282
Lys Ser Gly Leu Leu Arg Ala Asp Gly Gly Asp Gly Asn Leu Pro Phe
            705                 710                 715

ccg aag gtc ctt atc gac ggt cca tac ggt gct ccc gca caa gac tac    2330
Pro Lys Val Leu Ile Asp Gly Pro Tyr Gly Ala Pro Ala Gln Asp Tyr
        720                 725                 730

aag aaa tac gac gtg gta ctc ctc gta ggt ctc ggc att gga gcc acg    2378
Lys Lys Tyr Asp Val Val Leu Leu Val Gly Leu Gly Ile Gly Ala Thr
    735                 740                 745

cct atg atc agt atc ctt aag gac atc atc aac aac atg aaa ggt cct    2426
Pro Met Ile Ser Ile Leu Lys Asp Ile Ile Asn Asn Met Lys Gly Pro
750                 755                 760                 765

gac cgc gac agc gac att gag aac aat aac agt aac aac aat agt aaa    2474
Asp Arg Asp Ser Asp Ile Glu Asn Asn Asn Ser Asn Asn Asn Ser Lys
                770                 775                 780

ggg ttt aag aca agg aaa gct tat ttc tac tgg gtg act agg gaa caa    2522
Gly Phe Lys Thr Arg Lys Ala Tyr Phe Tyr Trp Val Thr Arg Glu Gln
            785                 790                 795

gga tca ttc gag tgg ttc aag gga ata atg gac gag att tcg gag tta    2570
Gly Ser Phe Glu Trp Phe Lys Gly Ile Met Asp Glu Ile Ser Glu Leu
        800                 805                 810

gac gag gaa gga atc atc gag ctt cac aat tat tgc acg agt gtg tac    2618
Asp Glu Glu Gly Ile Ile Glu Leu His Asn Tyr Cys Thr Ser Val Tyr
    815                 820                 825

gag gaa ggt gat gca aga gtg gct ctc att gcc atg ctt cag tcg ttg    2666
Glu Glu Gly Asp Ala Arg Val Ala Leu Ile Ala Met Leu Gln Ser Leu
830                 835                 840                 845

caa cac gct aag aac ggt gtg gat gtt gtg tcg ggt aca cgt gtc aag    2714
Gln His Ala Lys Asn Gly Val Asp Val Val Ser Gly Thr Arg Val Lys
                850                 855                 860
```

```
tcc cac ttc gct aaa cct aac tgg aga caa gtc tac aag aag atc gct    2762
Ser His Phe Ala Lys Pro Asn Trp Arg Gln Val Tyr Lys Lys Ile Ala
            865                 870                 875

gtt caa cat ccc ggc aaa aga ata gga gtc ttc tac tgt gga atg cca    2810
Val Gln His Pro Gly Lys Arg Ile Gly Val Phe Tyr Cys Gly Met Pro
        880                 885                 890

gga atg ata aag gaa tta aaa aat cta gct ttg gat ttt tct cga aag    2858
Gly Met Ile Lys Glu Leu Lys Asn Leu Ala Leu Asp Phe Ser Arg Lys
    895                 900                 905

aca act acc aag ttt gac ttc cac aaa gag aac ttc tagattaatt         2904
Thr Thr Thr Lys Phe Asp Phe His Lys Glu Asn Phe
910                 915                 920

atatacgttg tagaaaaata aaacaagaaa caactataca aataaatatt tattttaaat 2964

tcttttcatt ttatgtaaaa ttatctgagt tatctttttt tgttaaaaaa aaaaaaaaaa 3024

aaaaaaaaaa a                                                      3035
```

<210> 14
<211> 921
<212> PRT
<213> Arabidopsis thaliana

<400> 14

```
Met Lys Met Arg Arg Gly Asn Ser Ser Asn Asp His Glu Leu Gly Ile
 1               5                  10                  15

Leu Arg Gly Ala Asn Ser Asp Thr Asn Ser Asp Thr Glu Ser Ile Ala
            20                  25                  30

Ser Asp Arg Gly Ala Phe Ser Gly Pro Leu Gly Arg Pro Lys Arg Ala
        35                  40                  45

Ser Lys Lys Asn Ala Arg Phe Ala Asp Asp Leu Pro Lys Arg Ser Asn
    50                  55                  60

Ser Val Ala Gly Gly Arg Gly Asp Asp Asp Glu Tyr Val Glu Ile Thr
 65                  70                  75                  80

Leu Asp Ile Arg Asp Asp Ser Val Ala Val His Ser Val Gln Gln Ala
                 85                  90                  95

Ala Gly Gly Gly Gly His Leu Glu Asp Pro Glu Leu Ala Leu Leu Thr
            100                 105                 110

Lys Lys Thr Leu Glu Ser Ser Leu Asn Asn Thr Thr Ser Leu Ser Phe
        115                 120                 125

Phe Arg Ser Thr Ser Ser Arg Ile Lys Asn Ala Ser Arg Glu Leu Arg
    130                 135                 140

Arg Val Phe Ser Arg Arg Pro Ser Pro Ala Val Arg Arg Phe Asp Arg
145                 150                 155                 160

Thr Ser Ser Ala Ala Ile His Ala Leu Lys Gly Leu Lys Phe Ile Ala
                165                 170                 175

Thr Lys Thr Ala Ala Trp Pro Ala Val Asp Gln Arg Phe Asp Lys Leu
            180                 185                 190

Ser Ala Asp Ser Asn Gly Leu Leu Leu Ser Ala Lys Phe Trp Glu Cys
        195                 200                 205

Leu Gly Met Asn Lys Glu Ser Lys Asp Phe Ala Asp Gln Leu Phe Arg
    210                 215                 220
```

```
Ala Leu Ala Arg Arg Asn Asn Val Ser Gly Asp Ala Ile Thr Lys Glu
225                 230                 235                 240
Gln Leu Arg Ile Phe Trp Glu Gln Ile Ser Asp Glu Ser Phe Asp Ala
                245                 250                 255
Lys Leu Gln Val Phe Phe Asp Met Val Asp Lys Asp Glu Asp Gly Arg
            260                 265                 270
Val Thr Glu Glu Glu Val Ala Glu Ile Ile Ser Leu Ser Ala Ser Ala
        275                 280                 285
Asn Lys Leu Ser Asn Ile Gln Lys Gln Ala Lys Glu Tyr Ala Ala Leu
    290                 295                 300
Ile Met Glu Glu Leu Asp Pro Asp Asn Ala Gly Phe Ile Met Ile Glu
305                 310                 315                 320
Asn Leu Glu Met Leu Leu Leu Gln Ala Pro Asn Gln Ser Val Arg Met
                325                 330                 335
Gly Asp Ser Arg Ile Leu Ser Gln Met Leu Ser Gln Lys Leu Arg Pro
            340                 345                 350
Ala Lys Glu Ser Asn Pro Leu Leu Arg Trp Ser Glu Lys Ile Lys Tyr
        355                 360                 365
Phe Ile Leu Asp Asn Trp Gln Arg Leu Trp Ile Met Met Leu Trp Leu
    370                 375                 380
Gly Ile Cys Gly Gly Leu Phe Thr Tyr Lys Phe Ile Gln Tyr Lys Asn
385                 390                 395                 400
Lys Ala Ala Tyr Gly Val Met Gly Tyr Cys Val Cys Val Ala Lys Gly
                405                 410                 415
Gly Ala Glu Thr Leu Lys Phe Asn Met Ala Leu Ile Leu Leu Pro Val
            420                 425                 430
Cys Arg Asn Thr Ile Thr Trp Leu Arg Asn Lys Thr Lys Leu Gly Thr
        435                 440                 445
Val Val Pro Phe Asp Asp Ser Leu Asn Phe His Lys Val Ile Ala Ser
    450                 455                 460
Gly Ile Val Val Gly Val Leu Leu His Ala Gly Ala His Leu Thr Cys
465                 470                 475                 480
Asp Phe Pro Arg Leu Ile Ala Ala Asp Glu Asp Thr Tyr Glu Pro Met
                485                 490                 495
Glu Lys Tyr Phe Gly Asp Gln Pro Thr Ser Tyr Trp Trp Phe Val Lys
            500                 505                 510
Gly Val Glu Gly Trp Thr Gly Ile Val Met Val Val Leu Met Ala Ile
        515                 520                 525
Ala Phe Thr Leu Ala Thr Pro Trp Phe Arg Arg Asn Lys Leu Asn Leu
    530                 535                 540
Pro Asn Phe Leu Lys Lys Leu Thr Gly Phe Asn Ala Phe Trp Tyr Thr
545                 550                 555                 560
His His Leu Phe Ile Ile Val Tyr Ala Leu Leu Ile Val His Gly Ile
                565                 570                 575
Lys Leu Tyr Leu Thr Lys Ile Trp Tyr Gln Lys Thr Thr Trp Met Tyr
            580                 585                 590
Leu Ala Val Pro Ile Leu Leu Tyr Ala Ser Glu Arg Leu Leu Arg Ala
        595                 600                 605
```

```
Phe Arg Ser Ser Ile Lys Pro Val Lys Met Ile Lys Val Ala Val Tyr
    610                 615                 620

Pro Gly Asn Val Leu Ser Leu His Met Thr Lys Pro Gln Gly Phe Lys
625                 630                 635                 640

Tyr Lys Ser Gly Gln Phe Met Leu Val Asn Cys Arg Ala Val Ser Pro
                645                 650                 655

Phe Glu Trp His Pro Phe Ser Ile Thr Ser Ala Pro Gly Asp Asp Tyr
            660                 665                 670

Leu Ser Val His Ile Arg Thr Leu Gly Asp Trp Thr Arg Lys Leu Arg
        675                 680                 685

Thr Val Phe Ser Glu Val Cys Lys Pro Pro Thr Ala Gly Lys Ser Gly
    690                 695                 700

Leu Leu Arg Ala Asp Gly Gly Asp Gly Asn Leu Pro Phe Pro Lys Val
705                 710                 715                 720

Leu Ile Asp Gly Pro Tyr Gly Ala Pro Ala Gln Asp Tyr Lys Lys Tyr
                725                 730                 735

Asp Val Val Leu Leu Val Gly Leu Gly Ile Gly Ala Thr Pro Met Ile
            740                 745                 750

Ser Ile Leu Lys Asp Ile Ile Asn Asn Met Lys Gly Pro Asp Arg Asp
        755                 760                 765

Ser Asp Ile Glu Asn Asn Asn Ser Asn Asn Asn Ser Lys Gly Phe Lys
    770                 775                 780

Thr Arg Lys Ala Tyr Phe Tyr Trp Val Thr Arg Glu Gln Gly Ser Phe
785                 790                 795                 800

Glu Trp Phe Lys Gly Ile Met Asp Glu Ile Ser Glu Leu Asp Glu Glu
                805                 810                 815

Gly Ile Ile Glu Leu His Asn Tyr Cys Thr Ser Val Tyr Glu Glu Gly
            820                 825                 830

Asp Ala Arg Val Ala Leu Ile Ala Met Leu Gln Ser Leu Gln His Ala
        835                 840                 845

Lys Asn Gly Val Asp Val Val Ser Gly Thr Arg Val Lys Ser His Phe
    850                 855                 860

Ala Lys Pro Asn Trp Arg Gln Val Tyr Lys Lys Ile Ala Val Gln His
865                 870                 875                 880

Pro Gly Lys Arg Ile Gly Val Phe Tyr Cys Gly Met Pro Gly Met Ile
                885                 890                 895

Lys Glu Leu Lys Asn Leu Ala Leu Asp Phe Ser Arg Lys Thr Thr Thr
            900                 905                 910

Lys Phe Asp Phe His Lys Glu Asn Phe
        915                 920
```

<210> 15
<211> 3338
<212> DNA
<213> Nicotiana tabacum

<220>

<221> CDS
<222> (313)..(3129)
<223> coding for NADPH-oxidase

<400> 15

```
ggcacgagaa aatccccaat cttttatttg tttattaaaa ttagtacgcc aagaaagaaa 60
gaaagaaaga cagaaagact cggtcttctt tcttctcttg gtctgaaact ccaaaataga 120
ataccaatta ttaatctttt gtcatctttt tccttctcgc gttcatatat actggaatat 180
acatcttttt ttcaacctat cttctttcat tttcaagaat tcgggttcca taaatagtag 240
gttcactact tttatttcaa cctccttaaa gtttattcat tcatattttt tctcaaagaa 300
aaaactatag aa atg caa aat tcg gaa aat cat cat ccg cac cac cag cac 351
              Met Gln Asn Ser Glu Asn His His Pro His His Gln His
              1               5                   10

cac cat tcg gac aca gag ata att gga aat gat aga gcg tcg tac agt    399
His His Ser Asp Thr Glu Ile Ile Gly Asn Asp Arg Ala Ser Tyr Ser
    15                  20                  25

ggt ccg tta agc gga ccg tta aac aaa cga ggc ggc aaa aag agt gcg    447
Gly Pro Leu Ser Gly Pro Leu Asn Lys Arg Gly Gly Lys Lys Ser Ala
30                  35                  40                  45

aga ttt aac att cct gaa tct acc gac atc gga acc agt gtc gga acc    495
Arg Phe Asn Ile Pro Glu Ser Thr Asp Ile Gly Thr Ser Val Gly Thr
                50                  55                  60

ggc ggc aag tcc aat gat gat gcg tac gtt gaa atc act ctc gat gtc    543
Gly Gly Lys Ser Asn Asp Asp Ala Tyr Val Glu Ile Thr Leu Asp Val
            65                  70                  75

cgc gaa gat tcc gtc gct gtc cac agt gtc aaa act gcc ggc ggt gat    591
Arg Glu Asp Ser Val Ala Val His Ser Val Lys Thr Ala Gly Gly Asp
        80                  85                  90

gac gtg gaa gat ccc gag ctg gct tta ttg gct aaa ggc tta gag aag    639
Asp Val Glu Asp Pro Glu Leu Ala Leu Leu Ala Lys Gly Leu Glu Lys
    95                  100                 105

aag tcc act tta gga tct tca ctt gtt cga aat gct tcg tct aga att    687
Lys Ser Thr Leu Gly Ser Ser Leu Val Arg Asn Ala Ser Ser Arg Ile
110                 115                 120                 125

cgg caa gtg tca caa gag ctc agg cgt ttg gct tcc tta aat aaa cgc    735
Arg Gln Val Ser Gln Glu Leu Arg Arg Leu Ala Ser Leu Asn Lys Arg
                130                 135                 140

cca att cct act gga agg ttc gac agg aat aaa tca gct gct gct cat    783
Pro Ile Pro Thr Gly Arg Phe Asp Arg Asn Lys Ser Ala Ala Ala His
            145                 150                 155

gct ctt aaa ggt ctc aag ttt att agt aag acc gac ggc ggc gct ggt    831
Ala Leu Lys Gly Leu Lys Phe Ile Ser Lys Thr Asp Gly Gly Ala Gly
        160                 165                 170

tgg gcc gcc gtc gag aag cgg ttc gat gag att act gct tct act act    879
Trp Ala Ala Val Glu Lys Arg Phe Asp Glu Ile Thr Ala Ser Thr Thr
    175                 180                 185

ggt ttg ctt cct cgt gcc aaa ttt gga gaa tgt ata ggt atg aat aag    927
Gly Leu Leu Pro Arg Ala Lys Phe Gly Glu Cys Ile Gly Met Asn Lys
190                 195                 200                 205

gag tct aag gaa ttt gct gtt gag cta tat gat gcg cta gct cgg agg    975
Glu Ser Lys Glu Phe Ala Val Glu Leu Tyr Asp Ala Leu Ala Arg Arg
                210                 215                 220

aga aac att aca act gat tcc att aac aaa gca cag ctc aaa gag ttc    1023
Arg Asn Ile Thr Thr Asp Ser Ile Asn Lys Ala Gln Leu Lys Glu Phe
            225                 230                 235
```

```
tgg gac caa gtg gct gac caa agt ttt gat tct cgc ctt caa aca ttt    1071
Trp Asp Gln Val Ala Asp Gln Ser Phe Asp Ser Arg Leu Gln Thr Phe
        240                 245                 250

ttt gac atg gtt gat aaa gat gct gat ggt aga att aca gaa gaa gaa    1119
Phe Asp Met Val Asp Lys Asp Ala Asp Gly Arg Ile Thr Glu Glu Glu
    255                 260                 265

gtc aga gag att ata ggc ctt agc gcg tcg gcc aac agg ctg tca aca    1167
Val Arg Glu Ile Ile Gly Leu Ser Ala Ser Ala Asn Arg Leu Ser Thr
270                 275                 280                 285

atc cag aaa caa gct gat gaa tac gca gca atg atc atg gaa gag ttg    1215
Ile Gln Lys Gln Ala Asp Glu Tyr Ala Ala Met Ile Met Glu Glu Leu
                290                 295                 300

gat cct aac aac ctc gga tac att atg att gag aac ttg gaa atg ctt    1263
Asp Pro Asn Asn Leu Gly Tyr Ile Met Ile Glu Asn Leu Glu Met Leu
            305                 310                 315

tta ctg caa gca cca aat caa tca gtg caa aga gga ggc gaa agt cgg    1311
Leu Leu Gln Ala Pro Asn Gln Ser Val Gln Arg Gly Gly Glu Ser Arg
        320                 325                 330

aac ttg agt caa atg cta agt caa aaa cta aag cat aca caa gag aga    1359
Asn Leu Ser Gln Met Leu Ser Gln Lys Leu Lys His Thr Gln Glu Arg
    335                 340                 345

aat cca ata gta aga tgg tac aag agt ttc atg tac ttt ttg ctg gat    1407
Asn Pro Ile Val Arg Trp Tyr Lys Ser Phe Met Tyr Phe Leu Leu Asp
350                 355                 360                 365

aat tgg caa aga gtt tgg gta ttg tta ctg tgg att gga att atg gct    1455
Asn Trp Gln Arg Val Trp Val Leu Leu Leu Trp Ile Gly Ile Met Ala
                370                 375                 380

ggt cta ttt aca tgg aaa tat ata cag tat aaa gaa aaa gct gca tat    1503
Gly Leu Phe Thr Trp Lys Tyr Ile Gln Tyr Lys Glu Lys Ala Ala Tyr
            385                 390                 395

aaa gtc atg ggt ccc tgt gtg tgt ttt gcc aaa ggt gct gct gaa aca    1551
Lys Val Met Gly Pro Cys Val Cys Phe Ala Lys Gly Ala Ala Glu Thr
        400                 405                 410

ctc aag ctc aac atg gca att att tta ttt ccg gtt tgc aga aac acg    1599
Leu Lys Leu Asn Met Ala Ile Ile Leu Phe Pro Val Cys Arg Asn Thr
    415                 420                 425

atc aca tgg ctt cga aat aag acc aga tta ggt gct gct gtt cct ttt    1647
Ile Thr Trp Leu Arg Asn Lys Thr Arg Leu Gly Ala Ala Val Pro Phe
430                 435                 440                 445

gat gat aac ctt aat ttt cac aaa gtg ata gca gtg gca att gct ctt    1695
Asp Asp Asn Leu Asn Phe His Lys Val Ile Ala Val Ala Ile Ala Leu
                450                 455                 460

ggg gtt gga ata cac gga cta tct cac ttg aca tgt gat ttt cct cgg    1743
Gly Val Gly Ile His Gly Leu Ser His Leu Thr Cys Asp Phe Pro Arg
            465                 470                 475

ctt tta aat gct agt gaa gaa gaa tat gaa cca atg aag tac tat ttt    1791
Leu Leu Asn Ala Ser Glu Glu Glu Tyr Glu Pro Met Lys Tyr Tyr Phe
        480                 485                 490

gga gat cag cca gaa agc tat tgg tgg ttt ata aaa gga gta gaa ggg    1839
Gly Asp Gln Pro Glu Ser Tyr Trp Trp Phe Ile Lys Gly Val Glu Gly
    495                 500                 505
```

```
gta act gga att ata atg gtg gtt tta atg gca ata gca ttt act cta     1887
Val Thr Gly Ile Ile Met Val Val Leu Met Ala Ile Ala Phe Thr Leu
510                 515                 520                 525

gca acc cca tgg ttt aga agg aat aga gtt agt ttg cca aaa cca ttt     1935
Ala Thr Pro Trp Phe Arg Arg Asn Arg Val Ser Leu Pro Lys Pro Phe
                530                 535                 540

cac aaa ctc act gga tnt aat gcc ttt tgg tac tct cac cat ctc ttt     1983
His Lys Leu Thr Gly Xaa Asn Ala Phe Trp Tyr Ser His His Leu Phe
            545                 550                 555

gtt atc gtc tac act ctg ttc att gtg cat ggt gaa aag cta tac att     2031
Val Ile Val Tyr Thr Leu Phe Ile Val His Gly Glu Lys Leu Tyr Ile
        560                 565                 570

acc aaa gat tgg tac aag aga acc gac atg gat gta ctt tta act atc     2079
Thr Lys Asp Trp Tyr Lys Arg Thr Asp Met Asp Val Leu Leu Thr Ile
    575                 580                 585

cca atc ata ctc tat gct agt gaa agg ttg att agg gca ttc agg tca     2127
Pro Ile Ile Leu Tyr Ala Ser Glu Arg Leu Ile Arg Ala Phe Arg Ser
590                 595                 600                 605

agc att aaa gct gtt aag att ttg aag gtg gca gta tat cca gga aat     2175
Ser Ile Lys Ala Val Lys Ile Leu Lys Val Ala Val Tyr Pro Gly Asn
                610                 615                 620

gtg ttg gca ctt cac atg tca aaa cca cag ggc tac aaa tac aaa agt     2223
Val Leu Ala Leu His Met Ser Lys Pro Gln Gly Tyr Lys Tyr Lys Ser
            625                 630                 635

ggg caa tac atg ttt gtc aac tgt gct gca gtt tct cca ttt gag tgg     2271
Gly Gln Tyr Met Phe Val Asn Cys Ala Ala Val Ser Pro Phe Glu Trp
        640                 645                 650

cat cca ttt tca att act tcg gcc cca gga gat gac tat ctc agt gtc     2319
His Pro Phe Ser Ile Thr Ser Ala Pro Gly Asp Asp Tyr Leu Ser Val
    655                 660                 665

cat att cga act ctt ggt gat tgg acc agg caa ctt aaa act gtt ttc     2367
His Ile Arg Thr Leu Gly Asp Trp Thr Arg Gln Leu Lys Thr Val Phe
670                 675                 680                 685

tcc gag gtt tgc cag cca cca cct aat gga aaa agt gga ctc ctc aga     2415
Ser Glu Val Cys Gln Pro Pro Pro Asn Gly Lys Ser Gly Leu Leu Arg
                690                 695                 700

gct gac tac ttg caa gga gag aat aat cct aat ttc cca agg gtg tta     2463
Ala Asp Tyr Leu Gln Gly Glu Asn Asn Pro Asn Phe Pro Arg Val Leu
            705                 710                 715

ata gat gga cca tat gga gca cca gca caa gac tac aag aaa tat gag     2511
Ile Asp Gly Pro Tyr Gly Ala Pro Ala Gln Asp Tyr Lys Lys Tyr Glu
        720                 725                 730

gtg gtt ttg ttg gta ggt ctt gga att gga gct aca cca atg atc agt     2559
Val Val Leu Leu Val Gly Leu Gly Ile Gly Ala Thr Pro Met Ile Ser
    735                 740                 745

att gtt aaa gac att gtc aac aac atg aag gca atg gac gaa gaa gaa     2607
Ile Val Lys Asp Ile Val Asn Asn Met Lys Ala Met Asp Glu Glu Glu
750                 755                 760                 765

aat tcc ttg gaa gat gga cac aat aat aat atg gca cca aat tct agc     2655
Asn Ser Leu Glu Asp Gly His Asn Asn Asn Met Ala Pro Asn Ser Ser
                770                 775                 780
```

```
ccc aat att gca aaa aat aag ggt aat aaa tca ggt tca gca agt gga    2703
Pro Asn Ile Ala Lys Asn Lys Gly Asn Lys Ser Gly Ser Ala Ser Gly
            785                 790                 795

gga aat aat ttc aat aca agg aga gca tat ttc tat tgg gtt act aga    2751
Gly Asn Asn Phe Asn Thr Arg Arg Ala Tyr Phe Tyr Trp Val Thr Arg
        800                 805                 810

gaa caa ggt tca ttt gat tgg ttc aaa ggt ata atg aat gaa gct gct    2799
Glu Gln Gly Ser Phe Asp Trp Phe Lys Gly Ile Met Asn Glu Ala Ala
    815                 820                 825

gaa atg gac cat aag gga gta att gaa atg cat aat tat tgt act agt    2847
Glu Met Asp His Lys Gly Val Ile Glu Met His Asn Tyr Cys Thr Ser
830                 835                 840                 845

gtt tat gaa gaa ggt gat gct cgt tct gct ctt att act atg ctt cag    2895
Val Tyr Glu Glu Gly Asp Ala Arg Ser Ala Leu Ile Thr Met Leu Gln
                850                 855                 860

tct ctt cac cat gcc aaa aat ggt gtt gac att gtc tct ggc acc aga    2943
Ser Leu His His Ala Lys Asn Gly Val Asp Ile Val Ser Gly Thr Arg
            865                 870                 875

gtt aag tca cat ttt gct aaa cct aat tgg cgt aat gtc tac aaa cgc    2991
Val Lys Ser His Phe Ala Lys Pro Asn Trp Arg Asn Val Tyr Lys Arg
        880                 885                 890

att gct ctc aac cac cct gag gct aaa gtt ggg gtc ttc tat tgt ggg    3039
Ile Ala Leu Asn His Pro Glu Ala Lys Val Gly Val Phe Tyr Cys Gly
    895                 900                 905

gca cca gca ctg acc aaa gaa cta aga caa cac gcc ttg gat ttt tca    3087
Ala Pro Ala Leu Thr Lys Glu Leu Arg Gln His Ala Leu Asp Phe Ser
910                 915                 920                 925

cac aag aca tct acc aag ttt gat ttc cat aaa gaa aat ttt            3129
His Lys Thr Ser Thr Lys Phe Asp Phe His Lys Glu Asn Phe
                930                 935

tgagcaaaga atagaccatt aagcagagca ttaaaatttc atcaaaacag ctaaggacac 3189

aggttgtttt atagaagtct accaactctc cctattgtgt acagataatg ttgcacttca 3249

agttgatata tagttgtggt tgtgatgcta gtatattaca aaataataag attattttta 3309

tttgtagtaa aaaaaaaaaa aaaaaaaaa                                   3338
```

<210> 16
<211> 939
<212> PRT
<213> Nicotiana tabacum

<400> 16

```
Met Gln Asn Ser Glu Asn His His Pro His His Gln His His His Ser
 1               5                  10                  15

Asp Thr Glu Ile Ile Gly Asn Asp Arg Ala Ser Tyr Ser Gly Pro Leu
            20                  25                  30

Ser Gly Pro Leu Asn Lys Arg Gly Gly Lys Lys Ser Ala Arg Phe Asn
        35                  40                  45

Ile Pro Glu Ser Thr Asp Ile Gly Thr Ser Val Gly Thr Gly Gly Lys
    50                  55                  60

Ser Asn Asp Asp Ala Tyr Val Glu Ile Thr Leu Asp Val Arg Glu Asp
65                  70                  75                  80
```

Ser Val Ala Val His Ser Val Lys Thr Ala Gly Gly Asp Asp Val Glu
85 90 95

Asp Pro Glu Leu Ala Leu Leu Ala Lys Gly Leu Glu Lys Lys Ser Thr
100 105 110

Leu Gly Ser Ser Leu Val Arg Asn Ala Ser Ser Arg Ile Arg Gln Val
115 120 125

Ser Gln Glu Leu Arg Arg Leu Ala Ser Leu Asn Lys Arg Pro Ile Pro
130 135 140

Thr Gly Arg Phe Asp Arg Asn Lys Ser Ala Ala Ala His Ala Leu Lys
145 150 155 160

Gly Leu Lys Phe Ile Ser Lys Thr Asp Gly Gly Ala Gly Trp Ala Ala
165 170 175

Val Glu Lys Arg Phe Asp Glu Ile Thr Ala Ser Thr Thr Gly Leu Leu
180 185 190

Pro Arg Ala Lys Phe Gly Glu Cys Ile Gly Met Asn Lys Glu Ser Lys
195 200 205

Glu Phe Ala Val Glu Leu Tyr Asp Ala Leu Ala Arg Arg Arg Asn Ile
210 215 220

Thr Thr Asp Ser Ile Asn Lys Ala Gln Leu Lys Glu Phe Trp Asp Gln
225 230 235 240

Val Ala Asp Gln Ser Phe Asp Ser Arg Leu Gln Thr Phe Phe Asp Met
245 250 255

Val Asp Lys Asp Ala Asp Gly Arg Ile Thr Glu Glu Glu Val Arg Glu
260 265 270

Ile Ile Gly Leu Ser Ala Ser Ala Asn Arg Leu Ser Thr Ile Gln Lys
275 280 285

Gln Ala Asp Glu Tyr Ala Ala Met Ile Met Glu Glu Leu Asp Pro Asn
290 295 300

Asn Leu Gly Tyr Ile Met Ile Glu Asn Leu Glu Met Leu Leu Leu Gln
305 310 315 320

Ala Pro Asn Gln Ser Val Gln Arg Gly Gly Glu Ser Arg Asn Leu Ser
325 330 335

Gln Met Leu Ser Gln Lys Leu Lys His Thr Gln Glu Arg Asn Pro Ile
340 345 350

Val Arg Trp Tyr Lys Ser Phe Met Tyr Phe Leu Leu Asp Asn Trp Gln
355 360 365

Arg Val Trp Val Leu Leu Leu Trp Ile Gly Ile Met Ala Gly Leu Phe
370 375 380

Thr Trp Lys Tyr Ile Gln Tyr Lys Glu Lys Ala Ala Tyr Lys Val Met
385 390 395 400

Gly Pro Cys Val Cys Phe Ala Lys Gly Ala Ala Glu Thr Leu Lys Leu
405 410 415

Asn Met Ala Ile Ile Leu Phe Pro Val Cys Arg Asn Thr Ile Thr Trp
420 425 430

Leu Arg Asn Lys Thr Arg Leu Gly Ala Ala Val Pro Phe Asp Asp Asn
435 440 445

Leu Asn Phe His Lys Val Ile Ala Val Ala Ile Ala Leu Gly Val Gly
450 455 460

Ile His Gly Leu Ser His Leu Thr Cys Asp Phe Pro Arg Leu Leu Asn
465 470 475 480

Ala Ser Glu Glu Glu Tyr Glu Pro Met Lys Tyr Tyr Phe Gly Asp Gln
485 490 495

Pro Glu Ser Tyr Trp Trp Phe Ile Lys Gly Val Glu Gly Val Thr Gly
500 505 510

Ile Ile Met Val Val Leu Met Ala Ile Ala Phe Thr Leu Ala Thr Pro
515 520 525

Trp Phe Arg Arg Asn Arg Val Ser Leu Pro Lys Pro Phe His Lys Leu
530 535 540

Thr Gly Xaa Asn Ala Phe Trp Tyr Ser His His Leu Phe Val Ile Val
545 550 555 560

Tyr Thr Leu Phe Ile Val His Gly Glu Lys Leu Tyr Ile Thr Lys Asp
565 570 575

Trp Tyr Lys Arg Thr Asp Met Asp Val Leu Leu Thr Ile Pro Ile Ile
580 585 590

Leu Tyr Ala Ser Glu Arg Leu Ile Arg Ala Phe Arg Ser Ser Ile Lys
595 600 605

Ala Val Lys Ile Leu Lys Val Ala Val Tyr Pro Gly Asn Val Leu Ala
610 615 620

Leu His Met Ser Lys Pro Gln Gly Tyr Lys Tyr Lys Ser Gly Gln Tyr
625 630 635 640

Met Phe Val Asn Cys Ala Ala Val Ser Pro Phe Glu Trp His Pro Phe
645 650 655

Ser Ile Thr Ser Ala Pro Gly Asp Asp Tyr Leu Ser Val His Ile Arg
660 665 670

Thr Leu Gly Asp Trp Thr Arg Gln Leu Lys Thr Val Phe Ser Glu Val
675 680 685

Cys Gln Pro Pro Pro Asn Gly Lys Ser Gly Leu Leu Arg Ala Asp Tyr
690 695 700

Leu Gln Gly Glu Asn Asn Pro Asn Phe Pro Arg Val Leu Ile Asp Gly
705 710 715 720

Pro Tyr Gly Ala Pro Ala Gln Asp Tyr Lys Lys Tyr Glu Val Val Leu
725 730 735

Leu Val Gly Leu Gly Ile Gly Ala Thr Pro Met Ile Ser Ile Val Lys
740 745 750

Asp Ile Val Asn Asn Met Lys Ala Met Asp Glu Glu Glu Asn Ser Leu
755 760 765

Glu Asp Gly His Asn Asn Asn Met Ala Pro Asn Ser Ser Pro Asn Ile
770 775 780

Ala Lys Asn Lys Gly Asn Lys Ser Gly Ser Ala Ser Gly Gly Asn Asn
785 790 795 800

Phe Asn Thr Arg Arg Ala Tyr Phe Tyr Trp Val Thr Arg Glu Gln Gly
805 810 815

Ser Phe Asp Trp Phe Lys Gly Ile Met Asn Glu Ala Ala Glu Met Asp
820 825 830

His Lys Gly Val Ile Glu Met His Asn Tyr Cys Thr Ser Val Tyr Glu
835 840 845

Glu Gly Asp Ala Arg Ser Ala Leu Ile Thr Met Leu Gln Ser Leu His
    850                 855                 860

His Ala Lys Asn Gly Val Asp Ile Val Ser Gly Thr Arg Val Lys Ser
865                 870                 875                 880

His Phe Ala Lys Pro Asn Trp Arg Asn Val Tyr Lys Arg Ile Ala Leu
                885                 890                 895

Asn His Pro Glu Ala Lys Val Gly Val Phe Tyr Cys Gly Ala Pro Ala
            900                 905                 910

Leu Thr Lys Glu Leu Arg Gln His Ala Leu Asp Phe Ser His Lys Thr
        915                 920                 925

Ser Thr Lys Phe Asp Phe His Lys Glu Asn Phe
    930                 935

<210> 17
<211> 2532
<212> DNA
<213> Oryza sativa

<220>
<221> CDS
<222> (1)..(2529)
<223> coding for NADPH-oxidase

<400> 17

```
atg gcg tcg ccg tac gac cac cag tcg ccg cat gcg cag cac ccg tcg      48
Met Ala Ser Pro Tyr Asp His Gln Ser Pro His Ala Gln His Pro Ser
 1               5                  10                  15

ggg ttg ccg agg ccg ccg ggg gcg ggg gcg ggt gcg gcg gcg ggc ggg      96
Gly Leu Pro Arg Pro Pro Gly Ala Gly Ala Gly Ala Ala Ala Gly Gly
            20                  25                  30

ttc gcg cgg ggg ctg atg aag cag ccg tcg cgg ctg gcg tcc ggg gtg     144
Phe Ala Arg Gly Leu Met Lys Gln Pro Ser Arg Leu Ala Ser Gly Val
        35                  40                  45

agg cag ttc gcg tcg agg gtg tcg atg aag gtg ccg gag ggg gtg ggg     192
Arg Gln Phe Ala Ser Arg Val Ser Met Lys Val Pro Glu Gly Val Gly
    50                  55                  60

ggg atg cgg ccc ggt ggc ggg agg atg acg cgg atg cag tcc agc gcg     240
Gly Met Arg Pro Gly Gly Gly Arg Met Thr Arg Met Gln Ser Ser Ala
65                  70                  75                  80

cag gtg ggg ctc cgg ggg ctc cgc ttc ctc gac aag acg tcc ggc ggg     288
Gln Val Gly Leu Arg Gly Leu Arg Phe Leu Asp Lys Thr Ser Gly Gly
                85                  90                  95

aag gag ggg tgg aag tcc gtc gag cgc cgc ttc gac gag atg aac cgc     336
Lys Glu Gly Trp Lys Ser Val Glu Arg Arg Phe Asp Glu Met Asn Arg
            100                 105                 110

aac ggc cgc ctc ccc aag gag agc ttc ggc aag tgc atc ggc atg ggg     384
Asn Gly Arg Leu Pro Lys Glu Ser Phe Gly Lys Cys Ile Gly Met Gly
        115                 120                 125

gac tcc aag gag ttc gcc ggc gag ctg ttc gtg gcg ctg gcg cgg cgg     432
Asp Ser Lys Glu Phe Ala Gly Glu Leu Phe Val Ala Leu Ala Arg Arg
    130                 135                 140

agg aac ctg gag ccg gag gac ggc atc acc aag gag cag ctc aag gag     480
Arg Asn Leu Glu Pro Glu Asp Gly Ile Thr Lys Glu Gln Leu Lys Glu
145                 150                 155                 160
```

```
ttc tgg gag gag atg acc gac cag aac ttc gac tcg cgg ctt cgc att     528
Phe Trp Glu Glu Met Thr Asp Gln Asn Phe Asp Ser Arg Leu Arg Ile
                165                 170                 175

ttc ttt gac atg tgc gac aag aat ggc gat ggg atg ctc acg gaa gat     576
Phe Phe Asp Met Cys Asp Lys Asn Gly Asp Gly Met Leu Thr Glu Asp
            180                 185                 190

gag gtc aag gag gtt att ata ctg agt gcg tcg gcg aac aag ctg gcg     624
Glu Val Lys Glu Val Ile Ile Leu Ser Ala Ser Ala Asn Lys Leu Ala
        195                 200                 205

aag ctg aag gga cac gcg gcg acg tac gcg tcg ctg atc atg gag gag     672
Lys Leu Lys Gly His Ala Ala Thr Tyr Ala Ser Leu Ile Met Glu Glu
    210                 215                 220

ctg gac ccg gac gac cgc ggg tac atc gag atc tgg cag ctg gag acg     720
Leu Asp Pro Asp Asp Arg Gly Tyr Ile Glu Ile Trp Gln Leu Glu Thr
225                 230                 235                 240

ctg ctg cgc ggc atg gtg agc gcg cag gcg gcg ccg gag aag atg aag     768
Leu Leu Arg Gly Met Val Ser Ala Gln Ala Ala Pro Glu Lys Met Lys
                245                 250                 255

cgg acg acg tcg agc ctc gcg agg acg atg atc ccg tcg cgg tac cgg     816
Arg Thr Thr Ser Ser Leu Ala Arg Thr Met Ile Pro Ser Arg Tyr Arg
            260                 265                 270

agc ccg ctg aag cgg cac gtg tcc agg acg gtg gac ttc gtg cac gag     864
Ser Pro Leu Lys Arg His Val Ser Arg Thr Val Asp Phe Val His Glu
        275                 280                 285

aac tgg aag cgg atc tgg ctc gtc gcg ctg tgg ctc gcc gtc aac gtc     912
Asn Trp Lys Arg Ile Trp Leu Val Ala Leu Trp Leu Ala Val Asn Val
    290                 295                 300

ggc ctc ttc gcc tac aag ttc gag cag tac gag cgg cgc gcc gcg ttc     960
Gly Leu Phe Ala Tyr Lys Phe Glu Gln Tyr Glu Arg Arg Ala Ala Phe
305                 310                 315                 320

cag gtg atg ggc cac tgc gtg tgc gtg gcc aag ggc gcc gcc gag gtg     1008
Gln Val Met Gly His Cys Val Cys Val Ala Lys Gly Ala Ala Glu Val
                325                 330                 335

ctc aag ctc aac atg gcg ctc atc ctc ctc ccc gtg tgc cgg aac acg     1056
Leu Lys Leu Asn Met Ala Leu Ile Leu Leu Pro Val Cys Arg Asn Thr
            340                 345                 350

ctc acc acg ctc agg tcc acg gcg ctc agc cac gtc atc ccc ttc gac     1104
Leu Thr Thr Leu Arg Ser Thr Ala Leu Ser His Val Ile Pro Phe Asp
        355                 360                 365

gac aac atc aac ttc cac aag gtg atc gcg gcg acc atc gcc gcc gcc     1152
Asp Asn Ile Asn Phe His Lys Val Ile Ala Ala Thr Ile Ala Ala Ala
    370                 375                 380

acc gcc gtc cac acg ctg gcg cac gtc acc tgc gac ttc ccg agg ctg     1200
Thr Ala Val His Thr Leu Ala His Val Thr Cys Asp Phe Pro Arg Leu
385                 390                 395                 400

atc aac tgc ccc agc gac aag ttc atg gcg acg ctg ggg ccg aac ttc     1248
Ile Asn Cys Pro Ser Asp Lys Phe Met Ala Thr Leu Gly Pro Asn Phe
                405                 410                 415

ggg tac agg cag ccg acg tac gcc gac ctg ctg gag agc gcc ccc ggc     1296
Gly Tyr Arg Gln Pro Thr Tyr Ala Asp Leu Leu Glu Ser Ala Pro Gly
            420                 425                 430
```

```
gtc acc ggc atc ctc atg atc atc atc atg tcc ttc tcc ttc acg ctg    1344
Val Thr Gly Ile Leu Met Ile Ile Ile Met Ser Phe Ser Phe Thr Leu
        435                 440                 445

gcc acg cac tcc ttc cgc cgg agc gtc gtc aag ctg ccg tcg ccg ctg    1392
Ala Thr His Ser Phe Arg Arg Ser Val Val Lys Leu Pro Ser Pro Leu
    450                 455                 460

cac cac ctc gcc ggc ttc aac gcc ttc tgg tac gcg cac cac ctc ctg    1440
His His Leu Ala Gly Phe Asn Ala Phe Trp Tyr Ala His His Leu Leu
465                 470                 475                 480

gtg ctc gcc tac gtc ctc ctc gtc gtg cac tcc tac ttc ata ttc ctc    1488
Val Leu Ala Tyr Val Leu Leu Val Val His Ser Tyr Phe Ile Phe Leu
                485                 490                 495

acc agg gag tgg tac aag aaa acg aca tgg atg tac ctg ata gtc cca    1536
Thr Arg Glu Trp Tyr Lys Lys Thr Thr Trp Met Tyr Leu Ile Val Pro
            500                 505                 510

gtg ctc ttc tat gca tgc gag aga acg atc aga aaa gtt cga gag aac    1584
Val Leu Phe Tyr Ala Cys Glu Arg Thr Ile Arg Lys Val Arg Glu Asn
        515                 520                 525

aac tac cgc gtg agc atc gtc aag gca gcg att tac cca gga aat gtg    1632
Asn Tyr Arg Val Ser Ile Val Lys Ala Ala Ile Tyr Pro Gly Asn Val
    530                 535                 540

ctc tct ctt cac atg aag aag ccg ccg ggt ttc aag tac aag agc ggg    1680
Leu Ser Leu His Met Lys Lys Pro Pro Gly Phe Lys Tyr Lys Ser Gly
545                 550                 555                 560

atg tac ctg ttt gtg aag tgc cct gat gtc tct cct ttc gaa tgg cat    1728
Met Tyr Leu Phe Val Lys Cys Pro Asp Val Ser Pro Phe Glu Trp His
                565                 570                 575

ccc ttc tcc atc act tct gca cct gga gat gac tac ctg agt gtg cat    1776
Pro Phe Ser Ile Thr Ser Ala Pro Gly Asp Asp Tyr Leu Ser Val His
            580                 585                 590

atc cgt aca cta ggt gac tgg acg act gaa ctc aga aac ctg ttt ggg    1824
Ile Arg Thr Leu Gly Asp Trp Thr Thr Glu Leu Arg Asn Leu Phe Gly
        595                 600                 605

aag gct tgc gag gca cag gtt act tct aag aag gct acc ctt tca aga    1872
Lys Ala Cys Glu Ala Gln Val Thr Ser Lys Lys Ala Thr Leu Ser Arg
    610                 615                 620

ctt gaa act aca gtt gtg gcg gac gct cag aca gag gat act agg ttt    1920
Leu Glu Thr Thr Val Val Ala Asp Ala Gln Thr Glu Asp Thr Arg Phe
625                 630                 635                 640

cct aag gtc ctt att gat ggg ccc tat ggt gca ccg gcg caa aac tac    1968
Pro Lys Val Leu Ile Asp Gly Pro Tyr Gly Ala Pro Ala Gln Asn Tyr
                645                 650                 655

aag aag tat gac att ctt ttg ctt att ggt ctt gga att ggt gct act    2016
Lys Lys Tyr Asp Ile Leu Leu Leu Ile Gly Leu Gly Ile Gly Ala Thr
            660                 665                 670

cct ttc atc agc att ctg aag gat ctg ttg aac aac att aaa tcc aac    2064
Pro Phe Ile Ser Ile Leu Lys Asp Leu Leu Asn Asn Ile Lys Ser Asn
        675                 680                 685

gaa gag gtg gaa agc ata cat ggt tct gag ata ggc agc ttc aag aac    2112
Glu Glu Val Glu Ser Ile His Gly Ser Glu Ile Gly Ser Phe Lys Asn
    690                 695                 700
```

```
aat ggg cca gga aga gct tac ttc tac tgg gtg acc aga gag caa ggg     2160
Asn Gly Pro Gly Arg Ala Tyr Phe Tyr Trp Val Thr Arg Glu Gln Gly
705                 710                 715                 720

tcc ttc gag tgg ttt aaa gga gtc atg aac gat gtc gct gaa agt gat     2208
Ser Phe Glu Trp Phe Lys Gly Val Met Asn Asp Val Ala Glu Ser Asp
                725                 730                 735

cac aat aat att ata gag atg cac aat tac ctg acc agc gtg tat gaa     2256
His Asn Asn Ile Ile Glu Met His Asn Tyr Leu Thr Ser Val Tyr Glu
            740                 745                 750

gaa ggc gac gca agg tca gct ttg att gcc atg gtt cag tca ctt caa     2304
Glu Gly Asp Ala Arg Ser Ala Leu Ile Ala Met Val Gln Ser Leu Gln
        755                 760                 765

cat gcc aaa aat ggt gtg gat atc gtc tcc ggc agc agg att cgc aca     2352
His Ala Lys Asn Gly Val Asp Ile Val Ser Gly Ser Arg Ile Arg Thr
    770                 775                 780

cat ttt gcg agg cct aac tgg aga aag gtg ttc tct gac ttg gcg aat     2400
His Phe Ala Arg Pro Asn Trp Arg Lys Val Phe Ser Asp Leu Ala Asn
785                 790                 795                 800

gcc cac aaa aac tca cgc ata ggt gtt ttc tat tgt gga tcc cct aca     2448
Ala His Lys Asn Ser Arg Ile Gly Val Phe Tyr Cys Gly Ser Pro Thr
                805                 810                 815

ctc acg aaa caa ctc aag gat ctt tca aaa gaa ttc agc cag aca acc     2496
Leu Thr Lys Gln Leu Lys Asp Leu Ser Lys Glu Phe Ser Gln Thr Thr
            820                 825                 830

aca act aga ttc cac ttc cac aag gaa aac ttt taa                     2532
Thr Thr Arg Phe His Phe His Lys Glu Asn Phe
        835                 840
```

<210> 18
<211> 843
<212> PRT
<213> Oryza sativa

<400> 18

```
Met Ala Ser Pro Tyr Asp His Gln Ser Pro His Ala Gln His Pro Ser
 1               5                  10                  15
Gly Leu Pro Arg Pro Pro Gly Ala Gly Ala Gly Ala Ala Ala Gly Gly
            20                  25                  30
Phe Ala Arg Gly Leu Met Lys Gln Pro Ser Arg Leu Ala Ser Gly Val
        35                  40                  45
Arg Gln Phe Ala Ser Arg Val Ser Met Lys Val Pro Glu Gly Val Gly
    50                  55                  60
Gly Met Arg Pro Gly Gly Gly Arg Met Thr Arg Met Gln Ser Ser Ala
65                  70                  75                  80
Gln Val Gly Leu Arg Gly Leu Arg Phe Leu Asp Lys Thr Ser Gly Gly
                85                  90                  95
Lys Glu Gly Trp Lys Ser Val Glu Arg Arg Phe Asp Glu Met Asn Arg
            100                 105                 110
Asn Gly Arg Leu Pro Lys Glu Ser Phe Gly Lys Cys Ile Gly Met Gly
        115                 120                 125
Asp Ser Lys Glu Phe Ala Gly Glu Leu Phe Val Ala Leu Ala Arg Arg
    130                 135                 140
```

Arg Asn Leu Glu Pro Glu Asp Gly Ile Thr Lys Glu Gln Leu Lys Glu
145 150 155 160

Phe Trp Glu Glu Met Thr Asp Gln Asn Phe Asp Ser Arg Leu Arg Ile
165 170 175

Phe Phe Asp Met Cys Asp Lys Asn Gly Asp Gly Met Leu Thr Glu Asp
180 185 190

Glu Val Lys Glu Val Ile Ile Leu Ser Ala Ser Ala Asn Lys Leu Ala
195 200 205

Lys Leu Lys Gly His Ala Ala Thr Tyr Ala Ser Leu Ile Met Glu Glu
210 215 220

Leu Asp Pro Asp Asp Arg Gly Tyr Ile Glu Ile Trp Gln Leu Glu Thr
225 230 235 240

Leu Leu Arg Gly Met Val Ser Ala Gln Ala Ala Pro Glu Lys Met Lys
245 250 255

Arg Thr Thr Ser Ser Leu Ala Arg Thr Met Ile Pro Ser Arg Tyr Arg
260 265 270

Ser Pro Leu Lys Arg His Val Ser Arg Thr Val Asp Phe Val His Glu
275 280 285

Asn Trp Lys Arg Ile Trp Leu Val Ala Leu Trp Leu Ala Val Asn Val
290 295 300

Gly Leu Phe Ala Tyr Lys Phe Glu Gln Tyr Glu Arg Arg Ala Ala Phe
305 310 315 320

Gln Val Met Gly His Cys Val Cys Val Ala Lys Gly Ala Ala Glu Val
325 330 335

Leu Lys Leu Asn Met Ala Leu Ile Leu Leu Pro Val Cys Arg Asn Thr
340 345 350

Leu Thr Thr Leu Arg Ser Thr Ala Leu Ser His Val Ile Pro Phe Asp
355 360 365

Asp Asn Ile Asn Phe His Lys Val Ile Ala Ala Thr Ile Ala Ala Ala
370 375 380

Thr Ala Val His Thr Leu Ala His Val Thr Cys Asp Phe Pro Arg Leu
385 390 395 400

Ile Asn Cys Pro Ser Asp Lys Phe Met Ala Thr Leu Gly Pro Asn Phe
405 410 415

Gly Tyr Arg Gln Pro Thr Tyr Ala Asp Leu Leu Glu Ser Ala Pro Gly
420 425 430

Val Thr Gly Ile Leu Met Ile Ile Ile Met Ser Phe Ser Phe Thr Leu
435 440 445

Ala Thr His Ser Phe Arg Arg Ser Val Val Lys Leu Pro Ser Pro Leu
450 455 460

His His Leu Ala Gly Phe Asn Ala Phe Trp Tyr Ala His His Leu Leu
465 470 475 480

Val Leu Ala Tyr Val Leu Leu Val Val His Ser Tyr Phe Ile Phe Leu
485 490 495

Thr Arg Glu Trp Tyr Lys Lys Thr Thr Trp Met Tyr Leu Ile Val Pro
500 505 510

Val Leu Phe Tyr Ala Cys Glu Arg Thr Ile Arg Lys Val Arg Glu Asn
515 520 525

```
Asn Tyr Arg Val Ser Ile Val Lys Ala Ala Ile Tyr Pro Gly Asn Val
    530                 535                 540
Leu Ser Leu His Met Lys Lys Pro Pro Gly Phe Lys Tyr Lys Ser Gly
545                 550                 555                 560
Met Tyr Leu Phe Val Lys Cys Pro Asp Val Ser Pro Phe Glu Trp His
                565                 570                 575
Pro Phe Ser Ile Thr Ser Ala Pro Gly Asp Asp Tyr Leu Ser Val His
            580                 585                 590
Ile Arg Thr Leu Gly Asp Trp Thr Thr Glu Leu Arg Asn Leu Phe Gly
        595                 600                 605
Lys Ala Cys Glu Ala Gln Val Thr Ser Lys Lys Ala Thr Leu Ser Arg
    610                 615                 620
Leu Glu Thr Thr Val Val Ala Asp Ala Gln Thr Glu Asp Thr Arg Phe
625                 630                 635                 640
Pro Lys Val Leu Ile Asp Gly Pro Tyr Gly Ala Pro Ala Gln Asn Tyr
                645                 650                 655
Lys Lys Tyr Asp Ile Leu Leu Leu Ile Gly Leu Gly Ile Gly Ala Thr
            660                 665                 670
Pro Phe Ile Ser Ile Leu Lys Asp Leu Leu Asn Asn Ile Lys Ser Asn
        675                 680                 685
Glu Glu Val Glu Ser Ile His Gly Ser Glu Ile Gly Ser Phe Lys Asn
    690                 695                 700
Asn Gly Pro Gly Arg Ala Tyr Phe Tyr Trp Val Thr Arg Glu Gln Gly
705                 710                 715                 720
Ser Phe Glu Trp Phe Lys Gly Val Met Asn Asp Val Ala Glu Ser Asp
                725                 730                 735
His Asn Asn Ile Ile Glu Met His Asn Tyr Leu Thr Ser Val Tyr Glu
            740                 745                 750
Glu Gly Asp Ala Arg Ser Ala Leu Ile Ala Met Val Gln Ser Leu Gln
        755                 760                 765
His Ala Lys Asn Gly Val Asp Ile Val Ser Gly Ser Arg Ile Arg Thr
    770                 775                 780
His Phe Ala Arg Pro Asn Trp Arg Lys Val Phe Ser Asp Leu Ala Asn
785                 790                 795                 800
Ala His Lys Asn Ser Arg Ile Gly Val Phe Tyr Cys Gly Ser Pro Thr
                805                 810                 815
Leu Thr Lys Gln Leu Lys Asp Leu Ser Lys Glu Phe Ser Gln Thr Thr
            820                 825                 830
Thr Thr Arg Phe His Phe His Lys Glu Asn Phe
        835                 840
```

<210> 19
<211> 2604
<212> DNA
<213> Arabidopsis thaliana

<220>

<221> CDS
<222> (1)..(2601)
<223> coding for NADPH-oxidase

<400> 19

atg tct aga gtg agt ttt gaa gtg tca gga ggc tat cac tct gat gca 48
Met Ser Arg Val Ser Phe Glu Val Ser Gly Gly Tyr His Ser Asp Ala
1 5 10 15

gaa gcc gga aac agc gga cca atg agc ggt ggt caa tta cca ccg atc 96
Glu Ala Gly Asn Ser Gly Pro Met Ser Gly Gly Gln Leu Pro Pro Ile
20 25 30

tat aaa aaa ccg ggg aac tcc aga ttc act gct gag aac agt cag aga 144
Tyr Lys Lys Pro Gly Asn Ser Arg Phe Thr Ala Glu Asn Ser Gln Arg
35 40 45

aca cgt acg gca cca tac gtg gac ctc acg gta gat gta caa gac gat 192
Thr Arg Thr Ala Pro Tyr Val Asp Leu Thr Val Asp Val Gln Asp Asp
50 55 60

aca gtc tct gta cat agc ttg aaa atg gaa ggt gga tct agc gtt gaa 240
Thr Val Ser Val His Ser Leu Lys Met Glu Gly Gly Ser Ser Val Glu
65 70 75 80

gag agt ccg gag ctt act ttg ctg aaa cga aac cgt ctt gag aag aaa 288
Glu Ser Pro Glu Leu Thr Leu Leu Lys Arg Asn Arg Leu Glu Lys Lys
85 90 95

aca acg gtg gtg aaa cgt ttg gcg tct gtt tct cac gag ctt aag cgt 336
Thr Thr Val Val Lys Arg Leu Ala Ser Val Ser His Glu Leu Lys Arg
100 105 110

ttg aca tct gtt tct ggt ggt att ggt gga aga aag ccg cct cga ccg 384
Leu Thr Ser Val Ser Gly Gly Ile Gly Gly Arg Lys Pro Pro Arg Pro
115 120 125

gct aag tta gac cgg act aaa tcc gcc gcg agt caa gcg ttg aag gga 432
Ala Lys Leu Asp Arg Thr Lys Ser Ala Ala Ser Gln Ala Leu Lys Gly
130 135 140

ctt aag ttc att agt aaa acc gac ggt ggc gcc ggt tgg tct gcc gtg 480
Leu Lys Phe Ile Ser Lys Thr Asp Gly Gly Ala Gly Trp Ser Ala Val
145 150 155 160

gag aag cgg ttt aat cag att acc gcg act acc ggt gga cta ctt ctt 528
Glu Lys Arg Phe Asn Gln Ile Thr Ala Thr Thr Gly Gly Leu Leu Leu
165 170 175

cgg aca aag ttc ggt gaa tgc ata gga atg act tca aag gat ttt gct 576
Arg Thr Lys Phe Gly Glu Cys Ile Gly Met Thr Ser Lys Asp Phe Ala
180 185 190

ttg gaa ctg ttt gat gca ttg gct aga aga agg aat ata aca ggg gaa 624
Leu Glu Leu Phe Asp Ala Leu Ala Arg Arg Arg Asn Ile Thr Gly Glu
195 200 205

gtg att gat gga gat caa cta aag gag ttt tgg gaa caa att aat gat 672
Val Ile Asp Gly Asp Gln Leu Lys Glu Phe Trp Glu Gln Ile Asn Asp
210 215 220

caa agt ttt gat tct cgg ctt aag aca ttc ttt gac atg gtg gat aaa 720
Gln Ser Phe Asp Ser Arg Leu Lys Thr Phe Phe Asp Met Val Asp Lys
225 230 235 240

gat gct gat ggt aga ctt aca gaa gac gaa gtt aga gag ttg gag agt 768
Asp Ala Asp Gly Arg Leu Thr Glu Asp Glu Val Arg Glu Leu Glu Ser
245 250 255

ctt gag act ctg ctt ttg caa gcg gca aca cag tct gtg ata aca agt 816
Leu Glu Thr Leu Leu Leu Gln Ala Ala Thr Gln Ser Val Ile Thr Ser
260 265 270

```
act ggg gag aga aag aat ctg agt cat atg atg agt cag agg ctt aag     864
Thr Gly Glu Arg Lys Asn Leu Ser His Met Met Ser Gln Arg Leu Lys
        275                 280                 285

cct acg ttt aac cgc aac ccg ttg aag cga tgg tac cgt ggt ctt aga     912
Pro Thr Phe Asn Arg Asn Pro Leu Lys Arg Trp Tyr Arg Gly Leu Arg
    290                 295                 300

ttc ttc ttg tta gac aac tgg caa aga tgt tgg gtt ata gtg cta tgg     960
Phe Phe Leu Leu Asp Asn Trp Gln Arg Cys Trp Val Ile Val Leu Trp
305                 310                 315                 320

ttc ata gtt atg gct ata ctc ttc acc tac aaa tat atc caa tac agg    1008
Phe Ile Val Met Ala Ile Leu Phe Thr Tyr Lys Tyr Ile Gln Tyr Arg
                325                 330                 335

cgt agc cct gtg tat cca gtg atg ggt gat tgt gtg tgc atg gct aaa    1056
Arg Ser Pro Val Tyr Pro Val Met Gly Asp Cys Val Cys Met Ala Lys
            340                 345                 350

ggt gct gca gaa aca gtg aag ctg aac atg gct ttg att ctc tta cct    1104
Gly Ala Ala Glu Thr Val Lys Leu Asn Met Ala Leu Ile Leu Leu Pro
        355                 360                 365

gtt tgt aga aac acc atc aca tgg ctt aga aat aag acc agg ttg ggt    1152
Val Cys Arg Asn Thr Ile Thr Trp Leu Arg Asn Lys Thr Arg Leu Gly
    370                 375                 380

cgt gtt gtc cca ttt gat gac aat ctc aac ttc cac aag gtt ata gcg    1200
Arg Val Val Pro Phe Asp Asp Asn Leu Asn Phe His Lys Val Ile Ala
385                 390                 395                 400

gtg ggg att ata gtt gga gta acg atg cac gcc ggg gca cat tta gcg    1248
Val Gly Ile Ile Val Gly Val Thr Met His Ala Gly Ala His Leu Ala
                405                 410                 415

tgt gat ttc ccg cgg tta cta cat gca act cca gag gca tat agg cct    1296
Cys Asp Phe Pro Arg Leu Leu His Ala Thr Pro Glu Ala Tyr Arg Pro
            420                 425                 430

tta aga cag ttt ttt ggg gat gag caa cca aag agc tac tgg cat ttt    1344
Leu Arg Gln Phe Phe Gly Asp Glu Gln Pro Lys Ser Tyr Trp His Phe
        435                 440                 445

gta aac tcg gta gaa ggt ata acc gga ctt gtg atg gtt ttg tta atg    1392
Val Asn Ser Val Glu Gly Ile Thr Gly Leu Val Met Val Leu Leu Met
    450                 455                 460

gcg att gca ttc aca cta gcc acg cct tgg ttc aga aga ggg aag cta    1440
Ala Ile Ala Phe Thr Leu Ala Thr Pro Trp Phe Arg Arg Gly Lys Leu
465                 470                 475                 480

aac tat ctt cca gga cca tta aag aaa cta gct agc ttc aat gcc ttc    1488
Asn Tyr Leu Pro Gly Pro Leu Lys Lys Leu Ala Ser Phe Asn Ala Phe
                485                 490                 495

tgg tac act cat cat ttg ttt gtc ata gtc tac att ctt ctt gtt gct    1536
Trp Tyr Thr His His Leu Phe Val Ile Val Tyr Ile Leu Leu Val Ala
            500                 505                 510

cat gga tac tac ttg tat ctc acc aga gac tgg cac aat aaa acg act    1584
His Gly Tyr Tyr Leu Tyr Leu Thr Arg Asp Trp His Asn Lys Thr Thr
        515                 520                 525

tgg atg tat ttg gtg gta cca gtg gtt cta tac gcg tgt gaa agg ttg    1632
Trp Met Tyr Leu Val Val Pro Val Val Leu Tyr Ala Cys Glu Arg Leu
    530                 535                 540
```

ata aga gca ttc agg tcg agc atc aag gcg gtg act att agg aaa gta 1680
Ile Arg Ala Phe Arg Ser Ser Ile Lys Ala Val Thr Ile Arg Lys Val
545 550 555 560

gca gtt tat cca gga aac gtg ctg gca att cac ttg tca agg cct caa 1728
Ala Val Tyr Pro Gly Asn Val Leu Ala Ile His Leu Ser Arg Pro Gln
565 570 575

aac ttc aaa tac aag agt ggt caa tac atg ttt gtt aac tgt gct gct 1776
Asn Phe Lys Tyr Lys Ser Gly Gln Tyr Met Phe Val Asn Cys Ala Ala
580 585 590

gtt tct cca ttt gaa tgg cat cca ttt tca atc aca tct gca cca caa 1824
Val Ser Pro Phe Glu Trp His Pro Phe Ser Ile Thr Ser Ala Pro Gln
595 600 605

gat gat tac cta agt gtt cac att aga gtt ctt ggg gat tgg aca cga 1872
Asp Asp Tyr Leu Ser Val His Ile Arg Val Leu Gly Asp Trp Thr Arg
610 615 620

gct ctc aaa gga gtc ttc tct gag gtg tgt aag cca cca ccg gca gga 1920
Ala Leu Lys Gly Val Phe Ser Glu Val Cys Lys Pro Pro Pro Ala Gly
625 630 635 640

gtt agt ggt ctg ctt aga gcc gac atg ttg cat ggt gca aat aat ccc 1968
Val Ser Gly Leu Leu Arg Ala Asp Met Leu His Gly Ala Asn Asn Pro
645 650 655

gac ttc ccg aaa gtc ttg att gat ggt cca tat ggt gca cca gca caa 2016
Asp Phe Pro Lys Val Leu Ile Asp Gly Pro Tyr Gly Ala Pro Ala Gln
660 665 670

gac tac aag aag tac gag gtg gtt cta cta gtt ggt ctc ggg att gga 2064
Asp Tyr Lys Lys Tyr Glu Val Val Leu Leu Val Gly Leu Gly Ile Gly
675 680 685

gcc aca cca atg atc agt atc gtc aaa gac att gtt aat aac atc aag 2112
Ala Thr Pro Met Ile Ser Ile Val Lys Asp Ile Val Asn Asn Ile Lys
690 695 700

gcc aag gaa caa gcc caa cta aac cga atg gag aat gga aca agc gaa 2160
Ala Lys Glu Gln Ala Gln Leu Asn Arg Met Glu Asn Gly Thr Ser Glu
705 710 715 720

cca caa cga agt aag aaa gag agt ttc agg acc cgt aga gct tac ttc 2208
Pro Gln Arg Ser Lys Lys Glu Ser Phe Arg Thr Arg Arg Ala Tyr Phe
725 730 735

tat tgg gtt acg cgt gag caa ggc tct ttc gat tgg ttc aag aac ata 2256
Tyr Trp Val Thr Arg Glu Gln Gly Ser Phe Asp Trp Phe Lys Asn Ile
740 745 750

atg aac gaa gtc gcg gaa cga gat gcc aac cgc gtc atc gaa atg cat 2304
Met Asn Glu Val Ala Glu Arg Asp Ala Asn Arg Val Ile Glu Met His
755 760 765

aac tat tgt aca agt gtc tat gaa gaa ggt gac gct cgt tcc gca ctt 2352
Asn Tyr Cys Thr Ser Val Tyr Glu Glu Gly Asp Ala Arg Ser Ala Leu
770 775 780

ata cat atg ctt caa tca cta aac cat gca aag aac ggc gtc gac att 2400
Ile His Met Leu Gln Ser Leu Asn His Ala Lys Asn Gly Val Asp Ile
785 790 795 800

gtc tct gga aca aga gtt atg tcc cat ttc gct aaa cct aat tgg aga 2448
Val Ser Gly Thr Arg Val Met Ser His Phe Ala Lys Pro Asn Trp Arg
805 810 815

```
aat gtt tac aag cgt ata gct atg gat cat cct aac acc aaa gtt gga     2496
Asn Val Tyr Lys Arg Ile Ala Met Asp His Pro Asn Thr Lys Val Gly
            820                 825                 830

gtg ttt tac tgt gga gca cca gca ttg aca aag gag cta agg cat cta     2544
Val Phe Tyr Cys Gly Ala Pro Ala Leu Thr Lys Glu Leu Arg His Leu
        835                 840                 845

gct tta gat ttc acc cac aaa aca agc acc aga ttc tcc ttc cac aaa     2592
Ala Leu Asp Phe Thr His Lys Thr Ser Thr Arg Phe Ser Phe His Lys
    850                 855                 860

gag aat ttc taa                                                     2604
Glu Asn Phe
865
```

<210> 20
<211> 867
<212> PRT
<213> Arabidopsis thaliana

<400> 20

```
Met Ser Arg Val Ser Phe Glu Val Ser Gly Gly Tyr His Ser Asp Ala
 1               5                  10                  15

Glu Ala Gly Asn Ser Gly Pro Met Ser Gly Gly Gln Leu Pro Pro Ile
            20                  25                  30

Tyr Lys Lys Pro Gly Asn Ser Arg Phe Thr Ala Glu Asn Ser Gln Arg
        35                  40                  45

Thr Arg Thr Ala Pro Tyr Val Asp Leu Thr Val Asp Val Gln Asp Asp
    50                  55                  60

Thr Val Ser Val His Ser Leu Lys Met Glu Gly Gly Ser Ser Val Glu
65                  70                  75                  80

Glu Ser Pro Glu Leu Thr Leu Leu Lys Arg Asn Arg Leu Glu Lys Lys
                85                  90                  95

Thr Thr Val Val Lys Arg Leu Ala Ser Val Ser His Glu Leu Lys Arg
            100                 105                 110

Leu Thr Ser Val Ser Gly Gly Ile Gly Gly Arg Lys Pro Pro Arg Pro
        115                 120                 125

Ala Lys Leu Asp Arg Thr Lys Ser Ala Ala Ser Gln Ala Leu Lys Gly
    130                 135                 140

Leu Lys Phe Ile Ser Lys Thr Asp Gly Gly Ala Gly Trp Ser Ala Val
145                 150                 155                 160

Glu Lys Arg Phe Asn Gln Ile Thr Ala Thr Thr Gly Gly Leu Leu Leu
                165                 170                 175

Arg Thr Lys Phe Gly Glu Cys Ile Gly Met Thr Ser Lys Asp Phe Ala
            180                 185                 190

Leu Glu Leu Phe Asp Ala Leu Ala Arg Arg Arg Asn Ile Thr Gly Glu
        195                 200                 205

Val Ile Asp Gly Asp Gln Leu Lys Glu Phe Trp Glu Gln Ile Asn Asp
    210                 215                 220

Gln Ser Phe Asp Ser Arg Leu Lys Thr Phe Phe Asp Met Val Asp Lys
225                 230                 235                 240

Asp Ala Asp Gly Arg Leu Thr Glu Asp Glu Val Arg Glu Leu Glu Ser
                245                 250                 255
```

Leu Glu Thr Leu Leu Leu Gln Ala Ala Thr Gln Ser Val Ile Thr Ser
260 265 270

Thr Gly Glu Arg Lys Asn Leu Ser His Met Met Ser Gln Arg Leu Lys
275 280 285

Pro Thr Phe Asn Arg Asn Pro Leu Lys Arg Trp Tyr Arg Gly Leu Arg
290 295 300

Phe Phe Leu Leu Asp Asn Trp Gln Arg Cys Trp Val Ile Val Leu Trp
305 310 315 320

Phe Ile Val Met Ala Ile Leu Phe Thr Tyr Lys Tyr Ile Gln Tyr Arg
325 330 335

Arg Ser Pro Val Tyr Pro Val Met Gly Asp Cys Val Cys Met Ala Lys
340 345 350

Gly Ala Ala Glu Thr Val Lys Leu Asn Met Ala Leu Ile Leu Leu Pro
355 360 365

Val Cys Arg Asn Thr Ile Thr Trp Leu Arg Asn Lys Thr Arg Leu Gly
370 375 380

Arg Val Val Pro Phe Asp Asp Asn Leu Asn Phe His Lys Val Ile Ala
385 390 395 400

Val Gly Ile Ile Val Gly Val Thr Met His Ala Gly Ala His Leu Ala
405 410 415

Cys Asp Phe Pro Arg Leu Leu His Ala Thr Pro Glu Ala Tyr Arg Pro
420 425 430

Leu Arg Gln Phe Phe Gly Asp Glu Gln Pro Lys Ser Tyr Trp His Phe
435 440 445

Val Asn Ser Val Glu Gly Ile Thr Gly Leu Val Met Val Leu Leu Met
450 455 460

Ala Ile Ala Phe Thr Leu Ala Thr Pro Trp Phe Arg Arg Gly Lys Leu
465 470 475 480

Asn Tyr Leu Pro Gly Pro Leu Lys Lys Leu Ala Ser Phe Asn Ala Phe
485 490 495

Trp Tyr Thr His His Leu Phe Val Ile Val Tyr Ile Leu Leu Val Ala
500 505 510

His Gly Tyr Tyr Leu Tyr Leu Thr Arg Asp Trp His Asn Lys Thr Thr
515 520 525

Trp Met Tyr Leu Val Val Pro Val Val Leu Tyr Ala Cys Glu Arg Leu
530 535 540

Ile Arg Ala Phe Arg Ser Ser Ile Lys Ala Val Thr Ile Arg Lys Val
545 550 555 560

Ala Val Tyr Pro Gly Asn Val Leu Ala Ile His Leu Ser Arg Pro Gln
565 570 575

Asn Phe Lys Tyr Lys Ser Gly Gln Tyr Met Phe Val Asn Cys Ala Ala
580 585 590

Val Ser Pro Phe Glu Trp His Pro Phe Ser Ile Thr Ser Ala Pro Gln
595 600 605

Asp Asp Tyr Leu Ser Val His Ile Arg Val Leu Gly Asp Trp Thr Arg
610 615 620

Ala Leu Lys Gly Val Phe Ser Glu Val Cys Lys Pro Pro Pro Ala Gly
625 630 635 640

```
Val Ser Gly Leu Leu Arg Ala Asp Met Leu His Gly Ala Asn Asn Pro
                645                 650                 655

Asp Phe Pro Lys Val Leu Ile Asp Gly Pro Tyr Gly Ala Pro Ala Gln
            660                 665                 670

Asp Tyr Lys Lys Tyr Glu Val Val Leu Leu Val Gly Leu Gly Ile Gly
        675                 680                 685

Ala Thr Pro Met Ile Ser Ile Val Lys Asp Ile Val Asn Asn Ile Lys
    690                 695                 700

Ala Lys Glu Gln Ala Gln Leu Asn Arg Met Glu Asn Gly Thr Ser Glu
705                 710                 715                 720

Pro Gln Arg Ser Lys Lys Glu Ser Phe Arg Thr Arg Arg Ala Tyr Phe
                725                 730                 735

Tyr Trp Val Thr Arg Glu Gln Gly Ser Phe Asp Trp Phe Lys Asn Ile
            740                 745                 750

Met Asn Glu Val Ala Glu Arg Asp Ala Asn Arg Val Ile Glu Met His
        755                 760                 765

Asn Tyr Cys Thr Ser Val Tyr Glu Glu Gly Asp Ala Arg Ser Ala Leu
    770                 775                 780

Ile His Met Leu Gln Ser Leu Asn His Ala Lys Asn Gly Val Asp Ile
785                 790                 795                 800

Val Ser Gly Thr Arg Val Met Ser His Phe Ala Lys Pro Asn Trp Arg
                805                 810                 815

Asn Val Tyr Lys Arg Ile Ala Met Asp His Pro Asn Thr Lys Val Gly
            820                 825                 830

Val Phe Tyr Cys Gly Ala Pro Ala Leu Thr Lys Glu Leu Arg His Leu
        835                 840                 845

Ala Leu Asp Phe Thr His Lys Thr Ser Thr Arg Phe Ser Phe His Lys
    850                 855                 860

Glu Asn Phe
865
```

<210> 21
<211> 2709
<212> DNA
<213> Arabidopsis thaliana

<220>
<221> CDS
<222> (1)..(2706)
<223> coding for NADPH-oxidase

<400> 21

```
atg atg aat cga agt gaa atg caa aag tta ggt ttc gaa cac gtg aga    48
Met Met Asn Arg Ser Glu Met Gln Lys Leu Gly Phe Glu His Val Arg
  1               5                  10                  15

tac tac aca gag tcg ccg tac aac aga gga gag tcc tcg gcg aac gtg    96
Tyr Tyr Thr Glu Ser Pro Tyr Asn Arg Gly Glu Ser Ser Ala Asn Val
             20                  25                  30

gcg acg aca agc aac tat tac ggt gaa gat gaa cca tac gtg gag atc    144
Ala Thr Thr Ser Asn Tyr Tyr Gly Glu Asp Glu Pro Tyr Val Glu Ile
         35                  40                  45
```

acg cta gat atc cac gac gat tcc gtc tcc gtg tac ggc ttg aag tca 192
Thr Leu Asp Ile His Asp Asp Ser Val Ser Val Tyr Gly Leu Lys Ser
50 55 60

ccg aac cat cga ggg gcc ggg tct aat tat gag gat caa tcg ctt ctc 240
Pro Asn His Arg Gly Ala Gly Ser Asn Tyr Glu Asp Gln Ser Leu Leu
65 70 75 80

aga caa ggt cgt tca ggg agg agt aac tcg gta ttg aaa cgc ttg gct 288
Arg Gln Gly Arg Ser Gly Arg Ser Asn Ser Val Leu Lys Arg Leu Ala
85 90 95

tct tct gtt tcc acc gga ata aca cga gtt gct tct tct gtt tct tcg 336
Ser Ser Val Ser Thr Gly Ile Thr Arg Val Ala Ser Ser Val Ser Ser
100 105 110

tct tcc gcg aga aaa cca ccg cgg ccg cag ctg gct aag ctg cgc cgt 384
Ser Ser Ala Arg Lys Pro Pro Arg Pro Gln Leu Ala Lys Leu Arg Arg
115 120 125

tcg aaa tct aga gca gag cta gct ctc aaa ggt ctt aaa ttc atc acc 432
Ser Lys Ser Arg Ala Glu Leu Ala Leu Lys Gly Leu Lys Phe Ile Thr
130 135 140

aag act gat ggt gtc act ggt tgg cct gaa gtt gag aaa cgg ttt tat 480
Lys Thr Asp Gly Val Thr Gly Trp Pro Glu Val Glu Lys Arg Phe Tyr
145 150 155 160

gtg atg aca atg act aat aac gga tta tta cac cga tcc aga ttc ggt 528
Val Met Thr Met Thr Asn Asn Gly Leu Leu His Arg Ser Arg Phe Gly
165 170 175

gaa tgt ata ggg atg aaa tcg acg gag ttt gcg ttg gca ttg ttc gat 576
Glu Cys Ile Gly Met Lys Ser Thr Glu Phe Ala Leu Ala Leu Phe Asp
180 185 190

gct tta gcg agg agg gaa aac gta agc gga gat tca ata aac atg aat 624
Ala Leu Ala Arg Arg Glu Asn Val Ser Gly Asp Ser Ile Asn Met Asn
195 200 205

gag ctt aaa gag ttc tgg aag cag atc act gat caa gat ttt gat tca 672
Glu Leu Lys Glu Phe Trp Lys Gln Ile Thr Asp Gln Asp Phe Asp Ser
210 215 220

agg cta cga act ttc ttc gcc atg gtc gat aag gat tcg gat ggg cgg 720
Arg Leu Arg Thr Phe Phe Ala Met Val Asp Lys Asp Ser Asp Gly Arg
225 230 235 240

ttg aat gaa gcc gaa gta aga gag att ata act tta agt gct tct gca 768
Leu Asn Glu Ala Glu Val Arg Glu Ile Ile Thr Leu Ser Ala Ser Ala
245 250 255

aac gag ttg gat aac att cgg aga caa gct gat gaa tat gct gct ttg 816
Asn Glu Leu Asp Asn Ile Arg Arg Gln Ala Asp Glu Tyr Ala Ala Leu
260 265 270

att atg gaa gaa ctc gat cct tat cat tat gga tac atc atg ata gag 864
Ile Met Glu Glu Leu Asp Pro Tyr His Tyr Gly Tyr Ile Met Ile Glu
275 280 285

aat ctc gag ata ctt cta ttg caa gcg ccg atg cag gat gtg aga gat 912
Asn Leu Glu Ile Leu Leu Leu Gln Ala Pro Met Gln Asp Val Arg Asp
290 295 300

gga gag agt aag aag cta agc aag atg cta agt cag aat ctc atg gtt 960
Gly Glu Ser Lys Lys Leu Ser Lys Met Leu Ser Gln Asn Leu Met Val
305 310 315 320

```
ccg cag agt agg aat ctc ggg gca cgt ttt tgc aga ggg atg aag tat      1008
Pro Gln Ser Arg Asn Leu Gly Ala Arg Phe Cys Arg Gly Met Lys Tyr
                325                 330                 335

ttt ttg ttt gat aat tgg aag aga gtg tgg gtg atg gct cta tgg ata      1056
Phe Leu Phe Asp Asn Trp Lys Arg Val Trp Val Met Ala Leu Trp Ile
            340                 345                 350

ggt gct atg gcg ggt ttg ttc acg tgg aag ttt atg gag tat cga aaa      1104
Gly Ala Met Ala Gly Leu Phe Thr Trp Lys Phe Met Glu Tyr Arg Lys
        355                 360                 365

aga tcc gct tac gaa gtc atg gga gtt tgt gtt tgt ata gct aaa gga      1152
Arg Ser Ala Tyr Glu Val Met Gly Val Cys Val Cys Ile Ala Lys Gly
    370                 375                 380

gct gca gag acg ctt aaa cta aac atg gct atg att ttg tta cca gtt      1200
Ala Ala Glu Thr Leu Lys Leu Asn Met Ala Met Ile Leu Leu Pro Val
385                 390                 395                 400

tgt agg aac acc atc act tgg ctg cgg acc aaa acc aag tta agt gct      1248
Cys Arg Asn Thr Ile Thr Trp Leu Arg Thr Lys Thr Lys Leu Ser Ala
                405                 410                 415

att gtt cct ttc gat gac agc ctc aat ttt cac aag gtc ata gct ata      1296
Ile Val Pro Phe Asp Asp Ser Leu Asn Phe His Lys Val Ile Ala Ile
            420                 425                 430

gga att tca gtt gga gtt gga atc cat gct aca tct cac tta gca tgt      1344
Gly Ile Ser Val Gly Val Gly Ile His Ala Thr Ser His Leu Ala Cys
        435                 440                 445

gat ttc ccc cga ctg ata gct gca gac gaa gat cag tat gag cca atg      1392
Asp Phe Pro Arg Leu Ile Ala Ala Asp Glu Asp Gln Tyr Glu Pro Met
    450                 455                 460

gag aag tat ttt ggg cca cag aca aag aga tat ttg gac ttt gtt caa      1440
Glu Lys Tyr Phe Gly Pro Gln Thr Lys Arg Tyr Leu Asp Phe Val Gln
465                 470                 475                 480

tcg gta gaa gga gtt acc ggg att gga atg gtt gta cta atg acc ata      1488
Ser Val Glu Gly Val Thr Gly Ile Gly Met Val Val Leu Met Thr Ile
                485                 490                 495

gcc ttt aca ttg gct aca aca tgg ttc aga cgt aat aag ctc aac ctt      1536
Ala Phe Thr Leu Ala Thr Thr Trp Phe Arg Arg Asn Lys Leu Asn Leu
            500                 505                 510

cct gga cca ctg aag aaa ata aca ggc ttc aat gcc ttc tgg tac tct      1584
Pro Gly Pro Leu Lys Lys Ile Thr Gly Phe Asn Ala Phe Trp Tyr Ser
        515                 520                 525

cac cac tta ttt gtt atc gtc tac tcg ctt ctt gtc gtt cat gga ttc      1632
His His Leu Phe Val Ile Val Tyr Ser Leu Leu Val Val His Gly Phe
    530                 535                 540

tac gta tac ctc atc atc gag cca tgg tac aag aaa acg aca tgg atg      1680
Tyr Val Tyr Leu Ile Ile Glu Pro Trp Tyr Lys Lys Thr Thr Trp Met
545                 550                 555                 560

tat ttg atg gta ccg gtg gtt ctt tac ttg tgt gaa agg ctg att cgt      1728
Tyr Leu Met Val Pro Val Val Leu Tyr Leu Cys Glu Arg Leu Ile Arg
                565                 570                 575

gca ttc agg tca agc gtc gag gct gtt tca gtg cta aag gtt gct gtg      1776
Ala Phe Arg Ser Ser Val Glu Ala Val Ser Val Leu Lys Val Ala Val
            580                 585                 590
```

tta cca ggg aat gtc ttg tcg ctt cac ttg tca aga cca agc aac ttc 1824
Leu Pro Gly Asn Val Leu Ser Leu His Leu Ser Arg Pro Ser Asn Phe
595 600 605

aga tac aag agt gga caa tac atg tat ctc aac tgt tct gca gtt tct 1872
Arg Tyr Lys Ser Gly Gln Tyr Met Tyr Leu Asn Cys Ser Ala Val Ser
610 615 620

aca tta gaa tgg cat cca ttc tca att acc tca gct cca gga gat gac 1920
Thr Leu Glu Trp His Pro Phe Ser Ile Thr Ser Ala Pro Gly Asp Asp
625 630 635 640

tac ctc agt gtc cac atc agg gtt tta gga gac tgg act aag caa tta 1968
Tyr Leu Ser Val His Ile Arg Val Leu Gly Asp Trp Thr Lys Gln Leu
645 650 655

aga tca tta ttc tct gag gtg tgc aag cca cgc cct cct gat gaa cac 2016
Arg Ser Leu Phe Ser Glu Val Cys Lys Pro Arg Pro Pro Asp Glu His
660 665 670

aga ctg aac aga gcc gac tcg aag cac tgg gat tac atc cct gac ttt 2064
Arg Leu Asn Arg Ala Asp Ser Lys His Trp Asp Tyr Ile Pro Asp Phe
675 680 685

cca aga atc cta att gat ggt cca tat gga gca cca gca caa gac tac 2112
Pro Arg Ile Leu Ile Asp Gly Pro Tyr Gly Ala Pro Ala Gln Asp Tyr
690 695 700

aag aag ttt gaa gtt gtt ctg cta gtg ggt cta gga atc ggt gcc act 2160
Lys Lys Phe Glu Val Val Leu Leu Val Gly Leu Gly Ile Gly Ala Thr
705 710 715 720

ccg atg atc agc ata gtg agt gac ata atc aat aac ttg aaa ggc gtg 2208
Pro Met Ile Ser Ile Val Ser Asp Ile Ile Asn Asn Leu Lys Gly Val
725 730 735

gaa gaa ggc agt aac cga aga cag tca ccg atc cat aat atg gtc aca 2256
Glu Glu Gly Ser Asn Arg Arg Gln Ser Pro Ile His Asn Met Val Thr
740 745 750

cct cct gtt tct cca tca aga aaa agt gag acg ttc aga acc aag aga 2304
Pro Pro Val Ser Pro Ser Arg Lys Ser Glu Thr Phe Arg Thr Lys Arg
755 760 765

gct tac ttc tac tgg gtc aca aga gag cag ggg tcg ttt gac tgg ttc 2352
Ala Tyr Phe Tyr Trp Val Thr Arg Glu Gln Gly Ser Phe Asp Trp Phe
770 775 780

aag aac gtg atg gac gaa gtg act gaa aca gac cgc aaa aac gta att 2400
Lys Asn Val Met Asp Glu Val Thr Glu Thr Asp Arg Lys Asn Val Ile
785 790 795 800

gag ctg cat aat tac tgc acc agc gtt tac gag gaa ggg gac gcg agg 2448
Glu Leu His Asn Tyr Cys Thr Ser Val Tyr Glu Glu Gly Asp Ala Arg
805 810 815

tct gca ctt atc acg atg ctc cag tct cta aac cat gct aag cat gga 2496
Ser Ala Leu Ile Thr Met Leu Gln Ser Leu Asn His Ala Lys His Gly
820 825 830

gtg gac gtt gtg tca gga aca cgt gtc atg tcc cat ttc gct agg cca 2544
Val Asp Val Val Ser Gly Thr Arg Val Met Ser His Phe Ala Arg Pro
835 840 845

aac tgg aga agc gtt ttc aaa agg atc gct gtg aat cat cct aag act 2592
Asn Trp Arg Ser Val Phe Lys Arg Ile Ala Val Asn His Pro Lys Thr
850 855 860

```
aga gtc gga gtg ttt tat tgt gga gca gct ggg tta gtg aaa gag tta      2640
Arg Val Gly Val Phe Tyr Cys Gly Ala Ala Gly Leu Val Lys Glu Leu
865                 870                 875                 880

cga cac tta tca ctg gat ttc tct cat aag acc tcc acc aag ttc atc      2688
Arg His Leu Ser Leu Asp Phe Ser His Lys Thr Ser Thr Lys Phe Ile
                885                 890                 895

ttc cat aaa gag aat ttc taa                                          2709
Phe His Lys Glu Asn Phe
            900
```

<210> 22
<211> 902
<212> PRT
<213> Arabidopsis thaliana

<400> 22

Met Met Asn Arg Ser Glu Met Gln Lys Leu Gly Phe Glu His Val Arg
1 5 10 15

Tyr Tyr Thr Glu Ser Pro Tyr Asn Arg Gly Glu Ser Ser Ala Asn Val
20 25 30

Ala Thr Thr Ser Asn Tyr Tyr Gly Glu Asp Glu Pro Tyr Val Glu Ile
35 40 45

Thr Leu Asp Ile His Asp Asp Ser Val Ser Val Tyr Gly Leu Lys Ser
50 55 60

Pro Asn His Arg Gly Ala Gly Ser Asn Tyr Glu Asp Gln Ser Leu Leu
65 70 75 80

Arg Gln Gly Arg Ser Gly Arg Ser Asn Ser Val Leu Lys Arg Leu Ala
85 90 95

Ser Ser Val Ser Thr Gly Ile Thr Arg Val Ala Ser Ser Val Ser Ser
100 105 110

Ser Ser Ala Arg Lys Pro Pro Arg Pro Gln Leu Ala Lys Leu Arg Arg
115 120 125

Ser Lys Ser Arg Ala Glu Leu Ala Leu Lys Gly Leu Lys Phe Ile Thr
130 135 140

Lys Thr Asp Gly Val Thr Gly Trp Pro Glu Val Glu Lys Arg Phe Tyr
145 150 155 160

Val Met Thr Met Thr Asn Asn Gly Leu Leu His Arg Ser Arg Phe Gly
165 170 175

Glu Cys Ile Gly Met Lys Ser Thr Glu Phe Ala Leu Ala Leu Phe Asp
180 185 190

Ala Leu Ala Arg Arg Glu Asn Val Ser Gly Asp Ser Ile Asn Met Asn
195 200 205

Glu Leu Lys Glu Phe Trp Lys Gln Ile Thr Asp Gln Asp Phe Asp Ser
210 215 220

Arg Leu Arg Thr Phe Phe Ala Met Val Asp Lys Asp Ser Asp Gly Arg
225 230 235 240

Leu Asn Glu Ala Glu Val Arg Glu Ile Ile Thr Leu Ser Ala Ser Ala
245 250 255

Asn Glu Leu Asp Asn Ile Arg Arg Gln Ala Asp Glu Tyr Ala Ala Leu
260 265 270

```
Ile Met Glu Glu Leu Asp Pro Tyr His Tyr Gly Tyr Ile Met Ile Glu
        275                 280                 285
Asn Leu Glu Ile Leu Leu Leu Gln Ala Pro Met Gln Asp Val Arg Asp
    290                 295                 300
Gly Glu Ser Lys Lys Leu Ser Lys Met Leu Ser Gln Asn Leu Met Val
305                 310                 315                 320
Pro Gln Ser Arg Asn Leu Gly Ala Arg Phe Cys Arg Gly Met Lys Tyr
                325                 330                 335
Phe Leu Phe Asp Asn Trp Lys Arg Val Trp Val Met Ala Leu Trp Ile
                340                 345                 350
Gly Ala Met Ala Gly Leu Phe Thr Trp Lys Phe Met Glu Tyr Arg Lys
        355                 360                 365
Arg Ser Ala Tyr Glu Val Met Gly Val Cys Val Cys Ile Ala Lys Gly
    370                 375                 380
Ala Ala Glu Thr Leu Lys Leu Asn Met Ala Met Ile Leu Leu Pro Val
385                 390                 395                 400
Cys Arg Asn Thr Ile Thr Trp Leu Arg Thr Lys Thr Lys Leu Ser Ala
                405                 410                 415
Ile Val Pro Phe Asp Asp Ser Leu Asn Phe His Lys Val Ile Ala Ile
            420                 425                 430
Gly Ile Ser Val Gly Val Gly Ile His Ala Thr Ser His Leu Ala Cys
        435                 440                 445
Asp Phe Pro Arg Leu Ile Ala Ala Asp Glu Asp Gln Tyr Glu Pro Met
    450                 455                 460
Glu Lys Tyr Phe Gly Pro Gln Thr Lys Arg Tyr Leu Asp Phe Val Gln
465                 470                 475                 480
Ser Val Glu Gly Val Thr Gly Ile Gly Met Val Val Leu Met Thr Ile
                485                 490                 495
Ala Phe Thr Leu Ala Thr Thr Trp Phe Arg Arg Asn Lys Leu Asn Leu
            500                 505                 510
Pro Gly Pro Leu Lys Lys Ile Thr Gly Phe Asn Ala Phe Trp Tyr Ser
        515                 520                 525
His His Leu Phe Val Ile Val Tyr Ser Leu Leu Val Val His Gly Phe
    530                 535                 540
Tyr Val Tyr Leu Ile Ile Glu Pro Trp Tyr Lys Lys Thr Thr Trp Met
545                 550                 555                 560
Tyr Leu Met Val Pro Val Val Leu Tyr Leu Cys Glu Arg Leu Ile Arg
                565                 570                 575
Ala Phe Arg Ser Ser Val Glu Ala Val Ser Val Leu Lys Val Ala Val
            580                 585                 590
Leu Pro Gly Asn Val Leu Ser Leu His Leu Ser Arg Pro Ser Asn Phe
        595                 600                 605
Arg Tyr Lys Ser Gly Gln Tyr Met Tyr Leu Asn Cys Ser Ala Val Ser
    610                 615                 620
Thr Leu Glu Trp His Pro Phe Ser Ile Thr Ser Ala Pro Gly Asp Asp
625                 630                 635                 640
Tyr Leu Ser Val His Ile Arg Val Leu Gly Asp Trp Thr Lys Gln Leu
                645                 650                 655
```

```
Arg Ser Leu Phe Ser Glu Val Cys Lys Pro Arg Pro Pro Asp Glu His
            660                 665                 670

Arg Leu Asn Arg Ala Asp Ser Lys His Trp Asp Tyr Ile Pro Asp Phe
        675                 680                 685

Pro Arg Ile Leu Ile Asp Gly Pro Tyr Gly Ala Pro Ala Gln Asp Tyr
    690                 695                 700

Lys Lys Phe Glu Val Val Leu Leu Val Gly Leu Gly Ile Gly Ala Thr
705                 710                 715                 720

Pro Met Ile Ser Ile Val Ser Asp Ile Ile Asn Asn Leu Lys Gly Val
                725                 730                 735

Glu Glu Gly Ser Asn Arg Arg Gln Ser Pro Ile His Asn Met Val Thr
            740                 745                 750

Pro Pro Val Ser Pro Ser Arg Lys Ser Glu Thr Phe Arg Thr Lys Arg
        755                 760                 765

Ala Tyr Phe Tyr Trp Val Thr Arg Glu Gln Gly Ser Phe Asp Trp Phe
    770                 775                 780

Lys Asn Val Met Asp Glu Val Thr Glu Thr Asp Arg Lys Asn Val Ile
785                 790                 795                 800

Glu Leu His Asn Tyr Cys Thr Ser Val Tyr Glu Glu Gly Asp Ala Arg
                805                 810                 815

Ser Ala Leu Ile Thr Met Leu Gln Ser Leu Asn His Ala Lys His Gly
            820                 825                 830

Val Asp Val Val Ser Gly Thr Arg Val Met Ser His Phe Ala Arg Pro
        835                 840                 845

Asn Trp Arg Ser Val Phe Lys Arg Ile Ala Val Asn His Pro Lys Thr
    850                 855                 860

Arg Val Gly Val Phe Tyr Cys Gly Ala Ala Gly Leu Val Lys Glu Leu
865                 870                 875                 880

Arg His Leu Ser Leu Asp Phe Ser His Lys Thr Ser Thr Lys Phe Ile
                885                 890                 895

Phe His Lys Glu Asn Phe
            900
```

<210> 23
<211> 20
<212> DNA
<213> Künstliche Sequenz

<220>
<223> Beschreibung der künstlichen Sequenz: oligonucleotide primer

<400> 23
garcaaggct cttttgattg 20

<210> 24
<211> 21
<212> DNA
<213> Künstliche Sequenz

<220>
<223> Beschreibung der künstlichen Sequenz: oligonucleotide primer

<400> 24
gaaatgctcc ttatggaatt c 21

## Beschreibung

Die Erfindung betrifft Verfahren zur Erzeugung oder Erhöhung einer Resistenz gegen ein Pilzpathogen oder ein pilzähnliches Pathogen in monokotylen Pflanzen durch Verminderung der Expression, Aktivität oder Funktion einer NADPH Oxidase.

Ziel biotechnologischer Arbeiten an Pflanzen ist die Herstellung von Pflanzen mit vorteilhaften, neuen Eigenschaften zum Beispiel zur Steigerung der landwirtschaftlichen Produktivität. Oft sind die natürlichen Abwehrmechanismen der Pflanze gegen Pathogene unzureichend. Allein Pilzerkrankungen führen zu Ernteverlusten in der Höhe von vielen Milliarden US-$ jährlich. Die Einführung fremder Gene aus Pflanzen, Tieren oder mikrobiellen Quellen kann die Abwehr verstärken. Beispiele sind der Schutz gegen Insektenfrass durch Expression von *Bacillus thuringiensis* Endotoxinen (Vaeck et al. (1987) Nature 328:33-37) oder der Schutz gegen Pilzbefall durch Expression einer Chitinase aus Bohne (Broglie et al. (1991) Science 254:1194-1197). Die meisten der beschriebenen Ansätze gewähren jedoch nur eine Resistenz gegen ein einzelnes Pathogen oder gegen ein schmales Spektrum von Pathogenen.

Es gibt nur wenige Ansätze, die Pflanzen eine Resistenz gegen ein breiteres Spektrum von Pathogenen verleihen. Die systemische erworbene Resistenz ("systemic acquired resistance"; SAR) - ein Abwehrmechanismus bei verschiedenen Pflanze/Pathogen-Interaktionen - kann durch Applikation von endogene Botenstoffe wie Jasmonsäure (JA) oder Salicylsäure (SA) vermittelt werden (Ward et al. (1991) Plant Cell 3:1085-1094; Uknes et al. (1992) Plant Cell 4 (6) : 645-656). Ähnliche Effekte können auch durch synthetische Verbindungen wie 2,6-Dichlorisonikotinsäure (INA) oder Benzo(1,2,3)thiadiazol-7-thiocarbonsäure-S-methylester (BTH; Bionr) bewirkt werden (Friedrich et al. (1996) Plant J 10(1):61-70; Lawton et al. (1996) Plant J 10:71-82). Auch die Expression der im Rahmen eines SAR hochregulierten "pathogenesis related" (PR) Proteine vermag zum Teil eine Pathogenresistenz zu bewirken.

In Gerste ist der Mlo-Locus als negativer Regulator der Pathogenabwehr beschrieben. Der Verlust des Mlo-Gens bedingt eine erhöhte und rassen-unspezifische Resistenz gegen zahlreiche Arten von Mehltau (Büschges R et al. (1997) Cell 88:695-705; Jorgensen JH (1977) Euphytica 26:55-62; Lyngkjaer MF et al. (1995) Plant verwendet. Vermutlich aufgrund der Rezessivität hat sich trotz eines intensiven Anbaus die Resistenz als dauerhaft erwiesen. Mlo-ähnliche Resistenzen in anderen Pflanzen v.a. in Getreidearten sind nicht beschrieben. Das Mlo-Gen und verschiedene Homologe aus anderen Getreidearten wurde identifiziert und kloniert (Büschges R et al. (1997) Cell 88:695-705; WO 98/04586; Schulze-Lefert P, Vogel J (2000) Trends Plant Sci. 5:343-348). Verschiedene Verfahren unter Verwendung dieser Gene zum Erzielen einer Pathogenresistenz sind beschrieben (WO 98/04586; WO 00/01722; WO 99/47552). Nachteilig ist, dass der Mlo-vermittelte Abwehrmechanismus ein spontanes Absterben von Blattzellen umfasst (Wolter M et al. (1993) Mol Gen Genet 239:122-128). Nachteilig ist ferner, dass die Mlo-defizienten Genotypen eine Hypersuszeptibilität gegen hemibiotrophe Pathogene wie *Magnaporte grisea* (*M. grisea*) sowie *Cochliobolus sativus* (Bipolaris sorokiniana) zeigen (Jarosch B et al. (1999) Mol Plant Microbe Interact 12:508-514; Kumar J et al. (2001) Phytopathology 91:127-133).

Die Freisetzung reaktiver Sauerstoffspezies (ROS; z.B. Superoxid ($O_2$-), Hydroxylradikale und $H_2O_2$) wird eine wichtige protektive Funktion in der Reaktion auf pflanzliche Pathogene zugeordnet (Wojtaszek P (1997) Biochem J 322: 681-692). Es sind verschiedene Wege bekannt, wie eine Zelle ROS zu produzieren vermag. In den Makrophagen von Säugetieren ist hier insbesondere die NADPH Oxidase zu nennen, die Elektronen auf molekularen Sauerstoff zu übertragen vermag. Homologe Enzyme wurden auch in Pflanzen identifiziert (Lamb & Dixon (1997) Annu Rev Plant Physiol Plant Mol Biol 48:251).

Es wurde gezeigt, dass Mutationen in der katalytischen Untereinheit der NADPH-Oxidase in Arabidopsis thaliana eine verminderte Akkumulation reaktiver Sauerstoffintermediate (ROI) zeigen. In Bezug auf die Hypersensitive Reaktion (HR) war das Bild uneinheitlich: Bei einer Doppelmutante wurde bei Infektion mit dem avirulenten Pseudomonas syringae Bakterium eine verminderte HR gefunden, während mit dem virulenten Oomyceten Peronospora parasitica eine erhöhte HR detektiert wurde. Das Wachstum - sowohl von virulenten als auch von avirulenten P.syringae Stämmen - war jedoch - im Vergleich zu Wildtyp-Pflanzen - nicht verändert (Torres MA et al. (2002) Proc Natl Acad Sci USA 99: 517-522). Ebenso hatte die Inhibition der NADPH-Oxidase mittels des Inhibitors Diphenyleniodoniumchlorid (DPI) - bei Einsatz physiologisch relevanter Konzentrationen - keinen Effekt auf die Entwicklung pathogener Pilze (Hückelhoven R & Kogel KH (1998) Mol Plant Microbe Interact 11;292-300). Ein cDNA Fragment einer Phagozyten NADPH-Oxidase aus Gerste (*pNAox*, Homolog der großen Untereinheit *gp91phox* einer Phagozyten NADPHoxidase) ist unter der Gen-Bank Acc.-No.: AJ251717) beschrieben.

Der vorliegenden Erfindung liegt die Aufgabe zugrunde, neue Verfahren zur Pathogenabwehr in Pflanzen bereitzustellen, die eine effiziente Abwehr eines möglichst breiten Spektrum von Pathogenen in möglichst vielen verschiedene Pflanzenarten, bevorzugt den in der Landwirtschaft verwendeten Kulturpflanzen bewirken. Diese Aufgabe wird durch das erfindungsgemäße Verfahren gelöst.

Ein erster Gegenstand der Erfindung umfasst ein Verfahren zur Erzeugung oder Erhöhung der Resistenz gegen mindestens ein Pilz-Pathogen oder pilzähnliches Pathogen in monokotylen Pflanzen, dadurch gekennzeichnet, dass nachfolgende Arbeitsschritte umfasst sind

a) Verminderung der Proteinmenge, Aktivität oder Funktion einer NADPH-Oxidase in einer Pflanze oder einem Gewebe, Organ, Teil oder Zelle derselben und

b) Auswahl der Pflanzen, bei denen - im Unterschied oder Vergleich zur Ausgangspflanze - die Resistenz gegen mindestens ein Pathogen besteht oder erhöht ist.

Überraschenderweise zeigt die Verminderung der Expression einer NADPH-Oxidase aus Gerste (pNAox) in der epidermalen Zelle durch einen sequenzspezifischen RNA-Interferenz Ansatz unter Verwendung doppelsträngiger pNAox-dsRNA ("Gene-Silencing") einen signifikant reduzierten Befall infolge einer *Bgh*-Infektion (gemessen anhand der Haustorium-Ausbildung). Dieser Befund ist insbesondere deshalb überraschend, da der mit der NADPH-Oxidase in Verbindung gebrachten Freisetzung reaktiver Sauerstoffspezies ("oxidative burst") im allgemeinen eine protektive Funktion zugemessen wird.

Ähnlich wie Mlo vermittelt die Verminderung der NADPH-Oxidase Expression eine breite Resistenz gegen verschiedene Isolate von Blumeria graminis f.sp. hordei. In transienten "Gene-Silencing"-Experimenten wird dabei die Penetrationseffizienz (Haustorien-Bildung) von Bgh signifikant um mehr als 35 % reduziert - ein Effekt, der in seiner Stärke dem mittels Mlo-dsRNA erreichten entspricht (Schweizer P et al. (2000) Plant J 24:895-903). In der Wildtyp Gerstensorte Pallas führen ca. 40 % der Pilzpenetrationen zu einer Haustorien-Bildung, wohingegen die Penetrationsrate bei einer Verminderung der NADPH-Oxidase Expression mittels Einbringen einer doppelsträngigen RNA der NADPH-Oxidase (pNAox-dsRNA) nur ca. 25 % beträgt. Die Tatsache, dass auch in pathogenempfindlichen Wildtyp-Sorten wie Pallas nur eine Penetration von ca. 40 bis 50 % beobachtet werden kann, ist auf die stets vorhandene Basalresistenz zurückzuführen. Die NADPH-Oxidase ist aufgrund der dieser Befunde als Schlüsselelement für das erfolgreiche Eindringen eines Pathogens wie Bgh in die pflanzliche Zelle zu verstehen. Darüberhinaus ist das Verfahren allen Verfahren überlegen, bei denen ein pathogen-resistenter Phänotyp durch Überexpression eines resistenzvermittelnden Proteins realisiert wird. Das Ausschalten eines Gens, lässt sich ohne Expression eines (Fremd)-Proteins realisieren. Im Idealfall wird lediglich das endogene Gen deaktiviert. Dies hat nicht zu vernachlässigende Vorteile bei der Zulassung und der Akzeptanz durch den Verbraucher, der Pflanzen mit Fremdproteinen oft mit Vorbehalt begegnet. Ganz besonders vorteilhaft ist in diesem Zusammenhang die Verwendung von induzierbaren Promotoren zur Verminderung der NADPH-Oxidasemenge, Aktivität oder Funktion, was beispielsweise bei Verwendung von pathogeninduzierbaren Promotoren eine Expression nur im Bedarfsfall (d.h. Pathogenbefall) ermöglicht.

Das Verfahren kann im Prinzip auf alle Pflanzenarten angewendet werden. Bevorzugt auf solche, in denen natürlicherweise eine NADPH-Oxidase oder ein funktionelles Äquivalent derselben exprimiert wird.

"Pflanzen" im Rahmen der Erfindung meint alle Gattungen und Arten höherer und niedrigerer Pflanzen des Pflanzenreiches. Eingeschlossen unter dem Begriff sind die reifen Pflanzen, Saatgut, Sprossen und Keimlinge, sowie davon abgeleitete Teile, Vermehrungsgut, Pflanzenorgane, Gewebe, Protoplasten, Kallus und andere Kulturen, zum Beispiel Zellkulturen, sowie alle anderen Arten von Gruppierungen von Pflanzenzellen zu funktionellen oder strukturellen Einheiten. Reife Pflanzen meint Pflanzen zu jedem beliebigen Entwicklungsstadium jenseits des Keimlings. Keimling meint eine junge, unreife Pflanze in einem frühen Entwicklungsstadium. "Pflanze" umfasst alle einjährigen und mehrjährige, monokotyledonen Pflanzen und schließt beispielhaft jedoch nicht einschränkend solche der Gattunge Lotus, Onobrychis, Trigonella, Manihot, Hyoscyamus, Solarium, Heterocallis, Nemesis, Panieum, Pennisetum, Lolium, Oryza, Zea, Avena, Hordeum, Secale, Triticum, Sorghum, Picea ein.

Der Begriff "Pflanze umfasst bevorzugt monokotyle Kulturpflanzen, wie zum Beispiel Getreidearten wie Weizen, Gerste, Hirse, Roggen, Triticale, Mais, Reis, Sorghum oder Hafer sowie Zuckerrohr sowie Zwiebel, Knoblauch, Ananas, und den verschiedenen Baum-, Strauch-, Nuss- und Weinarten. Baumarten umfasst bevorzugt Banane, Mango.

Ferner umfasst sind Schmuckpflanzen, Nutz- oder Zierbäume, Blumen, Schnittblumen, Sträuchern oder Rasen wie beispielhaft aber nicht einschränkend die Familien der Rosaceae wie Rose, Ericaceae wie Rhododendrons und Azaleen, Euphorbiaceae wie Weihnachtssterne und Kroton, Caryophyllaceae wie Nelken, Solanaceae wie Petunien, Gesneriaceae wie das Usambaraveilchen, Balsaminaceae wie das Springkraut, Orchidaceae wie Orchideen, Iridaceae wie Gladiolen, Iris, Freesie und Krokus, Compositae wie Ringelblume, Geraniaceae wie Geranien, Liliaceae wie der Drachenbaum, Moraceae wie Ficus, Araceae wie Philodendron und andere mehr.

Im Rahmen der Erfindung sind solche Pflanzen bevorzugt, die als Nahrungs- oder Futtermittel zum Einsatz kommen, ganz besonders bevorzugt monokotyle Gattungen und Arten, wie die beschriebenen Getreidearten.

Ganz besonders bevorzugt wird das Verfahren auf monokotyle Pflanzen, am meisten bevorzugt auf Pflanzen mit landwirtschaftlicher Bedeutung wie Weizen, Hafer, Hirse, Gerste, Roggen, Mais, Reis, Buchweizen, Sorghum, Triticale, Dinkel, Leinsamen oder Zuckerrohr angewendet.

"Pathogenresistenz" meint das Vermindern oder Abschwächen von Krankheitssymptomen einer Pflanze infolge eines Befalls durch ein Pathogen. Die Symptome können vielfältiger Art sein, umfassen aber bevorzugt solche die direkt oder indirekt zu einer Beeinträchtigung Qualität der Pflanze, der Quantität des Ertrages, der Eignung zur Verwendung als Futter- oder Nahrungsmittel führen oder aber auch Aussaat, Anbau, Ernte oder Prozessierung des Erntegutes erschweren.

"Verleihen", "bestehen", "erzeugen" oder "erhöhen" einer Pathogenresistenz meint, dass die Abwehrmechanismen einer bestimmten Pflanzenart oder -sorte durch Anwendung des erfindungsgemäßen Verfahrens im Vergleich zu dem Wildtyp der Pflanze ("Ausgangspflanze"), auf den das erfindungsgemäße Verfahren nicht angewendet wurde, unter ansonsten gleichen Bedingungen (wie beispielsweise Klima- oder Anbaubedingungen, Pathogenart etc.) eine erhöhte Resistenz gegen ein und mehr Pathogene aufweist. Dabei äußert sich die erhöhte Resistenz bevorzugt in einer verminderten Ausprägung der Krankheitssymptome, wobei Krankheitssymptome - neben den oben erwähnten Beeinträchtigungen - auch beispielsweise die Penetrationseffizienz eines Pathogens in die Pflanze oder pflanzliche Zellen oder die Proliferationseffizienz in oder auf denselben umfasst. Dabei sind die Krankheitssymptome bevorzugt um mindestens 10 % oder mindestens 20 %, besonders bevorzugt um mindestens 40 % oder 60 %, ganz besonders bevorzugt um mindestens 70 % oder 80 %, am meisten bevorzugt um mindestens 90 % oder 95 % vermindert.

"Auswahl" meint in Bezug auf Pflanzen, bei denen - im Unterschied oder Vergleich zur Ausgangspflanze - die Resistenz gegen mindestens ein Pathogen besteht oder erhöht ist, all die Verfahren, die eine zur Erkennung einer vorliegenden oder erhöhten Pathogenresistenz geeignet sind. Dies können Symptome der Pathogeninfektion sein (z.B. Haustorium-Ausbildung bei Pilzinfektion) aber auch die oben beschriebenen Symptome umfassen, die die Qualität der Pflanze, die Quantität des Ertrages, die Eignung zur Verwendung als Futter- oder Nahrungsmittel usw. betreffen.

"Pathogen" meint beispielsweise jedoch nicht einschränkend Viren oder Viroide, Bakterien, Pilze, tierische Schädlinge, wie beispielsweise Insekten oder Nematoden. Besonders bevorzugt sind im Rahmen der Erfindung Pilze wie beispielsweise der Mehltau. Es ist jedoch anzunehmen, dass die Verminderung der Expression einer NADPH-Oxidase, ihrer Aktivität oder Funktion auch eine Resistenz gegen weitere Pathogene beispielsweise jedoch nicht einschränkend seien nachfolgende Pathogene zu nennen:

1. Pilzpathogene oder pilzähnliche Pathogene:

Pilzpathogene oder pilzähnliche Pathogene (wie z.B. Chromista) stammen vorzugsweise aus der Gruppe umfassend Plasmodiophoramycota, Oomycota, Ascomycota, Chytridiomyceten, Zygomyceten, Basidiomycota und Deuteromyceten (Fungi imperfecti). Beispielhaft jedoch nicht einschränkend seien die in Tabelle 1 und 2 genannten Pathogene und die mit ihnen in Zusammenhang gebrachten Erkrankungen zu nennen.

Tabelle 1: Pflanzliche Pilzerkrankungen

| Erkrankung | Pathogen |
|---|---|
| Braunrost | Puccinia recondita |
| Gelbrost | P. striiformis |
| Echter Mehltau | Erysiphe graminis / Blumeria graminis |
| Spelzenbräune | Septoria nodorum |
| Blattdürre | Septoria tritici |
| Ährenfusariosen | Fusarium spp. |
| Halmbruchkrankheit | Pseudocercosporella herpotrichoides |
| Flugbrand | Ustilago spp. |
| Weizensteinbrand | Tilletia caries |
| Schwarzbeinigkeit | Gaeumannomyces graminis |
| Anthrocnose leaf blight<br><br>Anthracnose stalk rot | Colletotrichum graminicola<br>(teleomorph: Glomerella graminicola<br>Politis); Glomerella tucumanensis<br>(anamorph: Glomerella falcatum Went) |
| Aspergillus ear and kernel rot | Aspergillus flavus |
| Banded leaf and sheath spot ("Murzeltöter") | Rhizoctonia solani Kuhn = Rhizoctonia microsclerotia J. Matz (telomorph: |
| Black bundle disease | Acremonium strictum W. Gams = Cephalosporium acremonium Auct. non |

(fortgesetzt)

| Erkrankung | Pathogen |
|---|---|
| | Corda |
| Black kernel rot | Lasiodiplodia theobromae = Botryodiplodia theobromae |
| Borde blanco | Marasmiellus sp. |
| Brown spot (black spot, stalk rot) | Physoderma maydis |
| Cephalosporium kernel rot | Acremonium strictum = Cephalosporium acremonium |
| Charcoal rot | Macrophomina phaseolina |
| Corticium ear rot | Thanatephorus cucumeris = Corticium sasakii |
| Curvularia leaf spot | Curvularia clavata, C. eragrostidis = C. maculans (teleomorph: Cochliobolus eragrostidis), Curvularia inaequalis, C. intermedia (teleomorph: Cochliobolus intermedius), Curvularia lunata (teleomorph: Cochliobolus lunatus), Curvularia pallescens (teleomorph: Cochliobolus pallescens), Curvularia senegalensis, C. tuberculata (teleomorph: Cochliobolus tuberculatus) |
| Didymella leaf spot | Didymella exitalis |
| Diplodia ear rot and stalk rot | Diplodia frumenti (teleomorph: Botryosphaeria festucae) |
| Diplodia ear rot, stalk rot, seed rot and seedling blight | Diplodia maydis = Stenocarpella maydis |
| Diplodia leaf spot or streak | Stenocarpella macrospora = Diplodialeaf macrospora |

Tabelle 2: Falscher Mehltau

| Erkrankung | Pathogen |
|---|---|
| Brown stripe downy mildew | Sclerophthora rayssiae var. zeae |
| Crazy top downy mildew | Sclerophthora macrospora = Sclerospora macrospora |
| Green ear downy mi ldew (graminicola downy mildew) | Sclerospora graminicola |
| Java downy mildew | Peronosclerospora maydis = Sclerospora maydis |
| Philippine downy mildew | Peronosclerospora philippinensis = Sclerospora philippinensis |
| Sorghum downy mildew | Peronosclerospora sorghi = Sclerospora sorghi |
| Spontaneum downy mildew | Peronosclerospora spontanea = Sclerospora spontanea |
| Sugarcane downy mildew | Peronosclerospora sacchari = Sclerospora sacchari |
| Dry ear rot (cob, | Nigrospora oryzae |

(fortgesetzt)

| Erkrankung | Pathogen |
|---|---|
| kernel and stalk rot) | (teleomorph: Khuskia oryzae) |
| Ear rots, minor | Alternaria alternata = A. tenuis, Aspergillus glaucus, A. niger, Aspergillus spp., Botrytis cinerea (teleomorph: Botryotinia fuckeliana), Cunninghamella sp., Curvularia pallescens, Doratomyces stemonitis = Cephalotrichum stemonitis, Fusarium culmorum, Gonatobotrys simplex, Pithomyces maydicus, Rhizopus microsporus Tiegh., R. stolonifer = R. nigricans, Scopulariopsis brumptii |
| Ergot(horse's tooth) | Claviceps gigantea (anamorph: Sphacelia sp.) |
| Eyespot | Aureobasidium zeae = Kabatiella zeae |
| Fusarium ear and stalk rot | Fusarium subglutinans = F. moniliforme var.subglutinans |
| Fusarium kernel, root and stalk rot, seed rot and seedling blight | Fusarium moniliforme (teleomorph: Gibberella fujikuroi) |
| Fusarium stalk rot, seedling root rot | Fusarium avenaceum (teleomorph: Gibberlla avenacea) |
| Gibberella ear and stalk rot (Ähren- u. Stengelfäule) | Gibberella zeae (anamorph: Fusarium graminearum) |
| Gray ear rot | Botryosphaeria zeae = Physalospora zeae (anamorph: Macrophoma zeae) |
| Gray leaf spot (Cercospora leaf spot) | Cercospora sorghi = C. sorghi var. maydis, C. zeae-maydis |
| Helminthosporium root rot | Exserohilum pedicellatum = Helminthosporium pedicellatum (teleomorph: Setosphaeria pedicellata) |
| Hormodendrum ear rot (Cladosporium rot) | Cladosporium cladosporioides = Hormodendrum cladosporioides, C. herbarum (teleomorph: Mycosphaerella tassiana) |
| Hyalothyridium leaf spot | Hyalothyridium maydis |
| Late wilt | Cephalosporium maydis |
| Leaf spots, minor | Alternaria alternata, Ascochyta maydis, A. tritici, A. zeicola, Bipolaris victoriae = Helminthosporium victoriae (teleomorph: Cochliobolus victoriae), |

(fortgesetzt)

| Erkrankung | Pathogen |
|---|---|
| | C. sativus (anamorph: Bipolaris sorokiniana = H. sorokinianum = H. sativum), Epicoccum nigrum, Exserohilum prolatum = Drechslera prolata (teleomorph: Setosphaeria prolata) Graphium penicillioides, Leptosphaeria maydis, Leptothyrium zeae, Ophiosphaerella herpotricha, (anamorph: Scolecosporiella sp.), Paraphaeosphaeria michotii, Phoma sp., Septoria zeae, S. zeicola, S. zeina |
| Northern corn leaf blight (white blast, crown stalk rot, stripe) | Setosphaeria turcica (anarnorph: Exserohilum turcicum = Helminthosporium turcicum) |
| Northern corn leaf spot Helminthosporium ear rot (race 1) | Cochliobolus carbonum (anamorph: Bipolaris zeicola = Helminthosporium carbonum) |
| Penicillium ear rot (blue eye, blue mold) | Penicillium spp., P. chrysogenum, P. expansum, P. oxalicum |
| Phaeocytostroma stalk rot and root rot | Phaeocytostroma ambiguum, = Phaeocytosporella zeae |
| Phaeosphaeria leaf spot | Phaeosphaeria maydis = Sphaerulina maydis |
| Physalospora ear rot (Botryosphaeria ear rot) | Botryosphaeria festucae = Physalospora zeicola (anamorph: Diplodia frumenti) |
| Purple leaf sheath | Hemiparasitic bacteria and fungi |
| Pyrenochaeta stalk rot and root rot | Phoma terrestris = Pyrenochaeta terrestris |
| Pythium root rot | Pythium spp., P. arrhenomanes, P. graminicola |
| Pythium stalk rot | Pythium aphanidermatum = P. butleri L. |
| Red kernel disease (ear mold, leaf and seed rot) | Epicoccum nigrum |
| Rhizoctonia ear rot (sclerotial rot) | Rhizoctonia zeae (teleomorph: Waitea circinata) |
| Rhizoctonia root rot and stalk rot | Rhizoctonia solani, Rhizoctonia zeae |
| Root rots, minor | Alternaria alternata, Cercospora sorghi, Dictochaeta fertilis, Fusarium acuminatum (teleomorph: Gibberella acuminata), F. equiseti (teleomorph: G. intricans), F. oxysporum, F. pallidoroseum, F. poae, F. roseum, G. cyanogena, (anamorph: F. sulphureum), Microdochium bolleyi, Mucor sp., Periconia circinata, Phytophthora cactorum, P. drechsleri, P. nicotianae |

(fortgesetzt)

| Erkrankung | Pathogen |
|---|---|
| | var. parasitica, Rhizopus arrhizus |
| Rostratum leaf spot (Helminthosporium leaf disease, ear and stalk rot) | Setosphaeria rostrata, (anamorph: Exserohilum rostratum = He/minthosporium rostratum) |
| Rust, common corn | Puccinia sorghi |
| Rust, southern corn | Puccinia polysora |
| Rust, tropical corn | Physopella pallescens, P. zeae = Angiopsora zeae |
| Sclerotium ear rot (sou-thern blight) | Sclerotium rolfsii Sacc. (teleomorph: Athelia rolfsii) |
| Seed rot-seedling blight | Bipolaris sorokiniana, B. zeicola = Helminthosporium carbonum, Diplodia maydis, Exserohilum pedicillatum, Exserohilum turcicum = Helminthosporium turcicum, Fusarium avenaceum, F. culmorum, F. moniliforme, Gibberella zeae (anamorph: F. graminearum), Macrophomina phaseolina, Penicillium spp., Phomopsis sp., Pythium spp., Rhizoctonia solani, R. zeae, Sclerotium rolfsii, Spicaria sp. |
| Selenophoma leaf spot | Selenophoma sp. |
| Sheath rot | Gaeumannomyces graminis |
| Shuck rot | Myrothecium gramineum |
| Silage mold | Monascus purpureus, M ruber |
| Smut, common | Ustilago zeae = U. maydis |
| Smut, false | Ustilaginoidea virens |
| Smut, head | Sphacelotheca reiliana = Sporisorium holcisorghi |
| Southern corn leaf blight and stalk rot | Cochliobolus heterostrophus (anamorph: Bipolaris maydis = Helminthosporium maydis) |
| Southern leaf spot | Stenocarpella macrospora = Diplodia macrospora |
| Stalk rots, minor | Cercospora sorghi, Fusarium episphaeria, F. merismoides, F. oxysporum Schlechtend, F. poae, F. roseum, F. solani (teleomorph: Nectria haematococca), F. tricinctum, Mariannaea elegans, Mucor sp., Rhopographus zeae, Spicaria sp. |
| Storage rots | Aspergillus spp., Penicillium spp. and other fungi |
| Tar spot | Phyllachora maydis |
| Trichoderma ear rot and root rot | Trichoderma viride = T. lignorum teleomorph: Hypocrea sp. |
| White ear rot, root and stalk rot | Stenocarpella maydis = Diplodia zeae |
| Yellow leaf blight | Ascochyta ischaemi, Phyllosticta maydis (teleomorph: Mycosphaerella zeae-maydis) |

(fortgesetzt)

| Erkrankung | Pathogen |
|---|---|
| Zonate leaf spot | Gloeocercospora sorghi |

Besonders bevorzugt sind

- Plasmodiophoromycota wie Plasmodiophora brassicae (Kohlhernie, clubroot of crucifers), Spongospora subterranea (powdery scab of potato tubers), Polymyxa graminis (root disease of cereals and grasses),

- Oomycota wie Bremia lactucae (Falscher Mehltau an Salat), Peronospora (Falscher Mehltau) bei snapdragon (P. antirrhini), Zwiebel (P. destructor), Spinat (P. effusa), Sojabohne (P. manchurica), Tabak ("blue mold" = Blauschimmel; P. tabacina) Alfalfa und Klee (P. trifolium), Pseudoperonospora humuli (Falscher Mehltau an Hopfen), Plasmopara (Falscher Mehltau bei Trauben) (P. viticola) und Sonnenblume (P. halstedii), Sclerophtohra macrospora (Falscher Mehltau bei Cerealien und Gäsern), Pythium (seed rot, seedling damping-off, and root rot and all types of plants, z.B. Wurzelbrand an Beta-Rübe durch P. debaryanum), Phytophthora infestans (Kraut- und Knollenfäule bei Kartoffel, Braunfäule bei Tomate etc.), Albugo spec. (white rust on cruciferous plants.

- Ascomycota wie Microdochium nivale (Schneeschimmel an Roggen und Weizen), Fusarium graminearum, Fusarium culmorum (Ährenfäule v.a. bei Weizen), Fusarium oxysporum (Fusarium-Welke an Tomate), Blumeria graminis (Echter Mehltau an Gerste (f.sp. hordei) und Weizen (f.sp. tritici)), Erysiphe pisi (Erbsenmehltau), Nectria galligena (Obstbaumkrebs), Unicnula necator (Echter Mehltau der Weinrebe), Pseudopeziza tracheiphila (Roter Brenner der Weinrebe), Claviceps purpurea (Mutterkorn an z.B. Roggen und Gräsern), Gaeumannomyces graminis (Schwarzbeinigkeit an Weizen, Roggen u.a. Gräsern), Magnaporthe grisea (rice blast disease), Pyrenophora graminea (Streifenkrankheit an Gerste), Pyrenophora teres (Netzfleckenkrankheit an Gerste), Pyrenophora tritici-repentis (Blattfleckenkrankheit (Blattdürre) an Weizen), Venturia inaequalis (Apfelschorf), Sclerotinia sclerotium (Weißstengeligkeit, Rapskrebs), Pseudopeziza medicaginis (Klappenschorf an Luzerne, Weiß- und Rotklee).

- Basidiomyceten wie Typhula incarnata (Typhula-Fäule an Gerste, Roggen, Weizen), Ustilago maydis (Beulenbrand an Mais), Ustilago nuda (Flugbrand an Gerste), Ustilago tritici (Flugbrand an Weizen, Dinkel), Ustilago avenae (Flugbrand an Hafer), Rhizoctonia solani (Wurzeltöter an Kartoffeln), Sphacelotheca spp. (head smut of sorghum), Melampsora lini (rust of flax), Puccinia graminis (Schwarzrost an Weizen, Gerste, Roggen, Hafer), Puccinia recondita (Braunrost an Weizen), Puccinia dispersa (Braunrost an Roggen), Puccinia hordei (Braunrost an Gerste), Puccinia coronata (Kronenrost an Hafer), Puccinia striiformis (Gelbrost an Weizen, Gerste, Roggen sowie zahlreichen Gräsern), Uromyces appendiculatus (Bohnenrost), Sclerotium rolfsii (root and stem rots of many plants).

- Deuteromyceten (Fungi imperfecti) wie Septoria nodorum (Spelzenbräune) an Weizen (Septoria tritici), Pseudocercosporella herpotrichoides (Halmbruchkrankheit an Weizen, Gerste, Roggen), Rynchosporium secalis (Blattfleckenkrankheit an Roggen und Gerste), Alternaria solani (Dürrfleckenkrankheit an Kartoffel, Tomate), Phoma betae (Wurzelbrand an Beta-Rübe), Cercospora beticola (Cercospora-Blattfleckenkrankheit an Beta-Rübe), (Alternaria brassicae (Rapsschwärze an Raps, Kohl u.a. Kreuzblütlern), Verticillium dahliae (Rapswelke und -stengelfäule), Colletotrichum lindemuthianum (Brennfleckenkrankheit an Bohne), Phoma lingam - Umfallkrankheit (Schwarzbeinigkeit an Kohl; Wurzelhals- oder Stengelfäule an Raps), Botrytis cinerea (Grauschimmel an Weinrebe, Erdbeere, Tomate, Hopfen etc.).

Am meisten bevorzugt sind Phytophthora infestans (Kraut- und Knollenfäule, Braunfäule bei Tomate etc.), Microdochium nivale (vormals Fusarium nivale; Schneeschimmel an Roggen und Weizen), Fusarium graminearum, Fusarium culmorum (Ährenfäule an Weizen), Fusarium oxysporum (Fusarium-Welke an Tomate), Blumeria graminis (Echter Mehltau an Gerste (f. sp. hordei) und Weizen (f. sp. tritici)), Magnaporthe grisea (rice blast disease), Sclerotinia sclerotium (Weißstengeligkeit, Rapskrebs), Septoria nodorum und Septoria tritici (Spelzenbräune an Weizen), Alternaria brassicae (Rapsschwärze an Raps, Kohl u.a. Kreuzblütlern), Phoma lingam (Umfallkrankheit, Schwarzbeinigkeit an Kohl; Wurzelhals- oder Stengelfäule an Raps).

2. Bakterielle Pathogene:

Beispielhaft jedoch nicht einschränkend seien die in Tabelle 3 genannten Pathogene und die mit ihnen in Zusammenhang gebrachten Erkrankungen zu nennen.

Tabelle 3: Bakterielle Erkrankungen

| Erkrankung | Pathogen |
|---|---|
| Bacterial leaf blight and stalk rot | Pseudomonas avenae subsp. avenae |
| Bacterial leaf spot | Xanthomonas campestris pv. holcicola |
| Bacterial stalk rot | Enterobacter dissolvens = Erwinia dissolvens |
| Schwarzbeinigkeit ("Bacterial stalk and top rot") | Erwinia carotovora subsp. carotovora, Erwinia chrysanthemi pv. zeae |
| Bacterial stripe | Pseudomonas andropogonis |
| Chocolate spot | Pseudomonas syringae pv. coronafaciens |
| Goss's bacterial wilt.and blight (leaf freckles and wilt) | Clavibacter michiganensis subsp. nebraskensis = Corynebacterium michiganense pv.andnebraskense |
| Holcus spot | Pseudomonas syringae pv. syringae |
| Purple leaf sheath | Hemiparasitic bacteria |
| Seed rot-seedling blight | Bacillus subtilis |
| Stewart's disease (bacterial wilt) | Pantoea stewartii = Erwinia stewartii |
| Corn stunt (achapparramiento,maize stunt, Mesa Central or Rio Grande maize stunt) | Spiroplasma kunkelii |

Ganz besonders bevorzugt sind nachfolgende pathogene Bakterien: Corynebacterium sepedonicum (Bakterienringfäule an Kartoffel), Erwinia carotovora (Schwarzbeinigkeit an Kartoffel), Erwinia amylovora (Feuerbrand an Birne, Apfel, Quitte), Streptomyces scabies (Kartoffelschorf), Pseudomonas syringae pv. tabaci (Wildfeuer an Tabak), Pseudomonas syringae pv. phaseolicola (Fettfleckenkrankheit an Buschbohne), Pseudomonas syringae pv. tomato ("bacterial speck" an Tomate), Xanthomonas campestris pv. malvacearum (Blattfleckenkrankheit an Baumwolle) und Xanthomonas campestris pv. oryzae (Bakterienfäule an Reis und anderen Gräsern).

3. Virale Pathogene:

"Virale Pathogene" schließt sämtliche Pflanzenviren ein wie beispielsweise Tabak- oder oder Cucumber-Mosaiv Virus, Ringspot-Virus, Nekrose-Virus, Mais Dwarf-Mosaic Virus etc.

Beispielhaft jedoch nicht einschränkend seien die in Tabelle 4 genannten Pathogene und die mit ihnen in Zusammenhang gebrachten Erkrankungen zu nennen.

Tabelle 4: Virale Erkrankungen

| Krankheit | Pathogen |
|---|---|
| American wheat striate (wheat striate mosaic) | American wheat striate mosaic virus (AWSMV) |
| Barley stripe mosaic | Barley stripe mosaic virus (BSMV) |
| Barley yellow dwarf | Barley yellow dwarf virus (BYDV) |
| Brome mosaic | Brome mosaic virus (BMV) |
| Cereal chlorotic mottle | Cereal chlorotic mottle virus (CCMV) |
| Corn chlorotic vein banding (Braizilian maize mosaic) | Corn chlorotic vein banding virus (CCVBV) |

(fortgesetzt)

| Krankheit | Pathogen |
|---|---|
| Corn lethal necrosis | Viruskomplex aus Maize chlorotic mottle virus (MCMV) und Maize dwarf mosaic virus (MDMV) A oder B oder Wheat streak mosaic virus(WSMV) |
| Cucumber mosaic | Cucumber mosaic virus (CMV) |
| Cynodon chlorotic streak | Cynodon chlorotic streak virus (CCSV) |
| Johnsongrass mosaic | Johnsongrass mosaic virus (JGMV) |
| Maize bushy stunt | Mycoplasma-like organism (MLO) associated |
| Maize chlorotic dwarf | Maize chlorotic dwarf virus (MCDV) |
| Maize chlorotic mottle | Maize chlorotic mottle virus (MCMV) |
| Maize dwarf mosaic | Maize dwarf mosaic virus (MDMV) strains A, D, E and F |
| Maize leaf fleck | Maize leaf fleck virus (MLFV) |
| Maize line | Maize line virus (MLV) |
| Maize mosaic (corn leaf stripe, enanismo rayado) | Maize mosaic virus (MMV) |
| Maize mottle and chlorotic stunt | Maize mottle and chlorotic stunt virus |
| Maize pellucid ringspot | Maize pellucid ringspot virus (MPRV) |
| Maize raya gruesa | Maize raya gruesa virus (MRGV) |
| maize rayado fino (fine striping disease) | Maize rayado fino virus (MRFV) |
| Maize red leaf and red stripe | Mollicute |
| Maize red stripe | Maize red stripe virus (MRSV) |
| Maize ring mottle | Maize ring mottle virus (MRMV) |
| Maize rio IV | Maize rio cuarto virus (MRCV) |
| Maize rough dwarf (nanismo ruvido) | Maize rough dwarf virus (MRDV) (Cereal tillering disease virus) |
| Maize sterile stunt | Maize sterile stunt virus (strains of barley yellow striate virus) |
| Maize streak | Maize streak virus (MSV) |
| Maize stripe (maize chlorotic stripe, maize hoja blanca) | Maize stripe virus |
| Maize stunting | Maize stunting virus |
| Maize tassel abortion | Maize tassel abortion virus (MTAV) |
| Maize vein enation | Maize vein enation virus (MVEV) |
| Maize wallaby ear | Maize wallaby ear virus (MWEV) |
| Maize white leaf | Maize white leaf virus |
| Maize white line mosaic | Maize white line mosaic virus (MWLMV) |
| Millet red leaf | Millet red leaf virus (MRLV) |
| Northern cereal mosaic | Northern cereal mosaic virus (NCMV) |
| Oat pseudorosette (zakuklivanie) | Oat pseudorosette virus |
| Oat sterile dwarf | Oat sterile dwarf virus (OSDV) |

(fortgesetzt)

| Krankheit | Pathogen |
|---|---|
| Rice black-streaked dwarf | Rice black-streaked dwarf virus (RBSDV) |
| Rice stripe | Rice stripe virus (RSV) |
| Sorghum mosaic | Sorghum mosaic virus (SrMV) (auch: sugarcane mosaic virus (SCMV) Stämme H, I and M) |
| Sugarcane Fiji disease | Sugarcane Fiji disease virus (FDV) |
| Sugarcane mosaic | Sugarcane mosaic virus (SCMV) strains A, B, D, E, SC, BC, Sabi and MB (formerly MDMV-B) |
| Wheat spot mosaic | Wheat spot mosaic virus (WSMV) |

4. Tierische Schädlinge

4.1 Insekten Pathogene:

Beispielhaft jedoch nicht einschränkend seien Insekten wie beispielsweise Käfer, Raupen, Läuse oder Milben zu nennen. Bevorzugt sind Insekten der Gattungen Coleoptera, Diptera, Hymenoptera, Lepidoptera, Mallophaga, Homoptera, Hemiptera, Orthoptera, Thysanoptera. Dermaptera, Isoptera, Anoplura, Siphonaptera, Trichoptera, etc.. Besonders bevorzugt sind Coleoptera and Lepidoptera Insekten, wie beispielsweise den Maiszünsler (European Corn Borer (ECB)), Diabrotica barberi ("northern corn rootworm"), Diabrotica undecimpunctata ("southern corn rootworm"), Diabrotica virgifera ("Western corn rootworm"), Agrotis ipsilon ("black cutworm"), Crymodes devastator ("glassy cutworm"), Feltia ducens ("dingy cutworm"), Agrotis gladiaria ("claybacked cutworm"), Melanotus spp., Aeolus mellillus ("wireworm"), Aeolus mancus ("wheat wireworm"), Horistonotus uhlerii ("sand wireworm"), Sphenophorus maidis ("maize billbug"), Sphenophorus zeae ("timothy billbug"), Sphenophorus parvulus ("bluegrass billbug"), Sphenophorus callosus ("southern corn billbug"), Phyllogphaga spp.("white grubs"), Anuraphis maidiradicis ("corn root aphid"), Delia platura ("seedcorn maggot"), Colaspis brunnea ("grape colaspis"), Stenolophus lecontei ("seedcorn beetle") und Clivinia impressifrons ("lender seedcorn beetle").

Ferner sind zu nennen: Das Getreidehähnchen (Oulema melanopus), die Fritfliege (Oscinella frit), Drahtwürmer (Agrotis lineatus) und Blattläuse (wie z.B. Haferblattlaus Rhopalosiphum padi, Große Getreideblattlaus Sitobion avenae).

4.2 Nematoden:

Beispielhaft jedoch nicht einschränkend seien die in Tabelle 6 genannten Pathogene und die mit ihnen in Zusammenhang gebrachten Erkrankungen zu nennen.

Tabelle 6: Parasitäre Nematoden

| Schädigung | Pathogene Nematode |
|---|---|
| Awl | Dolichodorus spp., D. heterocephalus |
| Stengel- oder Stockälchen, Rübenkopfälchen ("Bulb and stem"; Europe) | Ditylenchus dipsaci |
| Burrowing | Radopholus similis |
| Haferzystenälchen ("Cyst") | Heterodera avenae, H. zeae, Punctodera chalcoensis |
| Dagger | Xiphinema spp., X. americanum, X. mediterraneum |
| False root-knot | Nacobbus dorsalis |
| Lance, Columbia | Hoplolaimus columbus |
| Lance | Hoplolaimus spp., H. galeatus |

(fortgesetzt)

| Schädigung | Pathogene Nematode |
|---|---|
| Lesion | Paratylenchus spp., P. brachyurus, P. crenatus, P. hexincisus, P. neglectus, P. penetrans, P. scribneri, P. thornei, P. zeae |
| Needle | Longidorus spp., L. breviannulatus |
| Ring | Criconemella spp., C. ornata |
| Wurzelgallenälchen ("Root-knot") | Meloidogyne spp., M. chitwoodi, M. incognita, M. javanica |
| Spiral | Helicotylenchus spp. |
| Sting | Belonolaimus spp., B. longicaudatus |
| Stubby-root | Paratrichodorus spp., P. christiei, P. minor, Quinisulcius acutus, Trichodorus spp. |
| Stunt | Tylenchorhynchus dubius |

Ganz besonders bevorzugt sind Globodera rostochiensis und G. pallida (Zystenälchen an Kartoffel, Tomate u.a. Nachtschattengewächsen), Heterodera schachtii (Rübenzystenälchen an Zucker- und Futterrübe, Raps, Kohl etc.), Heterodera avenae (Haferzystenälchen an Hafer u.a. Getreidearten), Ditylenchus dipsaci (Stengel- oder Stockälchen, Rübenkopfälchen an Roggen, Hafer, Mais, Klee, Tabak, Rübe), Anguina tritici (Weizenälchen, Radekrankheit an Weizen (Dinkel, Roggen), Meloidogyne hapla (Wurzelgallenälchen an Möhre, Gurke, Salat, Tomate, Kartoffel, Zuckerrübe, Luzerne).

Als für die einzelnen Sorten bevorzugte Pilz- oder Virus-Pathogene sind beispielsweise zu nennen:

1. Gerste:

Pilz-, bakterielle und virale Pathogene: Puccinia graminis f.sp. hordei (barley stem rust), Blumeria (Erysiphe) graminis f.sp. hordei (Barley Powdery Mildew), barley yellow dwarf virus (BYDV),
Pathogene Insekten / Nematoden: Ostrinia nubilalis (European corn borer); Agrotis ipsilon (black cutworm); Schizaphis graminum (greenbug); Blissus leucopterus leucopterus (chinch bug); Acrosternum hilare (green stink bug); Euschistus servus (brown stink bug); Deliaplatura (seedcorn maggot); Mayetiola destructor (Hessian fly); Petrobia latens (brown wheat mite).

2. Sojabohne:

Pilz-, bakterielle oder virale Pathogene: Phytophthora megasperma fsp.glycinea, Macrophomina phaseolina, Rhizoctonia solani, Sclerotinia sclerotiorum, Fusarium oxysporum, Diaporthe phaseolorum var. sojae (Phomopsis sojae), Diaporthe phaseolorum var. caulivora, Sclerotium rolfsii, Cercospora kikuchii, Cercospora sojina, Peronospora manshurica, Colletotrichum dematium (Colletotrichum truncatum), Corynespora cassiicola, Septoria glycines, Phyllosticta sojicola, Alternaria alternata, Pseudomonas syringae p.v. glycinea, Xanthomonas campestris p.v. phaseoli, Microsphaera diffussa, Fusarium semitectum, Phialophora gregata, Sojabohnen Mosaikvirus, Glomerella glycines, Tobacco Ring spot virus, Tobacco Streak virus, Phakopsorapachyrhizi, Pythium aphanidermatum, Pythium ultimum, Pythium debaryanum, Tomato spotted wilt virus, Heterodera glycines Fusarium solani.

Pathogene Insekten / Nematoden: Pseudoplusia includens (soybean looper); Anticarsia gemmatalis (velvetbean caterpillar); Plathypena scabra (green cloverworm); Ostrinia nubilalis (European corn borer); Agrotis ipsilon (black cutworm); Spodoptera exigua (beet armyworm); Heliothis virescens (cotton budworm); Helicoverpa zea (cotton bollworm); Epilachna varivestis (Mexican bean beetle); Myzus persicae (green peach aphid); Empoasca fabae (potato leaf hopper); Acrosternum hilare (green stink bug); Melanoplus femurrubrum (redlegged grasshopper); Melanoplus differentialis (differential grasshopper); Hylemya platura (seedcom maggot); Sericothrips variabilis (soybean thrips); Thrips tabaci (onion thrips); Tetranychus turkestani (strawberry spider mite); Tetranychus urticae (twospotted spider mite);

3. Canola:

Pilz-, bakterielle oder virale Pathogene: Albugo candida, Alternaria brassicae, Leptosphaeria maculans, Rhizoctonia solani, Sclerotinia sclerotiorum, Mycosphaerella brassiccola, Pythium ultimum, Peronospora parasitica, Fusarium roseum, Alternaria alternata.

4. Alfalfa:

Pilz,, bakterielle oder virale Pathogene: Clavibater michiganese subsp. insidiosum, Pythium ultimum, Pythium irregulare, Pythium splendens, Pythium debaryanum, Pythium aphanidermatum, Phytophthora megasperma, Peronospora trifoliorum, Phoma medicaginis var. medicaginis, Cercospora medicaginis, Pseudopeziza medicaginis, Leptotrochila medicaginis, Fusarium, Xanthomonas campestris p.v. alfalfae, Aphanomyces euteiches, Stemphylium herbarum, Stemphylium alfalfae.

5. Weizen:

Pilz-,bakterielle oder virale Pathogene: Pseudomonas syringae p.v. atrofaciens, Urocystis agropyri, Xanthomonas campestris p.v. translucens, Pseudomonas syringae p.v. syringae, Alternaria alternata, Cladosporium herbarum, Fusarium graminearum, Fusarium avenaceum, Fusarium culmorum, Ustilago tritici, Ascochyta tritici, Cephalosporium gramineum, Collotetrichum graminicola, Erysiphe graminis f.sp. tritici, Puccinia graminis f.sp. tritici, Puccinia recondita f.sp. tritici, Puccinia striiformis, Pyrenophora tritici-repentis, Septoria nodorum, Septoria tritici, Septoria avenae, Pseudocercosporella herpotrichoides, Rhizoctonia solani, Rhizoctonia cerealis, Gaeumannomyces graminis var. tritici, Pythium aphanidermatum, Pythium arrhenomanes, Pythium ultimum, Bipolaris sorokiniana, Barley Yellow Dwarf Virus, Brome Mosaic Virus, Soil Borne Wheat Mosaic Virus, Wheat Streak Mosaic Virus, Wheat Spindle Streak Virus, American Wheat Striate Virus, Claviceps purpurea, Tilletia tritici, Tilletia laevis, Ustilago tritici, Tilletia indica, Rhizoctonia solani, Pythium arrhenomannes, Pythium gramicola, Pythium aphanidermatum, High Plains Virus, European wheat striate virus, Puccinia graminis f.sp. tritici (Wheat stem rust), Blumeria (Erysiphe) graminis f.sp. tritici (Wheat Powdery Mildew)

Pathogene Insekten / Nematoden: Pseudaletia unipunctata (army worm); Spodoptera, frugiperda (fall armyworm); Elasmopalpus lignosellus (lesser cornstalk borer); Agrotis orthogonia (western cutworm); Elasmopalpus Zignosellus (lesser cornstalk borer); Oulema melanopus (cereal leaf beetle); Hypera punctata (clover leaf weevil); Diabrotica undecimpunctata howardi (southern corn rootworm); Russian wheat aphid; Schizaphis graminum (greenbug); Macrosiphum avenae (English grain aphid); Melanoplus femurrubrum (redlegged grasshopper); Melanoplus differentialis (differential grasshopper); Melanoplus sanguinipes (migratory grasshopper); Mayetiola destructor (Hessian fly); Sitodiplosis mosellana (wheat midge); Meromyza americana (wheat stem maggot); Hylemya coarctata (wheat bulb fly); Frankliniella fusca (tobacco thrips); Cephus cinctus (wheat stem sawfly); Aceria tulipae (wheat curl mite);

6. Sonnenblume:

Pilz-, bakterielle oder virale Pathogene: Plasmophora halstedii, Sclerotinia sclerotiorum, Aster Yellows, Septoria helianthi, Phomopsis helianthi, Alternaria helianthi, Alternaria zinniae, Botrytis cinerea, Phoma macdonaldii, Macrophomina phaseolina, Erysiphe cichoracearum, Rhizopus oryzae, Rhizopus arrhizus, Rhizopus stolonifer, Puccinia helianthi, Verticillium dahliae, Erwinia carotovorum p.v. Carotovora, Cephalosporium acremonium, Phytophthora cryptogea, Albugo tragopogonis.

Pathogene Insekten / Nematoden: Suleima helianthana (sunflower bud moth); Homoeosoma electellum (sunflower moth); zygogramma exclamationis (sunflower beetle); Bothyrus gibbosus (carrot beetle); Neolasioptera murtfeldtiana (sunflower seed midge);

7. Mais:

Pilz-, bakterielle oder virale Pathogene: Fusarium moniliforme var. subglutinans, Erwinia stewartii, Fusarium moniliforme, Gibberella zeae (Fusarium graminearum), Stenocarpella maydi (Diplodia maydis), Pythium irregulare, Pythium debaryanum, Pythium graminicola, Pythium splendens, Pythium ultimum, Pythium aphanidermatum, Aspergillus flavus, Bipolaris maydis 0, T (Cochliobolus heterostrophus), Helminthosporium carbonum I, II & III (Cochliobolus carbonum), Exserohilum turcicum I, II & III, Helminthosporium pedicellatum, Physoderma maydis, Phyllosticta maydis, Kabatiella maydis, Cercospora sorghi, Ustilago maydis, Puccinia sorghi, Puccinia polysora, Macrophomina phaseolina, Penicillium oxalicum, Nigrospora oryzae, Cladosporium herbarum, Curvularia lunata, Curvularia inaequalis, Curvularia pallescens, Clavibacter michiganese subsp. nebraskense, Trichoderma viride, Maize Dwarf Mosaic Virus A & B, Wheat Streak Mosaic Virus, Maize Chlorotic Dwarf Virus, Claviceps sorghi, Pseudonomas avenae, Erwinia chrysanthemi p.v. Zea, Erwinia corotovora, Cornstunt spiroplasma, Diplodia macrospora, Sclerophthora macrospora, Peronosclerospora sorghi, Peronosclerospora philippinesis, Peronosclerospora maydis, Peronosclerospora sacchari, Spacelotheca reiliana, Physopella zeae, Cephalosporium maydis, Caphalosporium acremonium, Maize Chlorotic Mottle Virus, High Plains Virus, Maize Mosaic Virus, Maize Rayado Fino Virus, Maize Streak Virus (MSV, Maisstrichel-Virus), Maize Stripe Virus, Maize Rough Dwarf Virus.

Pathogene Insekten / Nematoden: Ostrinia nubilalis (European corn borer); Agrotis ipsilon (black cutworm); Helicoverpa zea (corn earworm); Spodoptera frugiperda. (fall armyworm); Diatraea grandiosella (southwestern corn borer); Elasmopalpus lignosellus (lesser cornstalk borer); Diatraea saccharalis (surgarcane borer); Diabrotica virgifera (western corn rootworm); Diabrotica longicornis barberi (northern corn rootworm); Diabrotica undecimpunctata howardi (southern corn rootworm); Melanotus spp. (wireworms); Cyclocephala borealis (northern masked chafer; white grub); Cyclocephala immaculata (southern masked chafer; white grub); Popillia japonica (Japanese beetle); Chaetocnema pulicaria (corn flea beetle); Sphenophorus maidis (maize billbug); Rhopalosiphum maidis (corn leaf aphid); Anuraphis maidiradicis (corn root aphid); Blissus leucopterus leucopterus (chinch bug); Melanoplus femurrubrum (redlegged grasshopper); Melanoplus sanguinipes (migratory grasshopper); Hylemva platura (seedcom maggot); Agromyza parvicornis (corn blot leafminer); Anaphothrips obscrurus (grass thrips); Solenopsis milesta (thief ant); Tetranychus urticae (twospotted spider mite).

8. Sorghum:

Pilz-, bakterielle oder virale Pathogene: Exserohilum turcicum, Colletotrichum graminicola (Glomerella graminicola), Cercospora sorghi, Gloeocercospora sorghi, Ascochyta sorghina, Pseudomonas syringae p.v. syringae, Xanthomonas campestris p.v. holcicola, Pseudomonas andropogonis, Puccinia purpurea, Macrophomina phaseolina, Perconia circinata, Fusarium monilifonne, Alternaria alternate, Bipolaris sorghicola, Helminthosporium sorghicola, Curvularia lunata, Phoma insidiosa, Pseudomonas avenae (Pseudomonas alboprecipitans), Ramulispora sorghi, Ramulispora sorghicola, Phyllachara sacchari, Sporisorium reilianum (Sphacelotheca reiliana), Sphacelotheca cruenta, Sporisorium sorghi, Sugarcane mosaic H, Maize Dwarf Mosaic Virus A & B, Claviceps sorghi, Rhizoctonia solani, Acremonium strictum, Sclerophthona macrospora, Peronosclerospora sorghi, Peronosclerospora philippinensis, Sclerospora graminicola, Fusarium graminearum, Fusarium oxysporum, Pythium arrhenomanes, Pythium graminicola.

Pathogene Insekten / Nematoden: Chilo partellus (sorghum borer); Spodoptera frugiperda (fall armyworm); Helicoverpa zea (corn ear-worm); Elasmopalpus lignosellus (lesser cornstalk borer); Feltia subterranea (granulate cutworm); Phvllophaga crinita (white grub); Eleodes, Conoderus und Aeolus spp. (wireworm); Oulema melanopus (cereal leaf beetle); Chaetocnema pulicaria (corn flea beetle); Sphenophorus maidis (maize billbug); Rhopalosiphum maidis (corn leaf aphid); Siphaflava (yellow sugarcane aphid); Blissus leucopterus leucopterus (chinch bug); Contarinia sorghicola (sorghummidge); Tetranychus cinnabarinus (carmine spider mite); Tetranychus urticae (two spotted spider mite).

9. Baumwolle:

Pathogene Insekten / Nematoden: Heliothis virescens (cotton budworm); Helicoverpa zea (cotton bollworm); Spodoptera exigua (beet armyworm); Pectinophora gossypiella (pink bollworm); Anthonomus grandis grandis (boll weevil); Aphis gossypii (cotton aphid); Pseudatomoscelis seriatus (cotton fleahopper); Trialeurodes abutilonea (bandedwinged whitefly) Lygus lineolaris (tarnished plant bug); Melanoplus femurrubrum (redlegged grasshopper); Melanoplus differentialis (differential grasshopper); Thrips tabaci (onion thrips); Franklinkiella fusca (tobacco thrips); Tetranychus cinnabarinus (carmine spider mite); Tetranychus urticae (twospotted spider mite);

10. Reis:

Pathogene Insekten / Nematoden: Diatraea saccharalis (sugarcane borer); Spodoptera frugiperda (fall armyworm); Helicoverpa zea (corn earworm); Colaspis brunnea (grape colaspis); Lissorhoptrus oryzophilus (rice water weevil); Sitophilus oryzae (rice weevil); Nephotettix nigropictus (rice leafhopper); Blissus leucopterus leucopterus (chinch bug); Acrosternum hilare (green stink bug);

11. Raps:

Pathogene Insekten / Nematoden: Brevicoryne brassicae (cabbage aphid); Phyilotreta cruciferae (Flea beetle); Mamestra conjgurata (Bertha armyworm); Plutella xylostella (Diamond-back snoth); Delia ssp. (Root maggots).

"NADPH-Oxidase" meint im Rahmen der Erfindung all solche Enzyme, die als wesentliche Eigenschaft befähigt sind mittels eines Einzelelektronentransfers molekularen Sauerstoff ($O_2$) zu Superoxid ($O_2^-$) umzusetzen. Bevorzugt sind die Enzyme die durch die EC-Klasse E.C.1.23.45.3 beschrieben werden. Dabei kann die NADPH-Oxidasen aus einem oder mehr Polypeptiden bestehen, die gleich oder unterschiedlich sein können.

Bevorzugt ist die NADPH-Oxidase ein Flavocytochromprotein und umfasst als prosthetische Gruppen ein Cytochrom b und/oder eine FAD Einheit. Die NADPH-Oxidase kann aus einem $\alpha 1 \beta 1$ Heterodimer bestehen, wobei die $\beta$ Untereinheit die funktionelle Untereinheit des Flavocytochroms darstellen und als Glykoprotein die Elektronentransportkomponenten umfassen kann (eine hydrophile, zytosolische, C-terminale Domäne, welche NADPH und FAD enthält, sowie 4 bis 6 N-terminale, putative Transmembrane-α-Helixes, welche zwei Histidin-komplexierte prosthetische Haem-Gruppen enthält). Die α-Untereinheit kann eine C-terminale, Prolin-reiche Sequenz umfassen, welche potentielle zytosoliche, aktivierende Faktoren der NADPH-Oxidase zu binden vermag. Durch die Bindung der zytosolische phox Proteine (z.B. p47-phox, p67-phox, p40-phox) und p21rac - ein GTP-bindendes Protein - kann Aktivierung erfolgen.

Dem Fachmann sind zahlreiche NADPH-Oxidasen aus pflanzlichen Organismen bekannt (u.a. Torres MA et al. (1998) Plant J 14: 365-370). Beispielhaft jedoch nicht einschränkend seien die Sequenzen mit nachfolgenden GenBnk Acc.-No. zu nennen: AJ251717 (Hordeum vulgare), AP003560 (Oryza sativa var. japonica), AJ320505 (Nicotiana tabacum), AB050660 (Solanum tuberosum), AF088276 (Lycopersicon esculentum), AB008111 (Arabidopsis thaliana; Atrboh F), AF055357 (Arabidopsis thaliana; RbohD), AJ309006 (Nicotiana tabacum; rboh), AP003271 (Oryza sativa cv. japonica), AF055355 (Arabidopsis thaliana; RbohC), AF055353 (Arabidopsis thaliana; RbohA). Insbesondere bevorzugt sind die NADPH-OXIDASEN, die eine Sequenz gemäß SEQ ID: 2, 4, 6, 8, 10, 12, 14, 16, 18, 20 oder 22 umfassen.

Die zu den im Rahmen dieser Erfindung offenbarten NADPH-Oxidase Sequenzen homologen Sequenzen aus anderen Pflanzen können z.B. durch Datenbanksuche oder Durchmustern von Gen-Banken - unter Verwendung der NADPH-Oxidase Sequenzen als Suchsequenz bzw. Sonde - leicht aufgefunden werden. Beispielhaft seien dabei Sequenzen mit nachfolgenden GenBnk Acc.-No. zu nennen: CAC51517.1, AJ251717, T03973, BAB68079.1, AP003560, T02024, CAC87256.1, AJ320505, BAB70750.1, AB050660, AF088276_1, NP_564821.1,N M_105079, T00265 AC007764_16, NP_192862.1, NM_117194, AF147783_1, AAM28891.1, AF506374, CAC84140.1, AJ309006, T51804, NP_199602.1, NM_124165, BAB89740.1, AP003271, AAC39477.1, AF055355, NP_199919.1, NM_124485, AAC39475.1, AF055353, NP_196356.1, NM_120821, NP_194239.1, NM_118641, BAB08369.1, AB015475, AAC39478.1, AF055356, AC069143_9, NP_173357.1, NM_101781, NP_172383.1, NM_100780, AAB70398.1, AC000106, AAC39476.1, AF055354, BAB70751.1, AB050661, BAB63664.1, AP003275, AAD24966.1, AF109150.

Besonders bevorzugt umfasst die Polypeptidsequenz der NADPH-Oxidase mindestens ein Sequenzmotiv ausgewählt aus der Gruppe von Sequenzmotiven bestehend aus

i) AL(K/R)GL(K/R)
ii) DK(N/D)XDG(R/K) (I/L/V) (T/N)E
iii) LSASAN
iv) IMEELDP
v) K(F/L)NMA(I/L)(I/V)LXPVCRN
vi) (E/Q)WHPFSIT
vii) S(A/S)PXDD(Q/Y) (L/I)S(I/V)H(V/I/L)R
viii) DGPYG(S/A)PAGDY
ix) L(I/V)GLGIGATP
x) FYWVTREQGSF
xi) GVFYCG

Ganz besonders bevorzugt enthält die Peptidsequenz mindestens 2 oder 3, ganz besonders bevorzugt mindestens 4 oder 5, am meisten bevorzugt alle der Sequenzmotive ausgewählt aus der Gruppe der Sequenzmotive i), ii), iii), iv), v), vi) vii), viii), ix) x) und xi). (Angaben in Klammern meinen alternativ mögliche Aminosäuren an dieser Position; z.B. mein (V/I), dass an dieser Position Valin oder Isoleucin möglich ist).

NADPH-Oxidase kann aber auch jede andere Einheit eines NADPH-Oxidase Enzymkomplexes meinen der wesentlich für Aktivität der NADPH-Oxidase ist.

"Proteinmenge" meint die Menge eines NADPH-Oxidase-Polypeptides in einem Organismus, einem Gewebe, einer Zelle oder einem Zellkompartiment. "Verminderung" der Proteinmenge meint die mengenmäßige Verminderung der Menge einer NADPH-Oxidase in einem Organismus, einem Gewebe, einer Zelle oder einem Zellkompartiment - beispielsweise durch eines der unten beschriebenen Verfahren - im Vergleich zu dem Wildtyp derselben Gattung und Art auf den dieses Verfahren nicht angewendet wurde, unter ansonst gleichen Rahmenbedingungen (wie beispielsweise Kulturbedingungen, Alter der Pflanzen etc.). Der Verminderung beträgt dabei mindestens 10 %, bevorzugt mindestens 10 % oder mindestens 20 %, besonders bevorzugt um mindestens 40 % oder 60 %, ganz besonders bevorzugt um mindestens 70 % oder 80 %, am meisten bevorzugt um mindestens 90 % oder 95 %.

"Aktivität" meint die Fähigkeit einer NADPH-Oxidase molekularen Sauerstoff ($O_2$) zu Superoxid ($O_2^-$) umzusetzen. "Verminderung" der Aktivität meint die Verminderung der Gesamt-Aktivität eines NADPH-Oxidase-Proteins in einem Organismus, einem Gewebe, einer Zelle oder einem Zellkompartiment - beispielsweise durch eines der unten beschriebenen Verfahren - im Vergleich zu dem Wildtyp derselben Gattung und Art auf den dieses Verfahren nicht angewendet wurde, unter ansonst gleichen Rahmenbedingungen (wie beispielsweise Kulturbedingungen, Alter der Pflanzen etc.). Der Verminderung beträgt dabei mindestens 10 %, bevorzugt mindestens 10 % oder mindestens 20 %, besonders bevorzugt um mindestens 40 % oder 60 %, ganz besonders bevorzugt um mindestens 70 % oder 80 %, am meisten bevorzugt um mindestens 90 % oder 95 %.

"Funktion" meint bevorzugt die Substratbindekapazität einer NADPH-Oxidase in einem Organismus, einem Gewebe, einer Zelle oder einem Zellkompartiment. Als Substrate kommen niedermolekulare Verbindungen wie NADPH oder FAD aber auch die Proteininteraktionspartner einer NADPH-Oxidase in Frage.

"Verminderung" der Funktion meint beispielsweise die mengenmäßige Verminderung der Bindekapazität oder Bindestärke einer NADPH-Oxidase zu mindestens einem Substrat in einem Organismus, einem Gewebe, einer Zelle oder einem Zellkompartiment - beispielsweise durch eines der unten beschriebenen Verfahren - im Vergleich zu dem Wildtyp derselben Gattung und Art auf den dieses Verfahren nicht angewendet wurde, unter ansonst gleichen Rahmenbedingungen (wie beispielsweise Kulturbedingungen, Alter der Pflanzen etc.). Unter Verminderung ist auch die Veränderung der Substratspezifität zu verstehen, wie sie beispielsweise durch den kcat/Km-Wert ausgedrückt werden kann. Der Verminderung beträgt dabei mindestens 10 %, bevorzugt mindestens 10 % oder mindestens 20 %, besonders bevorzugt um mindestens 40 % oder 60 %, ganz besonders bevorzugt um mindestens 70 % oder 80 %, am meisten bevorzugt um mindestens 90 % oder 95 %. Bindepartner für NADPH-Oxidase können beispielsweise durch das Hefe-2-Hybridsystem in der dem Fachmann geläufigen Weise identifiziert werden.

Verfahren zur Bestimmung der Proteinmenge, der Aktivität von NADPH Oxidasen oder der Substratbindekapazität sind dem Fachmann bekannt. Beispielsweise kann die NADPH abhängige, DPI-inhibierbare $O_2$- oder $H_2O_2$ Produktion (z.B. über Nitro-Blau-Tetrazolium [NBT] oder Cytochrom c Reduktion) gemessen werden. Die Proteinmenge kann beispielsweise immunologisch unter Verwendung entsprechender Antikörper bestimmt werden. Entsprechende Verfahren sind beschrieben (Yu L et al. (1999) Blood 94(7):2497-504; Doke N (1983a) Physiol Plant Pathol 23:345-357; Levine A et al. (1994) Cell 79:583-593; Tenhaken R et al. (1995) Proc Nat Acad Sci USA 92: 4158-4163; Sagi M & Fluhr R. (2001) Plant Physiol 126(3):1281-90; Hückelhoven R & Kogel KH (1998) Mol Plant Microbe Interact 11:292-300; so wie in den vorgenannten Artikeln zitierten Referenzen).

"Funktionelle Äquivalente" eines NADPH-Oxidase-Proteins meint bevorzugt solche Sequenzen, die von einer NADPH-Oxidase umfassend eine Polypeptidsequenz gemäß SEQ ID NO: 2, 4, 6, 8, 10, 12, 14, 16, 18, 20 oder 22 abgeleitet oder zu dieser homolog sind und die gleichen wesentlichen Eigenschaften aufweisen.

Dabei kann die Effizienz der Pathogenresistenz sowohl nach unten als auch nach oben im Vergleich zu einem Wert erhalten bei Verminderung einer der NADPH-Oxidasen umfassen eine Polypeptidsequenz gemäß SEQ ID NO: 2, 4, 6, 8, 10, 12, 14, 16, 18, 20 oder 22 abweichen. Bevorzugt sind solche funktionelle Äquivalente, bei denen sich die Effizienz der Pathogenresistenz - gemessen beispielsweise an der Penetrationseffizienz eines Pathogens (Haustoriumbildung) - um nicht mehr als 50 %, bevorzugt 25 %, besonders bevorzugt 10 % von einem Vergleichswert erhalten unter Verminderung einer NADPH-Oxidase umfassend eine Polypeptidsequenz gemäß SEQ ID NO: 2, 4, 6, 8, 10, 12, 14, 16, 18, 20 oder 22 unterscheidet. Besonders bevorzugt sind solche Sequenzen, bei deren Verminderung die Effizienz der Pathogenresistenz quantitativ um mehr als 50 %, bevorzugt 100 %, besonders bevorzugt 500 %, ganz besonders bevorzugt 1000 % einen Vergleichswert erhalten bei Verminderung einer der NADPH-Oxidasen umfassend eine Polypeptidsequenz gemäß SEQ ID NO: 2, 4, 6, 8, 10, 12, 14, 16, 18, 20 oder 22 übersteigt.

Der Vergleich wird bevorzugt unter analogen Bedingungen durch geführt. "Analoge Bedingungen" bedeutet, dass alle Rahmenbedingungen wie beispielsweise Kultur- oder Zuchtbedingungen, Assaybedingungen (wie Puffer, Temperatur, Substrate, Pathogenkonzentration etc.) zwischen den zu vergleichenden Versuchen identisch gehalten werden und die Ansätze sich allein durch die Sequenz der zu vergleichenden NADPH-Oxidasen, ihrem Ursprungsorganismus und gegebenenfalls dem Pathogen unterscheiden. Bei Wahl des Pathogens ist für den Vergleich jeweils das Pathogen zu wählen, das dem jeweils anderen - unter Berücksichtigung der Artspezifität - am nächsten kommt.

"Funktionelle Äquivalente" meint insbesondere natürliche oder künstliche Mutationen der NADPH-Oxidasen umfassend eine Polypeptidsequenz gemäß SEQ ID NO: 2, 4, 6, 8, 10, 12, 14, 16, 18, 20 oder 22 sowie homologe Polypeptide aus anderen Pflanzen, welche weiterhin im wesentlichen gleiche Eigenschaften aufweisen. Bevorzugt sind homologe Polypeptide aus oben beschriebenen bevorzugten Pflanzen. Die zu den im Rahmen dieser Erfindung offenbarten NADPH-Oxidase Sequenzen homologen Sequenzen aus anderen Pflanzen (beispielsweise Arabidopsis thaliana) können z.B. durch Datenbanksuche oder Durchmustern von Gen-Banken - unter Verwendung der NADPH-Oxidase-Sequenzen als Suchsequenz vzw. Sonde - leicht aufgefunden werden. Entsprechende Sequenzen sind oben mit GenBank Acc-No. beispielhaft aufgeführt.

Mutationen umfassen Substitutionen, Additionen, Deletionen, Inversion oder Insertionen eines oder mehrerer Aminosäurereste. Somit werden beispielsweise auch solche Polypeptide durch die vorliegende Erfindung mit umfasst, welche man durch Modifikation eines Polypeptides umfassend eine Polypeptidsequenz gemäß SEQ ID NO: 2, 4, 6, 8, 10, 12, 14, 16, 18, 20 oder 22 erhält.

Unter Homologie zwischen zwei Nukleinsäuresequenzen wird die Identität der Nukleinsäuresequenz über die jeweils gesamte Sequenzlänge verstanden, die durch Vergleich mit Hilfe des Programmalgorithmus GAP (Wisconsin Package Version 10.0, University of Wisconsin, Genetics Computer Group (GCG), Madison, USA; Altschul et al. (1997) Nucleic Acids Res. 25:3389ff) unter Einstellung folgender Parameter berechnet wird:

| | |
|---|---|
| Gap Weight: 50 | Length Weight: 3 |
| Average Match: 10 | Average Mismatch: 0 |

Beispielhaft wird unter einer Sequenz, die eine Homologie von mindestens 80 % auf Nukleinsäurebasis mit der Sequenz SEQ ID NO: 1 aufweist, eine Sequenz verstanden, die bei einem Vergleich mit der Sequenz SEQ ID NO: 1 nach obigem Programmalgorithmus mit obigem Parametersatz eine Homologie von mindestens 80 % aufweist.

Unter Homologie zwischen zwei Polypeptiden wird die Identität der Aminosäuresequenz über die jeweils gesamte Sequenzlänge verstanden, die durch Vergleich mit Hilfe des Programmalgorithmus GAP (Wisconsin Package Version 10.0, University of Wisconsin, Genetics Computer Group (GCG), Madison, USA) unter Einstellung folgender Parameter berechnet wird:

| | |
|---|---|
| Gap Weight: 8 | Length Weight: 2 |
| Average Match: 2,912 | Average Mismatch:-2,003 |

Beispielhaft wird unter einer Sequenz, die eine Homologie von mindestens 80 % auf Proteinbasis mit der Sequenz SEQ ID NO: 2 aufweist, eine Sequenz verstanden, die bei einem Vergleich mit der Sequenz SEQ ID NO: 2 nach obigem Programmalgorithmus mit obigem Parametersatz eine Homologie von mindestens 80 % aufweist.

Funktionelle Äquivalente, abgeleitet von einer NADPH-Oxidase umfassend eine Polypeptidsequenz gemäß SEQ ID NO: 2, 4, 6, 8, 10, 12, 14, 16, 18, 20 oder 22 durch Substitution, Insertion oder Deletion, haben eine Homologie von mindestens 50 %, bevorzugt mindestens 70 %, vorzugsweise mindestens 90 %, besonders bevorzugt mindestens 95 %, ganz besonders bevorzugt mindestens 98 % zu einem Polypeptid umfassend eine Polypeptidsequenz gemäß SEQ ID NO: 2, 4, 6, 8, 10, 12, 14, 16, 18, 20 oder 22 und zeichnen sich durch die gleichen wesentlichen Eigenschaften wie diese aus.

Funktionelle Äquivalente, abgeleitet einer eine Sequenz gemäß SEQ ID NO: 1, 3, 5, 7, 9, 11, 13, 15, 17, 19 oder 21 umfassenden NADPH-Oxidase Nukleinsäuresequenz durch Substitution, Insertion oder Deletion, haben eine Homologie von mindestens 50 %, bevorzugt 70 %, vorzugsweise mindestens 90 %, besonders bevorzugt mindestens 95 %, ganz besonders bevorzugt mindestens 98 % zu einem der erfindungsgemäßen Polypeptid gemäß SEQ ID NO: 1, 3, 5, 7, 9, 11, 13, 15, 17, 19 oder 21 und kodieren für Polypeptide mit den gleichen wesentlichen Eigenschaften wie ein Polypeptide umfassend eine Sequenz gemäß SEQ ID NO: 2, 4, 6, 8, 10, 12, 14, 16, 18, 20 oder 22.

Auch die Durchmusterung von cDNA- oder genomischen-Bibliotheken anderer Organismen, bevorzugt von den weiter unten genannten als Wirt zur Transformation geeigneten Pflanzenarten, unter Verwendung der unter SEQ ID NO: 1, 3, 5, 7, 9, 11, 13, 15, 17, 19 oder 21 beschriebene Nukleinsäuresequenzen oder Teilen derselben als Sonde, ist ein dem Fachmann geläufiges Verfahren, um Homologe in anderen Arte zu identifizieren. Dabei haben die von den Nukleinsäuresequenzen gemäß SEQ ID NO: 1, 3, 5, 7, 9, 11, 13, 15, 17, 19 oder 21 abgeleiteten Sonden eine Länge von mindestens 20 bp, bevorzugt mindestens 50 bp, besonders bevorzugt mindestens 100 bp, ganz besonders bevorzugt mindestens 200 bp, am meisten bevorzugt mindestens 400 bp. Für die Durchmusterung der Bibliotheken kann auch ein zu den unter SEQ ID NO: 1, 3, 5, 7, 9, 11, 13, 15, 17, 19 oder 21 beschriebenen Sequenzen komplementärer DNA-Strang eingesetzt werden.

Funktionelle Äquivalente umfasst DNA Sequenzen, die unter Standardbedingungen mit der durch SEQ ID NO: 1, 3, 5, 7, 9, 11, 13, 15, 17, 19 oder 21 beschriebenen NADPH-Oxidase Nukleinsäuresequenzen, der zu ihr komplementären Nukleinsäuresequenz oder teilen der vorgenannten hybridisieren und als vollständige Sequenzen für Proteine kodieren, die die gleichen wesentlichen Eigenschaften haben wie ein Polypeptide umfassend eine Sequenz gemäß SEQ ID NO: 2, 4, 6, 8, 10, 12, 14, 16, 18, 20 oder 22.

"Standardhybridisierungsbedingungen" ist breit zu verstehen und meint stringente als auch weniger stringente Hybridisierungsbedingungen. Solche Hybridisierungsbedingungen sind unter anderem bei Sambrook J, Fritsch EF, Maniatis T et al., in Molecular Cloning (A Laboratory Manual), 2. Auflage, Cold Spring Harbor Laboratory Press, 1989, Seiten 9.31-9.57) oder in Current Protocols in Molecular Biology, John Wiley &Sons, N.Y. (1989), 6.3.1-6.3.6. beschrieben.

Beispielhaft können die Bedingungen während des Waschschrittes ausgewählt sein aus dem Bereich von Bedingungen begrenzt von solchen mit geringer Stringenz (mit ungefähr 2X SSC bei 50°C) und solchen mit hoher Stringenz (mit ungefähr 0.2X SSC bei 50°C bevorzugt bei 65°C) (20X SSC: 0,3M Natriumcitrat, 3M NaCl, pH 7.0). Darüberhinaus kann die Temperatur während des Waschschrittes von niedrig stringenten Bedingungen bei Raumtemperatur, ungefähr 22°C, bis zu stärker stringenten Bedingungen bei ungefähr 65°C angehoben werden. Beide Parameter, Salzkonzentration und Temperatur, können gleichzeitig variiert werden, auch kann einer der beiden Parameter konstant gehalten und nur der andere variiert werden. Während der Hybridisierung können auch denaturierende Agenzien wie zum Beispiel Formamid oder SDS eingesetzt werden. In Gegenwart von 50% Formamid wird die Hybridisierung bevorzugt bei 42°C ausgeführt. Einige beispielhafte Bedingungen für Hybridisierung und Waschschritt sind infolge gegeben:

(1) Hybridisierungbedingungen zum Beispiel aus nachfolgenden Bedingungen ausgewählt sein:

a) 4X SSC bei 65°C (mit - optional - 100 μg/ml denaturierter, fragmentierte Fischsperma-DNA)
b) 6X SSC bei 45°C (mit - optional - 100 μg/ml denaturierter, fragmentierte Fischsperma-DNA),
c) 6X SSC, 0,5 % SDS, 50 % Formamid bei 42°C (mit - optional - 100 μg/ml denaturierter, fragmentierte Fischsperma-DNA)
d) 4XSSC, 50 % Formamid bei 42°C (mit - optional - 100 μg/ml denaturierter, fragmentierte Fischsperma-DNA)
e) 2X oder 4X SSC bei 50°C (schwach stringente Bedingung),
f) 30 bis 40 % Formamid, 2X oder 4X SSC bei 42°C (schwach stringente Bedingung).

(2) Waschschritte können zum Beispiel aus nachfolgenden Bedingungen ausgewählt sein:

a) 0,015 M NaCl/0,0015 M Natriumcitrat/0,1% SDS bei 50°C.
b) 0,1X SSC bei 65°C.
c) 0,1X SSC, 0,5% SDS bei 68°C.
d) 0,1X SSC, 0,5 % SDS, 50 % Formamid bei 42°C.
e) 0,2X SSC, 0,1 % SDS bei 42°C.
f) 2X SSC bei 65°C (schwach stringente Bedingung).

Die Verminderung der Expression eines NADPH-Oxidase-Proteins, der NADPH-Oxidase-Aktivität oder der NADPH-Oxidase-Funktion kann auf vielfältige Art und Weise realisiert werden.

"Verminderung" oder "vermindern" ist im Zusammenhang mit einer NADPH-Oxidase, einer NADPH-Oxidase Aktivität oder NADPH-Oxidase-Funktion weit auszulegen und umfasst die teilweise oder im wesentlichen vollständige, auf unterschiedliche zellbiologische Mechanismen beruhende Unterbindung oder Blockierung der Funktionalität einer NADPH-Oxidase in einer Pflanze oder einem davon abgeleiteten Teil, Gewebe, Organ, Zellen oder Samen. Eine Verminderung im Sinne der Erfindung umfasst auch eine mengenmäßige Verringerung einer NADPH-Oxidase bis hin zu einem im wesentlichen vollständigen Fehlen der NADPH-Oxidase (d.h. fehlende Nachweisbarkeit von NADPH-Oxidase-Aktivität bzw. NADPH-Oxidase-Funktion oder fehlende immunologische Nachweisbarkeit des NADPH-Oxidase-Proteins). Dabei können einer oder mehrere essentielle Einheiten der NADPH-Oxidase vermindert werden. Dabei wird die Expression eines bestimmter NADPH-Oxidase oder die NADPH-Oxidase-Aktivität bzw. NADPH-Oxidase-Funktion in einer Zelle oder einem Organismus bevorzugt um mehr als 50 %, besonders bevorzugt um mehr als 80 %, ganz besonders bevorzugt um mehr als 90% vermindert.

Erfindungsgemäß sind verschiedene Strategien zur Verminderung der Expression eines NADPH-Oxidase-Proteins, der NADPH-Oxidase-Aktivität oder NADPH-Oxidase-Funktion umfasst. Beispielhaft - jedoch nicht einschränkend - seien zu nennen:

a) Einbringen einer doppelsträngigen NADPH-Oxidase RNA-Nukleinsäuresequenz (NAox-dsRNA) oder einer deren Expression gewährleistenden Expressionskassette oder Expressionskassetten;

b) Einbringen einer NADPH-Oxidase antisense-Nukleinsäuresequenzen oder einer deren Expression gewährleistenden Expressionskassette. Umfasst sind solche Verfahren, bei denen die antisense-Nukleinsäuresequenz gegen ein NADPH-Oxidase-Gen (also genomische DNA-Sequenzen) oder ein NADPH-Oxidase-Gentranskript (also RNA-Sequenzen) gerichtet ist. Umfasst sind auch
α-anomere Nukleinsäuresequenzen.

c) Einbringen einer NADPH-Oxidase antisense-Nukleinsäuresequenzen kombiniert mit einem Ribozym oder einer deren Expression gewährleistenden Expressionskassette

d) Einbringen von NADPH-Oxidase sense-Nukleinsäuresequenzen zur Induktion einer Kosuppression oder einer deren Expression gewährleistenden Expressionskassette

e) Einbringen DNA-oder Protein-bindende Faktoren gegen NADPH-Oxidase -Gene, -RNAs oder -Proteine oder einer deren Expression gewährleistenden Expressionskassette

f) Einbringen von den NADPH-Oxidase RNA-Abbau bewirkende virale Nukleinsäuresequenzen und Expressionskonstrukten oder einer deren Expression gewährleistenden Expressionskassette

g) Einbringung von Konstrukten zur Induktion einer homologen Rekombination an endogenen NADPH-Oxidase-Genen beispielsweise zur Erzeugung von Knockout-Mutanten.

h) Einführen von Mutationen in endogenen NADPH-Oxidase Gene zur Erzeugung eines Funktionsverlustes (z.B. Generierung von Stopp-Kodons, Verschiebungen im Leseraster etc.)

Dabei kann jedes einzelne dieser Verfahren eine Verminderung der NADPH-Oxidase-Expression, NADPH-Oxidase-Aktivität oder NADPH-Oxidase-Funktion im Sinne der Erfindung bewirken. Auch eine kombinierte Anwendung ist denkbar. Weitere Methoden sind dem Fachmann bekannt und können die Behinderung oder Unterbindung der Prozessierung des NADPH-Oxidase-Proteins, des Transports des NADPH-Oxidase-Proteins oder dessen mRNA, Hemmung der Ribosomenanlagerung, Hemmung des RNA-Spleißens, Induktion eines NADPH-Oxidase-RNA abbauenden Enzyms und/oder Hemmung der Translationselongation oder -termination umfassen.

Die einzelnen bevorzugten Verfahren seien infolge kurz beschrieben:

a) Einbringung einer doppelsträngigen NADPH-Oxidase RNA-Nukleinsäuresequenz (NAox-dsRNA)

Das Verfahren der Genregulation mittels doppelsträngiger RNA ("double-stranded RNA interference"; dsRNAi) ist vielfach in tierischen und pflanzlichen Organismen beschrieben (z.B. Matzke MA et al. (2000) Plant Mol Biol 43: 401-415; Fire A. et al (1998) Nature 391:806-811; WO 99/32619; WO 99/53050; WO 00/68374; WO 00/44914; WO 00/44895; WO 00/49035; WO 00/63364). Auf die in den angegebenen Zitaten beschriebenen Verfahren und Methoden wird ausdrücklich Bezug genommen. Eine effiziente Gensuppression kann auch bei transienter Expression oder nach transienter Transformation beispielsweise infolge einer biolistischen Transformation gezeigt werden (Schweizer P et al. (2000) Plant J 2000 24:895-903). dsRNAi-Verfahren beruhen auf dem Phänomen, dass durch gleichzeitiges Einbringen von komplementären Strang- und Gegenstrang eines Gentranskriptes eine hocheffiziente Unterdrückung der Expression des entsprechenden Gens bewirkt wird. Der bewirkte Phänotyp kommt dem einer entsprechenden knock-out Mutanten sehr ähnlich (Waterhouse PM et al. (1998) Proc Natl Acad Sci USA 95:13959-64).

Das dsRNAi-Verfahren hat sich bei der Verminderung der NADPH-Oxidase-Expression als besonders effizient und vorteilhaft erwiesen. Wie u.a. in WO 99/32619 beschrieben sind dsRNAi-Ansätze klassischen antisense-Ansätzen deutlich überlegen.

Ein weiterer Gegenstand der Erfindung bezieht sich daher auf doppelsträngige RNA-Moleküle (dsRNA-Moleküle), die bei Einführung in eine Pflanze (oder eine davon abgeleitete Zelle, Gewebe, Organ oder Samen) die Verminderung eines NADPH-Oxidase bewirken.

Das doppelsträngiges RNA-Molekül zur Verminderung der Expression eines NADPH-Oxidase Proteins ist dadurch gekennzeichnet, dass

a) einen "sense"-RNA-Strang umfassend mindestens eine Ribonukleotidsequenz, die im wesentlichen identisch ist zu zumindest einem Teil einer NADPH-Oxidase-Nukleinsäuresequenz, und
b) einen "antisense"-RNA-Strang, der zu dem RNA-sense-Strang unter a) im wesentlichen - bevorzugt vollständig - komplementären ist.

In einer weiterhin bevorzugten Ausführungsform umfasst das doppelsträngige RNA-Molekül zur Verminderung der Expression eines NADPH-Oxidase Proteins

a) einen "sense"-RNA-Strang umfassend mindestens eine Ribonukleotidsequenz, die im wesentlichen identisch ist zu mindestens einem Teil des "sense"-RNA-Transkriptes einer Nukleinsäuresequenz kodierend für ein NADPH-Oxidase Protein, und

b) einen "antisense"-RNA-Strang, der zu dem RNA-sense-Strang unter a) im wesentlichen - bevorzugt vollständig - komplementären ist.
In Bezug auf die doppelsträngigen RNA-Moleküle meint NADPH-Oxidase-Nukleinsäuresequenz bevorzugt eine Sequenz umfassend eine Sequenz gemäß SEQ ID NO: 1, 3, 5, 7, 9, 11, 13, 15, 17, 19 oder 21.

Im wesentlichen identisch" meint, dass die dsRNA Sequenz auch Insertionen, Deletionen sowie einzelne Punktmutationen im Vergleich zu der NADPH-Oxidase Zielsequenz oder einer funktionell äquivalenten Zielsequenz aufweisen kann und dennoch eine effizient Verminderung der Expression bewirken. Bevorzugt beträgt die Homologie nach obiger Definition mindestens 75 %, bevorzugt mindestens 80 %, ganz besonders bevorzugt mindestens 90 % am meisten bevorzugt 100 % zwischen dem "sense"-Strang einer inhibitorischen dsRNA und mindestens einem Teil des "sense"-RNA-Transkriptes einer Nukleinsäuresequenz kodierend für ein NADPH-Oxidase Protein oder ein funktionelles Äquivalent desselben (bzw. zwischen dem "antisense"-Strang dem komplementären Strang einer Nukleinsäuresequenz kodierend für ein NADPH-Oxidase Protein oder ein funktionelles Äquivalent desselben).

Die Länge des Teilabschnittes beträgt mindestens 10 Basen, bevorzugt mindestens 25 Basen, besonders bevorzugt mindestens 50 Basen, ganz besonders bevorzugt mindestens 100 Basen, am meisten bevorzugt mindestens 200 Basen oder mindestens 300 Basen.

Alternativ, kann eine "im wesentlichen identische" dsRNA auch als Nukleinsäuresequenz definiert werden, die befähigt ist, mit einem Teil eines Speicherprotein Gentranskriptes zu hybridisieren (z.B. in 400 mM NaCl, 40 mM PIPES pH 6,4, 1 mM EDTA bei 50°C oder 70°C für 12 bis 16 h).

"Im wesentlichen komplementär" meint, dass der "antisense"-RNA-Strang auch Insertionen, Deletionen sowie einzelne Punktmutationen im Vergleich zu dem Komplement des "sense"-RNA-Stranges aufweisen kann. Bevorzugt beträgt die Homologie mindestens 80 %, bevorzugt mindestens 90 %, ganz besonders bevorzugt mindestens 95 %, am meisten bevorzugt 100% zwischen dem "anti-sense"-RNA-Strang und dem Komplement des "sense"-RNA-Strangs.

"Teil des "sense"-RNA-Transkriptes" einer Nukleinsäuresequenz kodierend für ein NADPH-Oxidase Protein oder ein funktionelles Äquivalent desselben meint Fragmente einer RNA oder mRNA transkribiert von einer für ein NADPH-Oxidase-Protein oder ein funktionelles Äquivalent desselben kodierenden Nukleinsäuresequenz, bevorzugt von einem NADPH-Oxidase-Gen. Dabei haben die Fragmente bevorzugt eine Sequenzlänge von mindestens 20 Basen, bevorzugt mindestens 50 Basen, besonders bevorzugt mindestens 100 Basen, ganz besonders bevorzugt mindestens 200 Basen, am meisten bevorzugt mindestens 500 Basen. Umfasst ist auch die vollständige transkribierte RNA oder mRNA.

Umfasst ist auch die Verwendung der erfindungsgemäßen dsRNA-Moleküle in den erfindungsgemäßen Verfahren zur Erzeugung einer Pathogenresistenz in Pflanzen.

Die dsRNA kann aus einem oder mehr Strängen polymerisierter Ribonukleotide bestehen. Es können ferner Modifikationen sowohl des Zucker-Phosphat-Gerüstes als auch der Nukleoside vorliegen. Beispielsweise können die Phosphodiesterbindungen der natürlichen RNA dahingehend modifiziert sein, dass sie zumindest ein Stickstoff oder Schwefel-Heteroatom umfassen. Basen können dahingehend modifiziert werden, dass die Aktivität beispielsweise von Adenosindeaminase eingeschränkt wird. Solche und weitere Modifikationen sind weiter unten bei den Verfahren zur Stabilisierung von antisense-RNA beschrieben.

Natürlich können, um den gleichen Zweck zu erreichen, auch mehrere individuelle dsRNA Moleküle, die jeweils einen der oben definierten Ribonukleotidsequenzabschnitte umfassen, in die Zelle oder den Organismus eingebracht werden.

Die dsRNA kann enzymatisch oder ganz oder teilweise chemischsynthetisch hergestellt werden.

Die doppelsträngige dsRNA Struktur kann ausgehend von zwei komplementären, separaten RNA-Strängen oder - bevorzugt - ausgehend von einem einzelnen, selbstkomplementären RNA-Strang gebildet werden.

Bei einem einzelnen, selbstkomplementären Strang, können "sense"- und "antisense"-Sequenz durch eine verbindende Sequenz ("Linker") verknüpft sein und beispielsweise eine Haarnadelstruktur ausbilden. Bevorzugt kann die verbindende Sequenz ein Intron sein, das nach Synthese der dsRNA herausgespleißt wird.

Die Nukleinsäuresequenz kodierend für eine dsRNA kann weitere Elemente beinhalten, wie beispielsweise Transkriptionsterminationssignale oder Polyadenylierungssignale.

Sollen die zwei Stränge der dsRNA in einer Zelle oder Pflanze zusammengebracht werden, so kann dies auf verschiedene Art geschehen:

a) Transformation der Zelle oder Pflanze mit einem Vektor, der beide Expressionskassetten umfasst,

b) Kotransformation der Zelle oder Pflanze mit zwei Vektoren, wobei der eine die Expressionskassetten mit dem "sense"-Strang, der andere die Expressionskassetten mit dem "antisense"-Strang umfasst.

c) Kreuzung von zwei Pflanzen, die mit jeweils einem Vektor transformiert wurden, wobei der eine die Expressionskassetten mit dem "sense"-Strang, der andere die Expressionskassetten mit dem "antisense"-Strang umfasst.

Die Bildung der RNA Duplex kann entweder außerhalb der Zelle oder innerhalb derselben initiiert werden. Wie in WO 99/53050 kann die dsRNA auch eine Haarnadelstruktur umfassen, indem "sense"- und "antisense"-Strang durch einen "Linker" (beispielsweise ein Intron) verbunden werden. Die selbstkomplementären dsRNA-Strukturen sind bevorzugt, da sie lediglich die Expression eines Konstruktes erfordern und die komplementären Stränge stets in einem äquimolaren Verhältnis umfassen.

Die Expressionskassetten kodierend für den "antisense"- oder "sense"-Strang einer dsRNA oder für den selbstkomplementären-Strang der dsRNA, werden bevorzugt in einen Vektor insertiert und mit den unten beschriebenen Verfahren stabil (beispielsweise unter Verwendung von Selektionsmarkern) in das Genom einer Pflanze insertiert, um eine dauerhafte Expression der dsRNA zu gewährleisten.

Die dsRNA kann unter Verwendung einer Menge eingeführt werden, die zumindest ein Kopie pro Zelle ermöglicht. Höhere Mengen (z.B. mindestens 5, 10, 100, 500 oder 1000 Kopien pro Zelle) können ggf. eine effizienter Verminderung bewirken.

Wie bereits beschrieben, ist eine 100%ige Sequenzidentität zwischen dsRNA und einem NADPH-Oxidase Gentranskript oder dem Gentranskript eines funktionell äquivalenten Gens nicht zwingend erforderlich, um eine effiziente Verminderung der NADPH-Oxidase Expression zu bewirken. Demzufolge besteht der Vorteil, dass das Verfahren tolerant ist gegenüber Sequenzabweichungen, wie sie infolge genetischer Mutationen, Polymorphismen oder evolutionärer Divergenzen vorliegen können. So ist es beispielsweise möglich mit der dsRNA, die ausgehend von der NADPH-Oxidase Sequenz des einen Organismus generiert wurde, die NADPH-Oxidase Expression in einem anderen Organismus zu unterdrücken. Die hohe Sequenzhomologie zwischen den NADPH-Oxidase Sequenzen aus Reis, Mais und Gerste lässt auf einen hohen Konservierungsgrad dieses Proteins innerhalb von Pflanzen schließen, so dass die Expression einer dsRNA abgeleitet von einer der NADPH-Oxidase Sequenzen umfassend eine Sequenz gemäß SEQ ID NO: 1, 3, 5, 7, 9, 11, 13, 15, 17, 19 oder 21 auch einen vorteilhaften Effekt in anderen Pflanzenarten haben dürfte.

Auch ist es aufgrund der hohen Homologie zwischen den einzelnen NADPH-Oxidase-Proteinen und ihren funktionellen Äquivalenten möglich mit einer einzigen dsRNA, die ausgehend von einer bestimmten NADPH-Oxidase-Sequenz eines Organismus generiert wurde, die Expression weiterer homologer NADPH-Oxidase-Proteine und/oder deren funktioneller Äquivalente des gleichen Organismus oder aber auch die Expression von NADPH-Oxidase-Proteinen in anderen verwandten Arten zu unterdrücken. Zu diesem Zweck umfasst die dsRNA bevorzugt Sequenzbereich von NADPH-Oxidase-Gentranskripten, die konservierten Bereichen entsprechen. Besagte konservierte Bereiche können aus Sequenzvergleichen leicht abgeleitet werden.

Die dsRNA kann entweder in vivo oder in vitro synthetisiert werden. Dazu kann eine DNA-Sequenz kodierend für eine dsRNA in eine Expressionskassette unter Kontrolle mindestens eines genetischen Kontrollelementes (wie beispielsweise Promotor, Enhancer, Silencer, Splice-Donor oder -Akzeptor, Polyadenylierungssignal) gebracht werden. Entsprechend vorteilhafte Konstruktionen sind weiter unten beschrieben. Eine Polyadenylierung ist nicht erforderlich, ebenso müssen keine Elemente zur Initiierung einer Translation vorhanden sein.

Eine dsRNA kann chemisch oder enzymatisch synthetisiert werden. Dazu können zelluläre RNA Polymerasen oder Bakteriophagen RNA Polymerasen (wie z.B. T3-, T7- oder SP6 RNA-Polymerase) verwendet werden. Entsprechende Verfahren zu in vitro Expression von RNA sind beschrieben (WO 97/32016; US 5,593,874; US 5,698,425, US 5,712,135, US 5,789,214, US 5,804,693). Eine chemisch oder enzymatisch in vitro syntetisierte dsRNA kann vor der Einführung in eine Zelle, Gewebe oder Organismus aus dem Reaktionsgemisch beispielsweise durch Extraktion, Präzipitation, Elektrophorese, Chromatographie oder Kombinationen dieser Verfahren ganz oder teilweise aufgereinigt werden. Die dsRNA kann unmittelbar in die Zelle eingeführt werden oder aber auch extrazellulär (z.B. in den interstitialen Raum) appliziert werden.

Bevorzugt wird die Pflanze jedoch stabil mit einem Expressionskonstrukt, das die Expression der dsRNA realisiert, transformiert. Entsprechende Verfahren sind weiter unten beschrieben.

b) Einbringung einer NADPH-Oxidase antisense-Nukleinsäuresequenz

Verfahren zur Suppression eines bestimmten Proteins durch Verhinderung der Akkumulation seiner mRNA durch die "antisense"-Technologie sind vielfach - auch in Pflanzen - beschrieben (Sheehy et al. (1988) Proc Natl Acad Sci USA 85: 8805-8809; US 4,801,340; Mol JN et al. (1990) FEBS Lett 268(2):427-430). Das antisense Nukleinsäuremolekül hybridisiert bzw. bindet mit der zellulären mRNA und/oder genomischen DNA kodierend für das zu supprimierende NADPH-Oxidase-Zielprotein. Dadurch wird die Transkription und/oder Translation des Zielproteins unterdrückt. Die Hybridisierung kann auf konventionelle Art über die Bildung einer stabilen Duplex oder - im Fall von genomischer DNA - durch Bindung des antisense Nukleinsäuremoleküls mit der Duplex der genomischen DNA durch spezifische Wechselwirkung in der großen Furche der DNA-Helix entstehen.

Eine antisense Nukleinsäuresequenz geeignet zur Verminderung eines NADPH-Oxidase-Proteins kann unter Verwendung der für dieses Protein kodierenden Nukleinsäuresequenz, beispielsweise der Nukleinsäuresequenz umfassend eine Sequenz gemäß SEQ ID NO: 1, 3, 5, 7, 9, 11, 13, 15, 17, 19 oder 21, nach den Basenpaarregeln von Watson und Crick abgeleitet werden. Die antisense Nukleinsäuresequenz kann zu der gesamten transkribierten mRNA des besagten Proteins komplementär sein, sich auf die kodierende Region beschränken oder nur aus einem Oligonukleotid bestehen, das zu einem Teil der kodierenden oder nicht-kodierenden Sequenz der mRNA komplementär ist. So kann das Oligonukleotid beispielsweise komplementär zu der Region sein, die den Translationsstart für das besagte Protein umfasst. Anti-sense-Nukleinsäuresequenzen können eine Länge von zum Beispiel 5, 10, 15, 20, 25, 30, 35, 40, 45 oder 50 Nukleotide haben, können aber auch länger sein und mindestens 100, 200, 500, 1000, 2000 oder 5000 Nukleotide umfassen. Antisense-Nukleinsäuresequenzen können rekombinant exprimiert oder chemisch bzw. enzymatisch unter Verwendung von dem Fachmann bekannten Verfahren synthetisiert werden. Bei der chemischen Synthese können natürlich oder modifizierte Nukleotide verwendet werden. Modifizierte Nukleotide können der antisense Nukleinsäuresequenz eine erhöhte biochemische Stabilität verleihen und zu einer erhöhten physikalischen Stabilität der Duplex gebildet aus antisense-Nukleinsäuresequenz und sense-Zielsequenz führen. Verwendet werden können beispielsweise Phosphorothioatderivative und Acridin-substitierte Nukleotide wie 5-Fluorouracil, 5-Bromouracil, 5-Chlorouracil, 5-Iodouracil, Hypoxanthin, Xanthin, 4-Acetylcytosin, 5-(Carboxyhydroxylmethyl)uracil, 5-Carboxymethylaminomethyl-2-thiouridin, 5-Carboxymethylaminomethyluracil, Dihydrouracil, β-D-Galactosylqueosin, Inosine, N6-Isopentenyladenin, 1-Methylguanin, 1-Methylinosin, 2,2-Dimethylguanin, 2-Methyladenin, 2-Methylguanin, 3-Methylcytosin, 5-Methylcytosin, N6-Adenin, 7-Methylguanin, 5-Methylaminomethyluracil, 5-Methoxyaminomethyl-2-thiouracil, β-D-mannosylqueosin, 5'-Methoxycarboxymethyluracil, 5-Methoxyuracil, 2-Methylthio-N6-isopentenyladenin, Uracil-5-oxyessigsäure, Pseudouracil, Queosine, 2-Thiocytosin, 5-Methyl-2-thiouracil, 2-Thiouracil, 4-Thiouracil, 5-Methyluracil, Uracil-5-oxyessigsäuremethylester, Uracil-5-oxyessigsäure, 5-Methyl- 2-thiouracil, 3-(3-Amino-3-N-2-carboxypropyl)uracil und 2,6-Diaminopurin.

In einer weiteren bevorzugten Ausführungsform kann die Expression eines NADPH-Oxidase-Proteins durch Nukleotidsequenzen inhibiert werden, die komplementär zu der regulatorischen Region eines NADPH-Oxidase-Gens (z.B. einem NADPH-Oxidase Promoter und/oder Enhancer) sind und triple-helikale Strukturen mit der dortigen DNA-Doppelhelix ausbilden, so dass die Transkription des NADPH-Oxidase-Gens vermindert wird. Entsprechende Verfahren sind beschrieben (Helene C (1991) Anticancer Drug Res 6(6):569-84; Helene C et al. (1992) Ann NY Acad Sci 660: 27-36; Maher LJ (1992) Bioassays 14(12):807-815).

In einer weiteren Ausführungsform kann das antisense Nukleinsäuremolekül eine α-anomere Nukleinsäure sein. Derartige α-anomere Nukleinsäuremoleküle bilden spezifische doppelsträngige Hybride mit komplementärer RNA in denen - im Unterschied zu den konventionellen β-Nukleinsäuren - die beiden Stränge parallel zueinander verlaufen (Gautier C et al. (1987) Nucleic Acids Res 15:6625-6641). Das antisense Nukleinsäuremolekül kann ferner auch 2'-O-Methylribonukleotide (Inoue et al. (1987) Nucleic Acids Res 15:6131-6148) oder chimäre RNA-DNA Analoge beinhalten (Inoue et al. (1987) FEBS Lett 215:327-330).

c) Einbringung einer NADPH-Oxidase antisense-Nukleinsäuresequenz kombiniert mit einem Ribozym

Vorteilhaft kann die oben beschriebene antisense-Strategie mit einem Ribozym-Verfahren gekoppelt werden. Katalytische RNA-Moleküle oder Ribozyme können an jede beliebige Ziel-RNA angepasst werden und spalten das Phosphodiester-Gerüst an spezifischen Positionen, wodurch die Ziel-RNA funktionell deaktiviert wird (Tanner NK (1999) FEMS Microbiol Rev 23(3):257-275). Das Ribozym wird dadurch nicht selber modifiziert, sondern ist in der Lage, weitere Ziel-RNA-Moleküle analog zu spalten, wodurch es die Eigenschaften eines Enzyms erhält. Der Einbau von Ribozymsequenzen in "antisense"-RNAs verleiht eben diesen "anti-sense"-RNAs diese enzymähnliche, RNA-spaltende Eigenschaft und steigert so deren Effizienz bei der Inaktivierung der Ziel-RNA. Die Herstellung und Verwendung entsprechender Ribozym-"anti-sense"-RNA-Moleküle ist beispielsweise beschrieben bei Haseloff et al. (1988) Nature 334:585-591.

Auf diese Art können Ribozyme (z.B. "Hammerhead"-Ribozyme; Haselhoff und Gerlach (1988) Nature 334: 585-591) verwendet werden, um die mRNA eines zu supprimierenden Enzyms - z.B. NADPH-Oxidase - katalytisch zu spalten und die Translation zu verhindern. Die Ribozym-Technologie kann die Effizienz einer antisense-Strategie erhöhen. Verfahren zur Expression von Ribozymen zur Verminderung bestimmter Proteine sind beschrieben in (EP 0 291 533, EP 0 321 201, EP 0 360 257). In pflanzlichen Zellen ist eine Ribozym-Expression ebenfalls beschrieben (Steinecke P et al. (1992) EMBO J 11(4):1525-1530; de Feyter R et al. (1996) Mol Gen Genet. 250(3):329-338). Geeignete Zielsequenzen und Ribozyme können zum Beispiel wie bei "Steinecke P, Ribozymes, Methods in Cell Biology 50, Galbraith et al. eds, Academic Press, Inc. (1995), S. 449-460" beschrieben, durch Sekundärstrukturberechnungen von Ribozym- und Ziel-RNA sowie durch deren Interaktion bestimmt werden (Bayley CC et al. (1992) Plant Mol Biol. 18(2):353-361; Lloyd AM and Davis RW et al. (1994) Mol Gen Genet. 242(6):653-657). Beispielsweise können Derivate der Tetrahymena L-19 IVS RNA konstruiert werden, die komplementäre Bereiche zu der mRNA des zu supprimierenden NADPH-Oxidase Proteins aufweisen (siehe auch US 4,987,071 und US 5,116,742). Alternativ können solche Ribozyme auch über einen Selektionsprozess aus einer Bibliothek diverser Ribozyme identifiziert werden (Bartel D und Szostak JW (1993) Science 261:1411-1418).

d) Einbringung einer NADPH-Oxidase sense-Nukleinsäuresequenz zur Induktion eines Kosuppression

Die Expression einer NADPH-Oxidase Nukleinsäuresequenz in sense-Orientierung kann zu einer Kosuppression des entsprechenden homologen, endogenen Gens führen. Die Expression von sense-RNA mit Homologie zu einem endogenen Gen kann die Expression desselben vermindern oder ausschalten , ähnlich wie es für antisense Ansätze beschrieben wurde (Jorgensen et al. (1996) Plant Mol Biol 31(5):957-973; Goring et al. (1991) Proc Natl Acad Sci USA 88:1770-1774; Smith et al. (1990) Mol Gen Genet 224:447-481; Napoli et al. (1990) Plant Cell 2:279-289; Van der Krol et al. (1990) Plant Cell 2:291-99). Dabei kann das eingeführte Konstrukt das zu vermindernde, homologe Gen ganz oder nur teilweise representieren. Die Möglichkeit zur Translation ist nicht erforderlich. Die Anwendung dieser Technologie auf Pflanzen ist beispielsweise beschrieben bei Napoli et al. (1990) The Plant Cell 2: 279-289 und in US 5,034,323.

Bevorzugt wird die Kosuppression unter Verwendung einer Sequenz realisiert, die im wesentlichen identisch ist zu zumindest einem Teil der Nukleinsäuresequenz kodierend für ein NADPH-Oxidase-Protein oder ein funktionelles Äquivalent desselben, beispielsweise der Nukleinsäuresequenz umfassend eine Sequenz gemäß SEQ ID NO: 1, 3, 5, 7, 9, 11, 13, 15, 17, 19 oder 21.

e) Einbringung DNA-oder Protein-bindende Faktoren gegen NADPH-Oxidase Gene, -RNAs oder Proteine

Eine Verminderung einer NADPH-Oxidase Genexpression ist auch mit spezifischen DNA-bindenden Faktoren z.B. mit Faktoren vom Typus der Zinkfingertranskriptionsfaktoren möglich. Diese Faktoren lagern sich an die genomische Sequenz des endogenen Zielgens, bevorzugt in den regulatorischen Bereichen, an und bewirken eine Repression des endogenen Gens. Die Verwendung eines solchen Verfahrens ermöglicht die Verminderung der Expression eines endogenen NADPH-Oxidase Gens, ohne dass dessen Sequenz gentechnisch manipuliert werden muss. Entsprechende Verfahren zur Herstellung entsprechender Faktoren sind beschrieben (Dreier B et al. (2001) J Biol Chem 276(31): 29466-78; Dreier B et al. (2000) J Mol Biol 303(4):489-502; Beerli RR et al. (2000) Proc Natl Acad Sci USA 97 (4): 1495-1500; Beerli RR et al. (2000) J Biol Chem 275(42):32617-32627; Segal DJ and Barbas CF 3rd. (2000) Curr Opin Chem Biol 4(1):34-39; Kang JS and Kim JS (2000) J Biol Chem 275(12):8742-8748; Beerli RR et al. (1998) Proc Natl Acad Sci USA 95(25):14628- 14633; Kim JS et al. (1997) Proc Natl Acad Sci USA 94(8):3616 -3620; Klug A (1999) J Mol Biol 293(2):215-218; Tsai SY et al. (1998) Adv Drug Deliv Rev 30(1-3):23-31; Mapp AK et al. (2000) Proc Natl Acad Sci USA 97(8):3930-3935; Sharrocks AD et al. (1997) Int J Biochem Cell Biol 29(12):1371-1387; Zhang L et al. (2000) J Biol Chem 275(43):33850-33860).

Die Selektion dieser Faktoren kann unter Verwendung eines beliebigen Stückes eines NADPH-Oxidase-Gens erfolgen. Bevorzugt liegt dieser Abschnitt im Bereich der Promotorregion. Für eine Genunterdrückung kann er aber auch im Bereich der kodierenden Exons oder Introns liegen. Die entsprechenden Abschnitte sind für den Fachmann mittels Datenbankabfrage aus der Genbank oder - ausgehend von einer NADPH-Oxidase cDNA, deren Gen nicht in der Genbank vorhanden ist, durch Durchmusterung einer genomischen Bibliothek nach korrespondierenden genomischen Klonen erhältlich. Die dazu erforderlichen Verfahren sind dem Fachmann geläufig.

Ferner können Faktoren in eine Zelle eingebracht werden, die das NADPH-Oxidase Zielprotein selber inhibieren. Die proteinbindenden Faktoren können z.B. Aptamere (Famulok M und Mayer G (1999) Curr Top Microbiol Immunol 243:123-36) oder Antikörper bzw. Antikörperfragmente oder einzelkettige Antikörper sein. Die Gewinnung dieser Faktoren ist beschrieben und dem Fachmann bekannt. Beispielsweise wurde ein cytoplasmatischer scFv Antikörper eingesetzt, um die Aktivität des Phytochrom A Proteins in gentechnisch veränderten Tabakpflanzen zu modulieren (Owen M et al. (1992) Biotechnology (N Y) 10(7):790-794; Franken E et al. (1997) Curr Opin Biotechnol 8(4): 411-416; Whitelam (1996) Trend Plant Sci 1:286-272).

Die Genexpression kann auch durch maßgeschneiderte, niedermolekulare synthetische Verbindungen unterdrückt werden, beispielsweise vom Polyamid-Typ (Dervan PB und Bürli RW (1999) Current Opinion in Chemical Biology 3:688-693; Gottesfeld JM et al. (2000) Gene Expr 9(1-2):77-91). Diese Oligomere bestehen aus den Bausteinen 3-(Dimethylamino)propylamin, N-Methyl-3-hydroxypyrrol, N-Methylimidazol und N-Methylpyrrole und können an jedes Stück doppelsträngiger DNA so angepasst werden, dass sie sequenzspezifisch in die große Furche binden und die Expression der dortigen Genesequenzen blockieren. Entsprechende Verfahren sind beschrieben (siehe unter anderem Bremer RE et al. (2001) Bioorg Med Chem. 9(8):2093-103; Ansari AZ et al. (2001) Chem Biol. 8(6):583-92; Gottesfeld JM et al. (2001) J Mol Biol. 309(3):615-29; Wurtz NR et al. (2001) Org Lett 3(8):1201-3; Wang CC et al. (2001) Bioorg Med Chem 9(3):653-7; Urbach AR und Dervan PB (2001) Proc Natl Acad Sci USA 98(8):4343-8; Chiang SY et al. (2000) J Biol Chem. 275(32):24246-54).

f) Einbringung von den NADPH-Oxidase RNA-Abbau bewirkenden viralen Nukleinsäuresequenzen und Expressionskonstrukten

Die NADPH-Oxidase Expression kann effektiv auch durch Induktion des spezifischen NADPH-Oxidase RNA-Abbaus durch die Pflanze mit Hilfe eines viralen Expressionssystems (Amplikon) (Angell, SM et al. (1999) Plant J. 20 (3):357-362) realisiert werden. Diese Systeme - auch als "VIGS" (viral induced gene silencing) bezeichnet - bringen Nukleinsäureseuqnzen mit Homologie zu den zu supprimierenden Transkripten mittels viraler Vektoren in die Pflanze ein. Die Transkription wird sodann - vermutlich mediiert durch pflanzliche Abwehrmechanismen gegen Viren - abgeschaltet. Entsprechende Techniken und Verfahren sind beschrieben (Ratcliff F et al. (2001) Plant J 25(2):237-45; Fagard M und Vaucheret H (2000) Plant Mol Biol 43(2-3):285-93; Anandalakshmi R et al. (1998) Proc Natl Acad Sci USA 95(22):13079-84; Ruiz MT (1998) Plant Cell 10(6): 937-46).

g) Einbringung von Konstrukten zur Induktion einer homologen Rekombination an endogenen NADPH-Oxidase-Genen beispielsweise zur Erzeugung von Knockout-Mutanten.

Zur Herstellung eines homolog rekombinanten Organismus mit verminderter NADPH-Oxidase-Aktivität verwendet man beispielsweise ein Nukleinsäurekonstrukt, das zumindest einen Teil eines endogenen NADPH-Oxidase Gens enthält, das durch eine Deletion, Addition oder Substitution mindestens eines Nukleotids so verändert wird, so dass die Funktionalität vermindert oder gänzlich aufgehoben wird. Die Veränderung kann auch die regulativen Elemente (z.B. den Promotor) des Gens betreffen, so dass die kodierende Sequenz unverändert bleibt, eine Expression (Transkription und/oder Translation) jedoch unterbleibt und vermindert wird.

Bei der konventionellen homologen Rekombination ist die veränderte Region an ihrem 5'- und 3'-Ende von weiteren Nukleinsäuresequenzen flankiert, die eine ausreichende Länge für die Ermöglichung der Rekombination aufweisen müssen. Die Länge liegt in der Regel in einem Bereich von mehreren einhundert Basen bis zu mehreren Kilobasen (Thomas KR und Capecchi MR (1987) Cell 51:503; Strepp et al. (1998) Proc Natl Acad Sci USA 95(8):4368-4373). Für die homologe Rekombination wird der Wirtsorganismus- zum Beispiel eine Pflanze - mit dem Rekombinationskonstrukt unter Verwendung der unten beschriebenen Verfahren transformiert und erfolgreich rekombinierte Klone unter Verwendung zum Beispiel einer Antibiotika- oder Herbizidresistenz selektioniert.

Homologe Rekombination ist ein relativ seltenes Ereignis in höheren Eukaryoten, vor allem in Pflanzen. Zufällige Integrationen in das Wirtsgenom überwiegen. Eine Möglichkeit die zufällig integrierten Sequenzen zu entfernen und so Zellklone mit einer korrekten homologen Rekombination anzureichern, besteht in der Verwendung eines sequenzspezifischen Rekombinationssystems wie in US 6,110,736 beschrieben, durch welche unspezifisch integrierte Sequenzen wieder deletiert werden können, was die Selektion erfolgreich über homologe Rekombination integrierter Ereignisse erleichtert. Eine Vielzahl von sequenzspezifischen Rekombinationssystemen kann verwendet werden, beispielhaft sind das Cre/lox-System des Bacteriophagen P1, das FLP/FRT System der Hefe, die Gin Rekombinase des Mu Phagen, die Pin Rekombinase aus E. coli und das R/RS System des pSR1 Plasmids genannt. Bevorzugt sind das Bacteriophagen P1 Cre/lox und das Hefe FLP/FRT System. Das FLP/FRT und cre/lox Rekombinasesystem wurde bereits in pflanzlichen Systemen angewendet (Odell et al. (1990) Mol Gen Genet 223: 369-378)

h) Einführung von Mutationen in endogene NADPH-Oxidase Gene zur Erzeugung eines Funktionsverlustes (z.B. Generierung von Stopp-Kodons, Verschiebungen im Leseraster etc.)

Weitere geeignete Methoden zur Verminderung der NADPH-Oxidase-Aktivität sind die Einführung von Nonsense-Mutationen in endogene NADPH-Oxidase Gene zum Beispiel mittels Einführung von RNA/DNA-Oligonukleotiden in die Pflanze (Zhu et al. (2000) Nat Biotechnol 18(5):555-558) sowie die Generierung von Knockout-Mutanten mit Hilfe von z.B. T-DNA-Mutagenese (Koncz et al. (1992) Plant Mol Biol 20(5):963-976), ENU-(N-Ethyl-N-nitrosoharnstoff) - Mutagenese oder homolger Rekombination (Hohn B und Puchta (1999) H Proc Natl Acad Sci USA 96:8321-8323.). Punktmutationen können auch mittels DNA-RNA Hybriden erzeugt werden, die auch als "chimeraplasty" bekannt sind (Cole-Strauss et al. (1999) Nucl Acids Res 27(5):1323-1330; Kmiec (1999) Gene therapy American Scientist 87(3): 240-247).

Die Methoden der dsRNAi, der Kosuppression mittels sense-RNA und der "VIGS" ("virus induced gene silencing") werden auch als "post-transcriptional gene silencing" (PTGS) bezeichnet. PTGS-Verfahren wie auch die Verminderung der NADPH-Oxidase-Funktion oder Aktivität mit dominant-negativen NADPH-Oxidase-Varianten sind besonders vorteilhaft, weil die Anforderungen an die Homologie zwischen dem zu supprimierenden endogenem Gen und der transgen exprimierten sense- oder dsRNA-Nukleinsäuresequenz (bzw. zwischen dem endogenen Gen und seiner dominant-negativen Variante) geringer sind als beispielsweise bei einem klassischen antisense-Ansatz. Entsprechende Homologie-Kriterien sind bei der Beschreibung des dsRNAI-Verfahrens genannt und allgemein für PTGS-Verfahren oder dominant-negative Ansätze übertragbar. Aufgrund der hohen Homologie zwischen den NADPH-Oxidase-Proteinen aus Mais, Reis und Gerste kann auf einen hohen Konservierungsgrad dieses Protein bei Pflanzen geschlossen werden. So kann man voraussichtlich unter Verwendung der NADPH-Oxidase-Nukleinsäuresequenzen aus Gerste, Mais oder Reis auch die Expression von homologen NADPH-Oxidase-Proteinen in anderen Arten effektiv supprimieren, ohne dass die Isolierung und Strukturaufklärung der dort vorkommenden NADPH-Oxidase-Homologen zwingend erforderlich wäre. Dies erleichtert erheblich den Arbeitsaufwand. Analog kann man voraussichtlich auch unter Verwendung von dominant-negativen Varianten eines NADPH-Oxidase-Proteins aus Reis, Mais oder Gerste die Funktion/Aktivität seines Homologs in anderen Pflanzenarten effektiv vermindern oder unterdrücken.

Alle Substanzen und Verbindungen die direkt oder indirekt eine Verminderung der Proteinmenge, RNA-Menge, Genaktivität oder Proteinaktivität eines NADPH-Oxidase-Proteins bewirken, seien infolge unter der Bezeichnung "anti-NADPH-Oxidase"-Verbindungen zusammengefasst. Der Begriff "anti-NADPH-Oxidase"-Verbindung schließt explizit die in den oben beschriebenen Verfahren zum Einsatz kommenden Nukleinsäuresequenzen, Peptide, Proteine oder andere Faktoren ein.

"Einbringung" umfasst im Rahmen der Erfindung alle Verfahren, die dazu geeignet eine "anti-NADPH-Oxidase"-Verbindung, direkt oder indirekt, in eine Pflanze oder eine Zelle, Kompartiment, Gewebe, Organ oder Samen derselben einzuführen oder dort zu generieren. Direkte und indirekte Verfahren sind umfasst. Die Einbringung kann zu einer vorübergehenden (transienten) Präsenz einer "anti-NADPH-Oxidase"-Verbindung (beispielsweise einer dsRNA) führen oder aber auch zu einer dauerhaften (stabilen).

Gemäß der unterschiedlichen Natur der oben beschriebenen Ansätze kann die "anti-NADPH-Oxidase"-Verbindung ihre Funktion direkt ausüben (zum Beispiel durch Insertion in ein endogenes NADPH-Oxidase Gen). Die Funktion kann aber auch indirekt nach Transkription in eine RNA (zum Beispiel bei antisense Ansätzen) oder nach Transkription und Translation in ein Protein (z.B. Bindungsfaktoren) ausgeübt werden. Sowohl direkte als auch indirekt wirkende "anti-NADPH-Oxidase"-Verbindungen sind erfindungsgemäß umfasst.

Einführen umfasst beispielsweise Verfahren wie Transfektion, Transduktion oder Transformation.

"Anti-NADPH-Oxidase" Verbindungen umfasst somit beispielsweise auch rekombinante Expressionskonstrukte, die eine Expression (d.h. Transkription und ggf. Translation) beispielsweise einer NADPH-Oxidase-dsRNA oder einer NADPH-Oxidase "antisense"-RNA - bevorzugt in einer Pflanze oder einem Teil, Gewebe, Organ oder Samen derselben - bedingen.

In besagten Expressionskonstrukten steht ein Nukleinsäuremolekül, dessen Expression (Transkription und ggf. Translation) eine "anti-NADPH-Oxidase"-Verbindung generiert, bevorzugt in funktioneller Verknüpfung mit mindestens einem genetischen Kontrollelement (beispielsweise einem Promotor), das eine Expression in einem Organismus, bevorzugt in Pflanzen, gewährleistet. Soll das Expressionskonstrukt direkt in die Pflanze eingeführt und die "anti-NADPH-Oxidase"-Verbindung (beispielsweise die NADPH-Oxidase dsRNA) dort in plantae erzeugt werden, so sind pflanzenspezifische genetische Kontrollelemente (beispielsweise Promotoren) bevorzugt. Die "anti-NADPH-Oxidase"-Verbindung kann jedoch auch in anderen Organismen oder in vitro erzeugt und dann in die Pflanze eingebracht werden (wie in Beispiel 6 und 7 beschrieben). In diesem sind all prokaryotischen oder eukaryotischen genetischen Kontrollelemente (beispielsweise Promotoren) bevorzugt, die die Expression in den jeweils für die Herstellung gewählten Organismus erlauben.

Unter einer funktionellen Verknüpfung versteht man zum Beispiel die sequentielle Anordnung eines Promotors mit der zu exprimierenden Nukleinsäuresequenz (zum Beispiel einer "anti-NAox-Verbindung) und ggf. weiterer regulativer Elemente wie zum Beispiel einem Terminator derart, dass jedes der regulativen Elemente seine Funktion bei der transgenen Expression der Nukleinsäuresequenz, je nach Anordnung der Nukleinsäuresequenzen zu sense oder antisense RNA, erfüllen kann. Dazu ist nicht unbedingt eine direkte Verknüpfung im chemischen Sinne erforderlich. Genetische Kontrollsequenzen, wie zum Beispiel Enhancer-Sequenzen, können ihre Funktion auch von weiter entfernten Positionen oder gar von anderen DNA-Molekülen aus auf die Zielsequenz ausüben. Bevorzugt sind Anordnungen, in denen die transgen zu exprimierende Nukleinsäuresequenz hinter der als Promoter fungierenden Sequenz positioniert wird, so dass beide Sequenzen kovalent miteinander verbunden sind. Bevorzugt ist dabei der Abstand zwischen der Promotorsequenz und der transgen zu exprimierende Nukleinsäuresequenz geringer als 200 Basenpaare, besonders bevorzugt kleiner als 100 Basenpaare, ganz besonders bevorzugt kleiner als 50 Basenpaare.

Die Herstellung einer funktionellen Verknüpfung als auch die Herstellung einer Expressionskassette kann mittels gängiger Rekombinations- und Klonierungstechniken realisiert werden, wie sie beispielsweise in Maniatis T, Fritsch EF und Sambrook J (1989) Molecular Cloning: A Laboratory Manual, Cold Spring Harbor Laboratory, Cold Spring Harbor (NY), in Silhavy TJ, Berman ML und Enquist LW (1984) Experiments with Gene Fusions, Cold Spring Harbor Laboratory, Cold Spring Harbor (NY), in Ausubel FM et al. (1987) Current Protocols in Molecular Biology, Greene Publishing Assoc. and Wiley Interscience und bei Gelvin et al. (1990) In: Plant Molecular Biology Manual beschrieben sind. Zwischen beide Sequenzen können aber auch weitere Sequenzen positioniert werden, die zum Beispiel die Funktion eines Linkers mit bestimmten Restriktionsenzymschnittstellen oder eines Signalpeptides haben. Auch kann die Insertion von Sequenzen zur Expression von Fusionsproteinen führen. Bevorzugt kann die Expressionskassette, bestehend aus einer Verknüpfung von Promoter und zu exprimierender Nukleinsäuresequenz, integriert in einem Vektor vorliegen und durch zum Beispiel Transformation in ein pflanzliches Genom insertiert werden.

Unter einer Expressionskassette sind aber auch solche Konstruktionen zu verstehen, bei denen ein Promoter - zum Beispiel durch eine homologe Rekombination - hinter ein endogenes NADPH-Oxidase-Gen platziert wird, und durch Expression einer antisense NADPH-Oxidase-RNA die erfindungsgemäße Verminderung eines NADPH-oxidase-Proteins bewirkt wird. Analog kann auch eine "anti-NADPH-Oxidase" Verbindung (zum Beispiel eine Nukleinsäuresequenz kodierend für eines NADPH-Oxidase dsRNA oder eine NADPH-Oxidase antisense RNA) derart hinter einen endogenen Promotor platziert werden, dass der gleiche Effekt auftritt. Beide Ansätze führen zu Expressionskassetten im Sinne der Erfindung.

Pflanzenspezifische Promotoren meint grundsätzlich jeden Promotor, der die Expression von Genen, insbesondere Fremdgenen, in Pflanzen oder Pflanzenteilen, -zellen, -geweben, -kulturen steuern kann. Dabei kann die Expression beispielsweise konstitutiv, induzierbar oder entwicklungsabhängig sein.

Bevorzugt sind:

a) Konstitutive Promotoren

Bevorzugt sind Vektoren, die eine konstitutive Expression in Pflanzen ermöglichen (Benfey et al. (1989) EMBO J 8:2195-2202). "Konstitutiver" Promotor meint solche Promotoren, die eine Expression in zahlreichen, bevorzugt allen, Geweben über einen größeren Zeitraum der Pflanzenentwicklung, bevorzugt zu allen Zeitpunkten der Pflanzenentwicklung, gewährleisten. Vorzugsweise verwendet man insbesondere einen pflanzlichen Promotor oder einen Promotor, der einem Pflanzenvirus entstammt. Insbesondere bevorzugt ist der Promotor des 35S-Transkriptes des CaMV Blumenkohlmosaikvirus (Franck et al. (1980) Cell 21:285-294; Odell et al. (1985) Nature 313:810-812; Shewmaker et al. (1985) Virology 140:281-288; Gardner et al. (1986) Plant Mol Biol 6:221- 228) oder der 19S CaMV Promotor (US 5,352,605; WO 84/02913; Benfey et al. (1989) EMBO J 8:2195-2202). Ein weiterer geeigneter konstitutiver Promotor ist der "Rubisco small subunit (SSU)"-Promotor (US 4,962,028), der LeguminB-Promotor (GenBank Acc.-Nr. X03677), der Promotor der Nopalinsynthase aus Agrobacterium, der TR-Doppelpromotor, der OCS (Octopin Synthase) Promotor aus Agrobacterium, der Ubiquitin Promotor (Holtorf S et al. (1995) Plant Mol Biol 29:637-649), den Ubiquitin 1 Promotor (Christensen et al. (1992) Plant Mol Biol 18:675-689; Bruce et al. (1989) Proc Natl Acad Sci USA 86:9692-9696), den Smas Promotor, den Cinnamylalkoholdehydrogenase-Promotor (US 5,683,439), die Promotoren der ATPase Untereinheiten oder der Promotor eines prolinreichen Proteins aus Weizen (WO 91/13991), sowie weitere Promotoren von Genen, deren konstitutive Expression in Pflanzen dem Fachmann bekannt ist. Als konstitutiver Promotor insbesondere bevorzugt ist der Promotor des Nitrilase-1 (nit1) Gens aus A. thaliana (GenBank Acc.-No.: Y07648.2, Nukleotide 2456-4340, Hillebrand et al. (1996) Gene 170:197-200).

b) Gewebespezifische Promotoren

Bevorzugt sind ferner Promotoren mit Spezifitäten für die Antheren, Ovarien, Blüten, Blätter, Stengel, Wurzeln und Samen.

Samenspezifische Promotoren umfassen zum Beispiel den Promotor des Phaseolins (US 5,504,200; Bustos MM et al. (1989) Plant Cell 1(9):839-53), des 2S Albumins (Joseffson LG et al. (1987) J Biol Chem 262:12196-12201), des Legumins (Shirsat A et al. (1989) Mol Gen Genet 215(2): 326-331), des USP (unknown seed protein; Bäumlein H et al. (1991) Mol Gen Genet 225(3):459-67), des Napin (US 5,608,152; Stalberg K et al. (1996) L Planta 199:515-519), des Saccharosebindeproteins (WO 00/26388), des Legumin B4 (LeB4; Bäumlein H et al. (1991) Mol Gen Genet 225: 121-128; Baeumlein et al. (1992) Plant Journal 2(2):233-9; Fiedler U et al. (1995) Biotechnology (NY) 13(10):1090f), des Oleosin aus Arabidopsis (WO 98/45461) und des Bce4 aus Brassica (WO 91/13980). Weitere geeignete samenspezifische Promotoren sind die der Gene kodierend für das "High Molecular Weight Glutenin" (HMWG), Gliadin, Verzweigungsenzym, ADP Glucose Pyrophosphatase (AGPase) oder die Stärkesynthase. Bevorzugt sind ferner Promotoren, die eine samenspezifische Expression in Monokotyledonen wie Mais, Gerste, Weizen, Roggen, Reis etc. erlauben. Vorteilhaft eingesetzt werden können der Promoter des lpt2- oder 1pt1-Gen (WO 95/15389, WO 95/23230) oder die Promotoren beschrieben in WO 99/16890 (Promotoren des Hordein-Gens, des Glutelin-Gens, des Oryzin-Gens, des Prolamin-Gens, des Gliadin-Gens, des Glutelin-Gens, des Zein-Gens, des Kasirin-Gens oder des Secalin-Gens).

Knollen-, Speicherwurzel- oder Wurzel-spezifische Promotoren umfassen beispielsweise den Patatin Promotor Klasse I (B33) und den Promotor des Cathepsin D Inhibitors aus Kartoffel.

Blattspezifische Promotoren umfassen beispielsweise den Promotor der cytosolischen FBPase aus Kartoffel (WO 97/05900), den SSU Promotor (small subunit) der Rubisco (Ribulose-1,5-bisphosphat-carbox-ylase) oder den ST-LSI Promotor aus Kartoffel (Stockhaus et al. (1989) EMBO J 8:2445-2451). Ganz besonders bevorzugt sind Epidermis-spezifische Promotoren, wie beispielsweise der Promotor des OXLP-Gens ("Oxalat-Oxidase like protein"; Wei et al. (1998) Plant Mol. Biol. 36:101-112).

Blütenspezifische Promotoren umfassen beispielsweise den Phytoen Synthase Promotor (WO 92/16635) oder den Promotor des P-rr Gens (WO 98/22593).

Antheren-spezifische Promotoren umfassen beispielsweise den 5126-Promotor (US 5,689,049, US 5,689,051), den glob-1 Promotor und den γ-Zein Promotor.

c) Chemisch induzierbare Promotoren

Die Expressionskassetten können auch einen chemisch induzierbaren Promotor enthalten (Übersichtsartikel: Gatz et al. (1997) Annu Rev Plant Physiol Plant Mol Biol 48:89-108), durch den die Expression des exogenen Gens in der Pflanze zu einem bestimmten Zeitpunkt gesteuert werden kann. Beispielhaft seien zu nennen der PRP1 Promotor (Ward et al. (1993) Plant Mol Biol 22:361-366), ein durch Salicylsäure induzierbarer Promotor (WO 95/19443), ein durch Benzolsulfonamid-induzierbarer Promotor (EP 0 388 186), ein durch Tetrazyklin-induzierbarer Promotor (Gatz et al. (1992) Plant J 2:397-404), ein durch Abscisinsäure induzierbarer Promotor (EP 0 335 528) bzw. ein durch Ethanol- oder Cyclohexanon-induzierbarer Promotor (WO 93/21334).

d) Stress- oder Pathogen-induzierbare Promotoren

Ferner sind Promotoren bevorzugt, die durch biotischen oder abiotischen Stress induziert werden wie beispielsweise der pathogen-induzierbare Promotor des PRP1-Gens (bzw. *gst1* Promotor) z.B. aus Kartoffel (WO 96/28561; Ward et al. (1993) Plant Mol Biol 22:361- 366), der hitzeinduzierbare hsp70- oder hsp80-Promoter aus Tomate (US 5,187,267), der kälteinduzierare alpha-Amylase Promoter aus der Kartoffel (WO 96/12814) oder der licht-induzierbare PPDK Promotor. Weitere pathogen-induzierbare Promotoren umfassen den Flachs *Fis1*-Promotor (WO 96/34949), den Vst1-Promotor (Schubert et al. (1997) Plant Mol Biol 34:417-426) sowie den EAS4 Sesquiterpene-Cyclase-Promotor aus Tabak (US 6,100,451).

Pathogen-induzierbare Promotoren umfassen ferner die Promotoren von Genen, die infolge eines Pathogenbefalls induziert werden, wie beispielsweise Promotoren der Gene von PR-Proteinen, SAR-Proteinen, β-1,3-Glucanase, Chitinase usw. (beispielsweise Redolfi et al. (1983) Neth J Plant Pathol 89:245-254; Uknes, et al. (1992) Plant Cell 4: 645-656; Van Loon (1985) Plant Mol Viral 4:111-116; Marineau et al. (1987) Plant Mol Biol 9:335-342; Matton et al. (1987) Molecular Plant-Microbe Interactions 2:325-342; Somssich et al. (1986) Proc Natl Acad Sci USA 83:2427-2430; Somssich et al. (1988) Mol Gen Genetics 2:93-98; Chen et al. (1996) Plant J 10:955-966; Zhang and Sing (1994) Proc Natl Acad Sci USA 91:2507-2511; Warner, et al. (1993) Plant J 3:191-201; Siebertz et al. (1989) Plant Cell 1:961-968 (1989).

Umfasst sind auch verwundungs-induzierbare Promotoren wie der des pinII Gens (EP-A 0 375 091; Ryan (1990) Ann Rev Phytopath 28:425-449; Duan et al. (1996) Nat Biotech 14:494-498), des wun1- und wun2-Gens (US 5,428,148), des win1- und win2-Gens (Stanford et al. (1989) Mol Gen Genet 215:200-208), des Systemin-Gens (McGurl et al. (1992) Science 225:1570-1573), des WIP1-Gens (Rohmeier et al. (1993) Plant Mol Biol 22:783-792; Eckelkamp et al. (1993) FEBS Letters 323:73-76), des MPI-Gens (Corderok et al. (1994) Plant J 6(2):141-150) und dergleichen.

Eine Quelle für weitere pathogen-induzierbare Promotoren stellt die PR-Genfamilie dar. Eine Reihe von Elementen in diesen Promotoren haben sich als vorteilhaft erwiesen. So vermittelt die Region -364 bis -288 im Promotor von PR-2d Salicylat-Spezifität (Buchel et al. (1996) Plant Mol Biol 30, 493-504). Die Sequenz 5'-TCATCTTCTT-3' taucht im Promotor der Gersten β-1,3-Glucanase und in mehr als 30 weiteren stress-induzierten Genen wiederholt auf. Diese Region bindet in Tabak ein nukleäres Protein, dessen Abundanz durch Salicylat erhöht wird. Die PR-1-Promotoren aus Tabak und Arabidopsis (EP-A 0 332 104, WO 98/03536) eignen sich ebenfalls als pathogen-induzierbare Promotoren. Bevorzugt, da besonders spezifisch durch Pathogen-induziert, sind die "acidic *PR-5*"-(aPR5)-Promotoren aus Gerste (Schweizer et al. (1997) Plant Physiol 114:79-88) und Weizen (Rebmann et al. (1991) Plant Mol Biol 16:329-331). aPR5-Proteine akkumulieren in ca. 4 bis 6 Stunden nach Pathogenbefall und zeigen nur eine sehr geringe Hintergrundsexpression (WO 99/66057). Ein Ansatz, um eine erhöhte pathogen-induzierte Spezifität zu erreichen, bildet die Herstellung synthetischer Promotoren aus Kombinationen von bekannten pathogen-responsiven Elementen (Rushton et al. (2002) Plant Cell 14, 749-762; WO 00/01830; WO 99/66057). Weitere pathogen-induzierbare Promotoren aus verschiedenen Arten sind dem Fachmann bekannt (EP-A 1 165 794; EP-A 1 062 356; EP-A 1 041 148; EP-A 1 032 684;

e) Entwicklungsabhängige Promotoren

Weitere geeignete Promotoren sind beispielsweise fruchtreifungsspezifische Promotoren, wie beispielsweise der fruchtreifungsspezifische Promotor aus Tomate (WO 94/21794, EP 409 625). Entwicklungsabhängige Promotoren schließt zum Teil die gewebespezifischen Promotoren ein, da die Ausbildung einzelner Gewebe naturgemäß entwicklungsabhängig erfolgt.

Besonders bevorzugt sind konstitutive, sowie Blatt und/oder Stengel-spezifische, pathogen-induzierbare und epidermis-spezifische Promotoren, wobei pathogen-induzierbar und epidermis-spezifische Promotoren am meisten bevorzugt sind.

Es können ferner weitere Promotoren funktionell mit der zu exprimierenden Nukleinsäuresequenz verknüpft sein, die eine Expression in weiteren Pflanzengeweben oder in anderen Organismen, wie zum Beispiel E.coli Bakterien ermöglichen. Als Pflanzen Promotoren kommen im Prinzip alle oben beschriebenen Promotoren in Frage.

Die in den erfindungsgemäßen Expressionskassetten oder Vektoren enthaltenen Nukleinsäuresequenzen können mit weiteren genetischen Kontrollsequenzen neben einem Promoter funktionell verknüpft sein. Der Begriff der genetischen Kontrollsequenzen ist breit zu verstehen und meint all solche Sequenzen, die einen Einfluss auf das Zustandekommen oder die Funktion der erfindungsgemäßen Expressionskassette haben. Genetische Kontrollsequenzen modifizieren zum Beispiel die Transkription und Translation in prokaryotischen oder eukaryotischen Organismen. Vorzugsweise umfassen die erfindungsgemäßen Expressionskassetten 5'-stromaufwärts von der jeweiligen transgen zu exprimierenden Nukleinsäuresequenz den Promoter mit Spezifität für die embryonale Epidermis und/oder die Blüte und 3'-stromabwärts eine Terminatorsequenz als zusätzliche genetische Kontrollsequenz, sowie gegebenenfalls weitere übliche regulative Elemente, und zwar jeweils funktionell verknüpft mit der transgen zu exprimierenden Nukleinsäuresequenz.

Genetische Kontrollsequenzen umfassen auch weitere Promotoren, Promotorelemente oder Minimalpromotoren, die die expressionssteuernden Eigenschaften modifizieren können. So kann durch genetische Kontrollsequenzen zum Beispiel die gewebespezifische Expression zusätzlich abhängig von bestimmten Stressfaktoren erfolgen. Entsprechende Elemente sind zum Beispiel für Wasserstress, Abscisinsäure (Lam E und Chua NH, J Biol Chem 1991; 266(26): 17131 -17135) und Hitzestress (Schoffl F et al., Molecular & General Genetics 217(2-3):246-53, 1989) beschrieben.

Prinzipiell können alle natürlichen Promotoren mit ihren Regulationssequenzen wie die oben genannten für das erfindungsgemäße Verfahren verwendet werden. Darüberhinaus können auch synthetische Promotoren vorteilhaft verwendet werden.

Genetische Kontrollsequenzen umfassen ferner auch die 5'-untranslatierte Regionen, Introns oder nichtkodierende 3'-Region von Genen wie beipielsweise das Actin-1 Intron, oder die Adh1-S Introns 1, 2 und 6 (allgemein: The Maize Handbook, Chapter 116, Freeling and Walbot, Eds., Springer, New York (1994)). Es ist gezeigt worden, dass diese eine signifikante Funktionen bei der Regulation der Genexpression spielen können. So wurde gezeigt, dass 5'-untranslatierte Sequenzen die transiente Expression heterologer Gene verstärken können. Beispielhaft für Translationsverstärker sei die 5'-Leadersequenz aus dem Tabak-Mosaik-Virus zu nennen (Gallie et al. (1987) Nucl Acids Res 15: 8693-8711) und dergleichen. Sie können ferner die Gewebsspezifität fördern (Rouster J et al. (1998) Plant J 15:435-440).

Die Expressionskassette kann vorteilhafterweise eine oder mehrere sogenannte "enhancer Sequenzen" funktionell verknüpft mit dem Promoter enthalten, die eine erhöhte transgene Expression der Nukleinsäuresequenz ermöglichen. Auch am 3'-Ende der transgen zu exprimierenden Nukleinsäuresequenzen können zusätzliche vorteilhafte Sequenzen inseriert werden, wie weitere regulatorische Elemente oder Terminatoren. Die transgen zu exprimierenden Nukleinsäuresequenzen können in einer oder mehreren Kopien im Genkonstrukt enthalten sein.

Als Kontrollsequenzen geeignete Polyadenylierungssignale sind pflanzliche Polyadenylierungssignale, vorzugsweise solche, die im wesentlichen T-DNA Polyadenylierungssignale aus *Agrobacterium tumefaciens*, insbesondere des Gens 3 der T-DNA (Octopin Synthese) des Ti-Plasmids pTiACHS entsprechen (Gielen et al. (1984) EMBO J 3:835 ff) oder funktionelle Äquivalente davon. Beispiele für besonders geeignete Terminatorsequenzen sind der OCS (Octopin-Synthase)-Terminator und der NOS (Nopalin-Synthase)-Terminator.

Als Kontrollsequenzen sind weiterhin solche zu verstehen, die eine homologe Rekombination bzw. Insertion in das Genom eines Wirtsorganismus ermöglichen oder die Entfernung aus dem Genom erlauben. Bei der homologen Rekombination kann zum Beispiel der natürliche Promoter eines bestimmten Gens gegen einen Promoter mit Spezifität für die embryonale Epidermis und/oder die Blüte ausgetauscht werden. Methoden wie die cre/lox-Technologie erlauben eine gewebespezifische, unter Umständen induzierbare Entfernung der Expressionskassette aus dem Genom des Wirtsorganismus (Sauer B (1998) Methods. 14(4):381-92). Hier werden bestimmte flankierende Sequenzen dem Zielgen angefügt (lox-Sequenzen), die später eine Entfernung mittels der cre-Rekombinase ermöglichen.

Eine Expressionskassetten und die von ihr abgeleiteten Vektoren können weitere Funktionselemente enthalten. Der Begriff Funktionselement ist breit zu verstehen und meint all solche Elemente, die einen Einfluss auf Herstellung, Vermehrung oder Funktion der erfindungsgemäßen Expressionskassetten, Vektoren oder transgenen Organismen haben. Beispielhaft aber nicht einschränkend seien zu nennen:

a) Selektionsmarker, die eine Resistenz gegen einen Metabolismusinhibitor wie 2-Desoxyglucose-6-phosphat (WO 98/45456), Antibiotika oder Biozide, bevorzugt Herbizide, wie zum Beispiel Kanamycin, G 418, Bleomycin, Hygromycin, oder Phosphinotricin etc. verleihen. Besonders bevorzugte Selektionsmarker sind solche die eine Resistenz gegen Herbizide verleihen. Beispielhaft seien genannt: DNA Sequenzen, die für Phosphinothricinacetyltransferasen (PAT) kodieren und Glutaminsynthaseinhibitoren inaktivieren (bar und pat Gen), 5-Enolpyruvylshikimat-3-phosphatsynthasegene (EPSP Synthasegene), die eine Resistenz gegen Glyphosat® (N-(phosphonomethyl)glycin) verleihen, das für das Glyphosat® degradierende Enzyme kodierende gox Gen (Glyphosatoxidoreduktase), das deh Gen (kodierend für eine Dehalogenase, die Dalapon inaktiviert), Sulfonylurea- und Imidazolinon inaktivierende Acetolactatsynthasen sowie bxn Gene, die für Bromoxynil degradierende Nitrilaseenzyme kodieren, das aasa-Gen, das eine Resistenz gegen das Antibiotikum Apectinomycin verleih, das Streptomycinphosphotransferase (SPT) Gen, das eine Resistenz gegen Streptomycin gewährt, das Neomycinphosphotransferas (NPTII) Gen, das eine Resistenz gegen Kanamycin oder Geneticidin verleiht, das Hygromycinphosphotransferase (HPT) Gen, das eine Resistenz gegen Hygromycin vermittelt, das Acetolactatsynthas Gen (ALS), das eine Resistenz gegen Sulfonylharnstoff-Herbizide verleiht (z.B. mutierte ALS-Varianten mit z.B. der S4 und/oder Hra Mutation).

b) Reportergene, die für leicht quantifizierbare Proteine kodieren und über Eigenfarbe oder Enzymaktivität eine Bewertung der Transformationseffizienz oder des Expressionsortes oder -zeitpunktes gewährleisten. Ganz besonders bevorzugt sind dabei Reporter-Proteine (Schenborn E, Groskreutz D. Mol Biotechnol. 1999; 13(1):29-44) wie das "green fluorescence protein" (GFP) (Sheen et al.(1995) Plant Journal 8(5):777-784; Haseloff et al.(1997) Proc Natl Acad Sci USA 94(6):2122-2127; Reichel et al.(1996) Proc Natl Acad Sci USA 93(12):5888-5893; Tian et al. (1997) Plant Cell Rep 16:267-271; WO 97/41228; Chui WL et al. (1996) Curr Biol 6:325-330; Leffel SM et al. (1997) Biotechniques. 23(5):912-8), die Chloramphenicoltransferase, eine Luziferase (Ow et al. (1986) Science 234:856-859; Millar et al. (1992) Plant Mol Biol Rep 10:324-414), das Aequoringen (Prasher et al. (1985) Biochem Biophys Res Commun 126(3):1259-1268), die β-Galactosidase, R-Locus Gen (kodieren ein Protein, das die Produktion von Anthocyaninpigmenten (rote Färbung) in pflanzlichen Gewebe reguliert und so eine direkte Analyse der Promotoraktivität ohne Zugabe zusätzlicher Hilfsstoffe oder chromogener Substrate ermöglicht; Dellaporta et al., In: Chromosome Structure and Function: Impact of New Concepts, 18th Stadler Genetics Symposium, 11:263-282, 1988), ganz besonders bevorzugt ist die β-Glucuronidase (Jefferson et al., EMBO J. 1987, 6, 3901-3907).

c) Replikationsursprünge, die eine Vermehrung der erfindungsgemäßen Expressionskassetten oder Vektoren in zum Beispiel E.coli gewährleisten. Beispielhaft seien genannt ORI (origin of DNA replication), der pBR322 ori oder der P15A ori (Sambrook et al.: Molecular Cloning. A Laboratory Manual, 2nd ed. Cold Spring Harbor Laboratory Press, Cold Spring Harbor, NY, 1989).

d) Elemente, die für eine Agrobakterium vermittelte Pflanzentransformation erforderlich sind, wie zum Beispiel die rechte oder linke Begrenzung der T-DNA oder die vir-Region.

Die Einführung einer erfindungsgemäßen Expressionskassette in einen Organismus oder Zellen, Geweben, Organe, Teile bzw. Samen desselben (bevorzugt in Pflanzen bzw. pflanzliche Zellen, Gewebe, Organe, Teile oder Samen), kann vorteilhaft unter Verwendung von Vektoren realisiert werden, in denen die Expressionskassetten enthalten sind. Die Expressionskassette kann in den Vektor (zum Beispiel ein Plasmid) über eine geeignete Restriktionsschnittstelle eingeführt werden. Das entstandene Plasmid wird zunächst in E.coli eingeführt. Korrekt transformierte E.coli werden selektioniert, gezüchtet und das rekombinante Plasmid mit dem Fachmann geläufigen Methoden gewonnen. Restriktionsanalyse und Sequenzierung können dazu dienen, den Klonierungsschritt zu überprüfen.

Vektoren können beispielhaft Plasmide, Cosmide, Phagen, Viren oder auch Agrobacterien sein. In einer vorteilhaften Ausführungsform wird die Einführung der Expressionskassette mittels Plasmidvektoren realisiert. Bevorzugt sind solche Vektoren, die eine stabile Integration der Expressionskassette in das Wirtsgenom ermöglichen.

Die Herstellung eines transformierten Organismus (bzw. einer transformierten Zelle oder Gewebes) erfordert, dass die entsprechende DNA, RNA oder Protein in die entsprechende Wirtszelle eingebracht wird.

Für diesen Vorgang, der als Transformation (oder Transduktion bzw. Transfektion) bezeichnet wird, steht eine Vielzahl von Methoden zur Verfügung (Keown et al. (1990) Methods in Enzymology 185:527-537). So kann die DNA oder RNA beispielhaft direkt durch Mikroinjektion oder durch Bombardierung mit DNA-beschichteten Mikropartikeln eingeführt werden. Auch kann die Zelle chemisch, zum Beispiel mit Polyethylenglycol, permeabilisiert werden, so dass die DNA durch Diffusion in die Zelle gelangen kann. Die DNA kann auch durch Protoplastenfusion mit anderen DNA-enthaltenden Einheiten wie Minicells, Zellen, Lysosomen oder Liposomen erfolgen. Elektroporation ist eine weitere geeignete Methode zur Einführung von DNA, bei der die Zellen reversibel durch einen elektrischen Impuls permeabilisert werden. Entsprechende Verfahren sind beschrieben (beispielsweise bei Bilang et al. (1991) Gene 100:247-250; Scheid et al. (1991) Mol Gen Genet 228:104-112; Guerche et al. (1987) Plant Science 52:111-116; Neuhause et al. (1987) Theor Appl Genet 75:30-36; Klein et al. (1987) Nature 327:70-73 ; Howell et al. (1980) Science 208:1265; Horsch et al. (1985) Science 227:1229-1231 ; DeBlock et al. (1989) Plant Physiology 91:694-701; Methods for Plant Molecular Biology (Weissbach and Weissbach, eds.) Academic Press Inc. (1988); and Methods in Plant Molecular Biology (Schuler and Zielinski, eds.) Academic Press Inc. (1989)).

Bei Pflanzen werden dabei die beschriebenen Methoden zur Transformation und Regeneration von Pflanzen aus Pflanzengeweben oder Pflanzenzellen zur transienten oder stabilen Transformation genutzt. Geeignete Methoden sind vor allem die Protoplastentransformation durch Polyethylenglykol-induzierte DNA-Aufnahme, das biolistische Verfahren mit der Genkanone, die sogenannte "particle bombardment" Methode, die Elektroporation, die Inkubation trokkener Embryonen in DNA-haltiger Lösung und die Mikroinjektion.

Neben diesen "direkten" Transformationstechniken kann eine Transformation auch durch bakterielle Infektion mittels Agrobacterium tumefaciens oder Agrobacterium rhizogenes durchgeführt werden. Die Agrobacterium-vermittelte Transformation ist am besten für dicotyledone Pflanzenzellen geeignet. Die Verfahren sind beispielsweise beschrieben bei Horsch RB et al. (1985) Science 225: 1229f).

Werden Agrobacterien verwendet, so ist die Expressionskassette in spezielle Plasmide zu integrieren, entweder in einen Zwischenvektor (englisch: shuttle or intermediate vector) oder einen binären Vektor. Wird ein Ti oder Ri Plasmid zur Transformation verwendet werden soll, ist zumindest die rechte Begrenzung, meistens jedoch die rechte und die linke Begrenzung der Ti oder Ri Plasmid T-DNA als flankierende Region mit der einzuführenden Expressionskassette verbunden.

Bevorzugt werden binäre Vektoren verwendet. Binäre Vektoren können sowohl in E.coli als auch in Agrobacterium replizieren. Sie enthalten in der Regel ein Selektionsmarkergen zur Selektion transformierter pflanzlicher Organismen (s.o.) und einen Linker oder Polylinker flankiert von der rechten und linken T-DNA Begrenzungssequenz. Sie können direkt in Agrobacterium transformiert werden (Holsters et al. (1978) Mol Gen Genet 163:181-187). Außerhalb der T-DNA Region können Elemente wie ein Selektionsmarkergen zur Selektion transformierter Agrobakteria oder E.coli (z.B. nptIII) umfasst sein. Das in diesem Fall als Wirtsorganismus fungierende Agrobacterium sollte bereits ein Plasmid mit der vir-Region enthalten. Diese ist für die Übertragung der T-DNA auf die pflanzliche Zelle erforderlich. Ein so transformiertes Agrobacterium kann zur Transformation pflanzlicher Zellen verwendet werden. Die Verwendung von T-DNA zur Transformation pflanzlicher Zellen ist intensiv untersucht und beschrieben (EP 120 516; Hoekema, In: The Binary Plant Vector System, Offsetdrukkerij Kanters B.V., Alblasserdam, Chapter V; An et al. (1985) EMBO J 4:277-287). Verschiedene binäre Vektoren sind bekannt und teilweise kommerziell erhältlich wie zum Beispiel pBI101.2 oder pBIN19 (Clontech Laboratories, Inc. USA).

Weitere zur Expression in Pflanzen geeignet Promotoren sind beschrieben (Rogers et al. (1987) Meth in Enzymol 153:253-277; Schardl et al. (1987) Gene 61:1-11; Berger et al. (1989) Proc Natl Acad Sci USA 86:8402-8406).

Direkte Transformationstechniken eignen sich für jeden Organismus und Zelltyp eignen.

Im Falle von Injektion oder Elektroporation von DNA bzw. RNA in pflanzliche Zellen sind keine besonderen Anforderungen an das verwendete Plasmid gestellt. Einfache Plasmide wie die der pUC-Reihe können verwendet werden. Sollen vollständige Pflanzen aus den transformierten Zellen regeneriert werden, so ist er erforderlich, das sich auf dem Plasmid ein zusätzliches selektionierbares Markergen befindet.

Stabil transformierte Zellen d.h. solche, die die eingeführte DNA integriert in die DNA der Wirtszelle enthalten, können von untransformierten selektioniert werden, wenn ein selektionierbarer Marker Bestandteil der eingeführten DNA ist. Als Marker kann beispielhaft jedes Gen fungieren, dass eine Resistenz gegen ein Biozid (zum Beispiel ein Antibiotikum, Herbizid oder ein Metabolismusinhibitor wie 2-Desoxyglucose-6-phosphat WO 98/45456) verleiht(s.o.). Transformierte Zellen, die ein solches Markergen exprimieren, sind in der Lage, in der Gegenwart von Konzentrationen eines entsprechenden Antibiotikums oder Herbizides zu überleben, die einen untransformierten Wildtyp abtöten. Beispiel sind oben genannt und umfassen bevorzugt das bar Gen, dass Resistenz gegen das Herbizid Phosphinotricin verleiht (Rathore KS et al. (1993) Plant Mol Biol 21(5):871-884), das nptII Gen, dass Resistenz gegen Kanamycin verleiht, das hpt Gen, das Resistenz gegen Hygromycin verleiht, oder das EPSP-Gen, das Resistenz gegen das Herbizid Glyphosat verleiht. Der Selektionsmarker erlaubt die Selektion von transformierten Zellen von untransformierten (McCormick et al. (1986) Plant Cell Reports 5:81-84). Die erhaltenen Pflanzen können in üblicher Weise gezüchtet und gekreuzt werden. Zwei oder mehr Generationen sollten kultiviert werden, um sicherzustellen, dass die genomische Integration stabil und vererblich ist.

Die oben genannten Verfahren sind beispielsweise beschrieben in Jenes B et al.(1993) Techniques for Gene Transfer, in: Transgenic Plants, Vol. 1, Engineering and Utilization, herausgegeben von SD Kung und R Wu, Academic Press, S. 128-143 sowie in Potrykus (1991) Annu Rev Plant Physiol Plant Molec Biol 42:205-225). Vorzugsweise wird das zu exprimierende Konstrukt in einen Vektor kloniert, der geeignet ist, Agrobacterium tumefaciens zu transformieren, beispielsweise pBin19 (Bevan et al. (1984) Nucl Acids Res 12:8711f).

Sobald eine transformierte Pflanzenzelle hergestellt wurde, kann eine vollständige Pflanze unter Verwendung von dem Fachmann bekannten Verfahren erhalten werden. Hierbei geht man beispielhaft von Kalluskulturen aus. Aus diesen noch undifferenzierten Zellmassen kann die Bildung von Spross und Wurzel in bekannter Weise induziert werden. Die erhaltenen Sprösslinge können ausgepflanzt und gezüchtet werden.

Dem Fachmann sind such Verfahren bekannt, um aus Pflanzenzellen, Pflanzenteile und ganze Pflanzen zu regenerieren. Beispielsweise werden hierzu Verfahren beschrieben von Fennell et al. (1992) Plant Cell Rep. 11: 567-570; Stoeger et al (1995) Plant Cell Rep. 14:273-278; Jahne et al. (1994) Theor Appl Genet 89:525-533 verwendet.

Das erfindungsgemäße Verfahren kann vorteilhaft mit weiteren Verfahren die eine Pathogenresistenz (beispielsweise gegen Insekten, Pilze, Bakterien, Nematoden etc.), Stressresistenz oder eine andere Verbesserung der pflanzlichen Eigenschaften bewirken kombiniert werden. Beispiele sind u.a. genannt bei Dunwell JM, Transgenic approaches to crop improvement, J Exp Bot. 2000;51 Spec No; Seite 487-96.

"Transgen" meint bezüglich zum Beispiel einer Nukleinsäuresequenz, einer Expressionskassette oder einem Vektor enthaltend besagte Nukleinsäuresequenz oder einem Organismus transformiert mit besagter Nukleinsäuresequenz, Expressionskassette oder Vektor alle solche durch gentechnische Methoden zustandegekommene Konstruktionen, in denen sich entweder

a) die NADPH-Oxidase Nukleinsäuresequenz, oder

b) eine mit der NADPH-Oxidase Nukleinsäuresequenz funktionell verknüpfte genetische Kontrollsequenz, zum Beispiel ein Promotor, oder

c) (a) und (b)

sich nicht in ihrer natürlichen, genetischen Umgebung befinden oder durch gentechnische Methoden modifiziert wurden, wobei die Modifikation beispielhaft eine Substitutionen, Additionen, Deletionen, Inversion oder Insertionen eines oder mehrerer Nukleotidreste sein kann. Natürliche genetische Umgebung meint den natürlichen chromosomalen Locus in dem Herkunftsorganismus oder das Vorliegen in einer genomischen Bibliothek. Im Fall einer genomischen Bibliothek ist die natürliche, genetische Umgebung der Nukleinsäuresequenz bevorzugt zumindest noch teilweise erhalten. Die Umgebung flankiert die Nukleinsäuresequenz zumindest an einer Seite und hat eine Sequenzlänge von mindestens 50 bp, bevorzugt mindestens 500 bp, besonders bevorzugt mindestens 1000 bp, ganz besonders bevorzugt mindestens 5000 bp. Eine natürlich vorkommende Expressionskassette - beispielsweise die natürlich vorkommende Kombination des NADPH-Oxidase-Promotors mit dem entsprechenden NADPH-Oxidase-Gen - wird zu einer transgenen Expressionskassette, wenn diese durch nicht-natürliche, synthetische ("künstliche") Verfahren wie beispielsweise einer Mutagenisierung geändert wird. Entsprechende Verfahren sind beschrieben (US 5,565,350; WO 00/15815; siehe auch oben).

Ein anderer Gegenstand der Erfindung betrifft transgene Organismen, transformiert mit wenigstens einer erfindungsgemäßen Nukleinsäuresequenzen, Expressionskassette oder einem erfindungsgemäßen Vektor, sowie Zellen, Zellkulturen, Gewebe, Teile - wie zum Beispiel bei pflanzlichen Organismen Blätter, Wurzeln usw.- oder Vermehrungsgut abgeleitet von solchen Organismen. Organismus ist breit zu verstehen und meint prokaryotische und eukaryotische Organismen, bevorzugt Bakterien, Hefen, Pilze, und pflanzliche Organismen.

Bevorzugt sind

a) Pilze, wie Aspergillus, Eremothecium, Trichoderma, Ashbya, Neurospora, Fusarium, Beauveria oder weitere in Indian Chem Eng. Section B. Vol 37, No 1,2 (1995) auf Seite 15, Tabelle 6 beschriebene Pilze. Besonders bevorzugt ist der filamentöse Hemiascomycet Ashbya gossypii oder Eremothecium ashbyii.

b) Hefen wie Candida, Saccharomyces, Hansenula oder Pichia, besonders bevorzugt sind Saccharomyces cerevisiae oder Pichia pastoris (ATCC Accession No. 201178),

c) Pflanzen gemäß der obengenannten Definition für "Pflanzen" prokaryontische Organismen wie gram-positive oder gramnegative Bakterien wie Acetobacter, Gluconobacter, Corynebacterium, Brevibacterium, Bacillus, Clostridium, Cyanobacter, Escherichia (vor allem Escherichia coli), Serratia, Staphylococcus, Aerobacter, Alcaligenes, Penicillium oder Klebsiella genannt.

Als transgene Organismen bevorzugte Wirts- oder Ausgangsorganismen sind vor allem Pflanzen gemäß der oben genannten Definition. Eingeschlossen sind im Rahmen der Erfindung alle Gattungen und Arten höherer und niedrigerer Pflanzen des Pflanzenreiches. Eingeschlossen sind ferner die reifen Pflanzen, Saatgut, Sprossen und Keimlinge, sowie davon abgeleitete Teile, Vermehrungsgut und Kulturen, zum Beispiel Zellkulturen. Reife Pflanzen meint Pflanzen zu jedem beliebigen Entwicklungsstadium jenseits des Keimlings. Keimling meint eine junge, unreife Pflanze in einem frühen Entwicklungsstadium. Insbesondere als Wirtsorganismen bevorzugte Pflanzen sind Pflanzen, auf die der erfindungsgemäße Verfahren zum Erzielen einer Pathogenresistenz gemäß oben genannten Kriterien angewendet werden kann. Ganz besonders bevorzugt sind monokotyle Pflanzen wie Weizen, Hafer, Hirse, Gerste, Roggen, Mais, Reis, Buchweizen, Sorghum, Triticale, Dinkel, Leinsamen, Zuckerrohr,

Die Herstellung der transgenen Organismen kann mit den oben beschriebenen Verfahren zur Transformation oder Transfektion von Organismen realisiert werden.

Ebenfalls offenbart ist die Verwendung der erfindungsgemäßen, transgenen Organismen und der von ihnen abgeleitete Zellen, Zellkulturen, Teile - wie zum Beispiel bei transgenen pflanzlichen Organismen Wurzeln, Blätter etc.-, und transgenes Vermehrungsgut wie Saaten oder Früchte, zur Herstellung von Nahrungs- oder Futtermitteln, Pharmazeutika oder Feinchemikalien.

Offenbart ist ferner ein Verfahren zur rekombinanten Herstellung von Pharmazeutika oder Feinchemikalien in Wirtsorganismen wobei ein Wirtsorganismus mit einer der oben beschriebenen Expressionskassetten transformiert wird und diese Expressionskassette ein oder mehrere Strukturgene enthält, die für die gewünschte Feinchemikalie kodieren oder die Biosynthese der gewünschten Feinchemikalie katalysieren, der transformierte Wirtsorganismus gezüchtet wird und die gewünschte Feinchemikalie aus dem Züchtungsmedium isoliert wird. Dieses Verfahren ist für Feinchemikalien wie Enzyme, Vitamine, Aminosäuren, Zucker, Fettsäuren, natürliche und synthetische Geschmacks-, Aroma- und Farbstoffe breit anwendbar. Besonders bevorzugt ist die Produktion von Tocopherolen und Tocotrienolen sowie Carotinoiden. Die Züchtung der transformierten Wirtsorganismen sowie die Isolierung aus den Wirtsorganismen bzw. aus dem Züchtungsmedium erfolgt mit dem Fachmann bekannten Verfahren. Die Produktion von Pharmazeutika, wie zum Beispiel Antikörpern oder Vakkzinen ist beschrieben bei Hood EE, Jilka JM (1999) Curr Opin Biotechnol 10(4):382-6; Ma JK, Vine ND (1999) Curr Top Microbiol Immunol 236:275-92.

Sequenzen

| | |
|---|---|
| 1. SEQ ID NO: 1 | Nukleinsäuresequenz kodierend für eine NADPH-Oxidase aus Gerste (Hordeum vulgare). |
| 2. SEQ ID NO: 2 | Aminosäuresequenz kodierend für eine NADPH-Oxidase aus Gerste (Hordeum vulgare). |
| 3. SEQ ID NO: 3 | Nukleinsäuresequenz kodierend für eine NADPH-Oxidase aus Reis (Oryza sativa var. japonica) |
| 4. SEQ ID NO: 4 | Aminosäuresequenz kodierend für eine NADPH-Oxidase aus Reis (Oryza sativa var. japonica) |
| 5. SEQ ID NO: 5 | Nukleinsäuresequenz kodierend für eine NADPH-Oxidase aus Nicotiana tabacum |
| 6. SEQ ID NO: 6 | Aminosäuresequenz kodierend für eine NADPH-Oxidase aus Nicotiana tabacum |
| 7. SEQ ID NO: 7 | Nukleinsäuresequenz kodierend für eine NADPH-Oxidase aus Kartoffel (Solanum tuberosum) |
| 8. SEQ ID NO: 8 | Aminosäuresequenz kodierend für eine NADPH-Oxidase aus Kartoffel (Solanum tuberosum) |
| 9. SEQ ID NO: 9 | Nukleinsäuresequenz kodierend für eine NADPH-Oxidase aus Tomate (Lycopersicon esculentum) |
| 10. SEQ ID NO: 10 | Aminosäuresequenz kodierend für eine NADPH-Oxidase aus NADPH-Oxidase aus Tomate (Lycopersicon esculentum) |
| 11. SEQ ID NO: 11 | Nukleinsäuresequenz kodierend für eine NADPH-Oxidase aus Arabidopsis thaliana (RbohF) |
| 12. SEQ ID NO: 12 | Aminosäuresequenz kodierend für eine NADPH-Oxidase aus NADPH-Oxidase Arabidopsis thaliana (RbohF) |
| 13. SEQ ID NO: 13 | Nukleinsäuresequenz kodierend für eine NADPH-Oxidase aus Arabidopsis thaliana (RbohD) |
| 14. SEQ ID NO: 14 | Aminosäuresequenz kodierend für eine NADPH-Oxidase aus NADPH-Oxidase Arabidopsis thaliana (RbohD) |
| 15. SEQ ID NO: 15 | Nukleinsäuresequenz kodierend für eine NADPH-Oxidase aus Nicotiana tabacum (rboh) |
| 16. SEQ ID NO: 16 | Aminosäuresequenz kodierend für eine NADPH-Oxidase aus Nicotiana tabacum (rboh) |
| 17. SEQ ID NO: 17 | Nukleinsäuresequenz kodierend für eine NADPH-Oxidase aus Reis (Oryza sativa var. japonica) |
| 18. SEQ ID NO: 18 | Aminosäuresequenz kodierend für eine NADPH-Oxidase aus Reis (Oryza sativa var. japonica) |
| 19. SEQ ID NO: 19 | Nukleinsäuresequenz kodierend für eine NADPH-Oxidase aus Arabidopsis thaliana (RbohC) |
| 20. SEQ ID NO: 20 | Aminosäuresequenz kodierend für eine NADPH-Oxidase aus NADPH-Oxidase Arabidopsis thaliana (RbohC) |
| 21. SEQ ID NO: 21 | Nukleinsäuresequenz kodierend für eine NADPH-Oxidase aus Arabidopsis thaliana (RbohA) |
| 22. SEQ ID NO: 22 | Aminosäuresequenz kodierend für eine NADPH-Oxidase aus NADPH-Oxidase Arabidopsis thaliana (RbohA) |
| 23. SEQ ID NO: 23 | Oligonukleotidprimer 5' NAOX<br>5'-GARCAAGGCTCTTTTGATTG-3' |
| 24. SEQ ID NO: 24 | Oligonukleotidprimer 3' Naox<br>5'-GAAATGCTCCTTATGGAATTC-3' |

Abbildungen

Fig. 1: "RNA Interference" mit pNAox-dsRNA vermindert die Penetrationseffizienz des Echten Gerstenmehltau BghA6 in Gerste.

Die relative Penetrationseffizienz (RPE) wurde in fünf individuellen Experimenten bei Inokulation mit *Bgh* aus Gerste cv Pallas bestimmt. Die RPE errechnet sich als Differenz aus der Penetrationseffizienz bei pNAox-dsRNA transformierten Zellen und der Penetrationseffizienz bei Kontroll-dsRNA transformierten Zellen (hier: durchschnittliche Penetrationseffizienz 38,74 %). Die prozentuale RPE (%-RPE) errechnet sich aus der RPE minus 1 und multipliziert mit 100.

$$\text{RPE} = \frac{[\text{PE bei pNAox-dsRNA transformierten Zellen}]}{[\text{PE bei Kontroll-dsRNA transformierten Zellen}]}$$

$$\%\text{-RPE} = 100 * (\text{RPE}-1)$$

Die Säulen "1" bis "5" stellen die %-RPE (d.h. die Abweichung der Penetrationseffizienz vom Durchschnitt der Penetrationseffizienz der Kontrolle) bei Evaluierung von mindesten 100 Interaktionsstellen für jeweils ein unabhängiges Experiment dar. Die Säule "m" stellt die durchschnittliche %-RPE der Experimente dar. Der Fehlerbalken gibt den Standardfehler an.

"Control-dsRNA" stellt die parallelen Experimente mit einer Kontroll-dsRNA. "pNAox"-dsRNA stellt die Expermente mit der dsRNA der NADPH-Oxidaseaus Gerste dar.

Die %-RPE war in Zellen, die mit pNAox-dsRNA beschossen wurden, deutlich (Signifikanz p=0,0054) vermindert im Vergleich zu Zellen, die mit einer Kontroll-dsRNA (TR: humaner Thyroidrezeptor-dsRNA) bombardiert wurden.

Beispiele

Allgemeine Methoden:

Die chemische Synthese von Oligonukleotiden kann beispielsweise, in bekannter Weise, nach der Phosphoamiditmethode (Voet, Voet, 2. Auflage, Wiley Press New York, Seite 896-897) erfolgen. Die im Rahmen der vorliegenden Erfindung durchgeführten Klonierungsschritte wie z.B. Restriktionsspaltungen, Agarosegelelektrophorese, Reinigung von DNA-Fragmenten, Transfer von Nukleinsäuren auf Nitrozellulose und Nylonmembranen, Verknüpfen von DNA-Fragmenten, Transformation von E. coli Zellen, Anzucht von Bakterien, Vermehrung von Phagen und Sequenzanalyse rekombinanter DNA werden wie bei Sambrook et al. (1989) Cold Spring Harbor Laboratory Press; ISBN 0-87969-309-6 beschrieben durchgeführt. Die Sequenzierung rekombinanter DNA-Moleküle erfolgt mit einem Laserfluoreszenz-DNA-Sequenzierer der Firma MWG-Licor nach der Methode von Sanger (Sanger et al. (1977) Proc Natl Acad Sci USA 74: 5463-5467).

Beispiel 1: Pflanzen, Pathogene und Inokulation

Die Sorte Pallas wurde von Lisa Munk, Department of Plant Pathology, Royal Veterinary and Agriculturai University, Kopenhagen, Dänemark zur Verfügung gestellt. Ihre Herstellung ist beschrieben (Kølster P et al. (1986)Crop Sci 26: 903-907).

Das 12 bis 36 h im Dunkeln auf feuchtem Filterpapier vorgekeimte Saatgut wurde, wenn nicht anders beschrieben, zu je 5 Körnern an den Rand eines Vierkanttopfes (8x8 cm) in Fruhstorfer Erde vom Typ P ausgelegt, mit Erde bedeckt und regelmäßig mit Leitungswasser gegossen. Alle Pflanzen wurden in Klimaschränken oder - kammern bei 16 bis 18°C, 50 bis 60 % relativer Luftfeuchte und einem 16-stündigen Licht / 8stündigen Dunkelheitszyklus mit 3000 bzw. 5000 lux (50 bzw. 60 $\mu mols^{-1}m^{-2}$ Photonenflussdichte) 5 bis 8 Tage lang kultiviert und im Keimlingsstadium in den Versuchen verwendet. Bei Experimenten, in denen Applikationen an Primärblättern durchgeführt wurden, waren diese vollständig entwickelt.

Vor Durchführung der transienten Transfektionsexperimente wurden die Pflanzen in Klimaschränken oder -kammern bei tagsüber 24°C, nachts 20°C, 50 bis 60 % relativer Luftfeuchte und einem 16stündigen Licht / 8stündigen Dunkelheitszyklus mit 30000 lux kultiviert.

Für die Inokulation von Gerstenpflanzen wurde Echte Gerstenmehltau *Blumeria graminis* (DC) Speer f.sp. *hordei* Em. Marchal der Rasse A6 (Wiberg A (1974) Hereditas 77: 89-148) (BghA6) verwendet. Dieser wurde vom Institut für Biometrie, JLU Gießen bereitgestellt. Die Nachzucht des Inokulums erfolgte in Klimakammern zu den gleichen Bedingungen, wie sie oben für die Pflanzen beschrieben sind, durch Übertragung der Konidien von befallenem Pflanzenmaterial auf regelmäßig angezogene, 7 Tage alte Gerstenpflanzen cv. Golden Promise bei einer Dichte von 100 Konidia/$mm^2$.

Die Inokulation mit BghA6 erfolgte unter Verwendung von 7 Tagen alten Keimlingen durch Abschütteln der Konidien bereits befallener Pflanzen in einem Inokulationsturm mit ca. 100 Konidien/$mm^2$ (soweit nicht anders angegeben).

Beispiel 2: Klonierung der pNAox cDNA Sequenz aus Gerste

Die zur Isolation der HvpNAox cDNA, ihrer Klonierung, Sequenzierung und Herstellung von Sonden benötigten cDNA Fragmente wurden mittel RT-PCR unter Verwendung des "One Step RT-PCR Kit" (Life Technologies, Karlsruhe, Deutschland oder Qiagen, Hilden, Deutschland) erhalten. Dazu wurde Gesamt-RNA aus Gerste-Sämlingen als Matrize verwendet. Die RNA wurde aus Pallas 3, 5 und 7 Tage nach Keimung isoliert. Darüberhinaus wurde RNA aus Pallas und den rückgekreuzten Linien mit *mlo5, Mlg* oder *Mla12* 1, 2 und 5 Tage nach Inokulation mit *Bgh*A6 am 7 Tag nach Keimung isoliert. Für die RT-PCR wurden Primer verwendet, die von konservierten Regionen der gp91phox Homologen aus Reis und Arabidopsis thaliana abgeleitet sind (GenBank Acc.-No.: X93301 bzw. AB008111):

5' NAOX: 5'-GARCAAGGCTCTTTTGATTG-3' (SEQ ID NO: 23) und

3' Naox: 5' GAAATGCTCCTTATGGAATTC 3'(SEQ ID NO: 24)

Für die Reaktion (25 µL-Ansatz) wurden je 1000 ng Gesamt-RNA, 0,4 mM dNTPs, je 0,6 mM OPN-1 und OPN-2 Primer, 10 µl RNase-Inhibitor und 1 µl Enzymmix in 1x RT-Puffer (*one step RT-PCR kit*, Qiagen, Hilden) eingesetzt.
Folgendes Temperaturprogramm wird verwendet (PTC-100TM Modell 96V; MJ Research, Inc., Watertown, Massachussetts):

| | |
|---|---|
| 1 | Zyklus mit 30 min bei 50°C |
| 1 | Zyklus mit 150 sec bei 94°C |
| 30 | Zyklen mit 94°C für 45 sec, 55°C für 1 min und 72°C für 2 min |
| 1 | Zyklus mit 72°C für 7 min |

Die PCR Produkt wurde mittels 2% w/v Agarosegelelektrophorese aufgetrennt. Es wurde ein RT-PCR Produkt von 378 bp erhalten (SEQ ID NO: 1), das ein teil des offenen Leseraster der NADPH-Oxidase aus Gerste kodiert. Die entsprechende cDNA wurde aus einem Agarosegel isoliert und in den pGEM-T-Vektor (Promega, Mannheim, Deutschland) mittels T-Überhang-Ligation kloniert. Die cDNAs wurden ausgehend von der Plasmid-DNA unter Verwendung des "Thermo Sequenase Fluorescent Labeled Primer Cycle Sequencing Kit" (Amersham, Freiburg, Deutschland) sequenziert. Das Konstrukt wurde mit pGEM-T-pNAox bezeichnet.

Beispiel 3: In vitro Synthese der pNAox dsRNA

Das Plasmid pGEM-T-pNAox, das für die in vitro RNA-Transkription eingesetzt wurde, beinhaltet den T7 und SP6 Promotor an den jeweiligen Enden der insertierten Nukleinsäuresequenz, was die Synthese von sense- bzw. antisense RNA ermöglicht. Das Plasmide kann mit geeigneten Restriktionsenzymen (ApaI für SP6- und PstI für T7-Polymerase) linearisiert werden, um eine korrekte Transkription der insertierten Nukleinsäuresequenz zu gewährleisten und ein Durchlesen in vektorielle Sequenzen zu verhindern. Dazu wurden 10 µg pGEM-T-pNAox Plasmid-DNA jeweils mit ApaI für SP6- und PstI für T7-Polymerase geschnitten. Die geschnittenen Plasmide werden in 200 µl Wasser mit gleichem Volumen Phenol/Chloroform/Isoamylalkohol extrahiert, in ein neues Eppendorfreaktionsgefäß (RNAse frei) transferiert und 5 min bei 20000 g zentrifugiert. 180 µl der Plasmid-Lösung wurden mit 420 µl Ethanol versetzt, auf Eis gestellt und anschließend durch Zentrifugation für 30 min bei 20000 g und - 4°C präzipitiert. Das Präzipitat wurde in 10 µl TE Puffer aufgenommen. Die jeweiligen Präparationen wurden direkt in eine in vitro Transkription mit T7-RNA-Polymerase bzw. SP6-RNA-Polymerase eingesetzt. RNA Polymerasen wurden von Roche Molecular Biology, Mannheim, Deutschland bezogen.
Jeder Transkriptionsansatz beinhaltete in einem Volumen of 40 µl:

| | |
|---|---|
| 2 µl | linearisierte Plasmid DNA (1 µg) |
| 2 µl | NTP's (25 mM) (1,25 mM von jedem NTP) |
| 4 µl | 10xReaktionspuffer (Roche Molecular Biology), |
| 1 µl | RNAsin RNAsin (27 Units; Roche Molecular Biology), |
| 2 µl | RNA Polymerase (40 Units) |
| 29 µl | DEPC-Wasser |

Nach einer Inkubation von 2 h bei 37°C wurde jeweils ein Teil der Reaktionsansätze aus der Transkription des "sense"- bzw. "anti-sense"-Stranges gemischt, für 5 min bei 95°C denaturiert und anschließend durch Abkühlung über 30 min auf eine Endtemperatur von 37°C miteinander hybridisiert ("annealing"). Alternativ kann nach der Denaturierung das Gemisch aus sense- und antisense-STrang auch für 30 min bei -20°C gekühlt werden. Das Proteinpräzipitat, das sich während Denaturierung und Hybridisierung bildet wurde durch kurze Zentrifugation bei 20800 g abgetrennt und der Überstand direkt zur Beschichtung von Wolframpartikeln verwendet (s. unten). Zur Analyse wurden jeweils 1 µl jeden RNA-Stranges und der dsRNA auf einem nicht-denaturierenden Agarosegel aufgetrennt. Eine erfolgreiche Hybridisierung zeigte sich, durch eine Bandenverschiebung zu höherem Molekulargewicht im Vergleich zu den Einzelsträngen.
4 µl der dsRNA wurden Ethanol-präzipitiert (durch Zugabe von 6 µl Wasser, 1 µl 3M Natriumacetat-Lösung und 25 µl Ethanol, sowie Zenrifugation für mindestens 5 min bei 20000 g und 4°C) und in 500 µl Wasser resuspendiert. Das Absorbtionsspektrum zwischen 230 und 300 nm wurde gemessen, bzw. die Absorption bei 280 und 260 nm bestimmt, um die Reinheit und die Konzentration der dsRNA zu bestimmen. In der Regel wurden 80 bis 100 µg dsRNA mit einem $OD_{260}/OD_{280}$-Verhältnis von 1,80 bis 1, 95 erhalten. Ein Verdau mit DNase I kann optional durchgeführt werden, beeinflusst jedoch nachfolgende Ergebnisse nicht wesentlich.

Als Kontroll-dsRNA fungierte die dsRNA des humanen Thyroidrezeptors (Ausgangsvektor pT7betaSal (Norman C et al. (1988) Cell 55(6):989-1003) zur Verfügung gestellt von Dr. Baniahmad, Institut für Genetik, Gießen, Deutschland; die Sequenz des Insert ist beschrieben unter der GenBank Acc.-No.: NM_000461). Für die Herstellung der sense-RNA wurde das Plasmid mit PvuII, für die antsense-RNA mit HindIII verdaut und die RNA dann mit T7- bzw. SP6 RNA-Polymerase transkribiert. Die einzelnen Verfahrensschritte zur Herstellung der Kontroll-dsRNA werden analog den oben für die pNAox-dsRNA beschriebenen durchgeführt.

Beispiel 4: Transiente Transformation, RNAi und Evaluation der Pilzpathogenentwicklung

Gerste cv Pallas Blattsegmente wurden mit einer *pNAox*-dsRNA zusammen mit einem GFP-Expressionsvektor transformiert. Anschließend wurden die Blätter mit Bgh inokuliert und das Ergebnis nach 48 h mittels Licht- und Fluoreszenzmikroskopie analysiert. Die Penetration in GFP-exprimierenden Zellen wurde mittels Detektion von Haustorien in lebenden Zellen und durch Bewertung der Pilzentwicklung in eben diesen Zellen beurteilt. In allen fünf Experimenten führte die Bombardierung von Gerste cv Pallas mit *pNAox*-dsRNA zu einer verminderten Anzahl von erfolgreich durch Bgh penetrierten Zellen im Vergleich zu Zellen die mit einer fremden Kontroll-dsRNA (humaner Thyroidhormonrezeptor dsRNA, TR) bombardiert wurden. Der resistenzinduzierende Effekt der *pNAox*-dsRNA bedingte eine durchschnittliche Verminderung der Penetrationseffizienz durch Bgh um 35 % (Fig. 4).

Es wurde ein Verfahren zur transienten Transformation eingesetzt das bereits für die biolistische Einführung von dsRNA in epidermale Zellen von Gerstenblättern beschrieben wurde (Schweizer P et al. (1999) Mol Plant Microbe Interact 12:647-54; Schweizer P et al. (2000) Plant J 2000 24: 895-903). Wolframpartikel mit einem Durchmesser von 1,1 µm (Partikeldichte 25 mg/ml) wurden mit dsRNA (Herstellung siehe oben) zusammen mit Plasmid-DNA des Vektors *pGFP* (GFP unter Kontrolle des CaMV 35S Promotors) als Transformationsmarker beschichtet. Dazu wurden pro Schuss die nachfolgender Mengen an dsRNA bzw. Reporterplasmid zur Beschichtung verwendet: 1 µg pGFP und 2 µg dsRNA. Dopplesträngige RNA wurde mittels Verschmelzens von "sense" und "antisense"-RNA *in vitro* synthetisiert (s.o.).

Für Microcarrier-Präparation wurden 55 mg Wolframpartikel (M 17, Durchmesser 1,1 µm; Bio-Rad, München) zweimal mit 1 ml autoklaviertem Destilliertem Wasser und einmal mit 1 mL absolutem Ethanol gewaschen, getrocknet und in 1 ml 50 %igem Glycerin aufgenommen (ca. 50 mg/ml Stammlösung) . Die Lösung wurde mit 50 %igem Glycerin auf 25 mg/ml verdünnt, vor Gebrauch gut gemischt und im Ultraschallbad suspendiert. Zur Microcarrier-Beschichtung wurden pro Schuss 1 µg Plasmid, 2 µg dsRNA (1 µL), 12,5 µl Wolframpartikel-Suspension (25 mg/ml), 12,5 µl 1 M $Ca(NO_3)_2$-Lösung (pH 10) tropfenweise unter ständigem Mischen zusammengegeben, 10 min bei RT stehengelassen, kurz zentrifugiert und 20 µl vom Überstand abgenommen. Der Rest mit den Wolframpartikel wird resuspendiert (Ultraschallbad) und ins Experimente eingesetzt.

Es wurden ca. 4 cm lange Segmente von Gerstenprimärblättern verwendet. Die Gewebe wurden auf 0,5 % Phytagar (GibcoBRL™ Life Technologies™, Karlsruhe) mit 20 µg/ml Benzimidazol in Petrischalen (6,5 cm Durchmesser) gelegt und direkt vor dem Partikelbeschuss an den Rändern mit einer Schablone mit einer rechteckigen Aussparung von 2,2 cm x 2,3 cm abgedeckt. Die Schalen wurden nacheinander auf den Boden der Vakuumkammer (Schweizer P et al. (1999) Mol Plant Microbe Interact 12:647-54) gestellt, über dem ein Nylonnetz (Maschenweite 0,2 mm, Millipore, Eschborn) als Diffusor auf einer Lochplatte eingeschoben war (5 cm über dem Boden, 11 cm unterhalb des Macrocarriers, s.u.), um Partikelklumpen zu zerstreuen und den Partikelstrom abzubremsen. Der oben an der Kammer angebrachte Macrocarrier (Plastik-Sterilfilterhalter, 13 mm, Gelman Sciences, Swinney, UK) wurde je Schuss mit 5,8 µL DNA-beschichteten Wolframpartikeln (Microcarrier, s.u.) beladen. Mit einer Membranvakuumpumpe (Vacuubrand, Wertheim) wurde der Druck um 0,9 bar in der Kammer reduziert und die Wolframpartikel mit 9 bar Heliumgasdruck auf die Oberfläche des Pflanzengewebes geschossen. Sofort danach wurde die Kammer belüftet. Zur Markierung transformierter Zellen wurden die Blätter mit dem Plasmid (pGFP; Vektor auf pUC18-Basis, CaMV 35S-Promoter/Terminator-Kassette mit insertiertem GFP-Gen; Schweizer P et al. (1999) Mol Plant Microbe Interact 12:647-54; zur Verfügung gestellt von Dr. P. Schweizer Schweizer P, Institut für Pflanzengenetik IPK, Gatersleben, Deutschland) beschossen. Vor dem Schießen eines anderen Plasmids wurde der Macrocarrier jeweils gründlich mit Wasser gereinigt. Nach vierstündiger Inkubation nach dem Beschuss bei leicht geöffneten Petrischalen, RT und Tageslicht wurden die Blätter mit 100 Konidien/$mm^2$ des Echten Gerstenmehltaupilzes (Rasse A6) inokuliert und für weitere 40 bis 48 h unter gleichen Bedingungen inkubiert.

Blattsegmente wurden mit den beschichteten Partikeln unter Verwendung einer "particle inflow gun" bombardiert. Pro Schuss wurden 312 µg Wolframpartikel appliziert. 4 h nach der Bombardierung wurde Inokulation mit *Blumeria graminis f.sp. hordei* Mehltau (Rasse A6) inokuliert und nach weiteren 40 h bezüglich der Infektionsanzeichen ausgewertet. Das Ergebnis (z.B. die Penetrationseffizienz, definiert als prozentualer Anteil angegriffener Zellen, die ein reifes Haustorium und eine Sekundärhyphae ("secondary elongating hyphae") wurde mittels Fluoreszens- und Lichtmikroskopie analysiert. Eine Inokulation mit 100 Conidia/$mm^2$ ergibt eine Angriffsfrequenz von ca. 50 % der transformierten Zellen. Für jedes einzelne Experiment wurde eine minimale Anzahl von 100 Interaktionsstellen ausgewertet. Transformierte (GFP exprimierende) Zellen wurden unter Anregung mit blauem Licht identifiziert. Drei verschiedene Katagorien von transformierten Zellen konnten unterschieden werden:

1. Penetrierte Zellen, die ein leicht erkennbares Haustorium beinhalten. Eine Zelle mit mehr als einem Haustorium wurde als eine Zelle gewertet.

2. Zellen, die durch ein Pilz-Appressorium zwar angegriffen wurden, aber kein Haustorium beinhalten. Eine Zelle die mehrfach von Bgh angegriffen wurden, aber kein Haustorium enthält, wurde als eine Zelle gewertet.

3. Zellen die nicht durch Bgh angegriffen sind.

Stomatazellen und Stomatanebenzellen wurden von der Bewertung ausgeschlossen. Oberflächenstrukturen von Bgh wurden mittels Lichtmikroskopie oder Fluoreszenzfärbung des Pilzes mit 0,1 % Calcofluor (w/v in Wasser) für 30 sec analysiert. Die Entwicklung des Pilzes kann leicht durch Fluoreszenzmikroskopie nach Anfärbung mit Calcofluor evaluiert werden. In pNAox-dsRNA transformierten Zellen entwickelt der Pilz zwar ein primäres und ein appressoriales Keimschlauch ("Germ-Tube") aber kein Haustorium. Haustoriumausbildung ist eine Vorbedingung für die Bildung einer Sekundärhyphae.

Die relative Penetrationseffizien (RPE) errechnet sich als Differenz aus der Penetrationseffizien bei transformierten Zellen (Transformation mit pNAox- oder Kontroll-dsRNA) und der Penetrationseffizienz bei untransformierten Zellen (hier: durchschnittliche Penetrationseffizienz 38,74 %). Die prozentuale RPE (%-RPE) errechnet sich aus der RPE minus 1 und multipliziert mit 100.

$$\text{RPE} = \frac{[\text{PE bei pNAox-dsRNA transformierten Zellen}]}{[\text{PE bei Kontroll-dsRNA transformierten Zellen}]}$$

$$\text{\%-RPE} = 100 * (\text{RPE}-1)$$

Der %-RPE-Wert (Abweichung von der durchschnittlichen Penetrationseffizienz der Kontrolle) dient der Bestimmung des Suszeptibilität von Zellen, die mit pNAox-dsRNA transfiziert sind (Fig. 4).

Bei der Kontroll-dsRNA wurde bei fünf unabhängigen Versuchen kein Unterschied zwischen der Transfektion mit der Kontroll dsRNA und Wasser bezüglich der Penetrationseffizienz von *Bgh* beobachtet.

Um einen Einfluss auf der dsRNA auf die Transformationsrate oder Überlebensrate der angegriffenen Zellen auszuschließen, wurde die Anzahl der GFP-exprimierenden Zellen zwischen Kontroll- und *pN*Aex-dsRNA Experimente verglichen. Die *pN*Aox-dsRNA hatten keinen Einfluss auf die Gesamtanzahl- oder die Anzahl der angegriffenen GFP-exrrimierenden Zellen.

Beispiel 5: Inhibition der NADPH-Oxidase mit Diphenyleniodoniumchlorid

Untermauert werden die Ergebnisse durch weitere Experimente mit dem NADPH-Oxidase Inhibitor Diphenyleniodoniumchlorid (DPI; Tabelle 1). Im allgemeinen wurden die Experimente durchgeführt wie von Hückelhoven und Kogel, 1998.

Tab. 1: Wirkung von DPI auf die Pathogenabwehr in Pallas[a]

| | Interaktionen | (% ± Standardfehler) |
|---|---|---|
| Art der Interaktion | Kontrolle[b] | 200 μM DPI[c] |
| Penetration | 68.25 ± 9.9 | 16.25 ± 0.5 |
| Nicht-Penetration | 24.25 ± 6.3 | 67.5 ± 9.5 |

(fortgesetzt)

| | Interaktionen | (% ± Standardfehler) |
|---|---|---|
| HR (Hypersensitive Reaktion) | 7.5 ± 3.7 | 16.25 ± 9.3 |

[a] DPI-Behandlung erfolgte 12 h nach Pathogen-Inokkulation, die Auswertung 36 h nach Inokkulation.
[b] Kontrolle mit 10 mM Kaliumphoshatpuffer, pH 7,8, mit DMSO Gehalt wie bei DPI Behandlung.
[c] DPI gelöst in 10 mM Kaliumphoshatpuffer, pH 7,8, ausgehend von einer 10 mg/ml DPI Stammlösung in DMSO.

## Patentansprüche

1. Verfahren zum Erzielen oder Erhöhen der Resistenz gegen mindestens ein Pilzpathogen oder pilzähnliches Pathogen in monokotylen Kulturpflanzen, **dadurch gekennzeichnet, dass** nachfolgende Arbeitsschritte umfasst sind

   a) Verminderung der Proteinmenge, Aktivität oder Funktion einer NADPH-Oxidase in einer Pflanze oder einem Gewebe, Organ, Teil oder Zelle derselben und
   b) Auswahl der Pflanzen, bei denen - im Unterschied oder Vergleich zur Ausgangspflanze - die Resistenz gegen mindestens ein Pathogen besteht oder erhöht ist,

   wobei die NADPH-Oxidase kodiert wird durch

   a) Polypeptidsequenzen umfassend eine Sequenz gemäß SEQ ID NO: 2, 4, 6, 8, 10, 12, 14, 16, 18, 20 oder 22, oder
   b) Polypeptidsequenzen eines funktionellen Äquivalentes eines Polypeptides, welches eine Sequenz gemäß SEQ ID NO: 2, 4, 6, 8, 10, 12, 14, 16, 18, 20 oder 22 umfasst.

2. Verfahren nach Anspruch 1, wobei das funktionelle Äquivalent eine Homologie von mindestens 50% zu einem der Polypeptide gemäss SEQ ID NO: 2, 4, 6, 8, 10, 12, 14, 16, 18, 20 oder 22 hat.

3. Verfahren nach einem der Ansprüche 1 oder 2, wobei die Verminderung der Proteinmenge, Aktivität oder Funktion einer NADPH-Oxidase gewährleistet wird durch Anwendung eines Verfahrens ausgewählt aus der Gruppe bestehend aus

   a) Einbringen einer doppelsträngigen NADPH-Oxidase Ribonukleinsäuresequenz oder einer deren Expression gewährleistenden Expressionskassette oder Expressionskassetten,
   b) Einbringen einer NADPH-Oxidase antisense-Nukleinsäuresequenz oder einer deren Expression gewährleistenden Expressionskassette,
   c) Einbringen einer NADPH-Oxidase antisense-Nukleinsäuresequenz kombiniert mit einem Ribozym oder einer deren Expression gewährleistenden Expressionskassette,
   d) Einbringen von NADPH-Oxidase sense-Nukleinsäuresequenzen zur Induktion einer Kosuppression oder einer deren Expression gewährleistenden Expressionskassette,
   e) Einbringen DNA-oder Protein-bindende Faktoren gegen NADPH-Oxidase-Gene, -RNAs oder -Proteine oder einer deren Expression gewährleistenden Expressionskassette,
   f) Einbringen von den NADPH-Oxidase RNA-Abbau bewirkende virale Nukleinsäuresequenzen und Expressionskonstrukten oder einer deren Expression gewährleistenden Expressionskassette,
   g) Einbringen von Konstrukten zur Induktion einer homologen Rekombination an endogenen NADPH-Oxidase-Genen und
   h) Einführung von Mutationen in ein endogenes NADPH-Oxidase Gen.

4. Verfahren nach einem der Ansprüche 1 bis 3, umfassend

   (i) die stabile Transformation einer pflanzlichen Zelle mit einer rekombinanten Expressiorlskassette enthaltend in funktioneller Verknüpfung mit einem in Pflanzen aktiven Promotor eine Nukleinsäuresequenz kodierend für

      a) eine doppelsträngigen NADPH-Oxidase RNA-Ribonukleinsäuresequenz oder
      b) eine NADPH-Oxidase antisense-Nukleinsäuresequenz oder
      c) eine NADPH-Oxidase antisense-Nukleinsäuresequenz kombiniert mit einem Ribozym oder
      d) eine NADPH-Oxidase sense-Nukleinsäuresequenzen zur Induktion einer Kosuppression oder
      e) DNA-oder Protein-bindende Faktoren gegen NADPH-Oxidase-Gene, -RNAs oder -Proteine
      f) den NADPH-Oxidase RNA-Abbau bewirkende virale Nukleinsäuresequenzen

(ii) Regeneration der Pflanze aus der pflanzlichen Zelle, und
(iii)Expression besagter Nukleinsäuresequenz in einer Menge und für eine Zeit hinreichend, um eine Pathogenresistenz in besagter Pflanze zu erzeugen oder zu erhöhen.

**5.** Verfahren nach einem der Ansprüche 1 bis 4, wobei das Pathogen Mehltau ist.

**6.** Verfahren nach einem der Ansprüche 1 bis 5, wobei die Pflanze ausgewählt ist aus der Gruppe der monokotyledonen Pflanzen bestehend aus Weizen, Hafer, Hirse, Gerste, Roggen, Mais, Reis, Sorghum, Triticale, Dinkel und Zuckerrohr.

**7.** Doppelsträngiges RNA-Molekül zur Verminderung der Expression einer NADPH-Oxidase umfassend

a) einen sense-RNA-Strang umfassend mindestens eine Ribonukleotidsequenz, die im wesentlichen identisch ist zu mindestens einem Teil des sense-RNA-Transkriptes einer Nukleinsäuresequenz kodierend für eine NADPH-Oxidase, und
b) einen antisense-RNA-Strang, der zu dem RNA-sense-Strang unter a) im wesentlichen komplementären ist,

wobei einer der beiden RNA-Stränge kodiert wird durch zumindest einen Teil einer Nukleinsäuresequenz kodierend für eine NADPH-Oxidase Sequenz gemäß SEQ ID NO: 1, 3, 5, 7, 9, 11, 13, 15, 17, 19 oder 21 oder ein funktionelles Äquivalent derselben.

**8.** Doppelsträngiges RNA-Molekül nach Anspruch 7, wobei die beiden RNA-Stränge der doppelsträngigen RNA kovalent miteinander verbunden sind.

**9.** Transgene Expressionskassette enthaltend in funktioneller Verknüpfung mit einem in pflanzlichen Organismen funktionellen Promotor eine Nukleinsäuresequenz kodierend für ein doppelsträngiges RNA-Molekül gemäß einem der Ansprüche 7 oder 8.

**10.** Transgene Expressionskassette enthaltend zumindest einen Teil einer Nukleinsäuresequenz kodierend für eine NADPH-Oxidase gemäß SEQ ID NO: l, 3, 5, 7, 9, 11, 13, 15, 17, 19 oder 21 oder ein funktionelles Äquivalent derselben, wobei besagte Nukleinaäuresequenz in antisense-Orientierung mit einem in pflanzlichen Organismen funktionellen Promotor funktionell verknüpft ist.

**11.** Transgene Expressionskassette nach Anspruch 9 oder 10, wobei der in Pflanzen funktionelle Promotor ein pathogeninduzierbarer Promotor ist.

**12.** Transgener Vektor enthaltend eine Expressionskassette gemäß einem der Ansprüche 9 bis 11.

**13.** Transgener Organismus enthaltend ein doppelsträngiges RNA-Molekül gemäß einem der Ansprüche 7 bis 8, eine Expressionskassette gemäß einem der Ansprüche 9 bis 11 oder einen Vektor gemäß Anspruch 12, ausgewählt aus der Gruppe bestehend aus Bakterien, Hefen und monokotylen Kulturpflanzen.

**14.** Transgener Organismus nach Anspruch 13, ausgewählt aus der Gruppe der Pflanzen bestehend aus Weizen, Gerste, Hirse, Roggen, Triticale, Mais, Reis, Sorghum, Hafer und Zuckerrohr.

**15.** Gewebe, Organ, Teil, Zelle, Zellkultur oder Vermehrungsgut abgeleitet von einem transgenen Organismus gemäß einem der Ansprüche 13 oder 14 und enthaltend eine Expressionskassette nach einem der Ansprüche 9 bis 11 oder enthaltend eine doppelsträngiges RNA-Molekül gemäß einem der Ansprüche 7 bis 8.

## Claims

**1.** A method for generating or increasing the resistance to at least one fungal pathogen or fungus-like pathogen in monocotyledonous crop plants, which comprises the following operating steps:

a) reduction of the protein quantity, activity or function of an NADPH oxidase in a plant or a tissue, organ, part or cell thereof, and
b) selection of the plants in which - in contrast or in comparison with the starting plant - the resistance to at least one pathogen exists or is increased,

wherein the NADPH oxidase is encoded by

a) polypeptide sequences comprising a sequence as shown in SEQ ID NO: 2, 4, 6, 8, 10, 12, 14, 16, 18, 20 or 22, or
b) polypeptide sequences of a functional equivalent of a polypeptide comprising a sequence as shown in SEQ ID NO: 2, 4, 6, 8, 10, 12, 14, 16, 18, 20 or 22.

**2.** The method according to claim 1, wherein the functional equivalent has at least 50% homology with one of the polypeptides as shown in SEQ ID NO: 2, 4, 6, 8, 10, 12, 14, 16, 18, 20 or 22.

**3.** The method according to either of claims 1 and 2, wherein the reduction of the protein quantity, activity or function of an NADPH oxidase is ensured by applying a method selected from the group consisting of

a) introducing a double-stranded NADPH oxidase ribonucleic acid sequence or (an) expression cassette(s) ensuring its expression,
b) introducing an NADPH oxidase antisense nucleic acid sequence or an expression cassette ensuring its expression,
c) introducing an NADPH oxidase antisense nucleic acid sequence in combination with a ribozyme or an expression cassette ensuring its expression,
d) introducing NADPH oxidase sense nucleic acid sequences for inducing a cosuppression or an expression cassette ensuring their expression,
e) introducing DNA- or protein-binding factors against NADPH oxidase genes, RNAs or proteins or an expression cassette ensuring their expression,
f) introducing viral nucleic acid sequences and expression constructs bringing about the degradation of NADPH oxidase RNA, or an expression cassette ensuring their expression,
g) introducing constructs for inducing a homologous recombination at endogenous NADPH oxidase genes, and
h) introducing mutations into an endogenous NADPH oxidase gene.

**4.** The method according to any of claims 1 to 3, comprising

(i) the stable transformation of a plant cell with a recombinant expression cassette comprising, in functional linkage with a promoter which is active in plants, a nucleic acid sequence encoding

a) a double-stranded NADPH oxidase RNA ribonucleic acid sequence or
b) an NADPH oxidase antisense nucleic acid sequence or
c) an NADPH oxidase antisense nucleic acid sequence in combination with a ribozyme or
d) an NADPH oxidase sense nucleic acid sequence for inducing a cosuppression or
e) DNA- or protein-binding factors against NADPH oxidase genes, RNAs or proteins
f) viral nucleic acid sequences which bring about the degradation of NADPH oxidase RNA,

(ii) regeneration of the plant from the plant cell, and
(iii) expression of said nucleic acid sequence in such a quantity and for such a time as suffices for generating or increasing a pathogen resistance in said plant.

**5.** The method according to any of claims 1 to 4, wherein the pathogen is mildew.

**6.** The method according to any of claims 1 to 5, wherein the plant is selected from the group of monocotyledonous plants consisting of wheat, oats, millet, barley, rye, maize, rice, sorghum, triticale, spelt and sugar cane.

**7.** A double-stranded RNA molecule for reducing the expression of an NADPH oxidase comprising

a) a sense RNA strand comprising at least one ribonucleotide sequence which is essentially identical to at least part of the sense RNA transcript of a nucleic acid sequence encoding an NADPH oxidase, and
b) an antisense RNA strand which is essentially complementary to the RNA sense strand of a),

wherein one of the two RNA strands is encoded by at least a part of a nucleic acid sequence encoding an NADPH oxidase sequence as shown in SEQ ID NO: 1, 3, 5, 7, 9, 11, 13, 15, 17, 19 or 21 or a functional equivalent thereof.

**8.** The double-stranded RNA molecule according to claim 7, wherein the two RNA strands of the double-stranded RNA are bonded covalently with one another.

**9.** A transgenic expression cassette comprising, in functional linkage with a promoter which is functional in plant organisms, a nucleic acid sequence encoding a double-stranded RNA molecule according to either of claims 7 and 8.

**10.** A transgenic expression cassette comprising at least a part of a nucleic acid sequence encoding an NADPH oxidase as shown in SEQ ID NO: 1, 3, 5, 7, 9, 11, 13, 15, 17, 19 or 21 or a functional equivalent thereof, where said nucleic acid sequence is linked functionally in antisense orientation with a promoter which is functional in plant organisms.

**11.** The transgenic expression cassette according to claim 9 or 10, wherein the promoter which is functional in plants is a pathogen-inducible promoter.

**12.** A transgenic vector comprising an expression cassette according to any of claims 9 to 11.

**13.** A transgenic organism comprising a double-stranded RNA molecule according to any of claims 7 to 8, an expression cassette according to any of claims 9 to 11 or a vector according to claim 12, selected from the group consisting of bacteria, yeasts and monocotyledonous crop plants.

**14.** The transgenic organism according to claim 13, selected from the group of plants consisting of wheat, barley, millet, rye, triticale, maize, rice, sorghum, oats and sugar cane.

**15.** A tissue, organ, part, cell, cell culture or propagation material derived from a transgenic organism according to either of claims 13 or 14 and comprising an expression cassette according to any of claims 9 to 11 or comprising a double-stranded RNA molecule according to either of claims 7 and 8.

## Revendications

**1.** Procédé pour atteindre ou augmenter la résistance à l'encontre d'au moins un microorganisme pathogène fongique ou un microorganisme pathogène analogue à un champignon dans des plantes de culture monocotylédones, **caractérisé en ce qu'**il comprend les étapes suivantes :

a) diminution de la quantité de protéines, de l'activité ou de la fonction d'une NADPH-oxydase dans une plante ou un tissu, un organe, une partie ou une cellule de ceux-ci, et
b) sélection des plantes dans lesquelles - au contraire de la plante de départ ou par rapport à cette dernière - la résistance à l'encontre d'au moins un microorganisme pathogène existe ou est augmentée,

la NADPH-oxydase étant codée par

a) des séquences polypeptidiques comprenant une séquence selon SEQ ID N° 2, 4, 6, 8, 10, 12, 14, 16, 18, 20 ou 22, ou
b) des séquences polypeptidiques d'un équivalent fonctionnel d'un polypeptide, qui comprend une séquence selon SEQ ID N° 2, 4, 6, 8, 10, 12, 14, 16, 18, 20 ou 22.

**2.** Procédé selon la revendication 1, dans lequel l'équivalent fonctionnel présente une homologie d'au moins 80 % avec l'un des polypeptides selon SEQ ID N° 2, 4, 6, 8, 10, 12, 14, 16, 18, 20 ou 22.

**3.** Procédé selon l'une des revendications 1 ou 2, dans lequel la diminution de la quantité de protéines, de l'activité ou de la fonction d'une NADPH-oxydase est assurée par l'utilisation d'un procédé choisi dans le groupe consistant en

a) l'introduction d'une séquence d'acides ribonucléiques de la NADPH-oxydase double brin, ou d'une ou plusieurs cassettes d'expression assurant son expression,
b) l'introduction d'une séquence d'acides nucléiques antisens de le NADPH-oxydase ou d'une cassette d'expression assurant son expression,
c) l'introduction d'une séquence d'acides nucléiques antisens de le NADPH-oxydase, combinée à un ribozyme, ou d'une cassette d'expression assurant son expression,
d) l'introduction de séquences d'acides nucléiques sens de le NADPH-oxydase pour l'induction d'une co-suppression, ou d'une cassette d'expression assurant son expression,
e) l'introduction de facteurs de liaison de l'ADN ou de protéines, contre des gènes, des ARN ou des protéines de la NADPH-oxydase, ou d'une cassette d'expression assurant son expression,
f) l'introduction de séquences d'acides nucléiques virales, provoquant la dégradation de l'ARN de la NADPH-oxydase, et de constructions d'expression, ou d'une cassette d'expression assurant son expression,
g) l'introduction de constructions pour l'induction d'une recombinaison homologue sur des gènes de la NADPH-oxydase endogènes, et
h) l'incorporation de mutations dans un gène de la NADPH-oxydase endogène.

**4.** Procédé selon l'une des revendications 1 à 3, comprenant

(i) la transformation stable d'une cellule végétale avec une cassette d'expression recombinante contenant, en liaison fonctionnelle avec un promoteur actif dans les plantes, une séquence d'acides nucléiques codant pour

a) une séquence d'acides ribonucléotidiques ARN de la NADPH-oxydase double brin, ou
b) une séquence d'acides nucléiques antisens de la NADPH-oxydase, ou
c) une séquence d'acides nucléiques antisens de la NADPH-oxydase, combinée à un ribozyme, ou
d) une séquence d'acides nucléiques sens de la NADPH-oxydase pour induction d'une co-suppression, ou
e) des facteurs de liaison de l'ADN ou de protéine, contre des gènes, des ARN ou des protéines de la NADPH-oxydase,
f) des séquences d'acides nucléiques virales provoquant la dégradation de l'ARN de la NADPH-oxydase,

ii) la régénération de la plante à partir de la cellule végétale, et
iii) l'expression de ladite séquence d'acides nucléiques en une quantité et pendant un laps de temps suffisants pour produire ou augmenter dans ladite plante une résistance aux microorganismes pathogènes.

**5.** Procédé selon l'une des revendications 1 à 4, dans lequel le microorganisme pathogène est le mildiou.

**6.** Procédé selon l'une des revendications 1 à 5, dans lequel la plante est choisie dans le groupe des plantes monocotylédones, consistant en le blé, l'avoine, le millet, l'orge, le seigle, le maïs, le riz, le sorgho, le triticale, l'épeautre et la canne à sucre.

**7.** Molécule d'ARN double brin pour diminuer l'expression d'une NADPH-oxydase, comprenant

a) un brin d'ARN sens comprenant au moins une séquence ribonucléotidique qui pour l'essentiel est identique à au moins une partie du produit de transcription de l'ARN sens d'une séquence d'acides nucléiques codant pour une NADPH-oxydase, et
b) un brin d'ARN antisens, qui pour l'essentiel est complémentaire du brin sens d'ARN de a),

l'un des deux brins d'ARN étant codé par au moins une partie de la séquence d'acides nucléiques codant pour une séquence de NADPH-oxydase selon SEQ ID N° 1, 3, 5, 7, 9, 11, 13, 15, 17, 19 ou 21 ou un équivalent fonctionnel de cette dernière.

**8.** Molécule d'ARN double brin selon la revendication 7, dans laquelle les deux brins d'ARN de l'ARN double brin sont liés d'une manière covalente l'un à l'autre.

**9.** Cassette d'expression transgénique contenant, en liaison fonctionnelle avec un promoteur fonctionnel dans des organismes végétaux, une séquence d'acides nucléiques codant pour une molécule d'ARN double brin selon l'une des revendications 7 ou 8.

**10.** Cassette d'expression transgénique contenant au moins une partie d'une séquence d'acides nucléiques codant pour une NADPH-oxydase selon SEQ ID N° 1, 3, 5, 7, 9, 11, 13, 15, 17, 19 ou 21 ou un équivalent fonctionnel de cette dernière, ladite séquence d'acides nucléiques étant fonctionnellement liée, selon une orientation anti-, à un promoteur fonctionnel dans des organismes végétaux.

**11.** Cassette d'expression transgénique selon la revendication 9 ou 10, dans laquelle le promoteur fonctionnel dans des plantes est un promoteur inductible par des microorganismes pathogènes.

**12.** Vecteur transgénique contenant une cassette d'expression selon l'une des revendications 9 à 11.

**13.** Organisme transgénique contenant une molécule d'ARN double brin selon l'une des revendications 7 à 8, une cassette d'expression selon l'une des revendications 9 à 11 ou un vecteur selon la revendication 11, choisi dans le groupe consistant en les bactéries, les levures et les plantes de culture monocotylédones.

**14.** Organisme transgénique selon la revendication 13, choisi dans le groupe des plantes consistant en le blé, l'orge, le millet, le seigle, le triticale, le maïs, le riz, le sorgho, l'avoine et la canne à sucre.

**15.** Tissu, organe, partie, cellule, culture cellulaire ou matériel de multiplication, dérivant d'un organisme transgénique selon l'une des revendications 13 ou 14 et contenant une cassette d'expression selon l'une des revendications 9 à 11 ou contenant une molécule d'ARN double brin selon l'une des revendications 7 à 8.

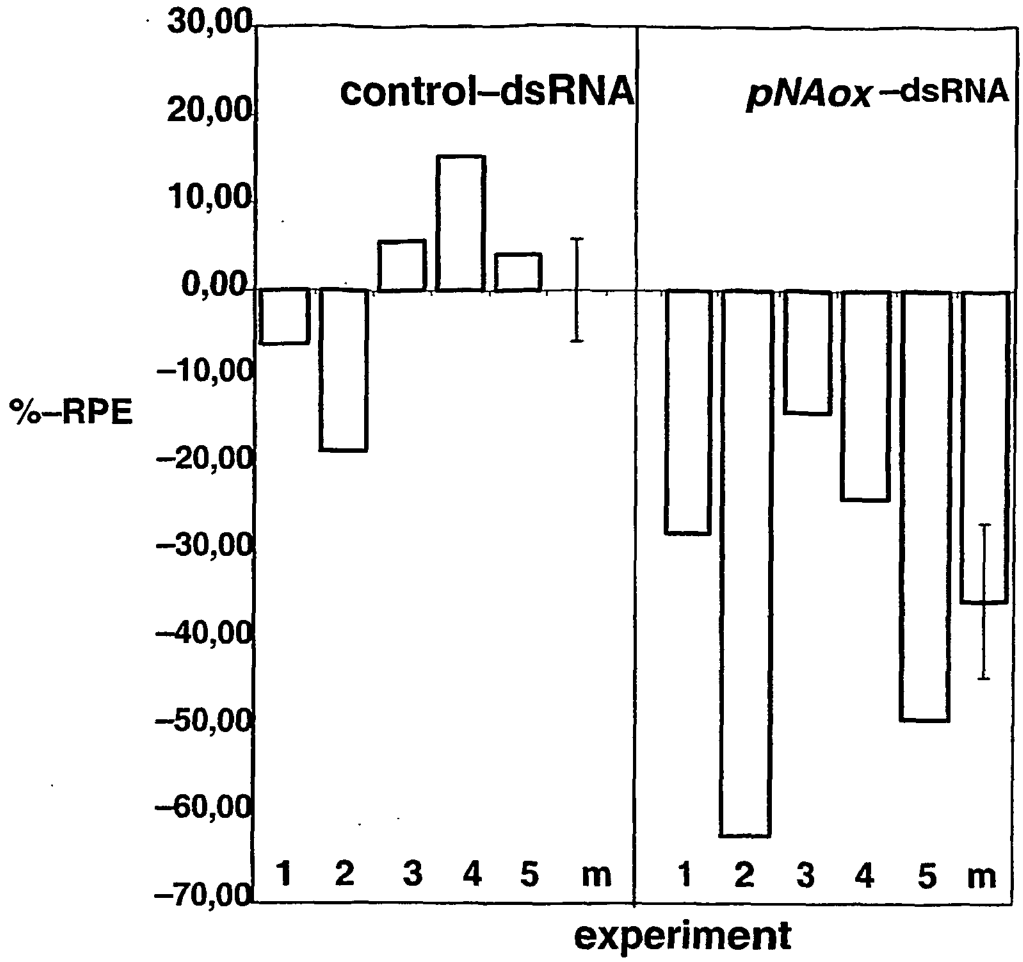
30,00
20,00
10,00
0,00
-10,00
-20,00
-30,00
-40,00
-50,00
-60,00
-70,00
%-RPE
control-dsRNA
pNAox-dsRNA
1 2 3 4 5 m
1 2 3 4 5 m
experiment

Fig. 1

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 9804586 A
- WO 0001722 A
- WO 9947552 A
- WO 9932619 A
- WO 9953050 A
- WO 0068374 A
- WO 0044914 A
- WO 0044895 A
- WO 0049035 A
- WO 0063364 A
- WO 9732016 A
- US 5593874 A
- US 5698425 A
- US 5712135 A
- US 5789214 A
- US 5804693 A
- US 4801340 A
- EP 0291533 A
- EP 0321201 A
- EP 0360257 A
- US 4987071 A
- US 5116742 A
- US 5034323 A
- US 6110736 A
- US 5352605 A
- WO 8402913 A
- US 4962028 A
- US 5683439 A
- WO 9113991 A
- US 5504200 A
- US 5608152 A
- WO 0026388 A
- WO 9845461 A
- WO 9113980 A
- WO 9515389 A
- WO 9523230 A
- WO 9916890 A
- WO 9705900 A
- WO 9216635 A
- WO 9822593 A
- US 5689049 A
- US 5689051 A
- WO 9519443 A
- EP 0388186 A
- EP 0335528 A
- WO 9321334 A
- WO 9628561 A
- US 5187267 A
- WO 9612814 A
- WO 9634949 A
- US 6100451 A
- EP 0375091 A
- US 5428148 A
- EP 0332104 A
- WO 9803536 A
- WO 9966057 A
- WO 0001830 A
- EP 1165794 A
- EP 1062356 A
- EP 1041148 A
- EP 1032684 A
- WO 9421794 A
- EP 409625 A
- WO 9845456 A
- WO 9741228 A
- EP 120516 A
- US 5565350 A
- WO 0015815 A


### In der Beschreibung aufgeführte Nicht-Patentliteratur


- **Vaeck et al.** *Nature,* 1987, vol. 328, 33-37
- **Broglie et al.** *Science,* 1991, vol. 254, 1194-1197
- **Ward et al.** *Plant Cell,* 1991, vol. 3, 1085-1094
- **Uknes et al.** *Plant Cell,* 1992, vol. 4 (6), 645-656
- **Friedrich et al.** *Plant J,* 1996, vol. 10 (1), 61-70
- **Lawton et al.** *Plant J,* 1996, vol. 10, 71-82
- **Büschges R et al.** *Cell,* 1997, vol. 88, 695-705
- **Jorgensen JH.** *Euphytica,* 1977, vol. 26, 55-62
- **Lyngkjaer MF et al.** *Plant,* 1995
- **Schulze-Lefert P ; Vogel J.** *Trends Plant Sci.,* 2000, vol. 5, 343-348
- **Wolter M et al.** *Mol Gen Genet,* 1993, vol. 239, 122-128
- **Jarosch B et al.** *Mol Plant Microbe Interact,* 1999, vol. 12, 508-514
- **Kumar J et al.** *Phytopathology,* 2001, vol. 91, 127-133

- **Wojtaszek P.** *Biochem J,* 1997, vol. 322, 681-692
- **Lamb ; Dixon.** *Annu Rev Plant Physiol Plant Mol Biol,* 1997, vol. 48, 251
- **Torres MA et al.** *Proc Natl Acad Sci USA,* 2002, vol. 99, 517-522
- **Hückelhoven R ; Kogel KH.** *Mol Plant Microbe Interact,* 1998, vol. 11, 292-300
- **Schweizer P et al.** *Plant J,* 2000, vol. 24, 895-903
- **Torres MA et al.** *Plant J,* 1998, vol. 14, 365-370
- **Yu L et al.** *Blood,* 1999, vol. 94 (7), 2497-504
- **Doke N.** *Physiol Plant Pathol,* 1983, vol. 23, 345-357
- **Levine A et al.** *Cell,* 1994, vol. 79, 583-593
- **Tenhaken R et al.** *Proc Nat Acad Sci USA,* 1995, vol. 92, 4158-4163
- **Sagi M ; Fluhr R.** *Plant Physiol,* 2001, vol. 126 (3), 1281-90
- **Altschul et al.** *Nucleic Acids Res.,* 1997, vol. 25, 3389ff
- **Sambrook J et al.** Molecular Cloning (A Laboratory Manual). Cold Spring Harbor Laboratory Press, 1989, 9.31-9.57
- Current Protocols in Molecular Biology. John Wiley &Sons, 1989, 6.3.1-6.3.6
- **Matzke MA et al.** *Plant Mol Biol,* 2000, vol. 43, 401-415
- **Fire A. et al.** *Nature,* 1998, vol. 391, 806-811
- **Schweizer P et al.** *Plant J 2000,* 2000, vol. 24, 895-903
- **Waterhouse PM et al.** *Proc Natl Acad Sci USA,* 1998, vol. 95, 13959-64
- **Sheehy et al.** *Proc Natl Acad Sci USA,* 1988, vol. 85, 8805-8809
- **Mol JN et al.** *FEBS Lett,* 1990, vol. 268 (2), 427-430
- **Helene C.** *Anticancer Drug Res,* 1991, vol. 6 (6), 569-84
- **Helene C et al.** *Ann NY Acad Sci,* 1992, vol. 660, 27-36
- **Maher LJ.** *Bioassays,* 1992, vol. 14 (12), 807-815
- **Gautier C et al.** *Nucleic Acids Res,* 1987, vol. 15, 6625-6641
- **Inoue et al.** *Nucleic Acids Res,* 1987, vol. 15, 6131-6148
- **Inoue et al.** *FEBS Lett,* 1987, vol. 215, 327-330
- **Tanner NK.** *FEMS Microbiol Rev,* 1999, vol. 23 (3), 257-275
- **Haseloff et al.** *Nature,* 1988, vol. 334, 585-591
- **Haselhoff ; Gerlach.** *Nature,* 1988, vol. 334, 585-591
- **Steinecke P et al.** *EMBO J,* 1992, vol. 11 (4), 1525-1530
- **de Feyter R et al.** *Mol Gen Genet.,* 1996, vol. 250 (3), 329-338
- **Steinecke P et al.** Ribozymes, Methods in Cell Biology 50. Academic Press, Inc, 1995, 449-460
- **Bayley CC et al.** *Plant Mol Biol.,* 1992, vol. 18 (2), 353-361
- **Lloyd AM ; Davis RW et al.** *Mol Gen Genet.,* 1994, vol. 242 (6), 653-657
- **Bartel D ; Szostak JW.** *Science,* 1993, vol. 261, 1411-1418
- **Jorgensen et al.** *Plant Mol Biol,* 1996, vol. 31 (5), 957-973
- **Goring et al.** *Proc Natl Acad Sci USA,* 1991, vol. 88, 1770-1774
- **Smith et al.** *Mol Gen Genet,* 1990, vol. 224, 447-481
- **Napoli et al.** *Plant Cell,* 1990, vol. 2, 279-289
- **Van der Krol et al.** *Plant Cell,* 1990, vol. 2, 291-99
- **Napoli et al.** *The Plant Cell,* 1990, vol. 2, 279-289
- **Dreier B et al.** *J Biol Chem,* 2001, vol. 276 (31), 29466-78
- **Dreier B et al.** *J Mol Biol,* 2000, vol. 303 (4), 489-502
- **Beerli RR et al.** *Proc Natl Acad Sci USA,* 2000, vol. 97 (4), 1495-1500
- **Beerli RR et al.** *J Biol Chem,* 2000, vol. 275 (42), 32617-32627
- **Segal DJ ; Barbas CF 3rd.** *Curr Opin Chem Biol,* 2000, vol. 4 (1), 34-39
- **Kang JS ; Kim JS.** *J Biol Chem,* 2000, vol. 275 (12), 8742-8748
- **Beerli RR et al.** *Proc Natl Acad Sci USA,* 1998, vol. 95 (25), 14628-14633
- **Kim JS et al.** *Proc Natl Acad Sci USA,* 1997, vol. 94 (8), 3616-3620
- **Klug A.** *J Mol Biol,* 1999, vol. 293 (2), 215-218
- **Tsai SY et al.** *Adv Drug Deliv Rev,* 1998, vol. 30 (1-3), 23-31
- **Mapp AK et al.** *Proc Natl Acad Sci USA,* 2000, vol. 97 (8), 3930-3935
- **Sharrocks AD et al.** *Int J Biochem Cell Biol,* 1997, vol. 29 (12), 1371-1387
- **Zhang L et al.** *J Biol Chem,* 2000, vol. 275 (43), 33850-33860
- **Famulok M ; Mayer G.** *Curr Top Microbiol Immunol,* 1999, vol. 243, 123-36
- **Owen M et al.** *Biotechnology (N Y),* 1992, vol. 10 (7), 790-794
- **Franken E et al.** *Curr Opin Biotechnol,* 1997, vol. 8 (4), 411-416
- **Whitelam.** *Trend Plant Sci,* 1996, vol. 1, 286-272
- **Dervan PB ; Bürli RW.** *Current Opinion in Chemical Biology,* 1999, vol. 3, 688-693
- **Gottesfeld JM et al.** *Gene Expr,* 2000, vol. 9 (1-2), 77-91

- **Bremer RE et al.** *Bioorg Med Chem.,* 2001, vol. 9 (8), 2093-103
- **Ansari AZ et al.** *Chem Biol.,* 2001, vol. 8 (6), 583-92
- **Gottesfeld JM et al.** *J Mol Biol.,* 2001, vol. 309 (3), 615-29
- **Wurtz NR et al.** *Org Lett,* 2001, vol. 3 (8), 1201-3
- **Wang CC et al.** *Bioorg Med Chem,* 2001, vol. 9 (3), 653-7
- **Urbach AR ; Dervan PB.** *Proc Natl Acad Sci USA,* 2001, vol. 98 (8), 4343-8
- **Chiang SY et al.** *J Biol Chem.,* 2000, vol. 275 (32), 24246-54
- **Angell, SM et al.** *Plant J.,* 1999, vol. 20 (3), 357-362
- **Ratcliff F et al.** *Plant J,* 2001, vol. 25 (2), 237-45
- **Fagard M ; Vaucheret H.** *Plant Mol Biol,* 2000, vol. 43 (2-3), 285-93
- **Anandalakshmi R et al.** *Proc Natl Acad Sci USA,* 1998, vol. 95 (22), 13079-84
- **Ruiz MT.** *Plant Cell,* 1998, vol. 10 (6), 937-46
- **Thomas KR ; Capecchi MR.** *Cell,* 1987, vol. 51, 503
- **Strepp et al.** *Proc Natl Acad Sci USA,* 1998, vol. 95 (8), 4368-4373
- **Odell et al.** *Mol Gen Genet,* 1990, vol. 223, 369-378
- **Zhu et al.** *Nat Biotechnol,* 2000, vol. 18 (5), 555-558
- **Koncz et al.** *Plant Mol Biol,* 1992, vol. 20 (5), 963-976
- **Hohn B ; Puchta.** *Proc Natl Acad Sci USA,* 1999, vol. 96, 8321-8323
- **Cole-Strauss et al.** *Nucl Acids Res,* 1999, vol. 27 (5), 1323-1330
- **Kmiec.** *Gene therapy American Scientist,* 1999, vol. 87 (3), 240-247
- **Maniatis T ; Fritsch EF ; Sambrook J.** Molecular Cloning: A Laboratory Manual. Cold Spring Harbor Laboratory, 1989
- **Silhavy TJ ; Berman ML ; Enquist LW.** Experiments with Gene Fusions. Cold Spring Harbor Laboratory, 1984
- **Ausubel FM et al.** Current Protocols in Molecular Biology. Greene Publishing Assoc. and Wiley Interscience, 1987
- **Gelvin et al.** Plant Molecular Biology Manual. 1990
- **Benfey et al.** *EMBO J,* 1989, vol. 8, 2195-2202
- **Franck et al.** *Cell,* 1980, vol. 21, 285-294
- **Odell et al.** *Nature,* 1985, vol. 313, 810-812
- **Shewmaker et al.** *Virology,* 1985, vol. 140, 281-288
- **Gardner et al.** *Plant Mol Biol,* 1986, vol. 6, 221-228
- **Holtorf S et al.** *Plant Mol Biol,* 1995, vol. 29, 637-649
- **Christensen et al.** *Plant Mol Biol,* 1992, vol. 18, 675-689
- **Bruce et al.** *Proc Natl Acad Sci USA,* 1989, vol. 86, 9692-9696
- **Hillebrand et al.** *Gene,* 1996, vol. 170, 197-200
- **Bustos MM et al.** *Plant Cell,* 1989, vol. 1 (9), 839-53
- **Joseffson LG et al.** *J Biol Chem,* 1987, vol. 262, 12196-12201
- **Shirsat A et al.** *Mol Gen Genet,* 1989, vol. 215 (2), 326-331
- **Bäumlein H et al.** *Mol Gen Genet,* 1991, vol. 225 (3), 459-67
- **Stalberg K et al.** *L Planta,* 1996, vol. 199, 515-519
- **Bäumlein H et al.** *Mol Gen Genet,* 1991, vol. 225, 121-128
- **Baeumlein et al.** *Plant Journal,* 1992, vol. 2 (2), 233-9
- **Fiedler U et al.** *Biotechnology (NY),* 1995, vol. 13 (10), 1090f
- **Stockhaus et al.** *EMBO J,* 1989, vol. 8, 2445-2451
- **Wei et al.** Oxalat-Oxidase like protein. *Plant Mol. Biol.,* 1998, vol. 36, 101-112
- **Gatz et al.** *Annu Rev Plant Physiol Plant Mol Biol,* 1997, vol. 48, 89-108
- **Ward et al.** *Plant Mol Biol,* 1993, vol. 22, 361-366
- **Gatz et al.** *Plant J,* 1992, vol. 2, 397-404
- **Ward et al.** *Plant Mol Biol,* 1993, vol. 22, 361-366
- **Schubert et al.** *Plant Mol Biol,* 1997, vol. 34, 417-426
- **Redolfi et al.** *Neth J Plant Pathol,* 1983, vol. 89, 245-254
- **Uknes et al.** *Plant Cell,* 1992, vol. 4, 645-656
- **Van Loon.** *Plant Mol Viral,* 1985, vol. 4, 111-116
- **Marineau et al.** *Plant Mol Biol,* 1987, vol. 9, 335-342
- **Matton et al.** *Molecular Plant-Microbe Interactions,* 1987, vol. 2, 325-342
- **Somssich et al.** *Proc Natl Acad Sci USA,* 1986, vol. 83, 2427-2430
- **Somssich et al.** *Mol Gen Genetics,* 1988, vol. 2, 93-98
- **Chen et al.** *Plant J,* 1996, vol. 10, 955-966
- **Zhang ; Sing.** *Proc Natl Acad Sci USA,* 1994, vol. 91, 2507-2511
- **Warner et al.** *Plant J,* 1993, vol. 3, 191-201
- **Siebertz et al.** *Plant Cell,* 1989, vol. 1, 961-968
- **Ryan.** *Ann Rev Phytopath,* 1990, vol. 28, 425-449

- **Duan et al.** *Nat Biotech,* 1996, vol. 14, 494-498
- **Stanford et al.** *Mol Gen Genet,* 1989, vol. 215, 200-208
- **McGurl et al.** *Science,* 1992, vol. 225, 1570-1573
- **Rohmeier et al.** *Plant Mol Biol,* 1993, vol. 22, 783-792
- **Eckelkamp et al.** *FEBS Letters,* 1993, vol. 323, 73-76
- **Corderok et al.** *Plant J,* 1994, vol. 6 (2), 141-150
- **Buchel et al.** *Plant Mol Biol,* 1996, vol. 30, 493-504
- **Schweizer et al.** *Plant Physiol,* 1997, vol. 114, 79-88
- **Rebmann et al.** *Plant Mol Biol,* 1991, vol. 16, 329-331
- **Rushton et al.** *Plant Cell,* 2002, vol. 14, 749-762
- **Lam E ; Chua NH.** *J Biol Chem,* 1991, vol. 266 (26), 17131-17135
- **Schoffl F et al.** *Molecular & General Genetics,* 1989, vol. 217 (2-3), 246-53
- The Maize Handbook. Springer, 1994
- **Gallie et al.** *Nucl Acids Res,* 1987, vol. 15, 8693-8711
- **Rouster J et al.** *Plant J,* 1998, vol. 15, 435-440
- **Gielen et al.** *EMBO J,* 1984, vol. 3, 835 ff
- **Sauer B.** *Methods,* 1998, vol. 14 (4), 381-92
- **Schenborn E ; Groskreutz D.** *Mol Biotechnol.,* 1999, vol. 13 (1), 29-44
- **Sheen et al.** *Plant Journal,* 1995, vol. 8 (5), 777-784
- **Haseloff et al.** *Proc Natl Acad Sci USA,* 1997, vol. 94 (6), 2122-2127
- **Reichel et al.** *Proc Natl Acad Sci USA,* 1996, vol. 93 (12), 5888-5893
- **Tian et al.** *Plant Cell Rep,* 1997, vol. 16, 267-271
- **Chui WL et al.** *Curr Biol,* 1996, vol. 6, 325-330
- **Leffel SM et al.** *Biotechniques,* 1997, vol. 23 (5), 912-8
- **Ow et al.** *Science,* 1986, vol. 234, 856-859
- **Millar et al.** *Plant Mol Biol Rep,* 1992, vol. 10, 324-414
- **Prasher et al.** *Biochem Biophys Res Commun,* 1985, vol. 126 (3), 1259-1268
- **Dellaporta et al.** Chromosome Structure and Function: Impact of New Concepts. *18th Stadler Genetics Symposium,* 1988, vol. 11, 263-282
- **Jefferson et al.** *EMBO J.,* 1987, vol. 6, 3901-3907
- **Sambrook et al.** Molecular Cloning. A Laboratory Manual. Cold Spring Harbor Laboratory Press, 1989
- **Keown et al.** *Methods in Enzymology,* 1990, vol. 185, 527-537
- **Bilang et al.** *Gene,* 1991, vol. 100, 247-250
- **Scheid et al.** *Mol Gen Genet,* 1991, vol. 228, 104-112
- **Guerche et al.** *Plant Science,* 1987, vol. 52, 111-116
- **Neuhause et al.** *Theor Appl Genet,* 1987, vol. 75, 30-36
- **Klein et al.** *Nature,* 1987, vol. 327, 70-73
- **Howell et al.** *Science,* 1980, vol. 208, 1265
- **Horsch et al.** *Science,* 1985, vol. 227, 1229-1231
- **DeBlock et al.** *Plant Physiology,* 1989, vol. 91, 694-701
- Methods for Plant Molecular Biology. Academic Press Inc, 1988
- Methods in Plant Molecular Biology. Academic Press Inc, 1989
- **Horsch RB et al.** *Science,* 1985, vol. 225, 1229f
- **Holsters et al.** *Mol Gen Genet,* 1978, vol. 163, 181-187
- **Hoekema.** The Binary Plant Vector System. Offset-drukkerij Kanters B.V,
- **An et al.** *EMBO J,* 1985, vol. 4, 277-287
- **Rogers et al.** *Meth in Enzymol,* 1987, vol. 153, 253-277
- **Schardl et al.** *Gene,* 1987, vol. 61, 1-11
- **Berger et al.** *Proc Natl Acad Sci USA,* 1989, vol. 86, 8402-8406
- **Rathore KS et al.** *Plant Mol Biol,* 1993, vol. 21 (5), 871-884
- **McCormick et al.** *Plant Cell Reports,* 1986, vol. 5, 81-84
- Techniques for Gene Transfer. **Jenes B et al.** Transgenic Plants, Vol. 1, Engineering and Utilization. Academic Press, 1993, vol. 1, 128-143
- **Potrykus.** *Annu Rev Plant Physiol Plant Molec Biol,* 1991, vol. 42, 205-225
- **Bevan et al.** *Nucl Acids Res,* 1984, vol. 12, 8711f
- **von Fennell et al.** *Plant Cell Rep.,* 1992, vol. 11, 567-570
- **Stoeger et al.** *Plant Cell Rep.,* 1995, vol. 14, 273-278
- **Jahne et al.** *Theor Appl Genet,* 1994, vol. 89, 525-533
- **Dunwell JM.** Transgenic approaches to crop improvement. *J Exp Bot.,* 2000, vol. 51, 487-96
- *Indian Chem Eng. Section B.,* 1995, vol. 37 (1,2), 15
- **Hood EE ; Jilka JM.** *Curr Opin Biotechnol,* 1999, vol. 10 (4), 382-6
- **Ma JK ; Vine ND.** *Curr Top Microbiol Immunol,* 1999, vol. 236, 275-92
- **Voet ; Voet.** Phosphoamiditmethode. Wiley Press, 896-897

- **Sanger et al.** *Proc Natl Acad Sci USA,* 1977, vol. 74, 5463-5467
- **Kølster P et al.** *Crop Sci,* 1986, vol. 26, 903-907
- **Wiberg A.** *Hereditas,* 1974, vol. 77, 89-148
- **Norman C et al.** *Cell,* 1988, vol. 55 (6), 989-1003
- **Schweizer P et al.** *Mol Plant Microbe Interact,* 1999, vol. 12, 647-54